# FASCICULE DE BREVET EUROPEEN

(11) **EP 3 237 644 B1**
(45) Date de publication et mention de la délivrance du brevet: **22.07.2020**
(21) Numéro de dépôt: 15830803.1
(22) Date de dépôt: 17.12.2015
(51) Int. Cl.: C12Q 1/6897, C12Q 1/70

(54) **MÉTHODE D'IDENTIFICATION DE CELLULES.**
ZELLIDENTIFIKATIONSVERFAHREN
CELL IDENTIFICATION METHOD

(30) Priorité: 22.12.2014 FR 1463138
(43) Date de publication de la demande: 01.11.2017
(73) Titulaire: Centre National de la Recherche Scientifique, 75794 Paris Cedex 16 (FR)
(72) Inventeur: BENKIRANE, Monsef, 34980 Saint Gely Du Fesc (FR); PETITJEAN, Gaël, 34090 Montpellier (FR)
(74) Mandataire: Monni, Richard
(86) Numéro de dépôt international: PCT/FR2015/053579
(87) Numéro de publication internationale: WO 2016/102829

(56) Documents cités:
- EP-A1- 1 715 053
- WO-A1-2013/148197
- WO-A2-2006/067572
- WO-A2-2006/067572
- Ilona Hauber ET AL: "Highly Significant Antiviral Activity of HIV-1 LTR-Specific Tre-Recombinase in Humanized Mice", PLoS Pathogens, vol. 9, no. 9 26 septembre 2013 (2013-09-26), pages 1-18, XP055093779, San Francisco DOI: 10.1371/journal.ppat.1003587 Extrait de l'Internet: URL:http://search.proquest.com/docview/144 2426778 [extrait le 2015-07-27]
- N. R. WALL ET AL: "Monosynaptic circuit tracing in vivo through Cre-dependent targeting and complementation of modified rabies virus", PROCEEDINGS OF THE NATIONAL ACADEMY OF SCIENCES, vol. 107, no. 50, 14 décembre 2010 (2010-12-14), pages 21848-21853, XP055204511, ISSN: 0027-8424, DOI: 10.1073/pnas.1011756107
- GREGORY M. LAIRD ET AL: "Rapid Quantification of the Latent Reservoir for HIV-1 Using a Viral Outgrowth Assay", PLOS PATHOGENS, vol. 9, no. 5, 30 mai 2013 (2013-05-30), page e1003398, XP055204081, DOI: 10.1371/journal.ppat.1003398
- N. R. Wall ET AL: "Monosynaptic circuit tracing in vivo through Cre-dependent targeting and complementation of modified rabies virus", Proceedings of the National Academy of Sciences, vol. 107, no. 50, 14 December 2010 (2010-12-14), pages 21848-21853, XP055204511, ISSN: 0027-8424, DOI: 10.1073/pnas.1011756107
- Gregory M. Laird ET AL: "Rapid Quantification of the Latent Reservoir for HIV-1 Using a Viral Outgrowth Assay", PLoS Pathogens, vol. 9, no. 5, 30 May 2013 (2013-05-30), page e1003398, XP055204081, DOI: 10.1371/journal.ppat.1003398

## Description

La présente invention concerne une méthode d'identification de cellules.

La recherche sur le syndrome d'immunodéficience acquise SIDA est un chemin semé d'embûches. Malgré les progrès des thérapies et les quelques rares cas de guérison fonctionnelle, l'infection par le virus de l'immunodéficience humaine VIH reste toujours un problème de santé publique. Les personnes séropositives peuvent aujourd'hui vivre aussi longtemps que les autres, mais doivent impérativement suivre leur traitement à vie. En effet, si elles arrêtent le traitement, la charge virale augmente rapidement, et les patients développent alors un SIDA.

Les antiviraux, et en particulier les thérapies hautement réactives HAART permettent de contrôler la charge virale et rendent le virus indétectable par les techniques de dépistage classiques. Cela ne veut pourtant pas dire que le virus a été éliminé de l'hôte.

Une partie des ADN viraux s'insèrent dans le génome de certaines cellules et y restent à l'état latent, c'est-à-dire que le virus est présent mais ne se réplique pas. Grâce à cette stratégie, le VIH devient «résistant» aux thérapies qui ciblent essentiellement les mécanismes d'infection et de multiplication du virus.

De plus, le VIH latent est invisible pour le système immunitaire, car la cellule infectée n'expose pas d'antigènes viraux, seuls signes pour que le système immunitaire considère que la cellule a été infectée et doit ainsi être éliminée.

Les cellules « résistantes » sont appelées les réservoirs du virus.

Aussi, il existe un besoin de déterminer la nature de ces cellules afin de proposer une thérapie appropriée pour éliminer ces réservoirs, en vue de l'éradication complète de l'infection.

La demande WO 2013/148197 propose l'utilisation d'un inhibiteur de bromodomaine en vue de réactiver le virus latent et ainsi d'éradiquer les cellules réservoirs.

La demande US 2009/010941 propose de traiter les cellules infectées par le VIH avec un agoniste de TRAIL, éventuellement en association avec des inhibiteurs d'histones désacétylases, afin d'induire l'apoptose des cellules réservoirs.

Wall et al. PNAS, 2010, 107(50), 21848-21853 décrit un système à deux virus qui vise à être utilisé dans des cerveaux d'animaux exprimant une Cre recombinase. Le premier virus (virus helper) est un virus modifié dans lequel une protéine rapporteur et le gène TVA sont placé sous contrôle d'un promoteur et encadré par deux paires de séquences lox. La séquence du rapporteur et du gène TVA est, avant recombinaison, dans une orientation 3'->5'. Une fois en présence de la recombinase, les séquences TVA et rapporteur sont inversées (orientation 5'->3'), ce qui permet l'expression de protéines fonctionnelles. La protéine TVA servira alors d'auxiliaire au virus de la rage pour l'infection neurale. Ce système permet de suivre le parcours neural du virus, et d'étudier l'impact du virus de la rage.

Laird et al. PLOS pathogens, 2013, 9(5), e1003398 vise à déterminer la taille du réservoir viral chez les individus infectés par le VIH. Pour ce faire, et afin d'améliorer les techniques classiquement utilisées, les auteurs proposent un procédé en deux étapes permettant d'isoler les cellules TCD4+ dormantes (c'est à dire les cellules infectées mais qui ne répliquent pas le virus). Une dilution limite est alors réalisée sur les cellules purifiées, et celles-ci sont activées avec du PHA et une irradiation afin de réactiver le virus. Les cellules activées sont alors mises en contact avec des lymphocytes TCD4 non infectés et l'expression de l'antigène p24 est mesurée. Les auteurs ont également proposé de remplacer les lymphocytes TCD4 non infectés par une lignée cellulaire compétente pour une infection par le VIH: la lignée MoIT4/CCR5 qui surexprime les récepteurs CXCR4 et CCR5, indispensables pour l'entrée du virus dans la cellule. Les auteurs montrent que ce système utilisant la lignée Molt4 est aussi efficace que l'infection des lymphocytes TCD4 non infectés. Ce système utilisant les cellules MoIT4/CCR5 est donc plus rapide et moins contraignant car la lignée peut être entretenue facilement dans des conditions de culture optimisées.

La demande WO2006067572 A2 décrit une méthode de diagnostic et de suivi de la population réservoir d'une infection virale utilisant des colorants capables d'interagir avec les ARN des leucocytes. Le principe de l'invention est de comparer la quantité d'acides nucléiques marqués entre des cellules saines et des cellules suspectées d'être infectées par un virus, notamment le virus VIH.

Toutefois, ces documents ne font que proposer des traitements qui visent toutes les cellules sans connaitre la nature exacte des cellules réservoirs. Aussi, il existe un risque de proposer des traitements peu spécifiques qui ne feront qu'augmenter les effets indésirables déjà associés aux thérapies antirétrovirales.

Par conséquent, le besoin de déterminer la nature des cellules réservoirs demeure.

Un de buts de l'invention est de palier ce manque.

L'invention a pour but de proposer une méthode permettant de déterminer de manière efficace les cellules réservoirs des virus induisant une immunodéficience.

Un autre but de l'invention est de proposer un modèle d'étude de ces cellules.

Il est décrit ci-après l'utilisation d'une molécule d'acides nucléiques comprenant une première séquence codant un premier rapporteur, sous contrôle d'au moins un élément nécessaire à la transcription, la première séquence étant bordée par
- au moins une première paire de séquences ciblant une recombinase spécifique de site, ladite première paire comprenant une séquence P1-1 et une séquence P1-2,
- au moins une seconde paire de séquences ciblant une recombinase spécifique de site, ladite seconde paire comprenant une séquence P2-1 et une séquence P2-2,
les séquences de chacune desdites première et seconde paires de séquences étant dans une orientation opposée l'une par rapport à l'autre,
où les séquences de la première paire de séquences ciblant une recombinase spécifique de site sont incapables de recombiner avec les séquences de la seconde paire de séquences ciblant une recombinase spécifique de site et où les séquences de la seconde paire de séquences ciblant une recombinase spécifique de site sont incapables de recombiner avec les séquences de la première paire de séquences ciblant une recombinase spécifique de site,
une séquence de la première paire et une séquence de la seconde paire de séquences ciblant une recombinase spécifique de site se trouvant en amont de ladite première séquence d'acide nucléiques et une séquence de la première paire et une séquence de la seconde paire de séquences ciblant une recombinase spécifique de site se trouvant en aval de ladite première séquence d'acide nucléiques,
de sorte que les séquences d'une même paire n'encadrent jamais les deux séquences de l'autre paire,
la séquence de ladite première molécule d'acides nucléiques étant telle que, en l'absence de combinaison induite par ladite recombinase spécifique de site, présente un cadre ouvert de lecture codant ledit premier rapporteur dans une orientation 3'-5', et donc incapable de permettre la transcription et la traduction du gène rapporteur pour obtenir ledit premier rapporteur,
éventuellement en association avec une recombinase, ou avantageusement en association avec un virus responsable d'une immunodéficience, ledit virus comprenant dans son génome un gène codant ladite recombinase spécifique de site,
pour la détection, notamment *in vitro,* de cellules d'un mammifère infecté par un virus responsable d'une immunodéficience chez ledit mammifère, lesdites cellules étant les cellules réservoirs dudit virus, ou pour la mise en œuvre d'une méthode de détection, notamment *in vitro,* de cellules d'un mammifère infecté par un virus responsable d'une immunodéficience chez ledit mammifère, lesdites cellules étant les cellules réservoirs dudit virus, lesdites cellules étant notamment des cellules hématopoïétiques.

Il est décrit en outre l'utilisation d'une molécule d'acides nucléiques comprenant une première séquence codant un premier rapporteur, sous contrôle d'au moins un élément nécessaire à la transcription, la première séquence étant bordée par
- au moins une première paire de séquences ciblant une recombinase spécifique de site, ladite première paire comprenant une séquence P1-1 et une séquence P1-2,
- au moins une seconde paire de séquences ciblant une recombinase spécifique de site, ladite seconde paire comprenant une séquence P2-1 et une séquence P2-2,
les séquences de chacune desdites première et seconde paires de séquences étant dans une orientation opposée l'une par rapport à l'autre,
les séquences de la première paire de séquences ciblant une recombinase spécifique de site sont incapables de recombiner avec les séquences de la seconde paire de séquences ciblant une recombinase spécifique de site et où les séquences de la seconde paire de séquences ciblant une recombinase spécifique de site sont incapables de recombiner avec les séquences de la première paire de séquences ciblant une recombinase spécifique de site,
une séquence de la première paire et une séquence de la seconde paire de séquences ciblant une recombinase spécifique de site se trouvant en amont de ladite première séquence d'acide nucléiques et une séquence de la première paire et une séquence de la seconde paire de séquences ciblant une recombinase spécifique de site se trouvant en aval de ladite première séquence d'acide nucléiques,
de sorte que les séquences d'une même paire n'encadrent jamais les deux séquences de l'autre paire,
la séquence de ladite première molécule d'acides nucléiques étant telle que, en l'absence de combinaison induite par ladite recombinase spécifique de site, présente un cadre ouvert de lecture codant pour ledit premier rapporteur dans une orientation 3'-5', et donc incapable de permettre la transcription et la traduction du gène rapporteur codant ledit premier rapporteur,
en association avec un virus responsable d'une immunodéficience, ledit virus comprenant dans son génome un gène codant ladite recombinase spécifique de site,
pour la détection, notamment *in vitro,* de cellules d'un mammifère infecté par un virus responsable d'une immunodéficience chez ledit mammifère, lesdites cellules étant les cellules réservoirs dudit virus.

L'invention repose sur la constatation surprenante faite par les inventeurs qu'une construction génique, qui permet l'expression d'un rapporteur, ciblant spécifiquement les cellules infectées par un virus induisant une immunodéficience, permet de détecter les cellules réservoirs dudit virus.

Avantageusement, en combinant une molécule d'acide nucléique comprenant une séquence codant un rapporteur, qui est capable d'être recombiné, et une recombinase, il est possible d'identifier spécifiquement les cellules cibles dudit virus, et de fait en isoler les cellules réservoirs.

L'invention se base donc sur l'utilisation d'une molécule d'acides nucléiques qui comprend une séquence d'un gène rapporteur codant un rapporteur. La séquence de ce gène rapporteur, ou plus exactement le cadre ouvert de lecture codant le rapporteur, est positionné dans le sens 3'->5'. Ainsi, dans des conditions normales, si la molécule d'acides nucléiques est introduite dans une cellule, ou si elle est mise en présence d'un système de transcription traduction *in vitro,* aucune protéine fonctionnelle correspondant au gène rapporteur ne pourra être exprimée. Ce principe est bien connu de l'homme de métier : la transcription et la traduction s'effectuent dans le sens 5'->3'.

Afin de permettre l'expression du gène rapporteur, il sera nécessaire de réaliser une recombinaison génétique au sein même de la molécule d'acides nucléiques.

Pour se faire, les inventeurs ont mis à profit la recombinaison spécifique de site utilisant les enzymes recombinases spécifiques de sites. Les mécanismes de recombinaisons impliqués dans le cadre d'une recombinaison s'une même molécule sont les suivants :
- l'excision et la résolution qui sont deux phénomènes relativement similaires dans le mécanisme mis en œuvre même s'ils représentent des événements biologiques non apparentés. Dans les deux cas, les deux sites de recombinaison sont portés par le même réplicon initial, et doivent être en répétition directe l'un par rapport à l'autre. Après synapse par la recombinase, deux réplicons indépendant seront produits, soit identiques dans le cas de la résolution de dimères, soit différents dans le cas de l'excision, et
- l'inversion qui nécessite également que les deux sites de recombinaison soient portés par le même réplicon. Cependant, pour qu'une inversion ait lieu, il faut que ces sites soient en répétition inversée l'un par rapport à l'autre. Leur assemblage dans la synapse va engendrer des contraintes topologiques qui conduiront à l'inversion du matériel génétique situé entre les deux sites plutôt qu'à leur délétion.

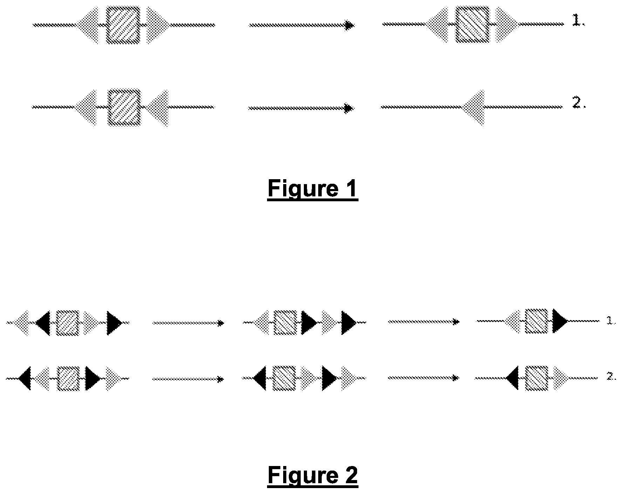

Ces deux mécanismes sont illustrés dans la **Figure 1****.**

Ainsi, comme la séquence codant le rapporteur est dans une orientation 3'->5', incompatible avec la transcription et la traduction, il sera nécessaire, pour que ledit gène rapporteur puisse s'exprimer, d'effectuer une inversion. Ainsi, comme cela est mentionné ci-dessus, la séquence codant le rapporteur est donc encadrée par une paire de séquences reconnaissables par une recombinase, les deux séquences étant en répétition inverse l'une par rapport à l'autre, ou dans une orientation opposée l'une par rapport à l'autre.

A titre d'exemple, dans la séquence de recombinaison appelée « SEQUENCE », la molécule d'acide nucléique s'écrirait artificiellement, pour le propos de cet exemple, de la manière suivante :
5' - SEQUENCE --- RUETROPPAP ENEG (qui correspond au gène rapporteur dans le sens 3'->5') --- ECNEUQES - 3'.

Après recombinaison, en présence de la recombinase appropriée, la séquence recombinée sera la suivante :
5' - ECNEUQES --- GENE RAPPORTEUR (qui correspond au gène rapporteur dans le sens 5'-> 3') --- SEQUENCE - 3'.

Toutefois, on comprend de cet exemple que tant que la recombinase sera présente, l'inversion par recombinaison sera possible, et la séquence du gène rapporteur pourra indéfiniment être inversée.

Afin de palier cet inconvénient, les inventeurs ont mis à profit la capacité qu'ont certaines recombinases spécifiques de site de reconnaitre des paires spécifiques de séquences. Dans ce cas, la recombinase est capable d'effectuer des recombinaisons à l'aide d'une première paire de séquences de recombinaison, ou à l'aide d'une deuxième ou troisième... paires de séquences, mais la recombinase est incapable d'effectuer une recombinaison en utilisant des séquences de deux paires différentes.

En d'autres termes, si la séquence codant le rapporteur de l'invention est encadrée par une séquence de recombinaison d'une première paire d'un côté et une séquence de recombinaison d'une deuxième paire de l'autre côté, sans que la seconde séquence de chacune des paires ne soit présente, aucune recombinaison (inversion ou excision) ne sera possible.

En mettant à profit ces propriétés, les inventeurs proposent d'utiliser une molécule d'acides nucléique codant un rapporteur dont le cadre de lecture est dans une orientation 3'->5', qui est bordée par deux paires de séquences de recombinaison reconnues par une même recombinase.

Comme cela a été mentionné ci-dessus, il est nécessaire que les séquences d'une même paire de séquence permettant la recombinaison spécifique de site par une recombinase soient dans une orientation opposée l'une par rapport à l'autre.

En outre, il est nécessaire que les séquences d'une même paire ne soient pas comprises entre les séquences de l'autre paire. En effet, si tel était le cas, la séquence du gène rapporteur serait alors inversée par une première paire, et inversée à nouveau par la deuxième paire.

Aussi, la molécule d'acide nucléique de l'invention est telle qu'elle comprend une première paire de séquences spécifiques de site constituée d'une séquence P1.1 et d'une séquence P1.2 et une seconde paire de séquences spécifiques de site constituée d'une séquence P2.1 et d'une séquence P2.2, de sorte que le gène rapporteur est encadré par les deux paires de séquences et que lesdites séquences sont orientées de la manière suivante :
5' - P1.1 - P2.1 - « gène rapporteur à recombiner » - P1.2 - P2.2 - 3', ou
5' - P1.1 - P2.2 - « gène rapporteur à recombiner » - P1.2 - P2.1 - 3', ou
5' - P1.2 - P2.1 - « gène rapporteur à recombiner » - P1.1 - P2.2 - 3', ou
5' - P1.2 - P2.2 - « gène rapporteur à recombiner » - P1.1 - P2.1 - 3', ou
5' - P2.1 - P1.1 - « gène rapporteur à recombiner » - P2.2 - P1.2 - 3', ou
5' - P2.1 - P1.2 - « gène rapporteur à recombiner » - P2.2 - P1.1 - 3', ou
5' - P2.2 - P1.1 - « gène rapporteur à recombiner » - P2.1 - P1.2 - 3', ou
5' - P2.2 - P1.2 - « gène rapporteur à recombiner » - P2.1 - P1.1 - 3'.

Dans cette configuration, et comme cela est illustré dans la **figure 2****,** une première inversion aura lieu grâce aux séquences d'une des deux paires de séquence spécifiques de site. A l'issue de cette recombinaison, les séquences de l'autre paire, initialement dans une orientation opposée l'une par rapport à l'autre, se retrouvent dans une même orientation. Ces séquences de l'autre paire, encadrent en outre une des séquences de la paire de séquence ayant permis l'inversion. Il sera alors possible de réaliser une excision à l'aide de l'autre paire de séquence et la molécule résultant comprendra une séquence de la paire ayant permis l'inversion, et une séquence ayant permis l'excision, entourant la séquence du gène rapporteur positionné dans le sens 5'->3'.

Comme les séquences des deux paires ne sont pas compatibles entre elles, c'est-à-dire qu'il n'est pas possible de de réaliser une recombinaison en utilisant une séquence d'une paire et une séquence d'une autre paire, la séquence du gène rapporteur est « fixée » dans une orientation 5'->3', et ne pourra plus être inversée. Le rapporteur pourra alors être exprimé.

Dans l'invention on entend par « gène rapporteur » la molécule d'acide nucléique codant le « rapporteur » protéique.

Dans le cadre de l'utilisation susmentionnée, c'est le rapporteur qui est détecté.

Les rapporteurs peut être notamment l'une quelconque des protéines connues de l'homme du métier qui permettent d'identifier des cellules, et notamment des protéines auto-fluorescentes comme la protéine fluorescente verte, rouge, jaune, rouge lointain, cyan... issues de méduse ou de corail, ou encore les enzymes telles que la luciférase de pensée de mer (*Renilla reniformis*), ou de luciole, ou encore des enzymes telles que la β-galactosidase. Cette liste n'est pas limitative.

Avantageusement, le premier rapporteur dans l'invention est la protéine fluorescente rouge RFP.

La séquence du gène rapporteur, codant le premier rapporteur susmentionné, est mise sous contrôle d'élément permettant sa transcription. Cela signifie que la séquence du gène rapporteur est sous le contrôle d'un promoteur et éventuellement d'enhanceurs, qui en présence des complexes de transcription permettront de produire un ARN messager correspondant audit gène rapporteur, ledit ARN étant par la suite traduit en ledit premier rapporteur.

Le(s) élément(s) nécessaire(s) à la transcription peut/peuvent être
- soit compris entre les séquences des paires de séquences de recombinaison spécifique de site, avantageusement en 3' de la séquence du gène rapporteur non inversé, mais en amont de la séquence de la paire de séquence qui servira à l'excision, de sorte qu'après inversion, le(s) élément(s) sera/seront en position 5'
- soit en amont desdites séquences, de sorte qu'il(s) ne sera/seront pas inversé(s) lors de la recombinaison.

La première molécule d'acides nucléiques susmentionnée, est donc prête à être recombinée.

Il est avantageux d'introduire dans les cellules cibles, c'est-à-dire les cellules qu'il est envisagé de détecter, une recombinase spécifique des sites de recombinaison spécifiques de sites contenus dans la première séquence d'acides nucléiques.

Comme l'objectif de l'invention est de détecter les cellules réservoirs de virus provoquant une immunodéficience chez un mammifère infecté, il est avantageux que les cellules cibles soient les cellules du système immunitaire. Aussi, la recombinase peut être introduite dans les cellules d'intérêt par tout moyen connu de l'homme du métier.

Un moyen avantageux de cibler l'expression de la recombinase dans les cellules d'intérêt est d'infecter les cellules avec un virus spécifique desdites cellules, ledit virus étant génétiquement modifié de sorte qu'en plus des gènes nécessaires à son cycle vital, il exprime ladite recombinase.

Il est donc particulièrement avantageux, dans la cadre de la détection des cellules réservoirs d'un virus de l'immunodéficience, comme le virus de l'immunodéficience humaine (VIH ou HIV en anglais), de l'immunodéficience simienne (VIS ou SIV en anglais), de l'immunodéficience féline (VIF ou FIV en anglais), d'utiliser ledit virus qui comprend dans son génome un gène supplémentaire codant la recombinase d'intérêt.

Du fait de sa spécificité pour les cellules hématopoïétiques, l'un des virus susmentionnés ciblera l'ensemble des cellules cibles et notamment les cellules réservoirs. Si ces cellules comprennent en outre la première molécule d'acides nucléiques susmentionnée, du fait de l'action de la recombinase, les cellules ciblées par le virus exprimeront le rapporteur et seront alors facilement détectable par l'homme du métier.

Il est par ailleurs précisé que la première séquence d'acides nucléiques est bordée
- en amont de son premier nucléotide dans sa partie 5' d'une des séquences de la première paire de séquences ciblant une recombinase spécifique de site et d'une des séquences de la seconde paire de séquences ciblant une recombinase spécifique de site, et
- en aval de son dernier nucléotide dans sa partie 3' de l'autre séquence de la première paire de séquences ciblant une recombinase spécifique de site et de l'autre séquence de la seconde paire de séquences ciblant une recombinase spécifique de site.

La recombinase spécifique de site est choisie parmi la recombinase Cre du bactériophage P1, la recombinase FLP de *Saccharomyces cerevisiae,* la recombinase R de *Zygosaccharomyces rouxii* pSR1, la recombinase A de *Kluyveromyces drosophilarium pKD1,* la recombinase A de *Kluyveromyces waltii* pKW1, l'intégrase λ Int, la recombinase du système de recombinaison GIN du phage Mu, la recombinase bactérienne β ou un variant de l'une quelconque de ces recombinases.

La molécule d'acides nucléiques comprend avantageusement en outre une seconde séquence codant un second rapporteur sous contrôle d'au moins un élément nécessaire à la transcription.

De plus, en dehors des régions participant à la recombinaison spécifique de site, la première molécule d'acides nucléiques susmentionnée comprend avantageusement une séquence d'un second gène rapporteur codant un second rapporteur. Contrairement à la séquence codant le premier rapporteur, la séquence du second gène rapporteur est d'emblée dans le sens 5'->3' et le second rapporteur s'exprime indépendamment de la présence ou non de la recombinase. Ce second rapporteur a notamment pour intérêt de détecter les cellules qui ont été transformées par la première molécule d'acides nucléiques susmentionnée.

En résumé de ce qui précède, il est décrit l'utilisation, pour la détection de cellules réservoirs d'un virus de l'immunodéficience des mammifères, notamment les primates (homme ou singe) et les félins, ou pour la mise en œuvre d'une méthode de détection in vitro desdites cellules réservoirs, lesdits virus étant notamment le VIH, le VIS ou le VIF :
- d'une molécule d'acide nucléique comprenant dans le sens 5'-3' :
   ∘ une première séquence d'une première paire de séquences de recombinaison spécifique de site, suivie par
   ∘ une première séquence d'une seconde paire de séquences de recombinaison spécifique de site, suivie par
   ∘ la séquence du premier gène rapporteur, dont le cadre ouvert de lecture est orienté dans le sens 3'->5', suivi par
   ∘ une seconde séquence d'une première paire de séquences de recombinaison spécifique de site, cette seconde séquence étant dans une orientation opposée à l'orientation de la première séquence de la première paire, suivie par
   ∘ une seconde séquence d'une seconde paire de séquences de recombinaison spécifique de site, cette seconde séquence étant dans une orientation opposée à l'orientation de la première séquence de la seconde paire,
- éventuellement un virus de l'immunodéficience susmentionné, modifié et permettant l'expression de la recombinase spécifique de site reconnaissant les séquences desdites première et seconde paire de séquences de recombinaison spécifique de site.

Cette séquence virale est avantageusement une des séquences suivantes :
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23),
- HIV NL4-3-Nef (codon opt)- CMV-Cre (SEQ ID NO: 24),
- SIVmac239-Nef-IRES-Cre (SEQ ID NO: 25),
- SIVmac239-Nef (codon opt)-IRES-Cre (SEQ ID NO : 26),
- SIVmac239-Nef-CMV-Cre (SEQ ID NO : 27), et
- SIVmac239-Nef (codon opt)-CMV-Cre (SEQ ID NO: 28).

Il est avantageusement décrit l'utilisation susmentionnée, dans laquelle la recombinase spécifique de site est la recombinase Cre du bactériophage P1.

Avantageusement, la Cre recombinase du bactériophage P1 comprend ou consiste en l'une quelconque des séquences SEQ ID NO ; 18 et SEQ ID NO : 20, respectivement codées par les séquences d'acides nucléiques suivantes : SEQ ID NO : 17 et SEQ ID NO : 19.

La séquence codant la recombinase Cre est soit insérée dans le génome du virus, sous contrôle d'un promoteur autonome, soit sous contrôle de l'expression de la protéine Nef, sous la forme d'une séquence Nef-IRES Cre.

IL est en outre avantageusement décrit l'utilisation susmentionnée, dans laquelle les séquences de la première paire de séquences ciblant une recombinase spécifique de site et les séquences de la seconde paire de séquences ciblant une recombinase spécifique de site sont choisie parmi Lox P1, Lox P2272, Lox 66, Lox 71, Lox 511, Lox 512, Lox 514 et des séquences mutées du site Lox P1, portant au moins une mutation ponctuelle dans la séquence espaceur.

Les paires de séquences ciblant une recombinase spécifique de site avantageuses de l'invention, reconnues par la Cre recombinase du phage P1, sont les séquences Lox P1 et Lox P2272, représentées par les séquences suivantes:
Lox P1 : ATAACTTCGTATAGCATACATTATACGAAGTTAT (SEQ ID NO : 1), et
Lox P2272 : ATAACTTCGTATAAAGTATCCTATACGAAGTTAT (SEQ ID NO : 2).

Leurs complémentaires (séquence dans l'orientation opposée) sont les séquences suivantes :
Complémentaire de Lox P1 : ATAACTTCGTATAATGTATGCTATACGAAGTTAT (SEQ ID NO : 3), et
Complémentaire de Lox P2272 : ATAACTTCGTATAGGATACTTTATACGAAGTTAT (SEQ ID NO : 4).

L'utilisation susmentionnée peut être réalisée en association avec un ou plusieurs composés inhibant la multiplication dudit virus.

Les mono, bi tri ou multi thérapie proposées pour le traitement des infections aux VIH sont transposables aux infections aux virus HIF et VIS. Des exemples sont donnés ci-après.

Aussi, est-il également décrit l'utilisation pour la détection, notamment *in vitro,* de cellules d'un mammifère infecté par un virus responsable d'une immunodéficience chez ledit mammifère, lesdites cellules étant les cellules réservoirs dudit virus, ou pour la mise en œuvre d'une méthode de détection notamment in vitro, de cellules d'un mammifère infecté par un virus responsable d'une immunodéficience chez ledit mammifère, lesdites cellules étant les cellules réservoirs dudit virus
- d'une molécule d'acides nucléiques comprenant une première séquence codant un premier rapporteur, sous contrôle d'au moins un élément nécessaire à la transcription, la première séquence étant bordée par
   - une première paire de séquences Lox P1 ciblant la Cre recombinase du phage P1, ladite première paire comprenant une séquence P1-1 et une séquence P1-2,
   - une seconde paire de séquences Lox P2272 ciblant la Cre recombinase du phage P1, ladite seconde paire comprenant une séquence P2-1 et une séquence P2-2,
   les séquences de chacune desdites paires de séquences Lox P1 et Lox P2272 étant dans une orientation opposée l'une par rapport à l'autre, les séquences de la paire Lox P1 étant incapables de recombiner avec les séquences de la paire Lox P2272,
   la séquence codant un premier rapporteur comprenant essentiellement ou consistant en la séquence SEQ ID NO: 5, ci-après représentée
- éventuellement en association avec la Cre recombinase du phage P1, ou avantageusement en association avec un virus responsable de ladite immunodéficience, ledit virus comprenant dans son génome un gène codant ladite recombinase Cre recombinase du phage P1,
- éventuellement en association avec un ou plusieurs composés inhibant la multiplication dudit virus, ou agents anti rétroviral, ou antirétroviraux.

La séquence SEQ ID NO : 5 correspond à la séquence du cadre ouvert de lecture codant la protéine fluorescente rouge (RFP), dans son orientation 3'->5'. Aussi, avant recombinaison, cette séquence, qui selon les principes de la transcription se lit dans le sens 5'->3', ne sera pas capable de coder le rapporteur RFP.

Avantageusement, il est également envisagé que la séquence codant un premier rapporteur comprenant essentiellement ou consistant en la séquence SEQ ID NO : 31, correspond à la séquence du cadre ouvert de lecture codant la luciférase de luciole, dans son orientation 3'->5'. Après recombinaison, la séquence sera orientée dans le sens 5'->3' (SEQ ID NO : 46) et codera une luciférase fonctionnelle de séquence SEQ ID NO : 47.

Avantageusement, la molécule d'acides nucléique comprend une seconde séquence codant un second rapporteur sous contrôle d'au moins un élément nécessaire à la transcription. Cette seconde séquence code notamment une protéine autofluorescente, une enzyme, ou tout autre peptide capable d'être détecté facilement avec les techniques de biologies moléculaire connues de l'homme du métier. Contrairement à la séquence du premier gène rapporteur, la séquence codant un second rapporteur est dans le sens 5'-> 3' et permet l'expression du rapporteur indépendamment de la recombinaison. Cette seconde séquence est sous contrôle d'un promoteur, éventuellement un ou plusieurs enhancer, qui permet une expression constitutive dudit second rapporteur. En outre cette séquence codant le second rapporteur se trouve en dehors des zones d'encadrement définies par les séquences cibles de recombinases spécifiques de site.

Avantageusement, ladite seconde séquence comprend essentiellement ou consiste en la séquence SEQ ID NO : 8, qui correspond au cadre ouvert de lecture codant la protéine fluorescente verte optimisée (eGFP).

Il est aussi décrit l'utilisation telle que définie ci-dessus, dans laquelle ladite molécule d'acide nucléique comprend essentiellement ou consiste essentiellement en l'une des séquences suivantes :
pHR-4lox-RFP/GFP-WPRE (SEQ ID NO : 6),
pHR-4lox-CMV-RFP-PGK-GFP-WPRE (SEQ ID NO : 32)
pHR-4lox-SFFV-RFP-PGK-GFP-WPRE (SEQ ID NO : 33)
pHR-4lox-SFFV-RFP-CMV-GFP-WPRE (SEQ ID NO : 34)
pHR-4lox-CMV-lucif-PGK-GFP-WPRE (SEQ ID NO : 35),
pSDT-4lox-RFP/GFP-WPRE (SEQ ID NO : 7),
HR-4lox-CMV-RFP-PGK-GFP-WPRE (SEQ ID NO : 36)
HR-4lox-SFFV-RFP-PGK-GFP-WPRE (SEQ ID NO : 37)
HR-4lox-SFFV-RFP-CMV-GFP-WPRE (SEQ ID NO : 38)
HR-4lox-CMV-lucif-PGK-GFP-WPRE (SEQ ID NO : 39), et
SDT-4lox-RFP/GFP-WPRE (SEQ ID NO : 40).

Avantageusement, les molécules pHR-4lox-RFP/GFP-WPRE (SEQ ID NO : 6), pHR-4lox-CMV-RFP-PGK-GFP-WPRE (SEQ ID NO : 32), pHR-4lox-SFFV-RFP-PGK-GFP-WPRE (SEQ ID NO : 33), pHR-4lox-SFFV-RFP-CMV-GFP-WPRE (SEQ ID NO : 34), pHR-4lox-CMV-lucif-PGK-GFP-WPRE (SEQ ID NO : 35), HR-4lox-CMV-RFP-PGK-GFP-WPRE (SEQ ID NO : 36), HR-4lox-SFFV-RFP-PGK-GFP-WPRE (SEQ ID NO : 37), HR-4lox-SFFV-RFP-CMV-GFP-WPRE (SEQ ID NO : 38) et HR-4lox-CMV-lucif-PGK-GFP-WPRE (SEQ ID NO : 39), sont utilisées pour la détection, ou la mise en œuvre d'une méthode de détection, de cellules humaines infectées par le VIH. Ces séquences sont telles que :
- la séquence SEQ ID NO : 6 ou 32 correspond à la séquence SEQ ID NO : 36 contenue dans le vecteur pHR.
- la séquence SEQ ID NO : 33 correspond à la séquence SEQ ID NO : 37 contenue dans le vecteur pHR,
- la séquence SEQ ID NO : 34 correspond à la séquence SEQ ID NO : 38 contenue dans le vecteur pHR, et
- la séquence SEQ ID NO : 35 correspond à la séquence SEQ ID NO : 39 contenue dans le vecteur pHR.

Le vecteur pHR est un vecteur comprenant une base génétique du virus VIH-1 dérivé du vecteur pHR-ET (Bachracha et al. 2005 Virology. 332(1), 418-429) lui-même dérivé du vecteur pHR-CMV-lacz (Naldini, et al. 1996. Science, 272(5259), pp. 263-267). La casette « lox-turboRFP-lox-promoteur » a été introduite dans le pHR-ET.

Avantageusement, les molécules pSDT-4lox-RFP/GFP-WPRE et SDT-4lox-RFP/GFP-WPRE sont utilisées pour la détection, ou la mise en œuvre d'une méthode de détection, de cellules simiennes infectées par le VIS. La séquence SEQ ID NO : 7 correspond à la séquence SEQ ID NO : 40 contenue dans le vecteur pSDT.

Le vecteur pSDT est un vecteur comprenant une base génétique du virus SIV déivé du vecteur le pGAE-SFFV-eGFP (Verhoeyen et al. 2012. Hum Gene Ther. 23(7):754-68.) lui-même dérivé du vecteur pSIV-RMES-GAE (Mangeot et al. 2002. Mol. Ther.; 5:283-290.). La cassette "lox-turboRFP-lox-promoteur" a été introduite dans le pGAE-SFFV-eGFP.

En outre il est décrit l'utilisation telle que définie ci-dessus, dans laquelle ladite molécule d'acide nucléique comprend essentiellement ou consiste essentiellement en l'une des séquences SEQ ID NO : 36, 37, 38, 39 ou 40.

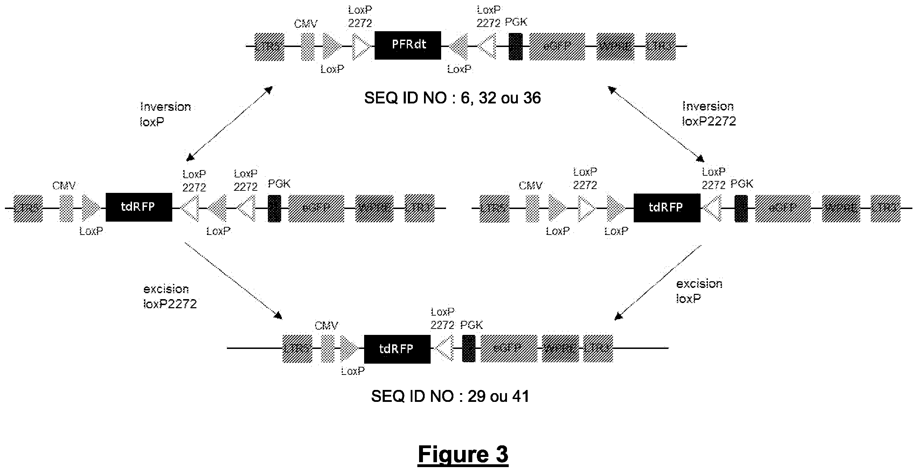

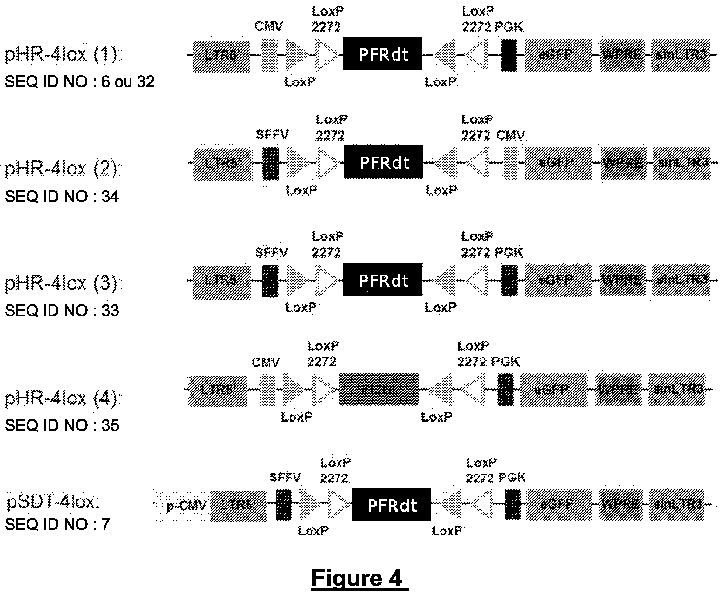

Des exemples de molécules d'acides nucléiques de l'invention sont illustrés à la **Figure 3****,** qui montre la recombinaison, et à la **Figure 4****.**

On décrit ci-après une cellule hématopoïétique comprenant dans son génome
a) une première séquence recombinée issue de la recombinaison
   - d'une première séquence codant un premier gène rapporteur, sous contrôle d'au moins un élément nécessaire à la transcription, la première séquence étant flaquée par
   - au moins une première paire de séquences ciblant une recombinase spécifique de site, ladite première paire comprenant une séquence P1-1 et une séquence P1-2,
   - au moins une seconde paire de séquences ciblant une recombinase spécifique de site, ladite seconde paire comprenant une séquence P2-1 et une séquence P2-2,
      les séquences de chacune desdites première et seconde paires de séquences étant dans une orientation opposée l'une par rapport à l'autre,
      les séquences de la première paire de séquences ciblant une recombinase spécifique de site sont incapables de recombiner avec les séquences de la seconde paire de séquences ciblant une recombinase spécifique de site et où les séquences de la seconde paire de séquences ciblant une recombinase spécifique de site sont incapables de recombiner avec les séquences de la première paire de séquences ciblant une recombinase spécifique de site,
      une séquence de la première paire et une séquence de la seconde paire de séquences ciblant une recombinase spécifique de site se trouvant en amont de ladite première séquence d'acide nucléiques et une séquence de la première paire et une séquence de la seconde paire de séquences ciblant une recombinase spécifique de site se trouvant en aval de ladite première séquence d'acide nucléiques,
      de sorte que les séquences d'une même paire n'encadrent jamais les deux séquences de l'autre paire,
      la séquence de ladite première molécule d'acides nucléiques étant telle que, en l'absence de combinaison induite par ladite une recombinase spécifique de site, présente un cadre ouvert de lecture codant pour ledit premier gène rapporteur dans une orientation 3'-5',
   - par la recombinase spécifique de site codée par un gène contenu dans le génome d'un virus provoquant une immunodéficience chez un mammifère,
   ladite séquence recombinée comprenant une première séquence présentant un cadre ouvert de lecture codant pour ledit premier gène rapporteur dans une orientation 5'-3', ladite première séquence étant bordée par une seule séquence d'une première paire de séquences ciblant une recombinase spécifique de site et une seule séquence d'une seconde paire de séquences ciblant une recombinase spécifique de site,
   de sorte que si en amont de son premier nucléotide dans sa partie 5' ladite première séquence recombinée est bordée par une séquence d'une première paire de séquence ciblant une recombinase spécifique de site, ladite séquence recombinée est bordée en aval de son dernier nucléotide 3' par une séquence d'une seconde paire de séquence ciblant une recombinase spécifique de site et si en amont de son premier nucléotide dans sa partie 5' ladite première séquence recombinée est bordée par une séquence d'une seconde paire de séquence ciblant une recombinase spécifique de site, ladite séquence recombinée est bordée en aval de son dernier nucléotide 3' par une séquence d'une première paire de séquence ciblant une recombinase spécifique de site, où lesdites séquences desdites première et seconde paires de séquences ciblant une recombinase spécifique de site sont dans une même orientation, et
b) éventuellement le génome d'un virus provoquant une immunodéficience chez un mammifère comprenant un gène codant pour une recombinase spécifique de site, ladite cellule hématopoïétique étant notamment résistante à une thérapie anti virale contre ledit virus.

Par cellule hématopoïétique, on entend ici et dans l'invention toute cellule sanguine du lignage myéloïde ou lymphoïde, ce qui regroupe les éosinophile, les neutrophiles, les basophiles, les monocytes, les macrophages, les lymphocytes B et T, les cellules NK, les mastocystes, les plasmocytes, les cellules les dérivés des proérythroblastes et des mégacaryocytes, ainsi que tous les précurseurs desdites cellules, y compris les cellules souches hématopoïétiques, notamment les cellules CD34+.
Par «comprenant dans son génome » on entend dans l'invention que les molécules d'acides nucléiques sont intégrées dans l'ADN de la cellule hématopoïétique. Ainsi, lorsque celle-ci se divisera, elle transmettra à sa descendance les molécules d'acides nucléiques qui ont été intégrée.

La cellule hématopoïétique susmentionnée est notamment une cellule réservoir dudit virus.

Les cellules susmentionnées sont les cellules qui ont subi la recombinaison par la recombinase, et dans laquelle la séquence du gène rapporteur qui était initialement dans le sens 3'->5' est désormais dans le sens 5'->3'. Cette cellule est donc capable d'exprimer le rapporteur. Si le rapporteur est une protéine auto-fluorescente, la cellule hématopoïétique sera donc auto-fluorescente.

Avantageusement, la cellule susmentionnée comprend une seule séquence Lox P1 de séquence SEQ ID NO : 1 ou 3, et une seule séquence Lox P2272 de séquence SEQ ID NO : 2 ou 4.

Avantageusement, la séquence du gène rapporteur, orienté dans le sens 5'->3' à l'issu de la recombinaison, comprend essentiellement ou consiste en la séquence SEQ ID NO : 9.

Il est par ailleurs décrit la cellule hématopoïétique susmentionnée, dans laquelle la molécule d'acide nucléique comprend essentiellement ou consiste en l'une des séquences suivantes :
- pHR-4lox-RFP/GFP flox SEQ ID NO : 29,
- HR-4lox-CMV-RFP-PGK-GFP-WPRE floxé (SEQ ID NO: 41)
- HR-4lox-SFFV-RFP-PGK-GFP-WPRE floxé (SEQ ID NO : 42)
- HR-4lox-SFFV-RFP-CMV-GFP-WPRE floxé (SEQ ID NO : 43)
- HR-4lox-CMV-lucif-PGK-GFP-WPRE flox (SEQ ID NO : 44),
- pSDT-4lox-RFP/GFP-WPRE-floxé (SEQ ID NO : 30), et
- SDT-4lox-CMV-RFP-PGK-GFP-WPRE floxé (SEQ ID NO: 45).

Dans encore un autre mode de réalisation avantageux, l'invention concerne la cellule hématopoïétique susmentionnée, dans laquelle la molécule d'acide nucléique comprend essentiellement ou consiste en l'une des séquences suivantes : SEQ ID NO : 41, SEQ ID NO : 42, SEQ IDNO : 43, SEQ ID NO : 44 ou SEQ ID NO : 45.

Il est aussi décrit la cellule hématopoïétique susmentionnée, comprenant en outre dans son génome l'une quelconque des séquences suivantes
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).
- SIVmac239-Nef-IRES-Cre (SEQ ID NO: 25),
- SIVmac239-Nef (codon opt)-IRES-Cre (SEQ ID NO : 26),
- SIVmac239-Nef-CMV-Cre (SEQ ID NO : 27), et
- SIVmac239-Nef (codon opt)-CMV-Cre (SEQ ID NO: 28).

Avantageusement, la cellule hématopoïétique susmentionnée comprend en outre la séquence d'un virus induisant une immunodéficience tel que défini ci-dessus.

Plus particulièrement l'invention concerne la cellule hématopoïétique comprenant :
- l'une quelconque des séquences SEQ ID NO : 41 à 44,
- l'une quelconque des séquences SEQ ID NO : 21 à 24.

Plus particulièrement il est décrit la cellule hématopoïétique comprenant :
- la séquence SEQ ID NO : 45 et
- l'une quelconque des séquences SEQ ID NO : 25 à 28.

Il est en outre décrit une cellule hématopoïétique comprenant dans son génome une molécule d'acides nucléiques telle que définie ci-dessus.

Dans le cadre du procédé permettant d'obtenir les cellules hématopoïétiques susmentionnées, les cellules hématopoïétiques de cet aspect sont les « produit intermédiaires » de la recombinaison par la recombinase.

La cellule hématopoïétique susmentionnée est notamment une cellule comprenant une molécule d'acides nucléiques comprenant une première séquence codant un premier rapporteur, sous contrôle d'au moins un élément nécessaire à la transcription, la première séquence étant bordée par
- au moins une première paire de séquences ciblant une recombinase spécifique de site, ladite première paire comprenant une séquence P1-1 et une séquence P1-2,
- au moins une seconde paire de séquences ciblant une recombinase spécifique de site, ladite seconde paire comprenant une séquence P2-1 et une séquence P2-2,
les séquences de chacune desdites première et seconde paires de séquences étant dans une orientation opposée l'une par rapport à l'autre,
les séquences de la première paire de séquences ciblant une recombinase spécifique de site sont incapables de recombiner avec les séquences de la seconde paire de séquences ciblant une recombinase spécifique de site et où les séquences de la seconde paire de séquences ciblant une recombinase spécifique de site sont incapables de recombiner avec les séquences de la première paire de séquences ciblant une recombinase spécifique de site,
une séquence de la première paire et une séquence de la seconde paire de séquences ciblant une recombinase spécifique de site se trouvant en amont de ladite première séquence d'acide nucléiques et une séquence de la première paire et une séquence de la seconde paire de séquences ciblant une recombinase spécifique de site se trouvant en aval de ladite première séquence d'acide nucléiques,
de sorte que les séquences d'une même paire n'encadrent jamais les deux séquences de l'autre paire,
la séquence de ladite première molécule d'acides nucléiques étant telle que, en l'absence de combinaison induite par ladite recombinase spécifique de site, présente un cadre ouvert de lecture codant ledit premier rapporteur dans une orientation 3'-5', et donc incapable de permettre la transcription et la traduction du gène rapporteur pour obtenir ledit premier rapporteur.

Cette cellule hématopoïétique comprend avantageusement la molécule d'acides nucléiques susmentionnés qui comprend en outre une seconde séquence codant un second rapporteur sous contrôle d'au moins un élément nécessaire à la transcription.

Les paires de séquences ciblant une recombinase spécifique de site avantageuses de l'invention, reconnues par la Cre recombinase du phage P1, sont les séquences Lox P1 et Lox P2272, représentées par les séquences suivantes:
Lox P1 : ATAACTTCGTATAGCATACATTATACGAAGTTAT (SEQ ID NO : 1), et
Lox P2272 : ATAACTTCGTATAAAGTATCCTATACGAAGTTAT (SEQ ID NO : 2).

Leurs complémentaires (séquence dans l'orientation opposée) sont les séquences suivantes :
Complémentaire de Lox P1 : ATAACTTCGTATAATGTATGCTATACGAAGTTAT (SEQ ID NO : 3), et
Complémentaire de Lox P2272 : ATAACTTCGTATAGGATACTTTATACGAAGTTAT (SEQ ID NO : 4).

Avantageusement, la séquence du gène rapporteur, dans son orientation 3'->5' est la séquence SEQ ID NO : 5 ou 31.

Plus avantageusement, la cellule hématopoïétique susmentionnée comprend une molécule d'acides nucléiques susmentionnée comprenant l'une quelconque des séquences SEQ ID NO : 6, SEQ ID NO : 7, SEQ ID NO : 32, SEQ ID NO : 33, SEQ ID NO : 34, SEQ ID NO : 35, SEQ ID NO : 36, SEQ ID NO : 37, SEQ ID NO : 38, SEQ ID NO : 39 et SEQ ID NO : 40,

En outre la cellule hématopoïétique susmentionnée peut comprendre la séquence d'un génome viral qui contient un gène codant pour une recombinase spécifique de site. Cette séquence virale est avantageusement une des séquences suivantes :
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23),
- HIV NL4-3-Nef (codon opt)- CMV-Cre (SEQ ID NO: 24),
- SIVmac239-Nef-IRES-Cre (SEQ ID NO: 25),
- SIVmac239-Nef (codon opt)-IRES-Cre (SEQ ID NO : 26),
- SIVmac239-Nef-CMV-Cre (SEQ ID NO : 27), et
- SIVmac239-Nef (codon opt)-CMV-Cre (SEQ ID NO: 28).

Avantageusement, la cellule susmentionnée est une cellule humaine comprenant, dans son génome,
- la séquence SEQ ID NO : 6, et avantageusement
- l'une quelconque des séquences SEQ ID NO : 10 à 12 et 21 à 24. Avantageusement, la cellule susmentionnée est une cellule humaine comprenant, dans son génome,
- la séquence SEQ ID NO : 32, et avantageusement
- l'une quelconque des séquences SEQ ID NO : 10 à 12 et 21 à 24. Avantageusement, la cellule susmentionnée est une cellule humaine comprenant, dans son génome,
- la séquence SEQ ID NO : 33, et avantageusement
- l'une quelconque des séquences SEQ ID NO : 10 à 12 et 21 à 24. Avantageusement, la cellule susmentionnée est une cellule humaine comprenant, dans son génome,
- la séquence SEQ ID NO : 34, et avantageusement
- l'une quelconque des séquences SEQ ID NO : 10 à 12 et 21 à 24. Avantageusement, la cellule susmentionnée est une cellule humaine comprenant, dans son génome,
- la séquence SEQ ID NO : 35, et avantageusement
- l'une quelconque des séquences SEQ ID NO : 10 à 12 et 21 à 24. Avantageusement, la cellule susmentionnée est une cellule humaine comprenant, dans son génome,
- la séquence SEQ ID NO : 36, et avantageusement
- l'une quelconque des séquences SEQ ID NO : 10 à 12 et 21 à 24. Avantageusement, la cellule susmentionnée est une cellule humaine comprenant, dans son génome,
- la séquence SEQ ID NO : 37, et avantageusement
- l'une quelconque des séquences SEQ ID NO : 10 à 12 et 21 à 24. Avantageusement, la cellule susmentionnée est une cellule humaine comprenant, dans son génome,
- la séquence SEQ ID NO : 38, et avantageusement
- l'une quelconque des séquences SEQ ID NO : 10 à 12 et 21 à 24. Avantageusement, la cellule susmentionnée est une cellule humaine comprenant, dans son génome,
- la séquence SEQ ID NO : 39, et avantageusement
- l'une quelconque des séquences SEQ ID NO : 10 à 12 et 21 à 24.

Avantageusement, la cellule susmentionnée est une cellule simienne comprenant, dans son génome,
- la séquence SEQ ID NO : 7, et avantageusement
- l'une quelconque des séquences SEQ ID NO : 13 à 16 et 25 à 28. Avantageusement, la cellule susmentionnée est une cellule simienne comprenant, dans son génome,
- la séquence SEQ ID NO : 40, et avantageusement
- l'une quelconque des séquences SEQ ID NO : 13 à 16 et 25 à 28.

Il est par ailleurs décrit un mammifère non humain dont le système hématopoïétique comprend, est essentiellement constitué ou est constitué de cellules hématopoïétiques telles que définies ci-dessus.

Les mammifères susmentionnés sont donc constitués de cellules de génotype essentiellement identique, à l'exception de tout ou partie de leurs cellules hématopoïétiques qui comprennent :
- soit la molécule d'acide nucléique susmentionnée ou la séquence du gène rapporteur est orientée dans le sens 3'->5', c'est notamment le cas de la totalité des cellules hématopoïétiques dudit animal,
- soit la molécule d'acide nucléique susmentionnée ou la séquence du gène rapporteur est orientée dans le sens 3'->5', et le génome dudit virus induisant l'immunodéficience, c'est notamment le cas des cellules hématopoïétiques qui expriment les récepteurs cellulaires auxdits virus
- soit la molécule d'acide nucléique susmentionnée ou la séquence du gène rapporteur est orientée dans le sens 5'->3', et le génome dudit virus induisant l'immunodéficience, c'est notamment le cas des cellules hématopoïétiques qui expriment les récepteurs cellulaires auxdits virus ; ces cellules sont les cellules issues de la recombinaison.

Les mammifères avantageux sont des singes, des souris ou des chats

Il est bien connu de l'état de la technique qu'il est possible de reconstituer l'ensemble du système hématopoïétique d'un mammifère en injectant chez cet animal des cellules souches hématopoïétiques, ou cellules CD34+, après une irradiation sub-létale.

En effet, lors que les mammifères sont irradiés aux rayons gamma à certaines doses, l'ensemble des cellules de la moelle osseuse est détruit (irradiation myélo-ablative), et le mammifère ne sera plus capable de produire de nouvelles cellules hématopoïétiques. Toutefois, l'injection de cellules souches CD34+, en respectant les règles histocompatibilité, a pour effet que les cellules souches CD34+ colonisent la moelle osseuse et donnent naissance à de nouvelles cellules hématopoïétiques qui sont capables de replacer les cellules mortes à l'issue de l'irradiation. Des exemples de protocoles expérimentaux sont donnés à titre indicatif dans les exemples pour la souris, le macaque et le chat.

La greffe de cellules souches hématopoïétique est
- soit une autogreffe, le mammifère est greffé par ses propres cellules souches hématopoïétiques, qui ont été génétiquement modifiées,
- soit une allogreffe, le mammifère est greffé par des cellules souches hématopoïétiques génétiquement modifiées d'un autre mammifère de la même espèce,
- soit une xénogreffe, le mammifère est greffé par des cellules souches hématopoïétiques génétiquement modifiées d'un autre mammifère d'espèce différente.

L'allogreffe et l'autogreffe sont particulièrement avantageuses dans le cadre de l'invention lorsque le mammifère est un singe ou un chat.

La xénogreffe permet quant à elle de reproduire un système hématopoïétique d'une espèce dans une autre espèce, et notamment de reproduire système hématopoïétique humain chez une souris immunodéficiente, en particulier une souris Nod Scid, des souris nude ou des souris Rag_{2-/-} γc_{-/-}, Dans cet exemple, après la greffe, les souris possèderont des cellules hématopoïétiques humaines, qui seront donc infectables par un virus de l'immunodéficience humaine.

On décrit ici par ailleurs l'utilisation d'un mammifère susmentionné pour la détection, notamment in vitro, de cellules réservoirs dudit virus induisant une immunodéficience chez ledit mammifère, ou pour la mise en œuvre d'un procédé permettant la détection, notamment in vitro, de cellules réservoirs dudit virus induisant une immunodéficience chez ledit mammifère.

Les mammifères susmentionnés peuvent être utilisés pour isoler les cellules réservoirs desdits virus induisant l'immunodéficience.

Après traitement avec un ou plusieurs agents antirétroviraux, les cellules infectées régressent et disparaissent à l'exception des cellules réservoirs. Aussi, à partir d'un échantillon sanguin ou un échantillon de moelle de desdits mammifères infectés et traités aux antirétroviraux, il sera possible, à l'aide de techniques appropriées, d'isoler les cellules qui expriment le rapporteur, ces cellules étant les cellules ayant été infectées par le virus qui exprime la recombinase, et dans lesquelles la molécule d'acides nucléiques a subi une recombinaison.

Si le rapporteur est une protéine auto fluorescente, il sera alors possible d'isoler les cellules réservoirs au moyen d'un cymomètre de flux / trieur de cellules, selon des protocoles de routine pour l'homme du métier.

Ainsi, l'invention concerne une méthode d'identification, notamment *in vitro,* de cellules réservoirs d'un virus induisant l'immunodéficience chez un mammifère, ladite méthode comprenant une étape de détection du gène rapporteur codé par une première séquence recombinée telle que définie précédemment dans une population de cellules hématopoïétiques.

Plus précisément l'invention concerne une méthode d'identification *in vitro* de cellules réservoirs d'un virus induisant l'immunodéficience chez un mammifère, comprenant les étapes suivantes :
- une étape de transformation de cellules souches hématopoïétiques avec une molécule d'acides nucléiques,
   ladite molécule d'acides nucléiques comprenant une séquence codant un gène rapporteur, sous contrôle d'au moins un élément nécessaire à la transcription, la séquence codant un gène rapporteur étant bordée par
      - au moins une première paire de séquences ciblant une recombinase spécifique de site, ladite première paire comprenant une séquence P1-1 et une séquence P1-2,
      - au moins une seconde paire de séquences ciblant une recombinase spécifique de site, ladite seconde paire comprenant une séquence P2-1 et une séquence P2-2,
   les séquences de chacune desdites première et seconde paires de séquences étant dans une orientation opposée l'une par rapport à l'autre,
   les séquences de la première paire de séquences ciblant une recombinase spécifique de site étant incapables de recombiner avec les séquences de la seconde paire de séquences ciblant une recombinase spécifique de site et les séquences de la seconde paire de séquences ciblant une recombinase spécifique de site étant incapables de recombiner avec les séquences de la première paire de séquences ciblant une recombinase spécifique de site,
   une séquence de la première paire et une séquence de la seconde paire de séquences ciblant une recombinase spécifique de site se trouvant en amont de ladite première séquence d'acide nucléiques et une séquence de la première paire et une séquence de la seconde paire de séquences ciblant une recombinase spécifique de site se trouvant en aval de ladite première séquence d'acide nucléiques,
   de sorte que les séquences d'une même paire n'encadrent jamais les deux séquences de l'autre paire,
   la séquence codant un gène rapporteur, en l'absence de recombinaison induite par ladite recombinase spécifique de site, étant telle qu'elle présente un cadre ouvert de lecture codant ledit gène rapporteur dans une orientation 3'-5', et donc incapable de permettre la transcription et la traduction du gène rapporteur
- une étape de reconstitution du système hématopoïétique d'un mammifère ayant subi une myéloablation, avec les cellules souches hématopoïétiques susmentionnées,
- l'infection du mammifère non humain ayant une moelle reconstituée à l'étape précédente avec un virus induisant une immunodéficience chez le mammifère dont sont issues les cellules souches hématopoïétiques, ledit virus exprimant une recombinase spécifique de sites,
- une étape de traitement du mammifère non humain infecté susmentionné avec un traitement inhibant le développement dudit virus, et
- une étape de détection des cellules hématopoïétiques exprimant le rapporteur recombiné, lesdites cellules hématopoïétiques exprimant le rapporteur étant des cellules réservoirs d'un virus induisant l'immunodéficience chez un mammifère.

De manière avantageuse, l'invention concerne la méthode susmentionnée, dans laquelle la recombinase spécifique de site est la recombinase Cre du bactériophage P1.

De manière plus avantageuse, l'invention concerne la méthode susmentionnée, dans laquelle les séquences de la première paire de séquences ciblant une recombinase spécifique de site et les séquences de la seconde paire de séquences ciblant une recombinase spécifique de site sont choisie parmi Lox P1, Lox P2272, Lox 66, Lox 71, Lox 511, Lox 512, Lox 514 et des séquences mutées du site Lox P1, portant au moins une mutation ponctuelle dans la séquence espaceur.

De manière plus avantageuse, l'invention concerne la méthode susmentionnée,dans laquelle ladite molécule d'acide nucléique comprend ou consiste essentiellement en l'une des séquences suivantes : SEQ ID NO : 6, SEQ ID NO : 7, SEQ ID NO : 32, SEQ ID NO : 33, SEQ ID NO : 34, SEQ ID NO : 35, SEQ ID NO : 36, SEQ ID NO : 37, SEQ ID NO : 38, SEQ ID NO : 39 et SEQ ID NO : 40.

Comme mentionné ci-dessus, les cellules hématopoïétiques susmentionnées ayant subit une recombinaison sont susceptibles d'être des cellules réservoirs pour le virus induisant l'immunodéficience.

Avantageusement, le procédé comprend une étape de sélection desdites cellules réservoirs contenues dans une population de cellules hématopoïétiques, en détectant, à l'aide des moyens appropriés, le rapporteur qui est exprimé dans les cellules qui sont :
- infectées par le virus induisant l'immunodéficience, et qui exprime en plus la recombinase.
- et transformées avec la molécule d'acides nucléiques susmentionnée.

Seules les cellules qui auront subi une recombinaison du gène rapporteur seront détectables car seules ces cellules exprimeront le rapporteur.

A l'issu de ce procédé, les cellules identifiées, sont des cellules hématopoïétiques, recombinée pour le gène rapporteur, infectées par le virus, et capable de « réactiver », et résistantes aux traitements contre ledit virus. Il s'agit des cellules réservoirs dudit virus. L'invention concerne aussi une cellule hématopoïétique susceptible d'être obtenue par la méthode susmentionnée.

L'invention concerne par ailleurs un mammifère non humain dont le système hématopoïétique est essentiellement constitué ou constitué de cellules hématopoïétiques telles que définies ci-dessus.

Dans un autre aspect de l'invention, il est aussi possible de proposer une méthode de caractérisation des cellules hématopoïétiques réservoirs obtenues par le procédé susmentionné, en utilisant une collection d'anticorps dirigés contre des marqueurs de différenciation CD exprimés à la surface de ces cellules.

La caractérisation des cellules réservoir permet ainsi de déterminer leur type génétique, et de proposer des compositions ou drogues qui tuent spécifiquement sans avoir d'effet sur la mortalité des autres cellules du mammifère.

Les modes de réalisations avantageux susmentionnés concernant l'utilisation, les cellules et les mammifères non humains s'appliquent *mutatis mutandis* à la présente méthode.

Il est également décrit un kit pour identifier et/ou isoler les cellules réservoirs d'un virus induisant une immunodéficience chez un mammifère comprenant
- une molécule d'acides nucléiques telle que définie ci-dessus, et notamment comprenant l'une quelconque des séquences SEQ ID NO : 6, SEQ ID NO : 7, SEQ ID NO : 32, SEQ ID NO : 33, SEQ ID NO : 34, SEQ ID NO : 35, SEQ ID NO : 36, SEQ ID NO : 37, SEQ ID NO : 38, SEQ ID NO : 39 et SEQ ID NO : 40, et
- au moins une molécule d'acides nucléiques comprenant la séquence dudit virus induisant une immunodéficience chez un mammifère, et comprenant dans son génome un gène codant pour ladite recombinase spécifique de site, et notamment au moins l'une quelconque des séquences suivantes :
   - pBR-NL4-3-Nef-IRES-Cre (SEQ ID NO : 10),
   - pBR-NL4-3-Nef (codon opt)-IRES-Cre (SEQ ID NO : 11),
   - pBR-NL4-3-Nef-CMV-Cre (SEQ ID NO : 12),
   - pBR-SIVmac239-Nef-IRES-Cre (SEQ ID NO: 13),
   - pBR-SIVmac239-Nef (codon opt)-IRES-Cre (SEQ ID NO : 14),
   - pBR-SIVmac239-Nef-CMV-Cre (SEQ ID NO : 15),
   - pBR-SIVmac239-Nef (codon opt)-CMV-Cre (SEQ ID NO: 16),
   - HIV NL4-3-Nef-IRES-Cre (SEQ ID NO : 21),
   - HIV NL4-3-Nef (codon opt)-IRES-Cre (SEQ ID NO : 22),
   - HIV NL4-3-Nef-CMV-Cre (SEQ ID NO : 23),
   - HIV NL4-3-Nef (codon opt)- CMV-Cre (SEQ ID NO: 24),
   - SIVmac239-Nef-IRES-Cre (SEQ ID NO: 25),
   - SIVmac239-Nef (codon opt)-IRES-Cre (SEQ ID NO : 26),
   - SIVmac239-Nef-CMV-Cre (SEQ ID NO : 27), et
   - SIVmac239-Nef (codon opt)-CMV-Cre (SEQ ID NO: 28),
   - éventuellement en association avec des moyens de détection dudit gène rapporteur.

Avantageusement, le kit susmentionné comprend : une molécule d'acides nucléiques comprenant la séquence SEQ ID NO : 6 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-NL4-3-Nef-IRES-Cre (SEQ ID NO : 10),
- pBR-NL4-3-Nef (codon opt)-IRES-Cre (SEQ ID NO : 11),
- pBR-NL4-3-Nef-CMV-Cre (SEQ ID NO : 12),
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).

Avantageusement, le kit susmentionné comprend : une molécule d'acides nucléiques comprenant la séquence SEQ ID NO : 32 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-NL4-3-Nef-IRES-Cre (SEQ ID NO : 10),
- pBR-NL4-3-Nef (codon opt)-IRES-Cre (SEQ ID NO : 11),
- pBR-NL4-3-Nef-CMV-Cre (SEQ ID NO : 12),
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).

Avantageusement, le kit susmentionné comprend : une molécule d'acides nucléiques comprenant la séquence SEQ ID NO : 33 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-NL4-3-Nef-IRES-Cre (SEQ ID NO : 10),
- pBR-NL4-3-Nef (codon opt)-IRES-Cre (SEQ ID NO : 11),
- pBR-NL4-3-Nef-CMV-Cre (SEQ ID NO : 12),
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).

Avantageusement, le kit susmentionné comprend : une molécule d'acides nucléiques comprenant la séquence SEQ ID NO : 34 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-NL4-3-Nef-IRES-Cre (SEQ ID NO : 10),
- pBR-NL4-3-Nef (codon opt)-IRES-Cre (SEQ ID NO : 11),
- pBR-NL4-3-Nef-CMV-Cre (SEQ ID NO : 12),
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).

Avantageusement, le kit susmentionné comprend : une molécule d'acides nucléiques comprenant la séquence SEQ ID NO : 35 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-NL4-3-Nef-IRES-Cre (SEQ ID NO : 10),
- pBR-NL4-3-Nef (codon opt)-IRES-Cre (SEQ ID NO : 11),
- pBR-NL4-3-Nef-CMV-Cre (SEQ ID NO : 12),
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).

Avantageusement, le kit susmentionné comprend : une molécule d'acides nucléiques comprenant la séquence SEQ ID NO : 36 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-NL4-3-Nef-IRES-Cre (SEQ ID NO : 10),
- pBR-NL4-3-Nef (codon opt)-IRES-Cre (SEQ ID NO : 11),
- pBR-NL4-3-Nef-CMV-Cre (SEQ ID NO : 12),
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).

Avantageusement, le kit susmentionné comprend : une molécule d'acides nucléiques comprenant la séquence SEQ ID NO : 37 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-NL4-3-Nef-IRES-Cre (SEQ ID NO : 10),
- pBR-NL4-3-Nef (codon opt)-IRES-Cre (SEQ ID NO : 11),
- pBR-NL4-3-Nef-CMV-Cre (SEQ ID NO : 12),
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).

Avantageusement, le kit susmentionné comprend : une molécule d'acides nucléiques comprenant la séquence SEQ ID NO : 38 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-NL4-3-Nef-IRES-Cre (SEQ ID NO : 10),
- pBR-NL4-3-Nef (codon opt)-IRES-Cre (SEQ ID NO : 11),
- pBR-NL4-3-Nef-CMV-Cre (SEQ ID NO : 12),
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).

Avantageusement, le kit susmentionné comprend : une molécule d'acides nucléiques comprenant la séquence SEQ ID NO : 39 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-NL4-3-Nef-IRES-Cre (SEQ ID NO : 10),
- pBR-NL4-3-Nef (codon opt)-IRES-Cre (SEQ ID NO : 11),
- pBR-NL4-3-Nef-CMV-Cre (SEQ ID NO : 12),
- HIV NL4-3-Nef-IRES-Cre (SEQ ID NO : 21),
- HIV NL4-3-Nef (codon opt)-IRES-Cre (SEQ ID NO : 22),
- HIV NL4-3-Nef-CMV-Cre (SEQ ID NO : 23), et
- HIV NL4-3-Nef (codon opt)- CMV-Cre (SEQ ID NO: 24).

Avantageusement, le kit susmentionné comprend : une molécule d'acides nucléiques comprenant la séquence SEQ ID NO : 7 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-SIVmac239-Nef-IRES-Cre (SEQ ID NO: 13),
- pBR-SIVmac239-Nef (codon opt)-IRES-Cre (SEQ ID NO : 14),
- pBR-SIVmac239-Nef-CMV-Cre (SEQ ID NO : 15),
- pBR-SIVmac239-Nef (codon opt)-CMV-Cre (SEQ ID NO: 16),
- SIVmac239-Nef-IRES-Cre (SEQ ID NO: 25),
- SIVmac239-Nef (codon opt)-IRES-Cre (SEQ ID NO : 26),
- SIVmac239-Nef-CMV-Cre (SEQ ID NO : 27), et
- SIVmac239-Nef (codon opt)-CMV-Cre (SEQ ID NO: 28).

Avantageusement, le kit susmentionné comprend : une molécule d'acides nucléiques comprenant la séquence SEQ ID NO : 40 et une molécule d'acides nucléiques comprenant la séquence dudit virus induisant une immunodéficience chez un mammifère, et comprenant dans son génome un gène codant pour ladite recombinase spécifique, ladite séquence de virus comprenant la séquence de la Cre recombinase comprenant l'une quelconque des séquences suivantes :
- pBR-SIVmac239-Nef-IRES-Cre (SEQ ID NO: 13),
- pBR-SIVmac239-Nef (codon opt)-IRES-Cre (SEQ ID NO : 14),
- pBR-SIVmac239-Nef-CMV-Cre (SEQ ID NO : 15),
- pBR-SIVmac239-Nef (codon opt)-CMV-Cre (SEQ ID NO: 16),
- SIVmac239-Nef-IRES-Cre (SEQ ID NO: 25),
- SIVmac239-Nef (codon opt)-IRES-Cre (SEQ ID NO : 26),
- SIVmac239-Nef-CMV-Cre (SEQ ID NO : 27), et
- SIVmac239-Nef (codon opt)-CMV-Cre (SEQ ID NO: 28).

Lorsque le kit comprend une molécule d'acides nucléiques permettant, après recombinaison, un rapporteur protéique auto fluorescent, les moyens de détection peuvent être des instructions sous forme d'un produit programme d'ordinateur sur un support approprié, permettant de sélectionnée, notamment par cytométrie de flux, les cellules réservoirs, c'est à dires les cellules hématopoïétiques exprimant le gène rapporteur.

Le kit peut comprendre également des moyens permettant de purifier des cellules souches hématopoïétiques, cellules qui seront transformées par ladite molécule d'acides nucléiques.

Le kit susmentionné comprend avantageusement en outre un ou plusieurs antirétroviraux, notamment les antirétroviraux indiqués dans les exemples.

L'invention sera mieux comprise à la lumière des quatre exemples et des vingt-et-une figures suivantes.

### Brève description des figures

La **figure 1** représente les modes de recombinaison par inversion (1.) et délétion (2.) par recombinaison spécifique de site.
La **figure 2** représente les modes de recombinaison par inversion possibles lorsque la molécule d'acides nucléiques comprend deux paires de séquences de recombinaison spécifique de site.
La **figure 3** représente une des constructions de l'invention et les mécanismes de la recombinaison du gène rapporteur.
La **figure 4** représente cinq exemples de construction (molécule d'acide nucléique) selon l'invention basée sur une détection des cellules réservoir du VIH1.
Les **figures 5A à 5H** sont des images de microscopie à fluorescence montrant la recombinaison du gène rapporteur, lorsque la molécule d'acides nucléiques est transduite dans des cellules humaines 293T, et que ces cellules ont été transfectées ou infectées par un virus VIH-1 exprimant la Cre recombinase.

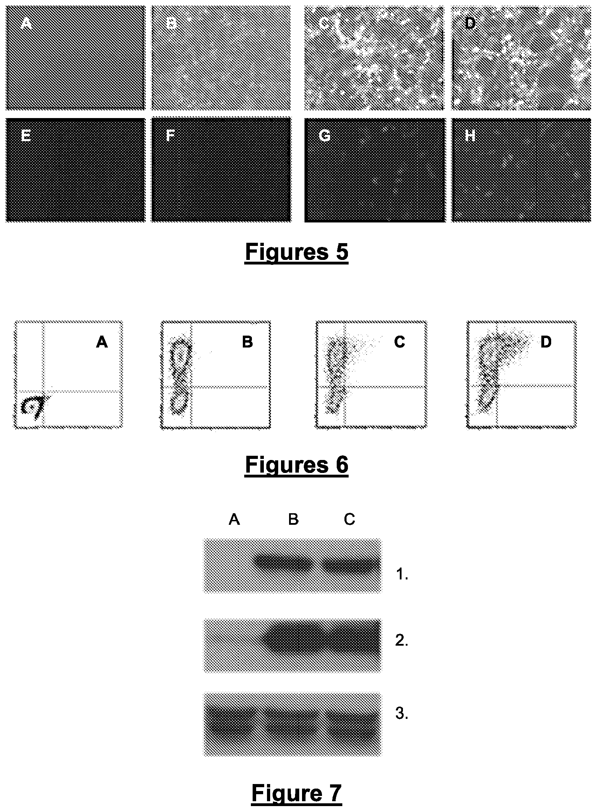

La figure 5A est une image de détection de la GFP dans les cellules non transduites et non transfectées ou non infectées (contrôle).
La figure 5B est une image de détection de la GFP dans les cellules transduites et non transfectées ou non infectées (contrôle de recombinaison).
La figure 5C est une image de détection de la GFP dans les cellules transduites et transfectées (avec un plasmide contenant le génome du virus).
La figure 5D est une image de détection de la GFP dans les cellules transduites et infectées avec un virus.
La figure 5E est une image de détection de la RFP dans les cellules non transduites et non transfectées ou non infectées (contrôle).
La figure 5F est une image de détection de la RFP cellules transduites et non transfectées ou non infectées (contrôle de recombinaison).
La figure 5G est une image de détection de la RFP dans les cellules transduites et transfectées (avec un plasmide contenant le génome du virus).
La figure 5H est une image de détection de la RFP dans les cellules transduites et infectées avec un virus.
Les **figures 6A à 6D** sont des graphiques montrant les images de cytométrie de flux pour les cellules exprimant la GFP (ordonnées) et la RFP (abscisses). Les cellules doublement marquées sont indiqués dans chaque figure dans le carré en haut à droite.
La figure 6A est le résultat obtenu par cytométrie de flux pour les cellules illustrées aux figures 5A et 5E.
La figure 6B est le résultat obtenu par cytométrie de flux pour les cellules illustrées aux figures 5B et 5F.
La figure 6C est le résultat obtenu par cytométrie de flux pour les cellules illustrées aux figures 5C et 5G.
La figure 6D est le résultat obtenu par cytométrie de flux pour les cellules illustrées aux figures 5D et 5H.
**La** **figure 7** est un western blot montrant l'expression de la Cre recombinase (1.), et de la protéine p24 du VIH (2.) dans des cellules illustrées aux figures 5B et 5F (A), aux figures 5C et 5G (B) et aux figures 5D et 5H (C). En contrôle, la charge est contrôlée par la détection des kinases Erk1/2 (3.).

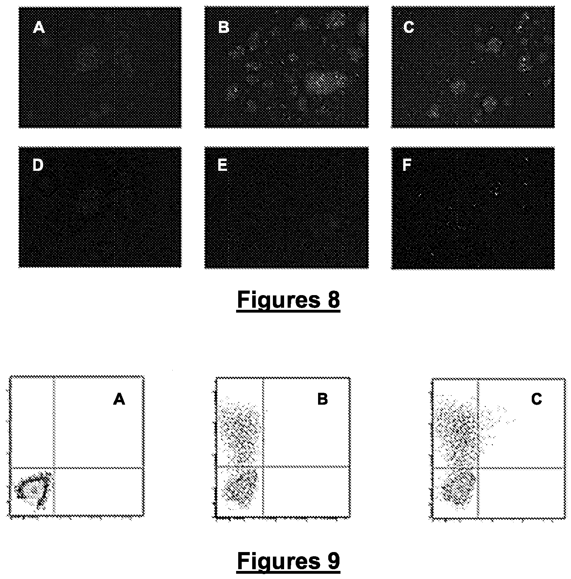

Les **figures 8A à 8F** sont des images de microscopie à fluorescence montrant la recombinaison du gène rapporteur, lorsque la molécule d'acides nucléiques est transduite dans des cellules humaines mononucléées de sang périphérique, et que ces cellules ont infectées par un virus VIH-1 exprimant la Cre recombinase.
La figure 8A est une image de détection de la GFP dans les cellules non transduites et non infectées (contrôle).
La figure 8B est une image de détection de la GFP dans les cellules transduites et non infectées (contrôle de recombinaison).
La figure 8C est une image de détection de la GFP dans les cellules transduites et infectées avec un virus.
La figure 8D est une image de détection de la RFP dans les cellules non transduites et non infectées (contrôle).
La figure 8E est une image de détection de la RFP dans les cellules transduites et non infectées (contrôle de recombinaison).
La figure 8F est une image de détection de la RFP dans les cellules transduites et infectées avec un virus.
Les **figures 9A à 9C** sont des graphiques montrant les images de cytométrie de flux pour les cellules exprimant la GFP (ordonnées) et la RFP (abscisses). Les cellules doublement marquées sont indiqués dans chaque figure dans le carré en haut à droite.
La figure 9A est le résultat obtenu par cytométrie de flux pour les cellules illustrées aux figures 8A et 8D.
La figure 9B est le résultat obtenu par cytométrie de flux pour les cellules illustrées aux figures 8B et 8E.
La figure 9C est le résultat obtenu par cytométrie de flux pour les cellules illustrées aux figures 5C et 5F.

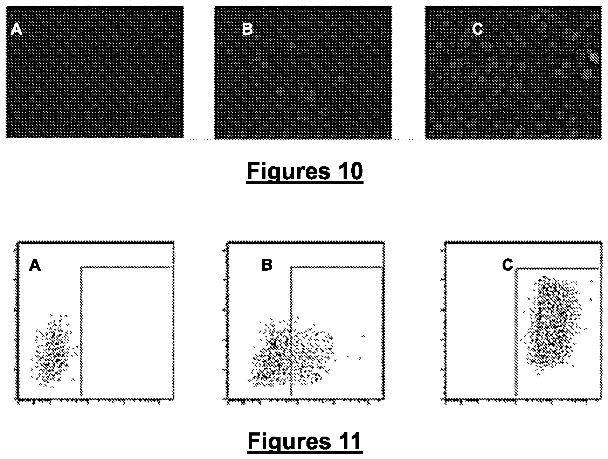

Les **figures 10A à 10C** sont des images de microscopie à fluorescence montrant le bon niveau de transduction (expression GFP) des cellules CD34+ humaines qui seront utilisées pour les reconstitutions. L'expression de la GFP dans des cellules CD34+ humaines transduites par la molécule d'acides nucléiques est contrôlées 24 et 48 heures post-transduction.
La figure 10A est une image de détection de la GFP dans les cellules non transduites et non infectées (contrôle).
La figure 10B est une image de détection de la GFP à 24 heures dans les cellules transduites à une multiplicité d'infection de 5 et non infectées.
La figure 10C est une image de détection de la GFP à 48 heures dans les cellules transduites à une multiplicité d'infection de 5 et non infectées.
La figure 10D est une image de détection de la RFP dans les cellules non transduites et non infectées (contrôle).
La figure 10E est une image de détection de la RFP à 24 heures dans les cellules transduites à une multiplicité d'infection de 5 et non infectées.
La figure 10F est une image de détection de la RFP à 48 heures dans les cellules transduites à une multiplicité d'infection de 5 et non infectées.
Les **figures 11A à 11C** sont des graphiques montrant les images de cytométrie de flux pour les cellules selon leur granulométrie (ordonnées) et la GFP (abscisses). Les cellules doublement marquées sont indiqués dans chaque figure dans le carré en haut à droite.
La figure 11A est le résultat obtenu par cytométrie de flux pour les cellules illustrées aux figures 10A et 10D.
La figure 11B est le résultat obtenu par cytométrie de flux pour les cellules illustrées aux figures 10B et 10E.
La figure 11C est le résultat obtenu par cytométrie de flux pour les cellules illustrées aux figures 10C et 10F.

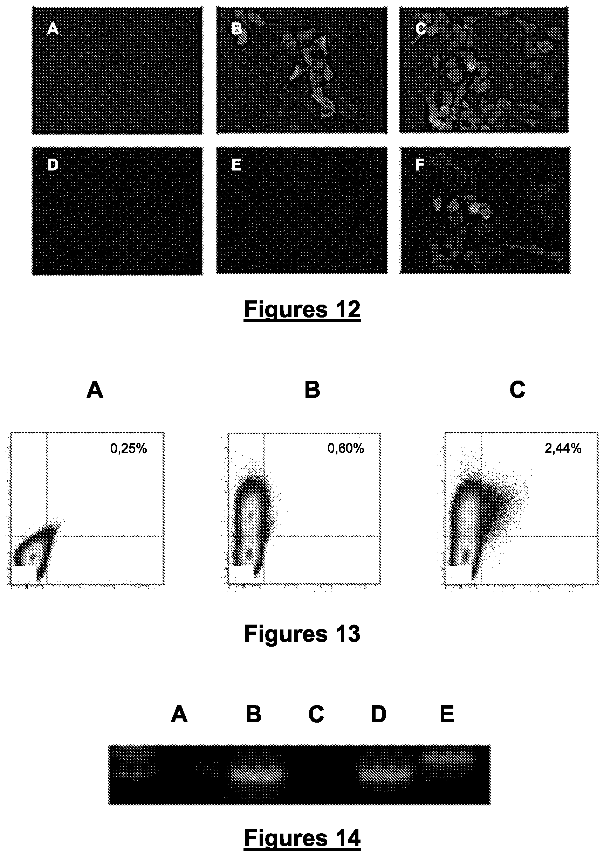

Les figures **12A à 12C** sont des images de microscopie à fluorescence montrant la recombinaison du gène rapporteur, lorsque la molécule d'acides nucléiques est transduite dans des cellules 293T, et que ces cellules ont été infectées par un virus SIVmac exprimant la Cre recombinase.
La figure 12A est une image de détection de la GFP dans les cellules non transduites et non infectées (contrôle).
La figure 12B est une image de détection de la GFP dans les cellules transduites et non infectées.
La figure 12C est une image de détection de la GFP dans les cellules transduites et infectées avec un virus.
La figure 12D est une image de détection de la RFP dans les cellules non transduites et non infectées (contrôle).
La figure 12E est une image de détection de la RFP dans les cellules transduites et non infectées.
La figure 12F est une image de détection de la RFP dans les cellules transduites et infectées avec un virus.
Les figures **13A à 13C** sont des graphiques montrant les images de cytométrie de flux pour les cellules exprimant la RFP (ordonnées) et la GFP (abscisses). Les cellules doublement marquées sont indiqués dans chaque figure dans le carré en haut à droite.
La figure 13A est le résultat obtenu par cytométrie de flux pour les cellules illustrées aux figures 12A et 12D.
La figure 13B est le résultat obtenu par cytométrie de flux pour les cellules illustrées aux figures 12B et 12E.
La figure 13C est le résultat obtenu par cytométrie de flux pour les cellules illustrées aux figures 12C et 12F.
La **figure 14** est une image de PCR montrant la détection des formes non floxées et floxées de la molécule d'acides nucléiques dans les cellules 293T transduites par la molécule d'acides nucléiques et infectées par le virus exprimant la Cre recombinase.
La figure 14A est une image de la détection des formes non floxées dans des cellules non transduites par la molécule d'acides nucléiques et non infectées (contrôle).
La figure 14B est une image de la détection des formes non floxées dans des cellules transduites par la molécule d'acides nucléiques et non infectées.
La figure 14C est une image de la détection des formes floxées dans des cellules transduites par la molécule d'acides nucléiques et non infectées.
La figure 14D est une image de la détection des formes non floxées dans des cellules transduites par la molécule d'acides nucléiques et infectées.
La figure 14E est une image de la détection des formes floxées dans des cellules transduites par la molécule d'acides nucléiques et infectées.

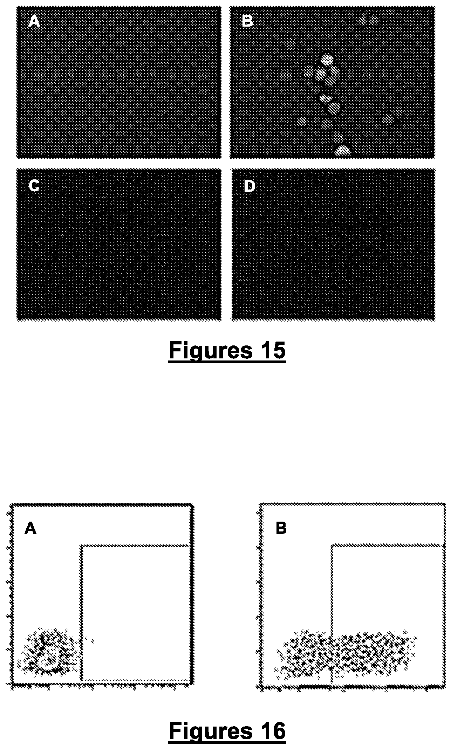

Les figures **15A à 15D** sont des images de microscopie à fluorescence montrant le bon niveau de transduction (expression GFP) des cellules CD34+ de macaque qui seront utilisées pour les reconstitutions. L'expression de la GFP dans des cellules CD34+ de macaque transduites par la molécule d'acides nucléiques est contrôlée 48 heures post-transduction.
La figure 15A est une image de détection de la GFP dans les cellules non transduites et non infectées (contrôle).
La figure 15B est une image de détection de la GFP dans les cellules transduites et non infectées par un virus après 48 heures de cultures.
La figure 15C est une image de détection de la RFP dans les cellules non transduites et non infectées (contrôle).
La figure 15D est une image de détection de la RFP dans les cellules transduites et non infectées par un virus après 48 heures de cultures.
Les figures **16A à 16B** sont des graphiques montrant les images de cytométrie de flux pour les cellules selon la granulosité (ordonnées) et la GFP (abscisses). Les cellules exprimant la GFP sont indiquées dans chaque figure dans le carré en haut à droite.
La figure 16A est le résultat obtenu par cytométrie de flux pour les cellules illustrées dans la figure 15A.
La figure 16B est le résultat obtenu par cytométrie de flux pour les cellules illustrées dans la figure 15B.

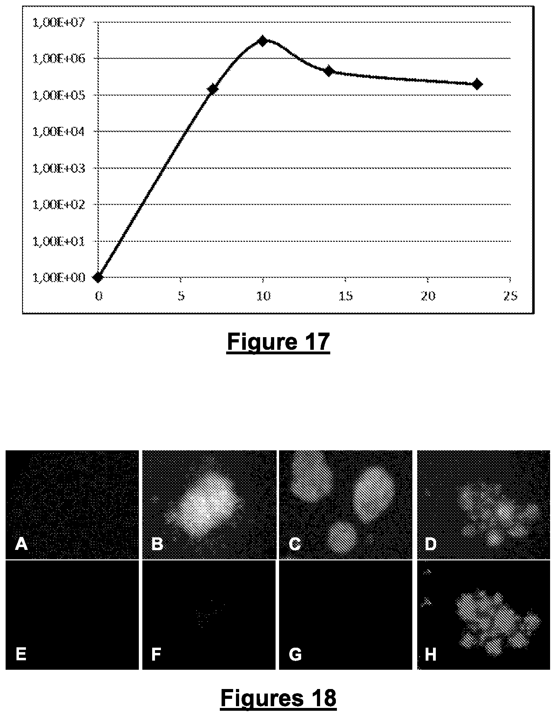

La **figure 17** est un graphique montrant la réplication du virus VIS exprimant la Cre recombinase évaluée par le nombre de copies d'ARN viral /mL de plasma (ordonnées) après infection chez le macaque, en fonction du temps (en jours).
Les **figures 18A à H** représentent des images de microscopie à fluorescence montrant le niveau de transduction (expression GFP) et de recombinaison (expression RFP) de cellules MT4C5.
La figure 18A représente une image de microscopie à fluorescence détectant la GFP (montrant la transfection avec le rapporteur de l'invention) dans des cellules MT4C5 non transfectées et non infectées par un virus du VIH.
La figure 18B représente une image de microscopie à fluorescence détectant la GFP (montrant la transfection avec le rapporteur de l'invention) dans des cellules MT4C5 transfectées avec le rapporteur et non infectées par un virus du VIH.
La figure 18C représente une image de microscopie à fluorescence détectant la GFP (montrant la transfection avec le rapporteur de l'invention) dans des cellules MT4C5 transfectées avec le rapporteur et infectées par un virus du VIH contrôle.
La figure 18D représente une image de microscopie à fluorescence détectant la GFP (montrant la transfection avec le rapporteur de l'invention) dans des cellules MT4C5 transfectées avec le rapporteur et infectées par un virus du VIH exprimant la recombinase CRE.
La figure 18E représente une image de microscopie à fluorescence détectant la RFP (montrant la recombinaison du rapporteur de l'invention) dans des cellules MT4C5 non transfectées et non infectées par un virus du VIH.
La figure 18F représente une image de microscopie à fluorescence détectant la RFP (montrant la recombinaison du rapporteur de l'invention) dans des cellules MT4C5 transfectées avec le rapporteur et non infectées par un virus du VIH.
La figure 18G représente une image de microscopie à fluorescence détectant la RFP (montrant la recombinaison du rapporteur de l'invention) dans des cellules MT4C5 transfectées avec le rapporteur et infectées par un virus du VIH contrôle.
La figure 18H représente une image de microscopie à fluorescence détectant la RFP (montrant la recombinaison du rapporteur de l'invention) dans des cellules MT4C5 transfectées avec le rapporteur et infectées par un virus du VIH exprimant la recombinase CRE.

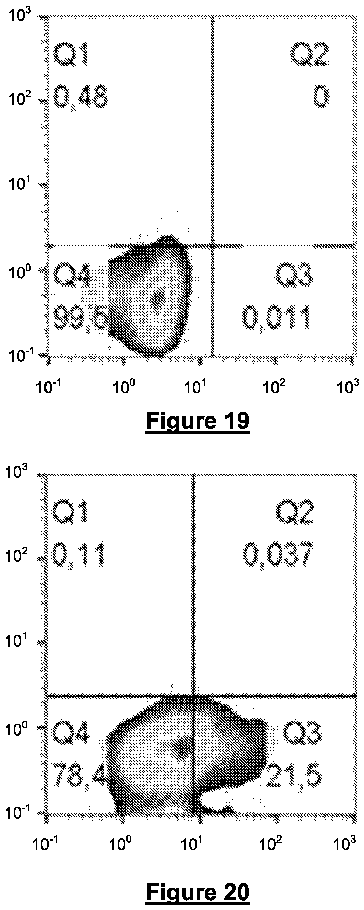

La **figure 19** représente une image de cytométrie de flux selon l'expression de RFP (ordonnées ; recombinaison du rapporteur) et l'expression de la protéine p24 du VIH (abscisses : réplication virale) à partir de cellules MT4C5 transfectées avec le rapporteur mais non infectées avec le VIH.
La **figure 20** représente une image de cytométrie de flux selon l'expression de RFP (ordonnées ; recombinaison du rapporteur) et l'expression de la protéine p24 du VIH (abscisses : réplication virale) à partir de cellules MT4C5 transfectées avec le rapporteur et infectées avec le VIH contrôle.

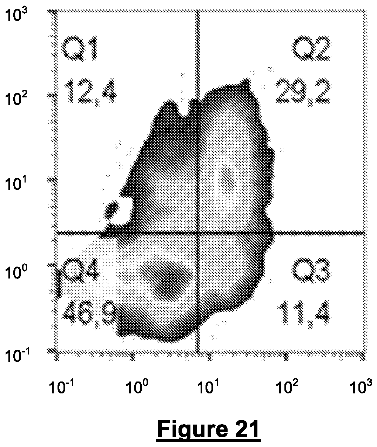

La **figure 21** représente une image de cytométrie de flux selon l'expression de RFP (ordonnées ; recombinaison du rapporteur) et l'expression de la protéine p24 du VIH (abscisses : réplication virale) à partir de cellules MT4C5 transfectées avec le rapporteur et infectées avec le VIH exprimant la recombinase CRE.

### EXEMPLES

### Exemple 1 : Méthode d'identification de cellules réservoirs humaines

### A- Matériel et méthodes

### 1. Isolement et pré-stimulation des cellules CD34+ à partir de la moelle osseuse ou de prélèvement de sang de cordon.

Les poches de sang de cordon frais sont récupérées via le réseau hospitalier
- Préparer 20mL de ficoll dans un tube Falcon 50.
- Diluer la moelle osseuse/sang de cordon en PBS jusqu'à un volume de 30mL (dilution au moins 1/2-1/3).
- Déposer doucement les 30mL de moelle sur le gel de Ficoll.
- Centrifuger 30min minimum à 1800rpm, RT et sans frein.
- Récupérer l'anneau de cellules à la pipette 10ml et le déposer dans un nouveau Falcon 50.
- Compléter en PBS le volume jusqu'à 50mL.
- Centrifuger 10 min 1800 rpm.
- Vider le surnageant à la pipette et casser le culot.
- S'il y a trop d'érythrocytes, ajouter 5mLde solution de lyse, bien homogénéiser et laisser 5 min maximum. Ajouter au moins 10mL de PBS pour arrêter la réaction.
- Centrifuger 10 min 1800rpm.
- Vider le surnageant, casser le culot.
- Re-suspendre dans 1mL final de tampon d'isolation (cf 2).
- Compter les cellules mononucléées (CBMNC/BMMC) avec exclusion des mortes en bleu trypan.
- Réserver dans microtube 2 x 100 000 cellules BMMC pour le marquage. On complète qsp 100µL de PBS pour éviter le séchage.

### Isolement des CD34+ par méthode billes magnétiques Dynal (sélection positive)

- Auparavant : préparer le tampon d'isolation : PBS 2%BSA 0.6%Citrate ou EDTA (100IU/mL Penicilline-Streptomycine), filtré sur membrane 0,2µm.
- Dans un eppendorf de 1.8mL : ajouter 100µL DynaBeads/ mL BMMC (capacité 4.107< CMMO<4.108 CBMNC/BMMC).
- Nettoyer 3 fois avec 1mL de tampon d'isolation sur l'aimant Dynal
   (ajouter 1mL de tampon ; bien homogénéiser ; appliquer l'aimant contre l'eppendorf et laisser les billes se rapprocher de l'aimant pendant 1 minute ; récolter à la P1000 la fraction négative - éloigner l'aimant de l'eppendorf ; resuspendre les cellules+billes dans 1mL de tampon d'isolation).
- Ajouter tout de suite les BMMC sur le culot de billes.
- Vortexer doucement 2-3 secondes.
- Incuber 30 minutes à 4°C (agitation douce 10-20 rpm).
- Pendant ce temps, identifier les microtubes de marquage de contrôle.
- Laver 5 à 7 fois sur l'aimant dans le tampon
   (appliquer l'aimant contre l'eppendorf et laisser les billes se rapprocher de l'aimant pendant 1 minute - récolter à la P1000 la fraction négative - éloigner l'aimant de l'eppendorf - resuspendre les cellules+billes dans 1mL de tampon d'isolation- bien homogénéiser)
- (garder la première fraction négative dans un eppendorf pour marquage de contrôle
- Réserver 100 000 cellules dans 100µL minimum)
- Retirer le tube de cellules CD34_billes de l'aimant
- Resuspendre dans un peu de tampon (100µL de volume total max)
- Ajouter 100µL de DETACHaBEAD
- Incuber 45 min à RT (>20°C) ou 15min à 37°C, en agitant doucement 10rpm ou manuellement toutes les 5 min.
- Ajouter 400µL de tampon d'isolation RT (neutralise la réaction).
- Placer le tube sur l'aimant et laisser une minute.
- Prélever la fraction non fixée1 dans un nouvel eppendorf identifié.
- Enlever l'eppendorf du champ magnétique.
- Rajouter 500µL de tampon d'isolation à l'eppendorf contenant les billes, agiter doucement.
- Replacer le tube sur l'aimant et prélever la fraction non fixée 2 et l'ajouter à la fraction 1.
- Placer le tube contenant les fractions 1+2 sur l'aimant et récupérer la fraction non fixée dans un nouvel eppendorf : fraction positive.
- Réserver un petit nombre de cellules pour marquage de contrôle (50 000) et ajuster le volume à 100µL minimum.
- Compter les cellules de la fraction positive en bleu trypan ½ (10µL cellules + 10µL BT). Concentrer la fraction positive si besoin par centrifugation.
- Marquage de CD34 (IgG1) pour vérifier la pureté des fractions positives et évaluer le rendement du tri : fraction CBMNC/BMMC, fraction négative, fraction positive marquées avec anti-CD34 PE, contrôle isotypique IgG1-PE. Analyser par cytométrie en flux.

### Pré-stimulation et transduction des CD34+ :

- Préparer extemporanément du milieu IMDM 1% « bovine sérum albumin » (BSA), complété avec de l'insuline pancréatique bovine (10 µg/ml), de la transferrine humaine (200 µg/ml), de la L-glutamine (2 mM). Ajouter 50 ng/ml (rh) SCF recombinante humaine (rh), 50 ng/ml de rh Flt3-L, 10 ng/ml de rh IL-3, et 10 ng/ml de rh IL-6. Préchauffer le milieu.
- Ensemencement à 0,5-1.106 cellules /mL et laisser en culture sur plaque 48 puits pendant 24heures à 37°C.
- Laver les cellules avec du milieu complet et ajouter du vecteur à une multiplicité d'infection (MOI) = 5-10 dans un volume final de 500µL. laisser en culture sur la nuit à 37°C.
- Ajouter 500¡JL de milieu complet et remettre en culture.
- Contrôler par cytométrie de flux l'expression de la GFP dans les cellules transduites.

### 2. Reconstitution du système immunitaire de souris Rag2-/-γc-/- par allogreffe de cellules CD34 transduites.

Les procédures expérimentales sont mise ne place en accord avec la réglementation locale pour l'expérimentation animale. Les prélèvements de sang de cordon sont collectés après signature d'une lettre de consentement éclairé et en accord avec les recommandations du comité éthique local. Une méthode est brièvement décrite ci-après :
- Irradier des souris NOG nouveau-nés (de 1-3 jours) avec 1 Gy.
- Injecter par voie intra-hépatique 2,5 ± 0,5 x 10⁵ cellules CD34 + transduites.
- Vérifier environ 15 semaines après transplantation, la prise de greffe de cellules immunitaires humaines par cytométrie de flux (marquage CD45, CD3, CD4, CD8 et CD19).

### 3. Infection des souris par le virus VIH-1-Cre.

La dose et la voie d'infection sont susceptibles d'être modifiées en fonction des objectifs scientifiques retenus lors de l'expérimentation. Une méthode est brièvement décrite ci-après :
- Infecter les souris par voie intrapéritonéale avec 20ng p24/animal.
- Suivre la charge virale en cinétique par quantification des ARN viraux dans le plasma des animaux infectés.

### B- Résultats

Afin de tester la construction de l'invention, le vecteur lentiviral pHR-4lox-RFP/GFP sur base VIH-1 a été construit par clonage de fragment issu du pHL-HH (Luche et al. 2007. Eur J Immunol. 2007 Jan;37(1):43-53.) et pHRET-GFP (fourni par C. Mettling). Ce vecteur non réplicatif (LTR3' inactivé), a été conçu de la manière suivante de 5' en 3':
- Plasmide procaryote/SV40.
- Gène lactamase.
- Eco1 origine.
- SV40 origine de réplication.
- xanthine-guanine phospho-ribosyle transférase.
- intron SV40.
- SV40 poly A.
- LTR5' actif VIH-1 (NL4-3).
- PBS-gag séquence d'encapsidation y du VIH-1 (NL4-3).
- SA et RRE du géne env du VIH-1 (NL4-3).
- DNAflap.
- promoteur CMV.
- cassette loxP-loxP2272-turboRFP (séquence inversée)-loxP-loxP2272.
- promoteur PGK.
- gène eGFP.
- séquence WPRE.
- LTR3' inactivé VIH-1 (NL4-3).

Cette construction est représentée par les séquences SEQ ID NO : 6 et SEQ ID NO : 32.

Le fonctionnement de la construction pHR-4lox-RFP/GFP a été validée sur lignée cellulaire humaine 293T et des cellules primaires (cellules mononucléées du sang périphérique de donneurs sains) en combinaison avec le pBR-VIH-1-NL4-3-Nef-IRES-Cre dans différentes conditions: surexpression par transfection/infection et transduction/infection. Les **Figures 5 à 7** présente la validation de la construction sur cellules 293T dans ces différentes conditions.

Les **Figures 8 et 9** présentent la validation de la construction pHR-4lox-RFP/GFP dans des cellules primaires de donneurs sains par microscopie de fluorescence et par cytométrie de flux. De même, le but étant d'utiliser cette construction pour transduire de précurseurs hématopoïétiques CD34+ issus de sang de cordon humain afin d'humaniser des souris Rag2-/- γc-/-, le lentivecteur a été testé en cinétique (24 et 48 heures post-transduction) à une multiplicité d'infection (MOI) de 5 **(****Figures 10 et 11****)** sur des cellules préalablement activées pendant 24h. Le vecteur s'est révélé parfaitement adapté à la transduction de ces cellules qui seront à l'origine de l'humanisation et de la reconstitution d'un système immunitaire complet chez les souris greffées.

La charge virale des souris humanisées et infectées par NL4-3-Nef-IRES-Cre a été testée. Les résultats sont confinés dans le tableau 1 suivant :

**Tableau 1**

| **animal** | **Nombre de semaines après infection** | **HIV-1-ARN (copies/mL)** |
|---|---|---|
| **#1824** | 2 semaines | 47940 |
| | 7 semaines | 473480 |
| **#1833** | 2 semaines | Non testé |
| | 2 semaines | 3540 |

Le traitement des souris humanisées se fait par voie alimentaire. Des granulés alimentaires ont été développés en mélangeant 2,5 g de 3TC, 2,5 g de TDF, 2,5 g d'AZT, 5 g de RTV dans 5 kg de protéines riches en terre (vitaminfortified food, Nafag 3432, Provimi Kliba AG, Suisse). Les granulés seront ensuite stérilisés par gamma-irradiation (25 kGy). Tous les lots de granulés de produits alimentaires seront analysés par HPLC afin de contrôler les doses médicamenteuses. L'alimentation et l'eau seront données ad *libitum.* Les molécules TMC278 - LA et TMC181 -LA sont administrées par voie sous-cutanée à 160 et 400 mg/kg, respectivement.
- Le traitement est administré en continu à partir de 30 à 40 jours post-infection.

Une fois traitées, la charge virale des souris est mesurée afin de vérifier l'efficacité du traitement, et les cellules réservoirs sont isolées par cryométrie de flux, en sélectionnant les cellules exprimant le rapporteur (RFP).

### Exemple 2 : Méthode d'identification de cellules réservoirs simiennes

### A- Matériel et méthodes

### 1. Isolement et pré-stimulation des cellules CD34+ à partir de la moelle osseuse.

Pour chaque singe, endormi par chlorhydrate de kétamine à 15mg/kg, on prélève 3mL de moelle osseuse à la crête iliaque (ou 8ml à l'humérus) dans un tube (10% citrate de sodium)
- Préparer 20mL de ficoll dans un tube Falcon 50.
- Diluer la moelle osseuse/sang de cordon en PBS jusqu'à un volume de 30mL (dilution au moins 1/2-1/3).
- Déposer doucement les 30mL de moelle sur le gel de Ficoll.
- Centrifuger 30min minimum à 1800rpm, RT et sans frein.
- Récupérer l'anneau de cellules à la pipette 10ml et le déposer dans un nouveau Falcon 50.
- Compléter en PBS le volume jusqu'à 50mL.
- Centrifuger 10 min 1800 rpm.
- Vider le surnageant à la pipette et casser le culot.
- S'il y a trop d'érythrocytes, ajouter 5mLde solution de lyse, bien homogénéiser et laisser 5 min maximum. Ajouter au moins 10mL de PBS pour arrêter la réaction.
- Centrifuger 10 min 1800rpm.
- Vider le surnageant, casser le culot.
- Re-suspendre dans 1mL final de tampon d'isolation (cf 2).
- Compter les cellules mononucléées (CBMNC/BMMC) avec exclusion des mortes en bleu trypan.
- Réserver dans microtube 2 x 100 000 cellules BMMC pour le marquage. On complète qsp 100µL de PBS pour éviter le séchage.

### Isolement des CD34+ par méthode billes magnétiques Dynal (sélection positive)

- Auparavant : préparer le tampon d'isolation : PBS 2%BSA 0.6%Citrate ou EDTA (100IU/mL Penicilline-Streptomycine), filtré sur membrane 0,2µm.
- Dans un eppendorf de 1.8mL : ajouter 100µL DynaBeads/ mL BMMC (capacité 4.10⁷< CMMO<4.10⁸ CBMNC/BMMC).
- Nettoyer 3 fois avec 1mL de tampon d'isolation sur l'aimant Dynal
   (ajouter 1mL de tampon ; bien homogénéiser ; appliquer l'aimant contre l'eppendorf et laisser les billes se rapprocher de l'aimant pendant 1 minute ; récolter à la P1000 la fraction négative - éloigner l'aimant de l'eppendorf ; resuspendre les cellules+billes dans 1mL de tampon d'isolation).
- Ajouter tout de suite les BMMC sur le culot de billes.
- Vortexer doucement 2-3 secondes.
- Incuber 30 minutes à 4°C (agitation douce 10-20 rpm).
- Pendant ce temps, identifier les microtubes de marquage de contrôle.
- Laver 5 à 7 fois sur l'aimant dans le tampon
   (appliquer l'aimant contre l'eppendorf et laisser les billes se rapprocher de l'aimant pendant 1 minute - récolter à la P1000 la fraction négative - éloigner l'aimant de l'eppendorf - resuspendre les cellules+billes dans 1mL de tampon d'isolation- bien homogénéiser)
- (garder la première fraction négative dans un eppendorf pour marquage de contrôle
- Réserver 100 000 cellules dans 100µL minimum)
- Retirer le tube de cellules CD34_billes de l'aimant
- Resuspendre dans un peu de tampon (100µL de volume total max)
- Ajouter 100µL de DETACHaBEAD
- Incuber 45 min à RT (>20°C) ou 15min à 37°C, en agitant doucement 10rpm ou manuellement toutes les 5 min.
- Ajouter 400µL de tampon d'isolation RT (neutralise la réaction).
- Placer le tube sur l'aimant et laisser une minute.
- Prélever la fraction non fixée1 dans un nouvel eppendorf identifié.
- Enlever l'eppendorf du champ magnétique.
- Rajouter 500¡JL de tampon d'isolation à l'eppendorf contenant les billes, agiter doucement.
- Replacer le tube sur l'aimant et prélever la fraction non fixée 2 et l'ajouter à la fraction 1.
- Placer le tube contenant les fractions 1+2 sur l'aimant et récupérer la fraction non fixée dans un nouvel eppendorf : fraction positive.
- Réserver un petit nombre de cellules pour marquage de contrôle (50 000) et ajuster le volume à 100µL minimum.
- Compter les cellules de la fraction positive en bleu trypan ½ (10µL cellules + 10µL BT). Concentrer la fraction positive si besoin par centrifugation.
- Marquage de CD34 (IgG1) pour vérifier la pureté des fractions positives et évaluer le rendement du tri : fraction CBMNC/BMMC, fraction négative, fraction positive marquées avec anti-CD34 PE, contrôle isotypique IgG1-PE. Analyser par cytométrie en flux.

### Pré-stimulation et transduction des CD34+ :

- Préparer extemporanément du milieu IMDM 1% « bovine sérum albumin » (BSA), complété avec de l'insuline pancréatique bovine (10 µg/ml), de la transferrine humaine (200 µg/ml), de la L-glutamine (2 mM). Ajouter 50 ng/ml (rh) SCF recombinante humaine (rh), 50 ng/ml de rh Flt3-L, 10 ng/ml de rh IL-3, et 10 ng/ml de rh IL-6. Préchauffer le milieu.
- Ensemencement à 0,5-1.10⁶ cellules /mL et laisser en culture sur plaque 48 puits pendant 24 heures à 37°C.
- Laver les cellules avec du milieu complet et ajouter du vecteur à une MOI=5-10 dans un volume final de 500µL. laisser en culture sur la nuit à 37°C.
- Ajouter 500¡JL de milieu complet et remettre en culture.
- Contrôler par cytométrie de flux l'expression de la GFP dans les cellules transduites.

### 2. Reconstitution du système immunitaire de macaque par autogreffe de cellules CD34 transduites.

- Les procédures expérimentales (susceptibles d'être modifiées), sont mise ne place en accord avec la règlementation Européenne sur l'expérimentation sur primate (Journal Officiel des Communautés Européennes, L358, 18 Décembre 1986).
- Placer l'animal sous sédation avec de la kétamine (Imalgène; 10 mg / kg, im), et placer le sur une chaise de contention.

- Appliquer le traitement myélo-ablatif sous la forme d'une exposition corporelle totale aux rayons gamma (60Co) avec une direction unilatérale antérieure. Délivrer une dose totale de 6 Gy un taux de 25,92 cGy/minute.
- Après le suivi clinique de l'animal, injecter la totalité des CD34 autologues transduits in vitro par voie intramédullaire au niveau des humérus.

### 3. Infection des macaques par le virus VISmac239-Cre.

La dose et la voie d'infection sont susceptibles d'être modifiées en fonction des objectifs scientifiques retenus lors de l'expérimentation. Une méthode est brièvement décrite ci-après :
- Infecter les macaques par voie intraveineuse (50 AID₅₀) ou par voie muqueuse (50-5000 AID₅₀).
- Suivre la charge virale en cinétique par quantification des ARN viraux dans le plasma des animaux infectés.

### B- Résultats

Le vecteur lentiviral pSDT-4lox-RFP/GFP sur base VISmac251 (Figure 5) a été obtenu par clonage de fragment issu du pHR-4lox-RFP/GFP et du pGAE-SFFV-GFP-WPRE (fourni par E. Verhoyen). Ce vecteur à haut titre (CMV-LTR5' VISmac251) et non réplicatif (LTR3' inactivé VISmac251), a été conçu de la manière suivante de 5' en 3':
- Plasmide procaryote.
- F1 origine.
- gène lactamase.
- promoteur CMV.
- LTR5' (VISmac251).
- PBS-gag séquence d'encapsidation du VISmac251.
- cPPT/CTS.
- séquence RRE du VISmac251.
- promoteur SFFV.
- cassette loxP-loxp2272-turboRFP-loxP-loxP2272.
- promoteur PGK.
- gène eGFP.
- WPRE.
- PPT et tract TTTTAT.
- LTR3' inactivé (VSImac251).

Cette construction est représentée par la séquence SEQ ID NO : 7.

De la même manière que pour le vecteur sur base VIH-1, le fonctionnement du vecteur SDT-4lox-RFP/GFP a été validé sur lignée 293T et transduction/infection avec le VISmac239-Nef-IRES-Cre **(****Figures 12, 13 et 14****).** La capacité de transduction des cellules CD34+ purifiées à partir de moelle osseuse de macaque, a été mise en évidence après 24h de pré-stimulation **(****Figures 15 et 16****).** Les CD34+ transduits seront utilisées pour des autogreffes de macaque préalablement irradiés, afin de reconstituer leur système immunitaire à partir de ces précurseurs porteur du transgène.

Ce vecteur SDT-4lox-RFP/GFP est sur le point d'être testé in vivo. Le virus VISmac239-Nef-IRES-Cre est en cours de validation. Un macaque a été infecté par ce virus qui montre une dynamique réplication normale **(****Figure 17****).**

Les animaux infectés seront traités avec AZT (4,5mg/kg) et 3TC (2,5mg/kg) deux fois par jour par injection sous cutanée ainsi qu'avec de l'indinavir (60mg/kg) deux fois par jour par voie orale.

Une fois traitées, la charge virale des macaques est mesurée afin de vérifier l'efficacité du traitement, et les cellules réservoirs sont isolées par cryométrie de flux, en sélectionnant les cellules exprimant le rapporteur (RFP).

### Exemple 3 : Méthode d'identification de cellules réservoirs félines

Les caractéristiques de l'infection par le virus de l'immunodéficience féline (VIF) sont similaires à celles de l'infection par le VIH-1 (pour revue McDonnel et al, Retrovirology 2013, 10:69.). Après une phase aigüe, l'infection est caractérisée par longue phase chronique menant vers un stade SIDA après déplétion du compartiment des lymphocytes T CD4+. De la même manière que pour le VIH-1, de cellules réservoirs ont été mise en évidence au cours de la phase chronique même en l'absence de traitement (McDonnel et al, Viruses 2012, 4:878-888). La méthode de l'exemple 1 est donc transposable au modèle VIF.

En outre il est possible d'obtenir des cellules hématopoïétiques félines transformées avec la molécule d'acides nucléiques. Ces expériences de transgénèse sont effectuées suivant le protocole décrit par Wongsrikeo et al Nat Methods. 2011 Sep 11;8(10):853-9), et brièvement résumé ci-après.

### Isolement de gamètes et génération d'embryon :

- Récupérer des gonades issues de stérilisation de chat.
- Récupérer les ovocytes-cumuls après découpages répétés des tissus ovariens dans du PBS complété avec de BSA à 4mg/mL et de la L gentamicine à 50µg/mL.
- Conserver les ovocytes de stade I et II.
- Maturer les ovocytes en les cultivant 28 heures à 38°C, dans du milieu modifié TCM-199 contenant 1 IU/mL de gonadotrophine chorionique humaine, 0.5 IU/mL de gonadotrophine chorionique équine, 10µg/mL de facteur de croissance épidermique et 4mg/mL des BSA.
- Eliminer les cellules du cumulus 18 à 20 heures après début de la maturation.
- Injecter un volume de 100pL de vecteur directement dans l'espace périvitellin de l'ovocyte, 12 heures avant la fertilisation.
- Laver et remettre les ovocytes en culture.
- Après 28 heures de culture, laver des spermatozoïdes dans du milieu Brakett-Oliphant complété avec 137µg/mL de sodium pyruvate, 4mg/mL de BSA et 50µg/mL de L-gentamicine en centrifugeant à 1800rpm pendant 5 minutes.
- Eliminer le surnageant et reprendre le culto dans 500¡JL de milieu de fertilisation (G-IVF Plus) et les placer 30 minutes en incubateur.
- Ajuster la concentration des spermatozoïdes à 2.10⁶/mL.
- Transférer 10 ovocytes pré-stimulés dans chaque 100µL de culot de spermatozoïde et cultiver pendant 12 heures.
- Récupérer les zygotes et les cultiver après lavage dans du milieu modifié Earl's balanced salt sodium (MK-1) complété avec 4mg/mL de BSA et 50µg/mL de gentamicine pour 3 jours.
- Récupérer les embryons et les cultiver dans du milieu MK-1 complété avec 5% de FBS et 50µg/mL de gentamicine pour 4 jours.

### Transfert des embryons porteur du transgène.

- Des femelles de 2 à 3 ans sont les receveuses.
- Stimuler les femelles par injection intraveineuse 96 à 120 heures avant la fertilisation in vitro, de 150 IU de gonadotrophine chorionique équine et de 100 IU de de gonadotrophine chorionique humaine 72 heures plus tard.
- Anesthésier les femelles avec 5mg/kg de kétamine, 0.03mg/kg de medetomidine et 0.01mg/kg de buprenorphine par voie intramusculaire et sous atmosphére de 1 à 3% d'isoflurane.
- Après incision ventrale et dégagement des trompes de Fallope, transférer 15 à 25 embryons dans 10 à 20µl de milieu par trompe.
- Suivre le déroulement de la grossesse suivant les critères habituels.
- Contrôler l'expression de la GFP chez les nouveaux nés.

### Infection des chats par le virus FIV.

La dose et la voie d'infection sont susceptibles d'être modifiées en fonction des objectifs scientifiques retenus lors de l'expérimentation. Une méthode est brièvement décrite ci-après :
- Infecter les chats par voie intraveineuse (50 AID50) ou par voie muqueuse (50-5000 AID50).
- Suivre la charge virale en cinétique par quantification des ARN viraux dans le plasma des animaux infectés.

Les animaux sont ensuite traités avec un ou plusieurs antirétroviraux. Après le traitement les cellules réservoirs sont détectées en détectant le rapporteur.

### Exemple 4 : caractérisation in vitro des cellules infectées ne produisant pas de virus : c'est-à-dire des cellules réservoirs

Les inventeurs ont ensuite isolé les cellules réservoirs :
10⁶ cellules MT4C5 (lignée lymphocytaire T) ont été transduites par le vecteur double couleur HR4lox à une multiplicité d'infection de 5. Les cellules ont été laissées en culture sur la nuit avant d'être lavées puis remises en culture à une concentration de 0.5.10⁶ cellules/mL.

Quatre jours post-transduction, la fréquence de transduction a été déterminée par cytométrie de flux en contrôlant le niveau d'expression de la GFP. Dans ces conditions, la fréquence de cellules porteuses du vecteur HR4lox était de 80%. Les cellules ont été conservées en culture à une concentration de 0.5.10⁶ cellules/mL.

10⁶ cellules HR4lox-MT4C5 ont été infectées par le HIV-1-NL4-3 (R5) à une concentration de 500ng p24/106 cellules en tant que contrôle, par le HIV-1-NL4-3-Nefopt-CMV-Cre à une concentration de 500ng p24/10⁶ cellules, ou non infectées (contrôle négatif). Les cellules ont été laissées en culture sur la nuit avant d'être lavées puis remises en culture à une concentration de 0.5.10⁶ cellules/mL.

De 4 à 10 jours post-infection, la fréquence de cellules exprimant la p24 intracellulaire (cellules infectées de façon productive) et/ou la RFP a été déterminée dans les cellules GFP+ par cytométrie de flux. Pour cela, les cellules ont été fixées, perméabilisées et marquées à l'aide d'un anticorps anti-protéine p24 Gag.

Les résultats sont montrés figures 20 et 21.

Ces résultats montrent clairement que le transgène rapporteur est recombiné et capable d'exprimer la RFP (Figure 21). En outre on constate qu'il existe deux populations de cellules où a eu lieu la recombinaison du transgène :
- les cellules où le virus se réplique (cadre Q2 de la figure 21), qui représentent environ 29% de la population, et
les cellules infectées et recombinées, mais où le virus ne se réplique pas (cadre Q1 de la figure 21), qui représentent environ 12% de la population, et qui correspondent aux cellules réservoir. L'invention n'est pas limitée aux modes de réalisation présentés et d'autres modes de réalisation apparaîtront clairement à l'homme du métier.

## Revendications

1. Méthode d'identification *in vitro* de cellules réservoirs d'un virus induisant l'immunodéficience chez un mammifère, comprenant les étapes suivantes :
- une étape de transformation de cellules souches hématopoïétiques avec une molécule d'acides nucléiques,
ladite molécule d'acides nucléiques comprenant une séquence codant un gène rapporteur, sous contrôle d'au moins un élément nécessaire à la transcription, la séquence codant un gène rapporteur étant bordée par
- au moins une première paire de séquences ciblant une recombinase spécifique de site, ladite première paire comprenant une séquence P1-1 et une séquence P1-2,
- au moins une seconde paire de séquences ciblant une recombinase spécifique de site, ladite seconde paire comprenant une séquence P2-1 et une séquence P2-2,
les séquences de chacune desdites première et seconde paires de séquences étant dans une orientation opposée l'une par rapport à l'autre,
les séquences de la première paire de séquences ciblant une recombinase spécifique de site étant incapables de recombiner avec les séquences de la seconde paire de séquences ciblant une recombinase spécifique de site et les séquences de la seconde paire de séquences ciblant une recombinase spécifique de site étant incapables de recombiner avec les séquences de la première paire de séquences ciblant une recombinase spécifique de site,
une séquence de la première paire et une séquence de la seconde paire de séquences ciblant une recombinase spécifique de site se trouvant en amont de ladite première séquence d'acide nucléiques et une séquence de la première paire et une séquence de la seconde paire de séquences ciblant une recombinase spécifique de site se trouvant en aval de ladite première séquence d'acide nucléiques,
de sorte que les séquences d'une même paire n'encadrent jamais les deux séquences de l'autre paire,
la séquence codant un gène rapporteur, en l'absence de recombinaison induite par ladite recombinase spécifique de site, étant telle qu'elle présente un cadre ouvert de lecture codant ledit gène rapporteur dans une orientation 3'-5', et donc incapable de permettre la transcription et la traduction du gène rapporteur
- une étape de reconstitution du système hématopoïétique d'un mammifère non humain ayant subi une myéloablation, avec les cellules souches hématopoïétiques susmentionnées,
- l'infection du mammifère non humain ayant une moelle reconstituée à l'étape précédente avec un virus induisant une immunodéficience chez le mammifère dont sont issues les cellules souches hématopoïétiques, ledit virus exprimant une recombinase spécifique de sites,
- une étape de traitement du mammifère non humain infecté susmentionné avec un traitement inhibant le développement dudit virus, et
- une étape de détection des cellules hématopoïétiques exprimant le rapporteur recombiné, lesdites cellules hématopoïétiques exprimant le rapporteur étant des cellules réservoirs d'un virus induisant l'immunodéficience chez un mammifère.

2. Méthode selon la revendication 1, dans laquelle la recombinase spécifique de site est la recombinase Cre du bactériophage P1.

3. Méthode selon la revendication 1 ou la revendication 2, dans laquelle les séquences de la première paire de séquences ciblant une recombinase spécifique de site et les séquences de la seconde paire de séquences ciblant une recombinase spécifique de site sont choisie parmi Lox P1, Lox P2272, Lox 66, Lox 71, Lox 511, Lox 512, Lox 514 et des séquences mutées du site Lox P1, portant au moins une mutation ponctuelle dans la séquence espaceur.

4. Méthode selon l'une quelconque des revendications 1 à 3, dans laquelle ladite molécule d'acide nucléique comprend ou consiste essentiellement en l'une des séquences suivantes : SEQ ID NO : 6, SEQ ID NO : 7, SEQ ID NO : 32, SEQ ID NO : 33, SEQ ID NO : 34, SEQ ID NO : 35, SEQ ID NO : 36, SEQ ID NO : 37, SEQ ID NO : 38, SEQ ID NO : 39 et SEQ ID NO : 40.

5. Cellule hématopoïétique susceptible d'être obtenue par la méthode selon l'une quelconque des revendications 1 à 4.

6. Mammifère non humain dont le système hématopoïétique est essentiellement constitué ou constitué de cellules hématopoïétiques telles que définies dans la revendication 5.

## Patentansprüche

1. Verfahren zur *in*-*vitro*-Identifizierung von Zellen, die Reservoire eines Virus darstellen, das bei einem Säugetier Immunschwäche induziert, umfassend die folgenden Schritte:
- einen Schritt des Transformierens hämatopoetischer Stammzellen mit einem Molekül aus Nukleinsäuren,
wobei das Molekül aus Nukleinsäuren eine Sequenz umfasst, die für ein Reportergen codiert, unter der Steuerung mindestens eines Elements, das für die Transkription erforderlich ist, wobei die Sequenz, die für ein Reportergen codiert, flankiert ist von
- mindestens einem ersten Paar von Sequenzen, die auf eine ortsspezifische Recombinase abzielen, wobei das erste Paar eine Sequenz P1-1 und eine Sequenz P1-2 umfasst,
- mindestens einem zweiten Paar von Sequenzen, die auf eine ortsspezifische Recombinase abzielen, wobei das zweite Paar eine Sequenz P2-1 und eine Sequenz P2-2 umfasst,
wobei die Sequenzen des ersten und des zweiten Paars von Sequenzen jeweils in Ausrichtungen vorliegen, die einander entgegengesetzt sind, wobei die Sequenzen des ersten Paars von Sequenzen, die auf eine ortsspezifische Recombinase abzielen, nicht dazu befähigt sind, eine Rekombination mit den Sequenzen des zweiten Paares von Sequenzen, die auf eine ortsspezifische Recombinase abzielen, einzugehen, und die Sequenzen des zweiten Paars von Sequenzen, die auf eine ortsspezifische Recombinase abzielen, nicht dazu befähigt sind, eine Rekombination mit den Sequenzen des ersten Paares von Sequenzen, die auf eine ortsspezifische Recombinase abzielen, einzugehen,
wobei eine Sequenz des ersten Paares und eine Sequenz des zweiten Paares von Sequenzen, die auf eine ortsspezifische Recombinase abzielen, sich strangaufwärts der ersten Sequenz aus Nukleinsäuren befinden und wobei eine Sequenz des ersten Paares und eine Sequenz des zweiten Paares von Sequenzen, die auf eine ortsspezifische Recombinase abzielen, sich strangabwärts der ersten Sequenz aus Nukleinsäuren befinden,
derart, dass die Sequenzen ein- und desselben Paares niemals die beiden Sequenzen des anderen Paares rahmenartig umschließen,
wobei die Sequenz, die für ein Reportergen codiert, bei Abwesenheit jedweder Rekombination, die durch die ortspezifische Recombinase induziert wird, derart beschaffen ist, dass sie ein offenes Leseraster aufweist, das für ein Reportergen in 3'-5'-Ausrichtung codiert, und somit nicht dazu befähigt ist, eine Transkription und eine Translation des Reportergens zu ermöglichen
- einen Schritt des Rekonstituierens des hämatopoetischen Systems eines nicht-menschlichen Säugetiers, das eine Myelosuppression erlitten hat, mit den oben genannten hämatopoetischen Stammzellen,
- wobei das Infizieren des nicht-menschlichen Säugetiers, dessen Knochenmark im vorhergehenden Schritt rekonstituiert wurde, mit einem Virus, das bei dem Säugetier, dem die hämatopoetischen Stammzellen entnommen wurden, eine Immunschwäche induziert, wobei das Virus eine ortsspezifische Recombinase exprimiert,
- einen Schritt des Behandelns des oben genannten infizierten nicht-menschlichen Säugetiers mit einem Behandlungsansatz, der die Entwicklung des Virus hemmt, und
- einen Schritt des Nachweisens der hämatopoetischen Stammzellen, welche das rekombinierte Reporterelement exprimieren, wobei es sich bei den hämatopoetischen Zellen, die das Reporterelement exprimieren, um Zellen handelt, die Reservoire eines Virus darstellen, das bei einem Säugetier Immunschwäche induziert.

2. Verfahren nach Anspruch 1, wobei es sich bei der ortsspezifischen Recombinase um die Recombinase Cre des Bakteriophagen P1 handelt.

3. Verfahren nach Anspruch 1 oder Anspruch 2, wobei die Sequenzen des ersten Paares von Sequenzen, die auf eine ortsspezifische Recombinase abzielen und die Sequenzen des zweiten Paares von Sequenzen, die auf eine ortsspezifische Recombinase abzielen, aus Lox P1, Lox P2272, Lox 66, Lox 71, Lox 511, Lox 512, Lox 514 und aus den die Sequenzen der Stelle Lox P1 ausgewählt sind, welche in der Abstandhaltersequenz mindestens eine Punktmutation aufweisen.

4. Verfahren nach einem der Ansprüche 1 bis 3, wobei das Molekül aus Nukleinsäuren eine der folgenden Sequenzen umfasst oder im Wesentlichen daraus besteht: SEQ ID Nr.: 6, SEQ ID Nr.: 7, SEQ ID Nr.: 32, SEQ ID Nr.: 33, SEQ ID Nr.: 34, SEQ ID Nr.: 35, SEQ ID Nr.: 36, SEQ ID Nr.: 37, SEQ ID Nr.: 38, SEQ ID Nr.: 39 und SEQ ID Nr.: 40.

5. Hämatopoetische Zelle, die mittels des Verfahrens nach einem der Ansprüche 1 bis 4 erhalten werden kann.

6. Nicht-menschliches Säugetier, dessen hämatopoetisches System aus den hämatopoetischen Zellen, die der Begriffsbestimmung in Anspruch 5 entsprechen, besteht oder im Wesentlichen besteht.

## Claims

1. Method for the *in vitro* identification of reservoir cells of a virus responsible for an immunodeficiency in a mammal, comprising the following steps:
- a step of transformation of hematopoietic stem cells with a nucleic acid molecule,
said nucleic acid molecule comprising a sequence coding for a reporter gene, under the control of at least one element necessary for transcription, the sequence being bordered by
- at least one first pair of sequences targeting a site-specific recombinase, said first pair comprising a P1-1 sequence and a P1-2 sequence,
- at least one second pair of sequences targeting a site-specific recombinase, said second pair comprising a P2-1 sequence and a P2-2 sequence,
the sequences of each of said first and second pairs of sequences being oppositely oriented relative to one another,
the sequences of the first pair of sequences targeting a site-specific recombinase being unable to recombine with the sequences of the second pair of sequences targeting a site-specific recombinase, and the sequences of the second pair of sequences targeting a site-specific recombinase being unable to recombine with the sequences of the first pair of sequences targeting a site-specific recombinase,
one sequence from the first pair and one sequence from the second pair of sequences targeting a site-specific recombinase being located upstream of said first nucleic acid sequence, and one sequence from the first pair and one sequence from the second pair of sequences targeting a site-specific recombinase being located downstream of said first nucleic acid sequence,
such that the sequences of the same pair never flank the two sequences of the other pair,
the sequence coding said reporter gene, in the absence of combination induced by said site-specific recombinase, being such that it has an open reading frame coding for said first reporter gene in a 3'-5' orientation, and thus is not able to carry out transcription and translation of the reporter gene,
- a step of reconstructing the hematopoietic system of a non human mammal which has undergone myeloablation, with the abovementioned hematopoietic stem cells,
- infection of the non human mammal having reconstructed marrow in the following step, with a virus inducing an immunodeficiency in the mammal from which the hematopoietic stem cells originate, said virus expressing a site-specific recombinase,
- a step of treating the abovementioned infected non human mammal with a treatment inhibiting the development of said virus, and
- a step of detecting hematopoietic cells expressing the recombined reporter, said hematopoietic cells expressing the reporter being reservoir cells of a virus responsible for an immunodeficiency in a mammal.

2. Method according to claim 1, in which the site-specific recombinase is the Cre recombinase from the P1 bacteriophage.

3. Method according to claim 1 or claim 2, in which the sequences of the first pair of sequences targeting a site-specific recombinase and the sequences of the second pair of sequences targeting a site-specific recombinase are chosen from Lox P1, Lox P2272, Lox 66, Lox 71, Lox 511, Lox 512, Lox 514 and mutated sequences of the Lox P1 site, bearing at least one point mutation in the spacer sequence.

4. Method according to any one of claims 1 to 3, in which said nucleic acid molecule essentially comprises, or essentially consists of, one of the following sequences: SEQ ID NO: 6, SEQ ID NO: 7, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, and SEQ ID NO: 40.

5. Hematopoietic cell liable to be obtained by the method according to any one of claims 1 to 4.

6. Nonhuman mammal, the hematopoietic system of which essentially consists of, or consists of, hematopoietic cells as defined in claim 5.


### SEQUENCE LISTING

<110> CENTRE NATIONAL DE LA RECHERCHE SCIENTIFIQUE
<120> Méthode d'identification de cellules
<130> BR75917
<140> FR 14/63138
   <141> 2014-12-22
<160> 47
<170> PatentIn version 3.5
<210> 1
   <211> 34
   <212> DNA
   <213> Artificial Sequence
<220>
   <223> LoxP1 forward sequence
<400> 1
   ataacttcgt atagcataca ttatacgaag ttat 34
<210> 2
   <211> 34
   <212> DNA
   <213> Artificial Sequence
<220>
   <223> Lox P2272 forward sequence
<400> 2
   ataacttcgt ataaagtatc ctatacgaag ttat 34
<210> 3
   <211> 34
   <212> DNA
   <213> Artificial Sequence
<220>
   <223> Lox P reverse sequence
<400> 3
   ataacttcgt ataatgtatg ctatacgaag ttat 34
<210> 4
   <211> 34
   <212> DNA
   <213> Artificial Sequence
<220>
   <223> Lox P2272 reverse sequence
<400> 4
   ataacttcgt ataggatact ttatacgaag ttat 34
<210> 5
   <211> 696
   <212> DNA
   <213> Artificial Sequence
<220>
   <223> reverse RFP
<400> 5
   ttatctgtgc cccagtttgc tagggaggtc gcagtacttg gccacagcca tctcgtgctg        60
   ctcgacgtag gtctctttgt cggcctcctt gattctttcc agtctgtggt ccacgaagtg       120
   gaagccgggc atcttgaggt tcttagcggg tttcttggat ctgtatgtgg tcttgaagga       180
   gcagtgcagg tagcccccgc ccacgagctt cagggccatc tggctgtggc ctctcaggcc       240
   gccgtcagcg gggtacagca tctcggtgtt ggcctcccag ccgcgtgttt tcttctgcat       300
   cacagggccg ttggatggga agttcacccc gttgatcttg acgttgtaga tgatgcagcc       360
   gttctggaag ctggtgtcct gggtagcggt cagcacgccc ccgtcttcgt atgtggtgat       420
   tctctcccat gtgaagccct cagggaagga ctgcttaaag aagtcgggga tgccctgggt       480
   gtggttgatg aaggctttgc tgccgtacat gaagctggta gccaggatgt cgaaggcgaa       540
   ggggagaggg ccgccctcga ccaccttgat cttcatggtc tgggtgccct cgtagggctt       600
   gccttcgccc tcggatgtgc acttgaagtg gtggttgttc acggtgccct ccatgtacag       660
   cttcatgtgc atgttctcct tgatcagctc gctcat                                 696
<210> 6
   <211> 11336
   <212> DNA
   <213> Artificial Sequence
<220>
   <223> sequence for screening human cells
<400> 6
   ttaattccgt gtattctata gtgtcaccta aatcgtatgt gtatgataca taaggttatg        60
   tattaattgt agccgcgttc taacgacaat atgtacaagc ctaattgtgt agcatctggc       120
   ttactgaagc agaccctatc atctctctcg taaactgccg tcagagtcgg tttggttgga       180
   cgaaccttct gagtttctgg taacgccgtc ccgcacccgg aaatggtcag cgaaccaatc       240
   agcagggtca tcgctagcca gatcctctac gccggacgca tcgtggccgg catcaccggc       300
   gccacaggtg cggttgctgg cgcctatatc gccgacatca ccgatgggga agatcgggct       360
   cgccacttcg ggctcatgag cgcttgtttc ggcgtgggta tggtggcagg ccccgtggcc       420
   gggggactgt tgggcgccat ctccttgcat gcaccattcc ttgcggcggc ggtgctcaac       480
   ggcctcaacc tactactggg ctgcttccta atgcaggagt cgcataaggg agagcgtcga       540
   atggtgcact ctcagtacaa tctgctctga tgccgcatag ttaagccagc cccgacaccc       600
   gccaacaccc gctgacgcgc cctgacgggc ttgtctgctc ccggcatccg cttacagaca 660
   agctgtgacc gtctccggga gctgcatgtg tcagaggttt tcaccgtcat caccgaaacg 720
   cgcgagacga aagggcctcg tgatacgcct atttttatag gttaatgtca tgataataat 780
   ggtttcttag acgtcaggtg gcacttttcg gggaaatgtg cgcggaaccc ctatttgttt 840
   atttttctaa atacattcaa atatgtatcc gctcatgaga caataaccct gataaatgct 900
   tcaataatat tgaaaaagga agagtatgag tattcaacat ttccgtgtcg cccttattcc 960
   cttttttgcg gcattttgcc ttcctgtttt tgctcaccca gaaacgctgg tgaaagtaaa 1020
   agatgctgaa gatcagttgg gtgcacgagt gggttacatc gaactggatc tcaacagcgg 1080
   taagatcctt gagagttttc gccccgaaga acgttttcca atgatgagca cttttaaagt 1140
   tctgctatgt ggcgcggtat tatcccgtat tgacgccggg caagagcaac tcggtcgccg 1200
   catacactat tctcagaatg acttggttga gtactcacca gtcacagaaa agcatcttac 1260
   ggatggcatg acagtaagag aattatgcag tgctgccata accatgagtg ataacactgc 1320
   ggccaactta cttctgacaa cgatcggagg accgaaggag ctaaccgctt ttttgcacaa 1380
   catgggggat catgtaactc gccttgatcg ttgggaaccg gagctgaatg aagccatacc 1440
   aaacgacgag cgtgacacca cgatgcctgt agcaatggca acaacgttgc gcaaactatt 1500
   aactggcgaa ctacttactc tagcttcccg gcaacaatta atagactgga tggaggcgga 1560
   taaagttgca ggaccacttc tgcgctcggc ccttccggct ggctggttta ttgctgataa 1620
   atctggagcc ggtgagcgtg ggtctcgcgg tatcattgca gcactggggc cagatggtaa 1680
   gccctcccgt atcgtagtta tctacacgac ggggagtcag gcaactatgg atgaacgaaa 1740
   tagacagatc gctgagatag gtgcctcact gattaagcat tggtaactgt cagaccaagt 1800
   ttactcatat atactttaga ttgatttaaa acttcatttt taatttaaaa ggatctaggt 1860
   gaagatcctt tttgataatc tcatgaccaa aatcccttaa cgtgagtttt cgttccactg 1920
   agcgtcagac cccgtagaaa agatcaaagg atcttcttga gatccttttt ttctgcgcgt 1980
   aatctgctgc ttgcaaacaa aaaaaccacc gctaccagcg gtggtttgtt tgccggatca 2040
   agagctacca actctttttc cgaaggtaac tggcttcagc agagcgcaga taccaaatac 2100
   tgtccttcta gtgtagccgt agttaggcca ccacttcaag aactctgtag caccgcctac 2160
   atacctcgct ctgctaatcc tgttaccagt ggctgctgcc agtggcgata agtcgtgtct 2220
   taccgggttg gactcaagac gatagttacc ggataaggcg cagcggtcgg gctgaacggg 2280
   gggttcgtgc acacagccca gcttggagcg aacgacctac accgaactga gatacctaca 2340
   gcgtgagcat tgagaaagcg ccacgcttcc cgaagggaga aaggcggaca ggtatccggt 2400
   aagcggcagg gtcggaacag gagagcgcac gagggagctt ccagggggaa acgcctggta 2460
   tctttatagt cctgtcgggt ttcgccacct ctgacttgag cgtcgatttt tgtgatgctc 2520
   gtcagggggg cggagcctat ggaaaaacgc cagcaacgcg gcctttttac ggttcctggc 2580
   cttttgctgg ccttttgctc acatgttctt tcctgcgtta tcccctgatt ctgtggataa 2640
   ccgtattacc gcctttgagt gagctgatac cgctcgccgc agccgaacga ccgagcgcag 2700
   cgagtcagtg agcgaggaag cggaagagcg cccaatacgc aaaccgcctc tccccgcgcg 2760
   ttggccgatt cattaatgca gctgtggaat gtgtgtcagt tagggtgtgg aaagtcccca 2820
   ggctccccag caggcagaag tatgcaaagc atgcatctca attagtcagc aaccaggtgt 2880
   ggaaagtccc caggctcccc agcaggcaga agtatgcaaa gcatgcatct caattagtca 2940
   gcaaccatag tcccgcccct aactccgccc atcccgcccc taactccgcc cagttccgcc 3000
   cattctccgc cccatggctg actaattttt tttatttatg cagaggccga ggccgcctcg 3060
   gcctctgagc tattccagaa gtagtgagga ggcttttttg gaggcctagg cttttgcaaa 3120
   aagcttggac acaagacagg cttgcgagat atgtttgaga ataccacttt atcccgcgtc 3180
   agggagaggc agtgcgtaaa aagacgcgga ctcatgtgaa atactggttt ttagtgcgcc 3240
   agatctctat aatctcgcgc aacctatttt cccctcgaac actttttaag ccgtagataa 3300
   acaggctggg acacttcaca tgagcgaaaa atacatcgtc acctgggaca tgttgcagat 3360
   ccatgcacgt aaactcgcaa gccgactgat gccttctgaa caatggaaag gcattattgc 3420
   cgtaagccgt ggcggtctgt accgggtgcg ttactggcgc gtgaactggg tattcgtcat 3480
   gtcgataccg tttgtatttc cagctacgat cacgacaacc agcgcgagct taaagtgctg 3540
   aaacgcgcag aaggcgatgg cgaaggcttc atcgttattg atgacctggt ggataccggt 3600
   ggtactgcgg ttgcgattcg tgaaatgtat ccaaaagcgc actttgtcac catcttcgca 3660
   aaaccggctg gtcgtccgct ggttgatgac tatgttgttg atatcccgca agatacctgg 3720
   attgaacagc cgtgggatat gggcgtcgta ttcgtcccgc caatctccgg tcgctaatct 3780
   tttcaacgcc tggcactgcc gggcgttgtt ctttttaact tcaggcgggt tacaatagtt 3840
   tccagtaagt attctggagg ctgcatccat gacacaggca aacctgagcg aaaccctgtt 3900
   caaaccccgc tttaaacatc ctgaaacctc gacgctagtc cgccgcttta atcacggcgc 3960
   acaaccgcct gtgcagtcgg cccttgatgg taaaaccatc cctcactggt atcgcatgat 4020
   taaccgtctg atgtggatct ggcgcggcat tgacccacgc gaaatcctcg acgtccaggc 4080
   acgtattgtg atgagcgatg ccgaacgtac cgacgatgat ttatacgata cggtgattgg 4140
   ctaccgtggc ggcaactgga tttatgagtg ggccccggat ctttgtgaag gaaccttact 4200
   tctgtggtgt gacataattg gacaaactac ctacagagat ttaaagctct aaggtaaata 4260
   taaaattttt aagtgtataa tgtgttaaac tactgattct aattgtttgt gtattttaga 4320
   ttccaaccta tggaactgat gaatgggagc agtggtggaa tgcctttaat gaggaaaacc 4380
   tgttttgctc agaagaaatg ccatctagtg atgatgaggc tactgctgac tctcaacatt 4440
   ctactcctcc aaaaaagaag agaaaggtag aagaccccaa ggactttcct tcagaattgc 4500
   taagtttttt gagtcatgct gtgtttagta atagaactct tgcttgcttt gctatttaca 4560
   ccacaaagga aaaagctgca ctgctataca agaaaattat ggaaaaatat tctgtaacct 4620
   ttataagtag gcataacagt tataatcata acatactgtt ttttcttact ccacacaggc 4680
   atagagtgtc tgctattaat aactatgctc aaaaattgtg tacctttagc tttttaattt 4740
   gtaaaggggt taataaggaa tatttgatgt atagtgcctt gactagagat cataatcagc 4800
   cataccacat ttgtagaggt tttacttgct ttaaaaaacc tcccacacct ccccctgaac 4860
   ctgaaacata aaatgaatgc aattgttgtt gttaacttgt ttattgcagc ttataatggt 4920
   tacaaataaa gcaatagcat cacaaatttc acaaataaag catttttttc actgcattct 4980
   agttgtggtt tgtccaaact catcaatgta tcttatcatg tctggatcaa ctggataact 5040
   caagctaacc aaaatcatcc caaacttccc accccatacc ctattaccac tgccaattac 5100
   ctagtggttt catttactct aaacctgtga ttcctctgaa ttattttcat tttaaagaaa 5160
   ttgtatttgt taaatatgta ctacaaactt agtagttgga agggctaatt cactcccaaa 5220
   gaagacaaga tatccttgat ctgtggatct accacacaca aggctacttc cctgattagc 5280
   agaactacac accagggcca ggggtcagat atccactgac ctttggatgg tgctacaagc 5340
   tagtaccagt tgagccagat aaggtagaag aggccaataa aggagagaac accagcttgt 5400
   tacaccctgt gagcctgcat gggatggatg acccggagag agaagtgtta gagtggaggt 5460
   ttgacagccg cctagcattt catcacgtgg cccgagagct gcatccggag tacttcaaga 5520
   actgctgata tcgagcttgc tacaagggac tttccgctgg ggactttcca gggaggcgtg 5580
   gcctgggcgg gactggggag tggcgagccc tcagatcctg catataagca gctgcttttt 5640
   gcctgtactg ggtctctctg gttagaccag atctgagcct gggagctctc tggctaacta 5700
   gggaacccac tgcttaagcc tcaataaagc ttgccttgag tgcttcaagt agtgtgtgcc 5760
   cgtctgttgt gtgactctgg taactagaga tccctcagac ccttttagtc agtgtggaaa 5820
   atctctagca gtggcgcccg aacagggact tgaaagcgaa agggaaacca gaggagctct 5880
   ctcgacgcag gactcggctt gctgaagcgc gcacggcaag aggcgagggg cggcgactgg 5940
   tgagtacgcc aaaaattttg actagcggag gctagaagga gagagatggg tgcgagagcg 6000
   tcagtattaa gcgggggaga attagatcgc gatgggaaaa aattcggtta aggccagggg 6060
   gaaagaaaaa atataaatta aaacatatag tatgggcaag cagggagcta gaacgattcg 6120
   cagttaatcc tggcctgtta gaaacatcag aaggctgtag acaaatactg ggacagctac 6180
   aaccatccct tcagacagga tcagaagaac ttagatcatt atataataca gtagcaaccc 6240
   tctattgtgt gcatcaaagg atagagataa aagacaccaa ggaagcttta gacaagatag 6300
   aggaagagca aaacaaaagt aagaccaccg cacagcaagc ggccgctgat cttcagacct 6360
   ggaggaggag atatgaggga caattggaga agtgaattat ataaatataa agtagtaaaa 6420
   attgaaccat taggagtagc acccaccaag gcaaagagaa gagtggtgca gagagaaaaa 6480
   agagcagtgg gaataggagc tttgttcctt gggttcttgg gagcagcagg aagcactatg 6540
   ggcgcagcgt caatgacgct gacggtacag gccagacaat tattgtctgg tatagtgcag 6600
   cagcagaaca atttgctgag ggctattgag gcgcaacagc atctgttgca actcacagtc 6660
   tggggcatca agcagctcca ggcaagaatc ctggctgtgg aaagatacct aaaggatcaa 6720
   cagctcctgg ggatttgggg ttgctctgga aaactcattt gcaccactgc tgtgccttgg 6780
   aatgctagtt ggagtaataa atctctggaa cagatttgga atcacacgac ctggatggag 6840
   tgggacagag aaattaacaa ttacacaagc ttaatacact ccttaattga agaatcgcaa 6900
   aaccagcaag aaaagaatga acaagaatta ttggaattag ataaatgggc aagtttgtgg 6960
   aattggttta acataacaaa ttggctgtgg tatataaaat tattcataat gatagtagga 7020
   ggcttggtag gtttaagaat agtttttgct gtactttcta tagtgaatag agttaggcag 7080
   ggatattcac cattatcgtt tcagacccac ctcccaaccc cgaggggacc cgacaggccc 7140
   gaaggaatag aagaagaagg tggagagaga gacagagaca gatccattcg attagtgaac 7200
   ggatctcgac ggtatcgccg aattcacaaa tggcagtatt catccacaat tttaaaagaa 7260
   aaggggggat tggggggtac agtgcagggg aaagaatagt agacataata gcaacagaca 7320
   tacaaactaa agaattacaa aaacaaatta caaaaattca aaattttcgg gtttattaca 7380
   gggacagcag agatccactt tatcgataag cttgggagtt ccgcgttaca taacttacgg 7440
   taaatggccc gcctggctga ccgcccaacg acccccgccc attgacgtca ataatgacgt 7500
   atgttcccat agtaacgcca atagggactt tccattgacg tcaatgggtg gagtatttac 7560
   ggtaaactgc ccacttggca gtacatcaag tgtatcatat gccaagtacg ccccctattg 7620
   acgtcaatga cggtaaatgg cccgcctggc attatgccca gtacatgacc ttacgggact 7680
   ttcctacttg gcagtacatc tacgtattag tcatcgctat taccatggtg atgcggtttt 7740
   ggcagtacac caatgggcgt ggatagcggt ttgactcacg gggatttcca agtctccacc 7800
   ccattgacgt caatgggagt ttgttttggc accaaaatca acgggacttt ccaaaatgtc 7860
   gtaacaactc cgccccattg acgcaaatgg gcggtaggcg tgtacggtgg gaggtctata 7920
   taagcagagc tcgtttagtg aaccgtcaga tcgcctggag acgccatcca cgctgttttg 7980
   acctccatag aagacaccga ctctagctag aggatccgga ctagtaactc gaggatgggg 8040
   actgacccgg gctgcaggaa ttcataactt cgtataatgt atgctatacg aagttatagt 8100
   taagccttat tgtttatcat cctcattcaa cgacgcgaag gtgtaccttg gcgagaataa 8160
   cttcgtatag gatactttat acgaagttat ttatctgtgc cccagtttgc tagggaggtc 8220
   gcagtacttg gccacagcca tctcgtgctg ctcgacgtag gtctctttgt cggcctcctt 8280
   gattctttcc agtctgtggt ccacgaagtg gaagccgggc atcttgaggt tcttagcggg 8340
   tttcttggat ctgtatgtgg tcttgaagga gcagtgcagg tagcccccgc ccacgagctt 8400
   cagggccatc tggctgtggc ctctcaggcc gccgtcagcg gggtacagca tctcggtgtt 8460
   ggcctcccag ccgcgtgttt tcttctgcat cacagggccg ttggatggga agttcacccc 8520
   gttgatcttg acgttgtaga tgatgcagcc gttctggaag ctggtgtcct gggtagcggt 8580
   cagcacgccc ccgtcttcgt atgtggtgat tctctcccat gtgaagccct cagggaagga 8640
   ctgcttaaag aagtcgggga tgccctgggt gtggttgatg aaggctttgc tgccgtacat 8700
   gaagctggta gccaggatgt cgaaggcgaa ggggagaggg ccgccctcga ccaccttgat 8760
   cttcatggtc tgggtgccct cgtagggctt gccttcgccc tcggatgtgc acttgaagtg 8820
   gtggttgttc acggtgccct ccatgtacag cttcatgtgc atgttctcct tgatcagctc 8880
   gctcatggtg gcatatgata acttcgtata gcatacatta tacgaagtta tattaagggt 8940
   tattgaatat gatcggaagt caacgggtcg atggtgatgc ttggctcgaa taacttcgta 9000
   taaagtatcc tatacgaagt tatactttgg ccgcggctcg agggggttgg ggttgcgcct 9060
   tttccaaggc agccctgggt ttgcgcaggg acgcggctgc tctgggcgtg gttccgggaa 9120
   acgcagcggc gccgaccctg ggtctcgcac attcttcacg tccgttcgca gcgtcacccg 9180
   gatcttcgcc gctacccttg tgggcccccc ggcgacgctt cctgctccgc ccctaagtcg 9240
   ggaaggttcc ttgcggttcg cggcgtgccg gacgtgacaa acggaagccg cacgtctcac 9300
   tagtaccctc gcagacggac agcgccaggg agcaatggca gcgcgccgac cgcgatgggc 9360
   tgtggccaat agcggctgct cagcagggcg cgccgagagc agcggccggg aaggggcggt 9420
   gcgggaggcg gggtgtgggg cggtagtgtg ggccctgttc ctgcccgcgc ggtgttccgc 9480
   attctgcaag cctccggagc gcacgtcggc agtcggctcc ctcgttgacc gaatcaccga 9540
   cctctctccc cagggggatc caccggtcgc caccatggtg agcaagggcg aggagctgtt 9600
   caccggggtg gtgcccatcc tggtcgagct ggacggcgac gtaaacggcc acaagttcag 9660
   cgtgtccggc gagggcgagg gcgatgccac ctacggcaag ctgaccctga agttcatctg 9720
   caccaccggc aagctgcccg tgccctggcc caccctcgtg accaccctga cctacggcgt 9780
   gcagtgcttc agccgctacc ccgaccacat gaagcagcac gacttcttca agtccgccat 9840
   gcccgaaggc tacgtccagg agcgcaccat cttcttcaag gacgacggca actacaagac 9900
   ccgcgccgag gtgaagttcg agggcgacac cctggtgaac cgcatcgagc tgaagggcat 9960
   cgacttcaag gaggacggca acatcctggg gcacaagctg gagtacaact acaacagcca 10020
   caacgtctat atcatggccg acaagcagaa gaacggcatc aaggtgaact tcaagatccg 10080
   ccacaacatc gaggacggca gcgtgcagct cgccgaccac taccagcaga acacccccat 10140
   cggcgacggc cccgtgctgc tgcccgacaa ccactacctg agcacccagt ccgccctgag 10200
   caaagacccc aacgagaagc gcgatcacat ggtcctgctg gagttcgtga ccgccgccgg 10260
   gatcactctc ggcatggacg agctgtacaa gtaagatatc aagcttatcg ataatcaacc 10320
   tctggattac aaaatttgtg aaagattgac tggtattctt aactatgttg ctccttttac 10380
   gctatgtgga tacgctgctt taatgccttt gtatcatgct attgcttccc gtatggcttt 10440
   cattttctcc tccttgtata aatcctggtt gctgtctctt tatgaggagt tgtggcccgt 10500
   tgtcaggcaa cgtggcgtgg tgtgcactgt gtttgctgac gcaaccccca ctggttgggg 10560
   cattgccacc acctgtcagc tcctttccgg gactttcgct ttccccctcc ctattgccac 10620
   ggcggaactc atcgccgcct gccttgcccg ctgctggaca ggggctcggc tgttgggcac 10680
   tgacaattcc gtggtgttgt cggggaaatc atcgtccttt ccttggctgc tcgcctgtgt 10740
   tgccacctgg attctgcgcg ggacgtcctt ctgctacgtc ccttcggccc tcaatccagc 10800
   ggaccttcct tcccgcggcc tgctgccggc tctgcggcct cttccgcgtc ttcgccttcg 10860
   ccctcagacg agtcggatct ccctttgggc cgcctccccg catcgatacc gtcgacctcg 10920
   agaacctaga aaaacatgga gcaatcacaa gtagcaatac agcagctacc aatgctgatt 10980
   gtgcctggct agaagcacaa gaggaggagg aggtgggttt tccagtcaca cctcaggtac 11040
   ctttaagacc aatgacttac aaggcagctg tagatcttag ccacttttta aaagaaaagg 11100
   ggggactgga agggctaatt cactcccaac gaagacaaga tctttttgct tgtactgggt 11160
   ctctctggtt agaccagatc tgagcctggg agctctctgg ctaactaggg aacccactgc 11220
   ttaagcctca ataaagcttg ccttgagtgc ttcaagtagt gtgtgcccgt ctgttgtgtg 11280
   actctggtaa ctagagatcc ctcagaccct tttagtcagt gtggaaaatc tctaga 11336
<210> 7
   <211> 9303
   <212> DNA
   <213> Artificial Sequence
<220>
   <223> sequence for screening simian cells
<400> 7
   ggaaattgta aacgttaata ttttgttaaa attcgcgtta aatttttgtt aaatcagctc       60
   attttttaac caataggccg aaatcggcaa aatcccttat aaatcaaaag aatagaccga     120
   gatagggttg agtgttgttc cagtttggaa caagagtcca ctattaaaga acgtggactc      180
   caacgtcaaa gggcgaaaaa ccgtctatca gggcgatggc ccactacgtg aaccatcacc      240
   ctaatcaagt tttttggggt cgaggtgccg taaagcacta aatcggaacc ctaaagggag      300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcg cgccattcgc cattcaggct 480
   gcgcaactgt tgggaagggc gatcggtgcg ggcctcttcg ctattacgcc agctggcgaa 540
   agggggatgt gctgcaaggc gattaagttg ggtaacgcca gggttttccc agtcacgacg 600
   ttgtaaaacg acggccagtg aattgtaata cgactcacta tagggcgaat tggagctcca 660
   ccgcggtggc ggccgctcta gaattcccat tgcatacgtt gtatccatat cataatatgt 720
   acatttatat tggctcatgt ccaacattac cgccatgttg acattgatta ttgactagtt 780
   attaatagta atcaattacg gggtcattag ttcatagccc atatatggag ttccgcgtta 840
   cataacttac ggtaaatggc ccgcctggct gaccgcccaa cgacccccgc ccattgacgt 900
   caataatgac gtatgttccc atagtaacgc caatagggac tttccattga cgtcaatggg 960
   tggagtattt acggtaaact gcccacttgg cagtacatca agtgtatcat atgccaagta 1020
   cgccccctat tgacgtcaat gacggtaaat ggcccgcctg gcattatgcc cagtacatga 1080
   ccttatggga ctttcctact tggcagtaca tctacgtatt agtcatcgct attaccatgg 1140
   tgatgcggtt ttggcagtac atcaatgggc gtggatagcg gtttgactca cggggatttc 1200
   caagtctcca ccccattgac gtcaatggga gtttgttttg gcaccaaaat caacgggact 1260
   ttccaaaatg tcgtaacaac tccgccccat tgacgcaaat gggcggtagg cgtgtacggt 1320
   gggaggtcta tataagcaga gctcgtttag tgaaccgcag tcgctctgcg gagaggctgg 1380
   cagattgagc cctgggaggt tctctccagc actagcaggt agagcctggg tgttccctgc 1440
   tagactctca ccagcacttg gccagtgctg ggcagagtgg ctccacgctt gcttgcttaa 1500
   agacctcttc aataaagctg ccattttaga agtaagccag tgtgtgttcc catctctcct 1560
   agtcgccgcc tggtcaactc ggtactcggt aataagaaga ccctggtctg ttaggaccct 1620
   ttctgctttg agaaaccgaa gcaggaaaat ccctagcaga ttggcgcccg aacagggact 1680
   tgaaggagag tgagagactc ctgagtacgg ctgagtgaag gcagtaaggg cggcaggaac 1740
   caaccacgac ggagtgctcc tataaaggcg cgggtcggta ccagacggcg tgaggagcgg 1800
   gagaggagga ggcctccggt tgcagtaagt gcaacacaaa aaagaaatag ctgtcttgtt 1860
   atccaggaag ggataataag atagagtggg agatgggcgc gagaaactcc gtcttgtcag 1920
   ggaagaaagc agatgaattg aaaaaattat taatcgcatg aattttaaaa gaaggggagg 1980
   aataggggat atgactccag cagaaagatt aattaacatg atcactacag aacaagaaat 2040
   acaatttcaa caatcaaaaa actcaaaatt taaaaatttt cgggtctatt acagagctca 2100
   cgcgtgattg gagttgggag attataaatt agtagagatc actccgattg gcttggcccc 2160
   cacagatgtg aagaggtaca ctactggtgg cacctcaaga aataaaagag gggtctttgt 2220
   gctagggttc ttgggttttc tcgcaacggc aggttctgca atgggcgcgg cgtcgttgac 2280
   gctgaccgct cagtcccgga ctttattggc tgggatagtg cagcaacagc aacagctgtt 2340
   ggacgtggtc aagagacaac aagaattgtt gcgactgacc gtctggggaa caaagaacct 2400
   ccagactagg gtcactgcca tcgagaagta cttaaaggac caggcgcagc taaatgcttg 2460
   gggatgtgcg tttagacaag tctgccacac tactgtacca tggccaaatg caagtctaac 2520
   accagactgg aacaatgata cttggcaaga gtgggagcga aaggttgact tcttggagga 2580
   aaatataaca gccctcctag aagaggcaca aattcaacaa gagaagaaca tgtatgaatt 2640
   acaaaagttg aatagctggg atgtgtttgg caattggttt gaccttgctt cttggataaa 2700
   gtatatacaa tatggaattt atgtagttgt aggagtaata ctgttaagaa tagtgatcta 2760
   tatagtacaa atgctagcta agttaaggca ggggtatagg ccagtgttct cttccccacc 2820
   ctcttatttc cagtagactc atacccaaca ggacccggca ctgccaacca gagaaggcaa 2880
   agaaggagac ggtggagaag gcggtggatc ctattatcga attcctgcag ccccgataaa 2940
   ataaaagatt ttatttagtc tccagaaaaa ggggggaatg aaagacccca cctgtaggtt 3000
   tggcaagcta gctgcagtaa cgccattttg caaggcatgg aaaaatacca aaccaagaat 3060
   agagaagttc agatcaaggg cgggtacatg aaaatagcta acgttgggcc aaacaggata 3120
   tctgcggtga gcagtttcgg ccccggcccg gggccaagaa cagatggtca ccgcagtttc 3180
   ggccccggcc cgaggccaag aacagatggt ccccagatat ggcccaaccc tcagcagttt 3240
   cttaagaccc atcagatgtt tccaggctcc cccaaggacc tgaaatgacc ctgcgcctta 3300
   tttgaattaa ccaatcagcc tgcttctcgc ttctgttcgc gcgcttctgc ttcccgagct 3360
   ctataaaaga gctcacaacc cctcactcgg cgcgccagtc ctccgacaga ctgagtcgcc 3420
   cgggggggat ccaccgggct gcaggaattc gatatcaagc ttatcgatga ttctagacat 3480
   gcatatctag cgtcccgggc tgcaggaatt cataacttcg tataatgtat gctatacgaa 3540
   gttatagtta agccttattg tttatcatcc tcattcaacg acgcgaaggt gtaccttggc 3600
   gagaataact tcgtatagga tactttatac gaagttattt atctgtgccc cagtttgcta 3660
   gggaggtcgc agtacttggc cacagccatc tcgtgctgct cgacgtaggt ctctttgtcg 3720
   gcctccttga ttctttccag tctgtggtcc acgaagtgga agccgggcat cttgaggttc 3780
   ttagcgggtt tcttggatct gtatgtggtc ttgaaggagc agtgcaggta gcccccgccc 3840
   acgagcttca gggccatctg gctgtggcct ctcaggccgc cgtcagcggg gtacagcatc 3900
   tcggtgttgg cctcccagcc gcgtgttttc ttctgcatca cagggccgtt ggatgggaag 3960
   ttcaccccgt tgatcttgac gttgtagatg atgcagccgt tctggaagct ggtgtcctgg 4020
   gtagcggtca gcacgccccc gtcttcgtat gtggtgattc tctcccatgt gaagccctca 4080
   gggaaggact gcttaaagaa gtcggggatg ccctgggtgt ggttgatgaa ggctttgctg 4140
   ccgtacatga agctggtagc caggatgtcg aaggcgaagg ggagagggcc gccctcgacc 4200
   accttgatct tcatggtctg ggtgccctcg tagggcttgc cttcgccctc ggatgtgcac 4260
   ttgaagtggt ggttgttcac ggtgccctcc atgtacagct tcatgtgcat gttctccttg 4320
   atcagctcgc tcatggtggc atatgataac ttcgtatagc atacattata cgaagttata 4380
   ttaagggtta ttgaatatga tcggaagtca acgggtcgat ggtgatgctt ggctcgaata 4440
   acttcgtata aagtatccta tacgaagtta tactttggcc gcggctcgag ggggttgggg 4500
   ttgcgccttt tccaaggcag ccctgggttt gcgcagggac gcggctgctc tgggcgtggt 4560
   tccgggaaac gcagcggcgc cgaccctggg tctcgcacat tcttcacgtc cgttcgcagc 4620
   gtcacccgga tcttcgccgc tacccttgtg ggccccccgg cgacgcttcc tgctccgccc 4680
   ctaagtcggg aaggttcctt gcggttcgcg gcgtgccgga cgtgacaaac ggaagccgca 4740
   cgtctcacta gtaccctcgc agacggacag cgccagggag caatggcagc gcgccgaccg 4800
   cgatgggctg tggccaatag cggctgctca gcagggcgcg ccgagagcag cggccgggaa 4860
   ggggcggtgc gggaggcggg gtgtggggcg gtagtgtggg ccctgttcct gcccgcgcgg 4920
   tgttccgcat tctgcaagcc tccggagcgc acgtcggcag tcggctccct cgttgaccga 4980
   atcaccgacc tctctcccca gggggatcca ccggtcgcca ccatggtgag caagggcgag 5040
   gagctgttca ccggggtggt gcccatcctg gtcgagctgg acggcgacgt aaacggccac 5100
   aagttcagcg tgtccggcga gggcgagggc gatgccacct acggcaagct gaccctgaag 5160
   ttcatctgca ccaccggcaa gctgcccgtg ccctggccca ccctcgtgac caccctgacc 5220
   tacggcgtgc agtgcttcag ccgctacccc gaccacatga agcagcacga cttcttcaag 5280
   tccgccatgc ccgaaggcta cgtccaggag cgcaccatct tcttcaagga cgacggcaac 5340
   tacaagaccc gcgccgaggt gaagttcgag ggcgacaccc tggtgaaccg catcgagctg 5400
   aagggcatcg acttcaagga ggacggcaac atcctggggc acaagctgga gtacaactac 5460
   aacagccaca acgtctatat catggccgac aagcagaaga acggcatcaa ggtgaacttc 5520
   aagatccgcc acaacatcga ggacggcagc gtgcagctcg ccgaccacta ccagcagaac 5580
   acccccatcg gcgacggccc cgtgctgctg cccgacaacc actacctgag cacccagtcc 5640
   gccctgagca aagaccccaa cgagaagcgc gatcacatgg tcctgctgga gttcgtgacc 5700
   gccgccggga tcactctcgg catggacgag ctgtacaagt aaagcggccg cgactctaga 5760
   gtcgacctgc aggcatgcaa gcttgatatc aagcttatcg ataatcaacc tctggattac 5820
   aaaatttgtg aaagattgac tggtattctt aactatgttg ctccttttac gctatgtgga 5880
   tacgctgctt taatgccttt gtatcatgct attgcttccc gtatggcttt cattttctcc 5940
   tccttgtata aatcctggtt gctgtctctt tatgaggagt tgtggcccgt tgtcaggcaa 6000
   cgtggcgtgg tgtgcactgt gtttgctgac gcaaccccca ctggttgggg cattgccacc 6060
   acctgtcagc tcctttccgg gactttcgct ttccccctcc ctattgccac ggcggaactc 6120
   atcgccgcct gccttgcccg ctgctggaca ggggctcggc tgttgggcac tgacaattcc 6180
   gtggtgttgt cggggaaatc atcgtccttt ccttggctgc tcgcctgtgt tgccacctgg 6240
   attctgcgcg ggacgtcctt ctgctacgtc ccttcggccc tcaatccagc ggaccttcct 6300
   tcccgcggcc tgctgccggc tctgcggcct cttccgcgtc ttcgccttcg ccctcagacg 6360
   agtcggatct ccctttgggc cgcctccccg catcgatacc gtcgacctcg agttttataa 6420
   aagaaaaggg gggactggaa gggatttatt acagtgcaag aagacataga atcttagaca 6480
   tgtacttaga aaaggaagaa ggcatcatac cagattggca ggattacacc tcaggaccag 6540
   gaattagata cccaaagaca tttggctggc tatggaaatt agtccctgta aatgtatcag 6600
   atgaggcaca ggaggatgag aggcattatt taatgcagcc agctcaaact tccaagtggg 6660
   atgacccttg gggagaggtt ctagcgtgga agtttgatcc aactctagcc tacacttatg 6720
   aggcatatgc tagataccca gaagagttgg aagcaagtca ggcctgtcag aactgcattt 6780
   cgctctgtat tcagtcgctc tgcggagagg ctggcagatt gagccctggg aggttctctc 6840
   cagcactagc aggtagagcc tgggtgttcc ctgctagact ctcaccagca cttggccagt 6900
   gctgggcaga gtggctccac gcttgcttgc ttaaagacct cttcaataaa gctgccattt 6960
   tagaagtaag ccagtgtgtg ttcccatctc tcctagtcgc cgcctggtca actcggtact 7020
   cggtaataag aagaccctgg tctgttagga ccctttctgc tttgagaaac cgaagcagga 7080
   aaatccctag catggtaccc agcttttgtt ccctttagtg agggttaatt ccgagcttgg 7140
   cgtaatcatg gtcatagctg tttcctgtgt gaaattgtta tccgctcaca attccacaca 7200
   acatacgagc cggaagcata aagtgtaaag cctggggtgc ctaatgagtg agctaactca 7260
   cattaattgc gttgcgctca ctgcccgctt tccagtcggg aaacctgtcg tgccagctgc 7320
   attaatgaat cggccaacgc gcggggagag gcggtttgcg tattgggcgc tcttccgctt 7380
   cctcgctcac tgactcgctg cgctcggtcg ttcggctgcg gcgagcggta tcagctcact 7440
   caaaggcggt aatacggtta tccacagaat caggggataa cgcaggaaag aacatgtgag 7500
   caaaaggcca gcaaaaggcc aggaaccgta aaaaggccgc gttgctggcg tttttccata 7560
   ggctccgccc ccctgacgag catcacaaaa atcgacgctc aagtcagagg tggcgaaacc 7620
   cgacaggact ataaagatac caggcgtttc cccctggaag ctccctcgtg cgctctcctg 7680
   ttccgaccct gccgcttacc ggatacctgt ccgcctttct cccttcggga agcgtggcgc 7740
   tttctcatag ctcacgctgt aggtatctca gttcggtgta ggtcgttcgc tccaagctgg 7800
   gctgtgtgca cgaacccccc gttcagcccg accgctgcgc cttatccggt aactatcgtc 7860
   ttgagtccaa cccggtaaga cacgacttat cgccactggc agcagccact ggtaacagga 7920
   ttagcagagc gaggtatgta ggcggtgcta cagagttctt gaagtggtgg cctaactacg 7980
   gctacactag aaggacagta tttggtatct gcgctctgct gaagccagtt accttcggaa 8040
   aaagagttgg tagctcttga tccggcaaac aaaccaccgc tggtagcggt ggtttttttg 8100
   tttgcaagca gcagattacg cgcagaaaaa aaggatctca agaagatcct ttgatctttt 8160
   ctacggggtc tgacgctcag tggaacgaaa actcacgtta agggattttg gtcatgagat 8220
   tatcaaaaag gatcttcacc tagatccttt taaattaaaa atgaagtttt aaatcaatct 8280
   aaagtatata tgagtaaact tggtctgaca gttaccaatg cttaatcagt gaggcaccta 8340
   tctcagcgat ctgtctattt cgttcatcca tagttgcctg actccccgtc gtgtagataa 8400
   ctacgatacg ggagggctta ccatctggcc ccagtgctgc aatgataccg cgagacccac 8460
   gctcaccggc tccagattta tcagcaataa accagccagc cggaagggcc gagcgcagaa 8520
   gtggtcctgc aactttatcc gcctccatcc agtctattaa ttgttgccgg gaagctagag 8580
   taagtagttc gccagttaat agtttgcgca acgttgttgc cattgctaca ggcatcgtgg 8640
   tgtcacgctc gtcgtttggt atggcttcat tcagctccgg ttcccaacga tcaaggcgag 8700
   ttacatgatc ccccatgttg tgcaaaaaag cggttagctc cttcggtcct ccgatcgttg 8760
   tcagaagtaa gttggccgca gtgttatcac tcatggttat ggcagcactg cataattctc 8820
   ttactgtcat gccatccgta agatgctttt ctgtgactgg tgagtactca accaagtcat 8880
   tctgagaata gtgtatgcgg cgaccgagtt gctcttgccc ggcgtcaata cgggataata 8940
   ccgcgccaca tagcagaact ttaaaagtgc tcatcattgg aaaacgttct tcggggcgaa 9000
   aactctcaag gatcttaccg ctgttgagat ccagttcgat gtaacccact cgtgcaccca 9060
   actgatcttc agcatctttt actttcacca gcgtttctgg gtgagcaaaa acaggaaggc 9120
   aaaatgccgc aaaaaaggga ataagggcga cacggaaatg ttgaatactc atactcttcc 9180
   tttttcaata ttattgaagc atttatcagg gttattgtct catgagcgga tacatatttg 9240
   aatgtattta gaaaaataaa caaatagggg ttccgcgcac atttccccga aaagtgccac 9300
   ctg 9303
<210> 8
   <211> 720
   <212> DNA
   <213> Artificial Sequence
<220>
   <223> recombinant enhanced GFP
<400> 8
   atggtgagca agggcgagga gctgttcacc ggggtggtgc ccatcctggt cgagctggac        60
   ggcgacgtaa acggccacaa gttcagcgtg tccggcgagg gcgagggcga tgccacctac       120
   ggcaagctga ccctgaagtt catctgcacc accggcaagc tgcccgtgcc ctggcccacc       180
   ctcgtgacca ccctgaccta cggcgtgcag tgcttcagcc gctaccccga ccacatgaag       240
   cagcacgact tcttcaagtc cgccatgccc gaaggctacg tccaggagcg caccatcttc       300
   ttcaaggacg acggcaacta caagacccgc gccgaggtga agttcgaggg cgacaccctg       360
   gtgaaccgca tcgagctgaa gggcatcgac ttcaaggagg acggcaacat cctggggcac       420
   aagctggagt acaactacaa cagccacaac gtctatatca tggccgacaa gcagaagaac       480
   ggcatcaagg tgaacttcaa gatccgccac aacatcgagg acggcagcgt gcagctcgcc       540
   gaccactacc agcagaacac ccccatcggc gacggccccg tgctgctgcc cgacaaccac       600
   tacctgagca cccagtccgc cctgagcaaa gaccccaacg agaagcgcga tcacatggtc       660
   ctgctggagt tcgtgaccgc cgccgggatc actctcggca tggacgagct gtacaagtaa       720
<210> 9
   <211> 696
   <212> DNA
   <213> Artificial Sequence
<220>
   <223> RFP
<400> 9
   atgagcgagc tgatcaagga gaacatgcac atgaagctgt acatggaggg caccgtgaac        60
   aaccaccact tcaagtgcac atccgagggc gaaggcaagc cctacgaggg cacccagacc       120
   atgaagatca aggtggtcga gggcggccct ctccccttcg ccttcgacat cctggctacc       180
   agcttcatgt acggcagcaa agccttcatc aaccacaccc agggcatccc cgacttcttt       240
   aagcagtcct tccctgaggg cttcacatgg gagagaatca ccacatacga agacgggggc       300
   gtgctgaccg ctacccagga caccagcttc cagaacggct gcatcatcta caacgtcaag       360
   atcaacgggg tgaacttccc atccaacggc cctgtgatgc agaagaaaac acgcggctgg       420
   gaggccaaca ccgagatgct gtaccccgct gacggcggcc tgagaggcca cagccagatg       480
   gccctgaagc tcgtgggcgg gggctacctg cactgctcct tcaagaccac atacagatcc       540
   aagaaacccg ctaagaacct caagatgccc ggcttccact tcgtggacca cagactggaa       600
   agaatcaagg aggccgacaa agagacctac gtcgagcagc acgagatggc tgtggccaag       660
   tactgcgacc tccctagcaa actggggcac agataa                                 696
<210> 10
   <211> 13978
   <212> DNA
   <213> Artificial Sequence
<220>
   <223> pBR HIV -NL4-3-Nef-IRES-Cre
<400> 10
   gcgttggaag ggctaattca ctcccaaaga agacaagata tccttgatct gtggatctac 60
   cacacacaag gctacttccc tgattggcag aactacacac cagggccagg ggtcagatat 120
   ccactgacct ttggatggtg ctacaagcta gtaccagttg agccagataa ggtagaagag 180
   gccaataaag gagagaacac cagcttgtta caccctgtga gcctgcatgg aatggatgac 240
   cctgagagag aagtgttaga gtggaggttt gacagccgcc tagcatttca tcacgtggcc 300
   cgagagctgc atccggagta ctacaaagac tgctgacatc gagctttcta caagggactt 360
   tccgctgggg actttccagg gaggtgtggc ctgggcggga ctggggagtg gcgagccctc 420
   agatgctaca tataagcagc tgctttttgc ctgtactggg tctctctggt tagaccagat 480
   ctgagcctgg gagctctctg gctaactagg gaacccactg cttaagcctc aataaagctt 540
   gccttgagtg ctcaaagtag tgtgtgcccg tctgttgtgt gactctggta actagagatc 600
   cctcagaccc ttttagtcag tgtggaaaat ctctagcagt ggcgcccgaa cagggacttg 660
   aaagcgaaag taaagccaga ggagatctct cgacgcagga ctcggcttgc tgaagcgcgc 720
   acggcaagag gcgaggggcg gcgactggtg agtacgccaa aaattttgac tagcggaggc 780
   tagaaggaga gagatgggtg cgagagcgtc ggtattaagc gggggagaat tagataaatg 840
   ggaaaaaatt cggttaaggc cagggggaaa gaaacaatat aaactaaaac atatagtatg 900
   ggcaagcagg gagctagaac gattcgcagt taatcctggc cttttagaga catcagaagg 960
   ctgtagacaa atactgggac agctacaacc atcccttcag acaggatcag aagaacttag 1020
   atcattatat aatacaatag cagtcctcta ttgtgtgcat caaaggatag atgtaaaaga 1080
   caccaaggaa gccttagata agatagagga agagcaaaac aaaagtaaga aaaaggcaca 1140
   gcaagcagca gctgacacag gaaacaacag ccaggtcagc caaaattacc ctatagtgca 1200
   gaacctccag gggcaaatgg tacatcaggc catatcacct agaactttaa atgcatgggt 1260
   aaaagtagta gaagagaagg ctttcagccc agaagtaata cccatgtttt cagcattatc 1320
   agaaggagcc accccacaag atttaaatac catgctaaac acagtggggg gacatcaagc 1380
   agccatgcaa atgttaaaag agaccatcaa tgaggaagct gcagaatggg atagattgca 1440
   tccagtgcat gcagggccta ttgcaccagg ccagatgaga gaaccaaggg gaagtgacat 1500
   agcaggaact actagtaccc ttcaggaaca aataggatgg atgacacata atccacctat 1560
   cccagtagga gaaatctata aaagatggat aatcctggga ttaaataaaa tagtaagaat 1620
   gtatagccct accagcattc tggacataag acaaggacca aaggaaccct ttagagacta 1680
   tgtagaccga ttctataaaa ctctaagagc cgagcaagct tcacaagagg taaaaaattg 1740
   gatgacagaa accttgttgg tccaaaatgc gaacccagat tgtaagacta ttttaaaagc 1800
   attgggacca ggagcgacac tagaagaaat gatgacagca tgtcagggag tggggggacc 1860
   cggccataaa gcaagagttt tggctgaagc aatgagccaa gtaacaaatc cagctaccat 1920
   aatgatacag aaaggcaatt ttaggaacca aagaaagact gttaagtgtt tcaattgtgg 1980
   caaagaaggg cacatagcca aaaattgcag ggcccctagg aaaaagggct gttggaaatg 2040
   tggaaaggaa ggacaccaaa tgaaagattg tactgagaga caggctaatt ttttagggaa 2100
   gatctggcct tcccacaagg gaaggccagg gaattttctt cagagcagac cagagccaac 2160
   agccccacca gaagagagct tcaggtttgg ggaagagaca acaactccct ctcagaagca 2220
   ggagccgata gacaaggaac tgtatccttt agcttccctc agatcactct ttggcagcga 2280
   cccctcgtca caataaagat aggggggcaa ttaaaggaag ctctattaga tacaggagca 2340
   gatgatacag tattagaaga aatgaatttg ccaggaagat ggaaaccaaa aatgataggg 2400
   ggaattggag gttttatcaa agtaggacag tatgatcaga tactcataga aatctgcgga 2460
   cataaagcta taggtacagt attagtagga cctacacctg tcaacataat tggaagaaat 2520
   ctgttgactc agattggctg cactttaaat tttcccatta gtcctattga gactgtacca 2580
   gtaaaattaa agccaggaat ggatggccca aaagttaaac aatggccatt gacagaagaa 2640
   aaaataaaag cattagtaga aatttgtaca gaaatggaaa aggaaggaaa aatttcaaaa 2700
   attgggcctg aaaatccata caatactcca gtatttgcca taaagaaaaa agacagtact 2760
   aaatggagaa aattagtaga tttcagagaa cttaataaga gaactcaaga tttctgggaa 2820
   gttcaattag gaataccaca tcctgcaggg ttaaaacaga aaaaatcagt aacagtactg 2880
   gatgtgggcg atgcatattt ttcagttccc ttagataaag acttcaggaa gtatactgca 2940
   tttaccatac ctagtataaa caatgagaca ccagggatta gatatcagta caatgtgctt 3000
   ccacagggat ggaaaggatc accagcaata ttccagtgta gcatgacaaa aatcttagag 3060
   ccttttagaa aacaaaatcc agacatagtc atctatcaat acatggatga tttgtatgta 3120
   ggatctgact tagaaatagg gcagcataga acaaaaatag aggaactgag acaacatctg 3180
   ttgaggtggg gatttaccac accagacaaa aaacatcaga aagaacctcc attcctttgg 3240
   atgggttatg aactccatcc tgataaatgg acagtacagc ctatagtgct gccagaaaag 3300
   gacagctgga ctgtcaatga catacagaaa ttagtgggaa aattgaattg ggcaagtcag 3360
   atttatgcag ggattaaagt aaggcaatta tgtaaacttc ttaggggaac caaagcacta 3420
   acagaagtag taccactaac agaagaagca gagctagaac tggcagaaaa cagggagatt 3480
   ctaaaagaac cggtacatgg agtgtattat gacccatcaa aagacttaat agcagaaata 3540
   cagaagcagg ggcaaggcca atggacatat caaatttatc aagagccatt taaaaatctg 3600
   aaaacaggaa aatatgcaag aatgaagggt gcccacacta atgatgtgaa acaattaaca 3660
   gaggcagtac aaaaaatagc cacagaaagc atagtaatat ggggaaagac tcctaaattt 3720
   aaattaccca tacaaaagga aacatgggaa gcatggtgga cagagtattg gcaagccacc 3780
   tggattcctg agtgggagtt tgtcaatacc cctcccttag tgaagttatg gtaccagtta 3840
   gagaaagaac ccataatagg agcagaaact ttctatgtag atggggcagc caatagggaa 3900
   actaaattag gaaaagcagg atatgtaact gacagaggaa gacaaaaagt tgtcccccta 3960
   acggacacaa caaatcagaa gactgagtta caagcaattc atctagcttt gcaggattcg 4020
   ggattagaag taaacatagt gacagactca caatatgcat tgggaatcat tcaagcacaa 4080
   ccagataaga gtgaatcaga gttagtcagt caaataatag agcagttaat aaaaaaggaa 4140
   aaagtctacc tggcatgggt accagcacac aaaggaattg gaggaaatga acaagtagat 4200
   aaattggtca gtgctggaat caggaaagta ctatttttag atggaataga taaggcccaa 4260
   gaagaacatg agaaatatca cagtaattgg agagcaatgg ctagtgattt taacctacca 4320
   cctgtagtag caaaagaaat agtagccagc tgtgataaat gtcagctaaa aggggaagcc 4380
   atgcatggac aagtagactg tagcccagga atatggcagc tagattgtac acatttagaa 4440
   ggaaaagtta tcttggtagc agttcatgta gccagtggat atatagaagc agaagtaatt 4500
   ccagcagaga cagggcaaga aacagcatac ttcctcttaa aattagcagg aagatggcca 4560
   gtaaaaacag tacatacaga caatggcagc aatttcacca gtactacagt taaggccgcc 4620
   tgttggtggg cggggatcaa gcaggaattt ggcattccct acaatcccca aagtcaagga 4680
   gtaatagaat ctatgaataa agaattaaag aaaattatag gacaggtaag agatcaggct 4740
   gaacatctta agacagcagt acaaatggca gtattcatcc acaattttaa aagaaaaggg 4800
   gggattgggg ggtacagtgc aggggaaaga atagtagaca taatagcaac agacatacaa 4860
   actaaagaat tacaaaaaca aattacaaaa attcaaaatt ttcgggttta ttacagggac 4920
   agcagagatc cagtttggaa aggaccagca aagctcctct ggaaaggtga aggggcagta 4980
   gtaatacaag ataatagtga cataaaagta gtgccaagaa gaaaagcaaa gatcatcagg 5040
   gattatggaa aacagatggc aggtgatgat tgtgtggcaa gtagacagga tgaggattaa 5100
   cacatggaaa agattagtaa aacaccatat gtatatttca aggaaagcta aggactggtt 5160
   ttatagacat cactatgaaa gtactaatcc aaaaataagt tcagaagtac acatcccact 5220
   aggggatgct aaattagtaa taacaacata ttggggtctg catacaggag aaagagactg 5280
   gcatttgggt cagggagtct ccatagaatg gaggaaaaag agatatagca cacaagtaga 5340
   ccctgaccta gcagaccaac taattcatct gcactatttt gattgttttt cagaatctgc 5400
   tataagaaat accatattag gacgtatagt tagtcctagg tgtgaatatc aagcaggaca 5460
   taacaaggta ggatctctac agtacttggc actagcagca ttaataaaac caaaacagat 5520
   aaagccacct ttgcctagtg ttaggaaact gacagaggac agatggaaca agccccagaa 5580
   gaccaagggc cacagaggga gccatacaat gaatggacac tagagctttt agaggaactt 5640
   aagagtgaag ctgttagaca ttttcctagg atatggctcc ataacttagg acaacatatc 5700
   tatgaaactt acggggatac ttgggcagga gtggaagcca taataagaat tctgcaacaa 5760
   ctgctgttta tccatttcag aattgggtgt cgacatagca gaataggcgt tactcgacag 5820
   aggagagcaa gaaatggagc cagtagatcc tagactagag ccctggaagc atccaggaag 5880
   tcagcctaaa actgcttgta ccaattgcta ttgtaaaaag tgttgctttc attgccaagt 5940
   ttgtttcatg acaaaagcct taggcatctc ctatggcagg aagaagcgga gacagcgacg 6000
   aagagctcat cagaacagtc agactcatca agcttctcta tcaaagcagt aagtagtaca 6060
   tgtaatgcaa cctataatag tagcaatagt agcattagta gtagcaataa taatagcaat 6120
   agttgtgtgg tccatagtaa tcatagaata taggaaaata ttaagacaaa gaaaaataga 6180
   caggttaatt gatagactaa tagaaagagc agaagacagt ggcaatgaga gtgaaggaga 6240
   agtatcagca cttgtggaga tgggggtgga aatggggcac catgctcctt gggatattga 6300
   tgatctgtag tgctacagaa aaattgtggg tcacagtcta ttatggggta cctgtgtgga 6360
   aggaagcaac caccactcta ttttgtgcat cagatgctaa agcatatgat acagaggtac 6420
   ataatgtttg ggccacacat gcctgtgtac ccacagaccc caacccacaa gaagtagtat 6480
   tggtaaatgt gacagaaaat tttaacatgt ggaaaaatga catggtagaa cagatgcatg 6540
   aggatataat cagtttatgg gatcaaagcc taaagccatg tgtaaaatta accccactct 6600
   gtgttagttt aaagtgcact gatttgaaga atgatactaa taccaatagt agtagcggga 6660
   gaatgataat ggagaaagga gagataaaaa actgctcttt caatatcagc acaagcataa 6720
   gagataaggt gcagaaagaa tatgcattct tttataaact tgatatagta ccaatagata 6780
   ataccagcta taggttgata agttgtaaca cctcagtcat tacacaggcc tgtccaaagg 6840
   tatcctttga gccaattccc atacattatt gtgccccggc tggttttgcg attctaaaat 6900
   gtaataataa gacgttcaat ggaacaggac catgtacaaa tgtcagcaca gtacaatgta 6960
   cacatggaat caggccagta gtatcaactc aactgctgtt aaatggcagt ctagcagaag 7020
   aagatgtagt aattagatct gccaatttca cagacaatgc taaaaccata atagtacagc 7080
   tgaacacatc tgtagaaatt aattgtacaa gacccaacaa caatacaaga aaaagtatcc 7140
   gtatccagag gggaccaggg agagcatttg ttacaatagg aaaaatagga aatatgagac 7200
   aagcacattg taacattagt agagcaaaat ggaatgccac tttaaaacag atagctagca 7260
   aattaagaga acaatttgga aataataaaa caataatctt taagcaatcc tcaggagggg      7320
   acccagaaat tgtaacgcac agttttaatt gtggagggga atttttctac tgtaattcaa      7380
   cacaactgtt taatagtact tggtttaata gtacttggag tactgaaggg tcaaataaca 7440
   ctgaaggaag tgacacaatc acactcccat gcagaataaa acaatttata aacatgtggc 7500
   aggaagtagg aaaagcaatg tatgcccctc ccatcagtgg acaaattaga tgttcatcaa 7560
   atattactgg gctgctatta acaagagatg gtggtaataa caacaatggg tccgagatct 7620
   tcagacctgg aggaggcgat atgagggaca attggagaag tgaattatat aaatataaag 7680
   tagtaaaaat tgaaccatta ggagtagcac ccaccaaggc aaagagaaga gtggtgcaga 7740
   gagaaaaaag agcagtggga ataggagctt tgttccttgg gttcttggga gcagcaggaa 7800
   gcactatggg cgcagcgtca atgacgctga cggtacaggc cagacaatta ttgtctgata 7860
   tagtgcagca gcagaacaat ttgctgaggg ctattgaggc gcaacagcat ctgttgcaac 7920
   tcacagtctg gggcatcaaa cagctccagg caagaatcct ggctgtggaa agatacctaa 7980
   aggatcaaca gctcctgggg atttggggtt gctctggaaa actcatttgc accactgctg 8040
   tgccttggaa tgctagttgg agtaataaat ctctggaaca gatttggaat aacatgacct 8100
   ggatggagtg ggacagagaa attaacaatt acacaagctt aatacactcc ttaattgaag 8160
   aatcgcaaaa ccagcaagaa aagaatgaac aagaattatt ggaattagat aaatgggcaa 8220
   gtttgtggaa ttggtttaac ataacaaatt ggctgtggta tataaaatta ttcataatga 8280
   tagtaggagg cttggtaggt ttaagaatag tttttgctgt actttctata gtgaatagag 8340
   ttaggcaggg atattcacca ttatcgtttc agacccacct cccaatcccg aggggacccg 8400
   acaggcccga aggaatagaa gaagaaggtg gagagagaga cagagacaga tccattcgat 8460
   tagtgaacgg atccttagca cttatctggg acgatctgcg gagcctgtgc ctcttcagct 8520
   accaccgctt gagagactta ctcttgattg taacgaggat tgtggaactt ctgggacgca 8580
   gggggtggga agccctcaaa tattggtgga atctcctaca gtattggagt caggaactaa 8640
   agaatagtgc tgttaacttg ctcaatgcca cagccatagc agtagctgag gggacagata 8700
   gggttataga agtattacaa gcagcttata gagctattcg ccacatacct agaagaataa 8760
   gacagggctt ggaaaggatt ttgctataag atgggtggca agtggtcaaa aagtagtgtg 8820
   attggatggc ctgctgtaag ggaaagaatg agacgagctg agccagcagc agatggggtg 8880
   ggagcagtat ctcgagacct agaaaaacat ggagcaatca caagtagcaa tacagcagct 8940
   aacaatgctg cttgtgcctg gctagaagca caagaggagg aagaggtggg ttttccagtc 9000
   acacctcagg tacctttaag accaatgact tacaaggcag ctgtagatct tagccacttt 9060
   ttaaaagaaa aggggggact ggaagggcta attcactccc aaagaaggca agatatcctt 9120
   gatctgtgga tctaccacac acaaggctac ttccctgatt ggcagaacta cacaccaggg 9180
   ccaggggtca gatatccact gacctttgga tggtgctaca agctagtacc agctgagcca 9240
   gataaggtag aagaggccaa taaaggagag aacaccagct tgttacaccc tgtgagcctg 9300
   catggaatgg atgaccctga gagagaagtg ttagagtgga ggtttgacag ccgcctagca 9360
   tttcatcacg tggcccgaga gctgcatccg gagtacttca agaactgctg aacgcgtcgg 9420
   atcccggagt actacaaaga ctgctgacgc gaattccgcc ccccccccta acgttactgg 9480
   ccgaagccgc ttggaataag gccggtgtgc gtttgtctat atgttatttt ccaccatatt 9540
   gccgtctttt ggcaatgtga gggcccggaa acctggccct gtcttcttga cgagcattcc 9600
   taggggtctt tcccctctcg ccaaaggaat gcaaggtctg ttgaatgtcg tgaaggaagc 9660
   agttcctctg gaagcttctt gaagacaaac aacgtctgta gcgacccttt gcaggcagcg 9720
   gaacccccca cctggcgaca ggtgcctctg cggccaaaag ccacgtgtat aagatacacc 9780
   tgcaaaggcg gcacaacccc agtgccacgt tgtgagttgg atagttgtgg aaagagtcaa 9840
   atggctctcc tcaagcgtat tcaacaaggg gctgaaggat gcccagaagg taccccattg 9900
   tatgggatct gatctggggc ctcggtgcac atgctttaca tgtgtttagt cgaggttaaa 9960
   aaacgtctag gccccccgaa ccacggggac gtggttttcc tttgaaaaac acgatgataa 10020
   taccatggtg cccaagaaga agaggaaagt ctccaacctg ctgactgtgc accaaaacct 10080
   gcctgccctc cctgtggatg ccacctctga tgaagtcagg aagaacctga tggacatgtt 10140
   cagggacagg caggccttct ctgaacacac ctggaagatg ctcctgtctg tgtgcagatc 10200
   ctgggctgcc tggtgcaagc tgaacaacag gaaatggttc cctgctgaac ctgaggatgt 10260
   gagggactac ctcctgtacc tgcaagccag aggcctggct gtgaagacca tccaacagca 10320
   cctgggccag ctcaacatgc tgcacaggag atctggcctg cctcgccctt ctgactccaa 10380
   tgctgtgtcc ctggtgatga ggagaatcag aaaggagaat gtggatgctg gggagagagc 10440
   caagcaggcc ctggcctttg aacgcactga ctttgaccaa gtcagatccc tgatggagaa 10500
   ctctgacaga tgccaggaca tcaggaacct ggccttcctg ggcattgcct acaacaccct 10560
   gctgcgcatt gccgaaattg ccagaatcag agtgaaggac atctcccgca ccgatggtgg 10620
   gagaatgctg atccacattg gcaggaccaa gaccctggtg tccacagctg gtgtggagaa 10680
   ggccctgtcc ctgggggtta ccaagctggt ggagagatgg atctctgtgt ctggtgtggc 10740
   tgatgacccc aacaactacc tgttctgccg ggtcagaaag aatggtgtgg ctgccccttc 10800
   tgccacctcc caactgtcca cccgggccct ggaagggatc tttgaggcca cccaccgcct 10860
   gatctatggt gccaaggatg actctgggca gagatacctg gcctggtctg gccactctgc 10920
   cagagtgggt gctgccaggg acatggccag ggctggtgtg tccatccctg aaatcatgca 10980
   ggctggtggc tggaccaatg tgaacattgt gatgaactac atcagaaacc tggactctga 11040
   gactggggcc atggtgaggc tgctcgagga tggggactga cccgggtagc cactttttaa 11100
   aagaaaaggg gggactggaa gggctaattc actcccaaag aagacaagat atcccatccg 11160
   gagtacttca agaactgctg acatcgagct tgctacaagg gactttccgc tggggacttt 11220
   ccagggaggc gtggcctggg cgggactggg gagtggcgag ccctcagatg ctgcatataa 11280
   gcagctgctt tttgcctgta ctgggtctct ctggttagac cagatctgag cctgggagct 11340
   ctctggctaa ctagggaacc cactgcttaa gcctcaataa agcttgcctt gagtgcttca 11400
   agtagtgtgt gcccgtctgt tgtgtgactc tggtaactag agatccctca gaccctttta 11460
   gtcagtgtgg aaaatctcta gcagttctag agcggccgct cgcgaattct tgaagacgaa 11520
   agggcctcgt gatacgccta tttttatagg ttaatgtcat gataataatg gtttcttaga 11580
   cgtcaggtgg cacttttcgg ggaaatgtgc gcggaacccc tatttgttta tttttctaaa 11640
   tacattcaaa tatgtatccg ctcatgagac aataaccctg ataaatgctt caataatatt 11700
   gaaaaaggaa gagtatgagt attcaacatt tccgtgtcgc ccttattccc ttttttgcgg 11760
   cattttgcct tcctgttttt gctcacccag aaacgctggt gaaagtaaaa gatgctgaag 11820
   atcagttggg tgcacgagtg ggttacatcg aactggatct caacagcggt aagatccttg 11880
   agagttttcg ccccgaagaa cgttttccaa tgatgagcac ttttaaagtt ctgctatgtg 11940
   gcgcggtatt atcccgtgtt gacgccgggc aagagcaact cggtcgccgc atacactatt 12000
   ctcagaatga cttggttgag tactcaccag tcacagaaaa gcatcttacg gatggcatga 12060
   cagtaagaga attatgcagt gctgccataa ccatgagtga taacactgcg gccaacttac 12120
   ttctgacaac gatcggagga ccgaaggagc taaccgcttt tttgcacaac atgggggatc 12180
   atgtaactcg ccttgatcgt tgggaaccgg agctgaatga agccatacca aacgacgagc 12240
   gtgacaccac gatgcctgca gcaatggcaa caacgttgcg caaactatta actggcgaac 12300
   tacttactct agcttcccgg caacaattaa tagactggat ggaggcggat aaagttgcag 12360
   gaccacttct gcgctcggcc cttccggctg gctggtttat tgctgataaa tctggagccg 12420
   gtgagcgtgg gtctcgcggt atcattgcag cactggggcc agatggtaag ccctcccgta 12480
   tcgtagttat ctacacgacg gggagtcagg caactatgga tgaacgaaat agacagatcg 12540
   ctgagatagg tgcctcactg attaagcatt ggtaactgtc agaccaagtt tactcatata 12600
   tactttagat tgatttaaaa cttcattttt aatttaaaag gatctaggtg aagatccttt 12660
   ttgataatct catgaccaaa atcccttaac gtgagttttc gttccactga gcgtcagacc 12720
   ccgtagaaaa gatcaaagga tcttcttgag atcctttttt tctgcgcgta atctgctgct 12780
   tgcaaacaaa aaaaccaccg ctaccagcgg tggtttgttt gccggatcaa gagctaccaa 12840
   ctctttttcc gaaggtaact ggcttcagca gagcgcagat accaaatact gtccttctag 12900
   tgtagccgta gttaggccac cacttcaaga actctgtagc accgcctaca tacctcgctc 12960
   tgctaatcct gttaccagtg gctgctgcca gtggcgataa gtcgtgtctt accgggttgg 13020
   actcaagacg atagttaccg gataaggcgc agcggtcggg ctgaacgggg ggttcgtgca 13080
   cacagcccag cttggagcga acgacctaca ccgaactgag atacctacag cgtgagctat 13140
   gagaaagcgc cacgcttccc gaagggagaa aggcggacag gtatccggta agcggcaggg 13200
   tcggaacagg agagcgcacg agggagcttc cagggggaaa cgcctggtat ctttatagtc 13260
   ctgtcgggtt tcgccacctc tgacttgagc gtcgattttt gtgatgctcg tcaggggggc 13320
   ggagcctatg gaaaaacgcc agcaacgcgg cctttttacg gttcctggcc ttttgctggc 13380
   cttttgctca catgttcttt cctgcgttat cccctgattc tgtggataac cgtattaccg 13440
   cctttgagtg agctgatacc gctcgccgca gccgaacgac cgagcgcagc gagtcagtga 13500
   gcgaggaagc ggaagagcgc ctgatgcggt attttctcct tacgcatctg tgcggtattt 13560
   cacaccgcat atggtgcact ctcagtacaa tctgctctga tgccgcatag ttaagccagt 13620
   atacactccg ctatcgctac gtgactgggt catggctgcg ccccgacacc cgccaacacc 13680
   cgctgacgcg ccctgacggg cttgtctgct cccggcatcc gcttacagac aagctgtgac 13740
   cgtctccggg agctgcatgt gtcagaggtt ttcaccgtca tcaccgaaac gcgcgaggca 13800
   gatcccgcaa gaggcccggc agtaccggca taaccaagcc tatgcctaca gcatccaggg 13860
   tgacggtgcc gaggatgacg atgagcgcat tgttagattt catacacggt gcctgactgc 13920
   gttagcaatt taactgtgat aaactaccgc attaaagctt gtcgacagcg ctacgcgc 13978
<210> 11
   <211> 13978
   <212> DNA
   <213> Artificial Sequence
<220>
   <223> pBR HIV NL4-3-Nef IRES-Cre
<400> 11
   gcgttggaag ggctaattca ctcccaaaga agacaagata tccttgatct gtggatctac       60
   cacacacaag gctacttccc tgattggcag aactacacac cagggccagg ggtcagatat      120
   ccactgacct ttggatggtg ctacaagcta gtaccagttg agccagataa ggtagaagag      180
   gccaataaag gagagaacac cagcttgtta caccctgtga gcctgcatgg aatggatgac      240
   cctgagagag aagtgttaga gtggaggttt gacagccgcc tagcatttca tcacgtggcc      300
   cgagagctgc atccggagta ctacaaagac tgctgacatc gagctttcta caagggactt      360
   tccgctgggg actttccagg gaggtgtggc ctgggcggga ctggggagtg gcgagccctc      420
   agatgctaca tataagcagc tgctttttgc ctgtactggg tctctctggt tagaccagat      480
   ctgagcctgg gagctctctg gctaactagg gaacccactg cttaagcctc aataaagctt      540
   gccttgagtg ctcaaagtag tgtgtgcccg tctgttgtgt gactctggta actagagatc      600
   cctcagaccc ttttagtcag tgtggaaaat ctctagcagt ggcgcccgaa cagggacttg      660
   aaagcgaaag taaagccaga ggagatctct cgacgcagga ctcggcttgc tgaagcgcgc      720
   acggcaagag gcgaggggcg gcgactggtg agtacgccaa aaattttgac tagcggaggc      780
   tagaaggaga gagatgggtg cgagagcgtc ggtattaagc gggggagaat tagataaatg 840
   ggaaaaaatt cggttaaggc cagggggaaa gaaacaatat aaactaaaac atatagtatg 900
   ggcaagcagg gagctagaac gattcgcagt taatcctggc cttttagaga catcagaagg 960
   ctgtagacaa atactgggac agctacaacc atcccttcag acaggatcag aagaacttag 1020
   atcattatat aatacaatag cagtcctcta ttgtgtgcat caaaggatag atgtaaaaga 1080
   caccaaggaa gccttagata agatagagga agagcaaaac aaaagtaaga aaaaggcaca 1140
   gcaagcagca gctgacacag gaaacaacag ccaggtcagc caaaattacc ctatagtgca 1200
   gaacctccag gggcaaatgg tacatcaggc catatcacct agaactttaa atgcatgggt 1260
   aaaagtagta gaagagaagg ctttcagccc agaagtaata cccatgtttt cagcattatc 1320
   agaaggagcc accccacaag atttaaatac catgctaaac acagtggggg gacatcaagc 1380
   agccatgcaa atgttaaaag agaccatcaa tgaggaagct gcagaatggg atagattgca 1440
   tccagtgcat gcagggccta ttgcaccagg ccagatgaga gaaccaaggg gaagtgacat 1500
   agcaggaact actagtaccc ttcaggaaca aataggatgg atgacacata atccacctat 1560
   cccagtagga gaaatctata aaagatggat aatcctggga ttaaataaaa tagtaagaat 1620
   gtatagccct accagcattc tggacataag acaaggacca aaggaaccct ttagagacta 1680
   tgtagaccga ttctataaaa ctctaagagc cgagcaagct tcacaagagg taaaaaattg 1740
   gatgacagaa accttgttgg tccaaaatgc gaacccagat tgtaagacta ttttaaaagc 1800
   attgggacca ggagcgacac tagaagaaat gatgacagca tgtcagggag tggggggacc 1860
   cggccataaa gcaagagttt tggctgaagc aatgagccaa gtaacaaatc cagctaccat 1920
   aatgatacag aaaggcaatt ttaggaacca aagaaagact gttaagtgtt tcaattgtgg 1980
   caaagaaggg cacatagcca aaaattgcag ggcccctagg aaaaagggct gttggaaatg 2040
   tggaaaggaa ggacaccaaa tgaaagattg tactgagaga caggctaatt ttttagggaa 2100
   gatctggcct tcccacaagg gaaggccagg gaattttctt cagagcagac cagagccaac 2160
   agccccacca gaagagagct tcaggtttgg ggaagagaca acaactccct ctcagaagca 2220
   ggagccgata gacaaggaac tgtatccttt agcttccctc agatcactct ttggcagcga 2280
   cccctcgtca caataaagat aggggggcaa ttaaaggaag ctctattaga tacaggagca 2340
   gatgatacag tattagaaga aatgaatttg ccaggaagat ggaaaccaaa aatgataggg 2400
   ggaattggag gttttatcaa agtaggacag tatgatcaga tactcataga aatctgcgga 2460
   cataaagcta taggtacagt attagtagga cctacacctg tcaacataat tggaagaaat 2520
   ctgttgactc agattggctg cactttaaat tttcccatta gtcctattga gactgtacca 2580
   gtaaaattaa agccaggaat ggatggccca aaagttaaac aatggccatt gacagaagaa 2640
   aaaataaaag cattagtaga aatttgtaca gaaatggaaa aggaaggaaa aatttcaaaa 2700
   attgggcctg aaaatccata caatactcca gtatttgcca taaagaaaaa agacagtact 2760
   aaatggagaa aattagtaga tttcagagaa cttaataaga gaactcaaga tttctgggaa 2820
   gttcaattag gaataccaca tcctgcaggg ttaaaacaga aaaaatcagt aacagtactg 2880
   gatgtgggcg atgcatattt ttcagttccc ttagataaag acttcaggaa gtatactgca 2940
   tttaccatac ctagtataaa caatgagaca ccagggatta gatatcagta caatgtgctt 3000
   ccacagggat ggaaaggatc accagcaata ttccagtgta gcatgacaaa aatcttagag 3060
   ccttttagaa aacaaaatcc agacatagtc atctatcaat acatggatga tttgtatgta 3120
   ggatctgact tagaaatagg gcagcataga acaaaaatag aggaactgag acaacatctg 3180
   ttgaggtggg gatttaccac accagacaaa aaacatcaga aagaacctcc attcctttgg 3240
   atgggttatg aactccatcc tgataaatgg acagtacagc ctatagtgct gccagaaaag 3300
   gacagctgga ctgtcaatga catacagaaa ttagtgggaa aattgaattg ggcaagtcag 3360
   atttatgcag ggattaaagt aaggcaatta tgtaaacttc ttaggggaac caaagcacta 3420
   acagaagtag taccactaac agaagaagca gagctagaac tggcagaaaa cagggagatt 3480
   ctaaaagaac cggtacatgg agtgtattat gacccatcaa aagacttaat agcagaaata 3540
   cagaagcagg ggcaaggcca atggacatat caaatttatc aagagccatt taaaaatctg 3600
   aaaacaggaa aatatgcaag aatgaagggt gcccacacta atgatgtgaa acaattaaca 3660
   gaggcagtac aaaaaatagc cacagaaagc atagtaatat ggggaaagac tcctaaattt 3720
   aaattaccca tacaaaagga aacatgggaa gcatggtgga cagagtattg gcaagccacc 3780
   tggattcctg agtgggagtt tgtcaatacc cctcccttag tgaagttatg gtaccagtta 3840
   gagaaagaac ccataatagg agcagaaact ttctatgtag atggggcagc caatagggaa 3900
   actaaattag gaaaagcagg atatgtaact gacagaggaa gacaaaaagt tgtcccccta 3960
   acggacacaa caaatcagaa gactgagtta caagcaattc atctagcttt gcaggattcg 4020
   ggattagaag taaacatagt gacagactca caatatgcat tgggaatcat tcaagcacaa 4080
   ccagataaga gtgaatcaga gttagtcagt caaataatag agcagttaat aaaaaaggaa 4140
   aaagtctacc tggcatgggt accagcacac aaaggaattg gaggaaatga acaagtagat 4200
   aaattggtca gtgctggaat caggaaagta ctatttttag atggaataga taaggcccaa 4260
   gaagaacatg agaaatatca cagtaattgg agagcaatgg ctagtgattt taacctacca 4320
   cctgtagtag caaaagaaat agtagccagc tgtgataaat gtcagctaaa aggggaagcc 4380
   atgcatggac aagtagactg tagcccagga atatggcagc tagattgtac acatttagaa 4440
   ggaaaagtta tcttggtagc agttcatgta gccagtggat atatagaagc agaagtaatt 4500
   ccagcagaga cagggcaaga aacagcatac ttcctcttaa aattagcagg aagatggcca 4560
   gtaaaaacag tacatacaga caatggcagc aatttcacca gtactacagt taaggccgcc 4620
   tgttggtggg cggggatcaa gcaggaattt ggcattccct acaatcccca aagtcaagga 4680
   gtaatagaat ctatgaataa agaattaaag aaaattatag gacaggtaag agatcaggct 4740
   gaacatctta agacagcagt acaaatggca gtattcatcc acaattttaa aagaaaaggg 4800
   gggattgggg ggtacagtgc aggggaaaga atagtagaca taatagcaac agacatacaa 4860
   actaaagaat tacaaaaaca aattacaaaa attcaaaatt ttcgggttta ttacagggac 4920
   agcagagatc cagtttggaa aggaccagca aagctcctct ggaaaggtga aggggcagta 4980
   gtaatacaag ataatagtga cataaaagta gtgccaagaa gaaaagcaaa gatcatcagg 5040
   gattatggaa aacagatggc aggtgatgat tgtgtggcaa gtagacagga tgaggattaa 5100
   cacatggaaa agattagtaa aacaccatat gtatatttca aggaaagcta aggactggtt 5160
   ttatagacat cactatgaaa gtactaatcc aaaaataagt tcagaagtac acatcccact 5220
   aggggatgct aaattagtaa taacaacata ttggggtctg catacaggag aaagagactg 5280
   gcatttgggt cagggagtct ccatagaatg gaggaaaaag agatatagca cacaagtaga 5340
   ccctgaccta gcagaccaac taattcatct gcactatttt gattgttttt cagaatctgc 5400
   tataagaaat accatattag gacgtatagt tagtcctagg tgtgaatatc aagcaggaca 5460
   taacaaggta ggatctctac agtacttggc actagcagca ttaataaaac caaaacagat 5520
   aaagccacct ttgcctagtg ttaggaaact gacagaggac agatggaaca agccccagaa 5580
   gaccaagggc cacagaggga gccatacaat gaatggacac tagagctttt agaggaactt 5640
   aagagtgaag ctgttagaca ttttcctagg atatggctcc ataacttagg acaacatatc 5700
   tatgaaactt acggggatac ttgggcagga gtggaagcca taataagaat tctgcaacaa 5760
   ctgctgttta tccatttcag aattgggtgt cgacatagca gaataggcgt tactcgacag 5820
   aggagagcaa gaaatggagc cagtagatcc tagactagag ccctggaagc atccaggaag 5880
   tcagcctaaa actgcttgta ccaattgcta ttgtaaaaag tgttgctttc attgccaagt 5940
   ttgtttcatg acaaaagcct taggcatctc ctatggcagg aagaagcgga gacagcgacg 6000
   aagagctcat cagaacagtc agactcatca agcttctcta tcaaagcagt aagtagtaca 6060
   tgtaatgcaa cctataatag tagcaatagt agcattagta gtagcaataa taatagcaat 6120
   agttgtgtgg tccatagtaa tcatagaata taggaaaata ttaagacaaa gaaaaataga 6180
   caggttaatt gatagactaa tagaaagagc agaagacagt ggcaatgaga gtgaaggaga 6240
   agtatcagca cttgtggaga tgggggtgga aatggggcac catgctcctt gggatattga 6300
   tgatctgtag tgctacagaa aaattgtggg tcacagtcta ttatggggta cctgtgtgga 6360
   aggaagcaac caccactcta ttttgtgcat cagatgctaa agcatatgat acagaggtac 6420
   ataatgtttg ggccacacat gcctgtgtac ccacagaccc caacccacaa gaagtagtat 6480
   tggtaaatgt gacagaaaat tttaacatgt ggaaaaatga catggtagaa cagatgcatg 6540
   aggatataat cagtttatgg gatcaaagcc taaagccatg tgtaaaatta accccactct 6600
   gtgttagttt aaagtgcact gatttgaaga atgatactaa taccaatagt agtagcggga 6660
   gaatgataat ggagaaagga gagataaaaa actgctcttt caatatcagc acaagcataa 6720
   gagataaggt gcagaaagaa tatgcattct tttataaact tgatatagta ccaatagata 6780
   ataccagcta taggttgata agttgtaaca cctcagtcat tacacaggcc tgtccaaagg 6840
   tatcctttga gccaattccc atacattatt gtgccccggc tggttttgcg attctaaaat 6900
   gtaataataa gacgttcaat ggaacaggac catgtacaaa tgtcagcaca gtacaatgta 6960
   cacatggaat caggccagta gtatcaactc aactgctgtt aaatggcagt ctagcagaag 7020
   aagatgtagt aattagatct gccaatttca cagacaatgc taaaaccata atagtacagc 7080
   tgaacacatc tgtagaaatt aattgtacaa gacccaacaa caatacaaga aaaagtatcc 7140
   gtatccagag gggaccaggg agagcatttg ttacaatagg aaaaatagga aatatgagac 7200
   aagcacattg taacattagt agagcaaaat ggaatgccac tttaaaacag atagctagca 7260
   aattaagaga acaatttgga aataataaaa caataatctt taagcaatcc tcaggagggg 7320
   acccagaaat tgtaacgcac agttttaatt gtggagggga atttttctac tgtaattcaa 7380
   cacaactgtt taatagtact tggtttaata gtacttggag tactgaaggg tcaaataaca 7440
   ctgaaggaag tgacacaatc acactcccat gcagaataaa acaatttata aacatgtggc 7500
   aggaagtagg aaaagcaatg tatgcccctc ccatcagtgg acaaattaga tgttcatcaa 7560
   atattactgg gctgctatta acaagagatg gtggtaataa caacaatggg tccgagatct 7620
   tcagacctgg aggaggcgat atgagggaca attggagaag tgaattatat aaatataaag 7680
   tagtaaaaat tgaaccatta ggagtagcac ccaccaaggc aaagagaaga gtggtgcaga 7740
   gagaaaaaag agcagtggga ataggagctt tgttccttgg gttcttggga gcagcaggaa 7800
   gcactatggg cgcagcgtca atgacgctga cggtacaggc cagacaatta ttgtctgata 7860
   tagtgcagca gcagaacaat ttgctgaggg ctattgaggc gcaacagcat ctgttgcaac 7920
   tcacagtctg gggcatcaaa cagctccagg caagaatcct ggctgtggaa agatacctaa 7980
   aggatcaaca gctcctgggg atttggggtt gctctggaaa actcatttgc accactgctg 8040
   tgccttggaa tgctagttgg agtaataaat ctctggaaca gatttggaat aacatgacct 8100
   ggatggagtg ggacagagaa attaacaatt acacaagctt aatacactcc ttaattgaag 8160
   aatcgcaaaa ccagcaagaa aagaatgaac aagaattatt ggaattagat aaatgggcaa 8220
   gtttgtggaa ttggtttaac ataacaaatt ggctgtggta tataaaatta ttcataatga 8280
   tagtaggagg cttggtaggt ttaagaatag tttttgctgt actttctata gtgaatagag 8340
   ttaggcaggg atattcacca ttatcgtttc agacccacct cccaatcccg aggggacccg 8400
   acaggcccga aggaatagaa gaagaaggtg gagagagaga cagagacaga tccattcgat 8460
   tagtgaacgg atccttagca cttatctggg acgatctgcg gagcctgtgc ctcttcagct 8520
   accaccgctt gagagactta ctcttgattg taacgaggat tgtggaactt ctgggacgca 8580
   gggggtggga agccctcaaa tattggtgga atctcctaca gtattggagt caggaactaa 8640
   agaatagtgc tgttaacttg ctcaatgcca cagccatagc agtagctgag gggacagata 8700
   gggttataga agtattacaa gcagcttata gagctattcg ccacatacct agaagaataa 8760
   gacagggctt ggaaaggatt ttgctataag atgggtggca agtggtcaaa aagtagtgtg 8820
   attggatggc ctgctgtaag ggaaagaatg agacgagctg agccagcagc agatggggtg 8880
   ggagcagtat ctcgagacct agaaaaacat ggagcaatca caagtagcaa tacagcagct 8940
   aacaatgctg cttgtgcctg gctagaagca caagaggagg aagaggtggg ttttccagtc 9000
   acacctcagg tacctttaag accaatgact tacaaggcag ctgtagatct gagccatttc 9060
   ctgaaggaga agggaggcct tgagggcctg attcacagcc agcggcgcca agacatcctt 9120
   gacttgtgga tttaccatac acaggggtac ttccccgact ggcagaacta tacaccagga 9180
   cccggagtga gataccccct gaccttcgga tggtgttaca aactggttcc agtggagcct 9240
   gataaggtcg aagaggcaaa caaaggcgag aatacatctt tgctgcatcc tgtgtcactg 9300
   cacgggatgg acgaccctga gcgggaggtg cttgagtgga ggttcgactc tcgactggcc 9360
   tttcaccacg tagcaaggga gctgcaccct gagtatttta aaaattgttg aacgcgtcgg 9420
   atcccggagt actacaaaga ctgctgacgc gaattccgcc ccccccccta acgttactgg 9480
   ccgaagccgc ttggaataag gccggtgtgc gtttgtctat atgttatttt ccaccatatt 9540
   gccgtctttt ggcaatgtga gggcccggaa acctggccct gtcttcttga cgagcattcc 9600
   taggggtctt tcccctctcg ccaaaggaat gcaaggtctg ttgaatgtcg tgaaggaagc 9660
   agttcctctg gaagcttctt gaagacaaac aacgtctgta gcgacccttt gcaggcagcg 9720
   gaacccccca cctggcgaca ggtgcctctg cggccaaaag ccacgtgtat aagatacacc 9780
   tgcaaaggcg gcacaacccc agtgccacgt tgtgagttgg atagttgtgg aaagagtcaa 9840
   atggctctcc tcaagcgtat tcaacaaggg gctgaaggat gcccagaagg taccccattg 9900
   tatgggatct gatctggggc ctcggtgcac atgctttaca tgtgtttagt cgaggttaaa 9960
   aaacgtctag gccccccgaa ccacggggac gtggttttcc tttgaaaaac acgatgataa 10020
   taccatggtg cccaagaaga agaggaaagt ctccaacctg ctgactgtgc accaaaacct 10080
   gcctgccctc cctgtggatg ccacctctga tgaagtcagg aagaacctga tggacatgtt 10140
   cagggacagg caggccttct ctgaacacac ctggaagatg ctcctgtctg tgtgcagatc 10200
   ctgggctgcc tggtgcaagc tgaacaacag gaaatggttc cctgctgaac ctgaggatgt 10260
   gagggactac ctcctgtacc tgcaagccag aggcctggct gtgaagacca tccaacagca 10320
   cctgggccag ctcaacatgc tgcacaggag atctggcctg cctcgccctt ctgactccaa 10380
   tgctgtgtcc ctggtgatga ggagaatcag aaaggagaat gtggatgctg gggagagagc 10440
   caagcaggcc ctggcctttg aacgcactga ctttgaccaa gtcagatccc tgatggagaa 10500
   ctctgacaga tgccaggaca tcaggaacct ggccttcctg ggcattgcct acaacaccct 10560
   gctgcgcatt gccgaaattg ccagaatcag agtgaaggac atctcccgca ccgatggtgg 10620
   gagaatgctg atccacattg gcaggaccaa gaccctggtg tccacagctg gtgtggagaa 10680
   ggccctgtcc ctgggggtta ccaagctggt ggagagatgg atctctgtgt ctggtgtggc 10740
   tgatgacccc aacaactacc tgttctgccg ggtcagaaag aatggtgtgg ctgccccttc 10800
   tgccacctcc caactgtcca cccgggccct ggaagggatc tttgaggcca cccaccgcct 10860
   gatctatggt gccaaggatg actctgggca gagatacctg gcctggtctg gccactctgc 10920
   cagagtgggt gctgccaggg acatggccag ggctggtgtg tccatccctg aaatcatgca 10980
   ggctggtggc tggaccaatg tgaacattgt gatgaactac atcagaaacc tggactctga 11040
   gactggggcc atggtgaggc tgctcgagga tggggactga cccgggtagc cactttttaa 11100
   aagaaaaggg gggactggaa gggctaattc actcccaaag aagacaagat atcccatccg 11160
   gagtacttca agaactgctg acatcgagct tgctacaagg gactttccgc tggggacttt 11220
   ccagggaggc gtggcctggg cgggactggg gagtggcgag ccctcagatg ctgcatataa 11280
   gcagctgctt tttgcctgta ctgggtctct ctggttagac cagatctgag cctgggagct 11340
   ctctggctaa ctagggaacc cactgcttaa gcctcaataa agcttgcctt gagtgcttca 11400
   agtagtgtgt gcccgtctgt tgtgtgactc tggtaactag agatccctca gaccctttta 11460
   gtcagtgtgg aaaatctcta gcagttctag agcggccgct cgcgaattct tgaagacgaa 11520
   agggcctcgt gatacgccta tttttatagg ttaatgtcat gataataatg gtttcttaga 11580
   cgtcaggtgg cacttttcgg ggaaatgtgc gcggaacccc tatttgttta tttttctaaa 11640
   tacattcaaa tatgtatccg ctcatgagac aataaccctg ataaatgctt caataatatt 11700
   gaaaaaggaa gagtatgagt attcaacatt tccgtgtcgc ccttattccc ttttttgcgg 11760
   cattttgcct tcctgttttt gctcacccag aaacgctggt gaaagtaaaa gatgctgaag 11820
   atcagttggg tgcacgagtg ggttacatcg aactggatct caacagcggt aagatccttg 11880
   agagttttcg ccccgaagaa cgttttccaa tgatgagcac ttttaaagtt ctgctatgtg 11940
   gcgcggtatt atcccgtgtt gacgccgggc aagagcaact cggtcgccgc atacactatt 12000
   ctcagaatga cttggttgag tactcaccag tcacagaaaa gcatcttacg gatggcatga 12060
   cagtaagaga attatgcagt gctgccataa ccatgagtga taacactgcg gccaacttac 12120
   ttctgacaac gatcggagga ccgaaggagc taaccgcttt tttgcacaac atgggggatc 12180
   atgtaactcg ccttgatcgt tgggaaccgg agctgaatga agccatacca aacgacgagc 12240
   gtgacaccac gatgcctgca gcaatggcaa caacgttgcg caaactatta actggcgaac 12300
   tacttactct agcttcccgg caacaattaa tagactggat ggaggcggat aaagttgcag 12360
   gaccacttct gcgctcggcc cttccggctg gctggtttat tgctgataaa tctggagccg 12420
   gtgagcgtgg gtctcgcggt atcattgcag cactggggcc agatggtaag ccctcccgta 12480
   tcgtagttat ctacacgacg gggagtcagg caactatgga tgaacgaaat agacagatcg 12540
   ctgagatagg tgcctcactg attaagcatt ggtaactgtc agaccaagtt tactcatata 12600
   tactttagat tgatttaaaa cttcattttt aatttaaaag gatctaggtg aagatccttt 12660
   ttgataatct catgaccaaa atcccttaac gtgagttttc gttccactga gcgtcagacc 12720
   ccgtagaaaa gatcaaagga tcttcttgag atcctttttt tctgcgcgta atctgctgct 12780
   tgcaaacaaa aaaaccaccg ctaccagcgg tggtttgttt gccggatcaa gagctaccaa 12840
   ctctttttcc gaaggtaact ggcttcagca gagcgcagat accaaatact gtccttctag 12900
   tgtagccgta gttaggccac cacttcaaga actctgtagc accgcctaca tacctcgctc 12960
   tgctaatcct gttaccagtg gctgctgcca gtggcgataa gtcgtgtctt accgggttgg 13020
   actcaagacg atagttaccg gataaggcgc agcggtcggg ctgaacgggg ggttcgtgca 13080
   cacagcccag cttggagcga acgacctaca ccgaactgag atacctacag cgtgagctat 13140
   gagaaagcgc cacgcttccc gaagggagaa aggcggacag gtatccggta agcggcaggg 13200
   tcggaacagg agagcgcacg agggagcttc cagggggaaa cgcctggtat ctttatagtc 13260
   ctgtcgggtt tcgccacctc tgacttgagc gtcgattttt gtgatgctcg tcaggggggc 13320
   ggagcctatg gaaaaacgcc agcaacgcgg cctttttacg gttcctggcc ttttgctggc 13380
   cttttgctca catgttcttt cctgcgttat cccctgattc tgtggataac cgtattaccg 13440
   cctttgagtg agctgatacc gctcgccgca gccgaacgac cgagcgcagc gagtcagtga 13500
   gcgaggaagc ggaagagcgc ctgatgcggt attttctcct tacgcatctg tgcggtattt 13560
   cacaccgcat atggtgcact ctcagtacaa tctgctctga tgccgcatag ttaagccagt 13620
   atacactccg ctatcgctac gtgactgggt catggctgcg ccccgacacc cgccaacacc 13680
   cgctgacgcg ccctgacggg cttgtctgct cccggcatcc gcttacagac aagctgtgac 13740
   cgtctccggg agctgcatgt gtcagaggtt ttcaccgtca tcaccgaaac gcgcgaggca 13800
   gatcccgcaa gaggcccggc agtaccggca taaccaagcc tatgcctaca gcatccaggg 13860
   tgacggtgcc gaggatgacg atgagcgcat tgttagattt catacacggt gcctgactgc 13920
   gttagcaatt taactgtgat aaactaccgc attaaagctt gtcgacagcg ctacgcgc 13978
<210> 12
   <211> 14057
   <212> DNA
   <213> Artificial Sequence
<220>
   <223> pBR-HIV NL4-3-Nef-CMV-Cre
<400> 12
   gcgttggaag ggctaattca ctcccaaaga agacaagata tccttgatct gtggatctac      60
   cacacacaag gctacttccc tgattggcag aactacacac cagggccagg ggtcagatat     120
   ccactgacct ttggatggtg ctacaagcta gtaccagttg agccagataa ggtagaagag     180
   gccaataaag gagagaacac cagcttgtta caccctgtga gcctgcatgg aatggatgac     240
   cctgagagag aagtgttaga gtggaggttt gacagccgcc tagcatttca tcacgtggcc     300
   cgagagctgc atccggagta ctacaaagac tgctgacatc gagctttcta caagggactt     360
   tccgctgggg actttccagg gaggtgtggc ctgggcggga ctggggagtg gcgagccctc     420
   agatgctaca tataagcagc tgctttttgc ctgtactggg tctctctggt tagaccagat     480
   ctgagcctgg gagctctctg gctaactagg gaacccactg cttaagcctc aataaagctt     540
   gccttgagtg ctcaaagtag tgtgtgcccg tctgttgtgt gactctggta actagagatc     600
   cctcagaccc ttttagtcag tgtggaaaat ctctagcagt ggcgcccgaa cagggacttg     660
   aaagcgaaag taaagccaga ggagatctct cgacgcagga ctcggcttgc tgaagcgcgc     720
   acggcaagag gcgaggggcg gcgactggtg agtacgccaa aaattttgac tagcggaggc     780
   tagaaggaga gagatgggtg cgagagcgtc ggtattaagc gggggagaat tagataaatg     840
   ggaaaaaatt cggttaaggc cagggggaaa gaaacaatat aaactaaaac atatagtatg     900
   ggcaagcagg gagctagaac gattcgcagt taatcctggc cttttagaga catcagaagg     960
   ctgtagacaa atactgggac agctacaacc atcccttcag acaggatcag aagaacttag    1020
   atcattatat aatacaatag cagtcctcta ttgtgtgcat caaaggatag atgtaaaaga    1080
   caccaaggaa gccttagata agatagagga agagcaaaac aaaagtaaga aaaaggcaca    1140
   gcaagcagca gctgacacag gaaacaacag ccaggtcagc caaaattacc ctatagtgca    1200
   gaacctccag gggcaaatgg tacatcaggc catatcacct agaactttaa atgcatgggt    1260
   aaaagtagta gaagagaagg ctttcagccc agaagtaata cccatgtttt cagcattatc    1320
   agaaggagcc accccacaag atttaaatac catgctaaac acagtggggg gacatcaagc    1380
   agccatgcaa atgttaaaag agaccatcaa tgaggaagct gcagaatggg atagattgca    1440
   tccagtgcat gcagggccta ttgcaccagg ccagatgaga gaaccaaggg gaagtgacat    1500
   agcaggaact actagtaccc ttcaggaaca aataggatgg atgacacata atccacctat    1560
   cccagtagga gaaatctata aaagatggat aatcctggga ttaaataaaa tagtaagaat    1620
   gtatagccct accagcattc tggacataag acaaggacca aaggaaccct ttagagacta 1680
   tgtagaccga ttctataaaa ctctaagagc cgagcaagct tcacaagagg taaaaaattg 1740
   gatgacagaa accttgttgg tccaaaatgc gaacccagat tgtaagacta ttttaaaagc 1800
   attgggacca ggagcgacac tagaagaaat gatgacagca tgtcagggag tggggggacc 1860
   cggccataaa gcaagagttt tggctgaagc aatgagccaa gtaacaaatc cagctaccat 1920
   aatgatacag aaaggcaatt ttaggaacca aagaaagact gttaagtgtt tcaattgtgg 1980
   caaagaaggg cacatagcca aaaattgcag ggcccctagg aaaaagggct gttggaaatg 2040
   tggaaaggaa ggacaccaaa tgaaagattg tactgagaga caggctaatt ttttagggaa 2100
   gatctggcct tcccacaagg gaaggccagg gaattttctt cagagcagac cagagccaac 2160
   agccccacca gaagagagct tcaggtttgg ggaagagaca acaactccct ctcagaagca 2220
   ggagccgata gacaaggaac tgtatccttt agcttccctc agatcactct ttggcagcga 2280
   cccctcgtca caataaagat aggggggcaa ttaaaggaag ctctattaga tacaggagca 2340
   gatgatacag tattagaaga aatgaatttg ccaggaagat ggaaaccaaa aatgataggg 2400
   ggaattggag gttttatcaa agtaggacag tatgatcaga tactcataga aatctgcgga 2460
   cataaagcta taggtacagt attagtagga cctacacctg tcaacataat tggaagaaat 2520
   ctgttgactc agattggctg cactttaaat tttcccatta gtcctattga gactgtacca 2580
   gtaaaattaa agccaggaat ggatggccca aaagttaaac aatggccatt gacagaagaa 2640
   aaaataaaag cattagtaga aatttgtaca gaaatggaaa aggaaggaaa aatttcaaaa 2700
   attgggcctg aaaatccata caatactcca gtatttgcca taaagaaaaa agacagtact 2760
   aaatggagaa aattagtaga tttcagagaa cttaataaga gaactcaaga tttctgggaa 2820
   gttcaattag gaataccaca tcctgcaggg ttaaaacaga aaaaatcagt aacagtactg 2880
   gatgtgggcg atgcatattt ttcagttccc ttagataaag acttcaggaa gtatactgca 2940
   tttaccatac ctagtataaa caatgagaca ccagggatta gatatcagta caatgtgctt 3000
   ccacagggat ggaaaggatc accagcaata ttccagtgta gcatgacaaa aatcttagag 3060
   ccttttagaa aacaaaatcc agacatagtc atctatcaat acatggatga tttgtatgta 3120
   ggatctgact tagaaatagg gcagcataga acaaaaatag aggaactgag acaacatctg 3180
   ttgaggtggg gatttaccac accagacaaa aaacatcaga aagaacctcc attcctttgg 3240
   atgggttatg aactccatcc tgataaatgg acagtacagc ctatagtgct gccagaaaag 3300
   gacagctgga ctgtcaatga catacagaaa ttagtgggaa aattgaattg ggcaagtcag 3360
   atttatgcag ggattaaagt aaggcaatta tgtaaacttc ttaggggaac caaagcacta 3420
   acagaagtag taccactaac agaagaagca gagctagaac tggcagaaaa cagggagatt 3480
   ctaaaagaac cggtacatgg agtgtattat gacccatcaa aagacttaat agcagaaata 3540
   cagaagcagg ggcaaggcca atggacatat caaatttatc aagagccatt taaaaatctg 3600
   aaaacaggaa aatatgcaag aatgaagggt gcccacacta atgatgtgaa acaattaaca 3660
   gaggcagtac aaaaaatagc cacagaaagc atagtaatat ggggaaagac tcctaaattt 3720
   aaattaccca tacaaaagga aacatgggaa gcatggtgga cagagtattg gcaagccacc 3780
   tggattcctg agtgggagtt tgtcaatacc cctcccttag tgaagttatg gtaccagtta 3840
   gagaaagaac ccataatagg agcagaaact ttctatgtag atggggcagc caatagggaa 3900
   actaaattag gaaaagcagg atatgtaact gacagaggaa gacaaaaagt tgtcccccta 3960
   acggacacaa caaatcagaa gactgagtta caagcaattc atctagcttt gcaggattcg 4020
   ggattagaag taaacatagt gacagactca caatatgcat tgggaatcat tcaagcacaa 4080
   ccagataaga gtgaatcaga gttagtcagt caaataatag agcagttaat aaaaaaggaa 4140
   aaagtctacc tggcatgggt accagcacac aaaggaattg gaggaaatga acaagtagat 4200
   aaattggtca gtgctggaat caggaaagta ctatttttag atggaataga taaggcccaa 4260
   gaagaacatg agaaatatca cagtaattgg agagcaatgg ctagtgattt taacctacca 4320
   cctgtagtag caaaagaaat agtagccagc tgtgataaat gtcagctaaa aggggaagcc 4380
   atgcatggac aagtagactg tagcccagga atatggcagc tagattgtac acatttagaa 4440
   ggaaaagtta tcttggtagc agttcatgta gccagtggat atatagaagc agaagtaatt 4500
   ccagcagaga cagggcaaga aacagcatac ttcctcttaa aattagcagg aagatggcca 4560
   gtaaaaacag tacatacaga caatggcagc aatttcacca gtactacagt taaggccgcc 4620
   tgttggtggg cggggatcaa gcaggaattt ggcattccct acaatcccca aagtcaagga 4680
   gtaatagaat ctatgaataa agaattaaag aaaattatag gacaggtaag agatcaggct 4740
   gaacatctta agacagcagt acaaatggca gtattcatcc acaattttaa aagaaaaggg 4800
   gggattgggg ggtacagtgc aggggaaaga atagtagaca taatagcaac agacatacaa 4860
   actaaagaat tacaaaaaca aattacaaaa attcaaaatt ttcgggttta ttacagggac 4920
   agcagagatc cagtttggaa aggaccagca aagctcctct ggaaaggtga aggggcagta 4980
   gtaatacaag ataatagtga cataaaagta gtgccaagaa gaaaagcaaa gatcatcagg 5040
   gattatggaa aacagatggc aggtgatgat tgtgtggcaa gtagacagga tgaggattaa 5100
   cacatggaaa agattagtaa aacaccatat gtatatttca aggaaagcta aggactggtt 5160
   ttatagacat cactatgaaa gtactaatcc aaaaataagt tcagaagtac acatcccact 5220
   aggggatgct aaattagtaa taacaacata ttggggtctg catacaggag aaagagactg 5280
   gcatttgggt cagggagtct ccatagaatg gaggaaaaag agatatagca cacaagtaga 5340
   ccctgaccta gcagaccaac taattcatct gcactatttt gattgttttt cagaatctgc 5400
   tataagaaat accatattag gacgtatagt tagtcctagg tgtgaatatc aagcaggaca 5460
   taacaaggta ggatctctac agtacttggc actagcagca ttaataaaac caaaacagat 5520
   aaagccacct ttgcctagtg ttaggaaact gacagaggac agatggaaca agccccagaa 5580
   gaccaagggc cacagaggga gccatacaat gaatggacac tagagctttt agaggaactt 5640
   aagagtgaag ctgttagaca ttttcctagg atatggctcc ataacttagg acaacatatc 5700
   tatgaaactt acggggatac ttgggcagga gtggaagcca taataagaat tctgcaacaa 5760
   ctgctgttta tccatttcag aattgggtgt cgacatagca gaataggcgt tactcgacag 5820
   aggagagcaa gaaatggagc cagtagatcc tagactagag ccctggaagc atccaggaag 5880
   tcagcctaaa actgcttgta ccaattgcta ttgtaaaaag tgttgctttc attgccaagt 5940
   ttgtttcatg acaaaagcct taggcatctc ctatggcagg aagaagcgga gacagcgacg 6000
   aagagctcat cagaacagtc agactcatca agcttctcta tcaaagcagt aagtagtaca 6060
   tgtaatgcaa cctataatag tagcaatagt agcattagta gtagcaataa taatagcaat 6120
   agttgtgtgg tccatagtaa tcatagaata taggaaaata ttaagacaaa gaaaaataga 6180
   caggttaatt gatagactaa tagaaagagc agaagacagt ggcaatgaga gtgaaggaga 6240
   agtatcagca cttgtggaga tgggggtgga aatggggcac catgctcctt gggatattga 6300
   tgatctgtag tgctacagaa aaattgtggg tcacagtcta ttatggggta cctgtgtgga 6360
   aggaagcaac caccactcta ttttgtgcat cagatgctaa agcatatgat acagaggtac 6420
   ataatgtttg ggccacacat gcctgtgtac ccacagaccc caacccacaa gaagtagtat 6480
   tggtaaatgt gacagaaaat tttaacatgt ggaaaaatga catggtagaa cagatgcatg 6540
   aggatataat cagtttatgg gatcaaagcc taaagccatg tgtaaaatta accccactct 6600
   gtgttagttt aaagtgcact gatttgaaga atgatactaa taccaatagt agtagcggga 6660
   gaatgataat ggagaaagga gagataaaaa actgctcttt caatatcagc acaagcataa 6720
   gagataaggt gcagaaagaa tatgcattct tttataaact tgatatagta ccaatagata 6780
   ataccagcta taggttgata agttgtaaca cctcagtcat tacacaggcc tgtccaaagg 6840
   tatcctttga gccaattccc atacattatt gtgccccggc tggttttgcg attctaaaat 6900
   gtaataataa gacgttcaat ggaacaggac catgtacaaa tgtcagcaca gtacaatgta 6960
   cacatggaat caggccagta gtatcaactc aactgctgtt aaatggcagt ctagcagaag 7020
   aagatgtagt aattagatct gccaatttca cagacaatgc taaaaccata atagtacagc 7080
   tgaacacatc tgtagaaatt aattgtacaa gacccaacaa caatacaaga aaaagtatcc 7140
   gtatccagag gggaccaggg agagcatttg ttacaatagg aaaaatagga aatatgagac 7200
   aagcacattg taacattagt agagcaaaat ggaatgccac tttaaaacag atagctagca 7260
   aattaagaga acaatttgga aataataaaa caataatctt taagcaatcc tcaggagggg     7320
   acccagaaat tgtaacgcac agttttaatt gtggagggga atttttctac tgtaattcaa      7380
   cacaactgtt taatagtact tggtttaata gtacttggag tactgaaggg tcaaataaca 7440
   ctgaaggaag tgacacaatc acactcccat gcagaataaa acaatttata aacatgtggc 7500
   aggaagtagg aaaagcaatg tatgcccctc ccatcagtgg acaaattaga tgttcatcaa 7560
   atattactgg gctgctatta acaagagatg gtggtaataa caacaatggg tccgagatct 7620
   tcagacctgg aggaggcgat atgagggaca attggagaag tgaattatat aaatataaag 7680
   tagtaaaaat tgaaccatta ggagtagcac ccaccaaggc aaagagaaga gtggtgcaga 7740
   gagaaaaaag agcagtggga ataggagctt tgttccttgg gttcttggga gcagcaggaa 7800
   gcactatggg cgcagcgtca atgacgctga cggtacaggc cagacaatta ttgtctgata 7860
   tagtgcagca gcagaacaat ttgctgaggg ctattgaggc gcaacagcat ctgttgcaac 7920
   tcacagtctg gggcatcaaa cagctccagg caagaatcct ggctgtggaa agatacctaa 7980
   aggatcaaca gctcctgggg atttggggtt gctctggaaa actcatttgc accactgctg 8040
   tgccttggaa tgctagttgg agtaataaat ctctggaaca gatttggaat aacatgacct 8100
   ggatggagtg ggacagagaa attaacaatt acacaagctt aatacactcc ttaattgaag 8160
   aatcgcaaaa ccagcaagaa aagaatgaac aagaattatt ggaattagat aaatgggcaa 8220
   gtttgtggaa ttggtttaac ataacaaatt ggctgtggta tataaaatta ttcataatga 8280
   tagtaggagg cttggtaggt ttaagaatag tttttgctgt actttctata gtgaatagag 8340
   ttaggcaggg atattcacca ttatcgtttc agacccacct cccaatcccg aggggacccg 8400
   acaggcccga aggaatagaa gaagaaggtg gagagagaga cagagacaga tccattcgat 8460
   tagtgaacgg atccttagca cttatctggg acgatctgcg gagcctgtgc ctcttcagct 8520
   accaccgctt gagagactta ctcttgattg taacgaggat tgtggaactt ctgggacgca 8580
   gggggtggga agccctcaaa tattggtgga atctcctaca gtattggagt caggaactaa 8640
   agaatagtgc tgttaacttg ctcaatgcca cagccatagc agtagctgag gggacagata 8700
   gggttataga agtattacaa gcagcttata gagctattcg ccacatacct agaagaataa 8760
   gacagggctt ggaaaggatt ttgctataag atgggtggca agtggtcaaa aagtagtgtg 8820
   attggatggc ctgctgtaag ggaaagaatg agacgagctg agccagcagc agatggggtg 8880
   ggagcagtat ctcgagacct agaaaaacat ggagcaatca caagtagcaa tacagcagct 8940
   aacaatgctg cttgtgcctg gctagaagca caagaggagg aagaggtggg ttttccagtc 9000
   acacctcagg tacctttaag accaatgact tacaaggcag ctgtagatct tagccacttt 9060
   ttaaaagaaa aggggggact ggaagggcta attcactccc aaagaaggca agatatcctt 9120
   gatctgtgga tctaccacac acaaggctac ttccctgatt ggcagaacta cacaccaggg 9180
   ccaggggtca gatatccact gacctttgga tggtgctaca agctagtacc agctgagcca 9240
   gataaggtag aagaggccaa taaaggagag aacaccagct tgttacaccc tgtgagcctg 9300
   catggaatgg atgaccctga gagagaagtg ttagagtgga ggtttgacag ccgcctagca 9360
   tttcatcacg tggcccgaga gctgcatccg gagtacttca agaactgctg aacgcgtatt 9420
   gcattactag ttattaatag taatcaatta cggggtcatt agttcatagc ccatatatgg 9480
   agttccgcgt tacataactt acggtaaatg gcccgcctgg ctgaccgccc aacgaccccc 9540
   gcccattgac gtcaataatg acgtatgttc ccatagtaac gccaataggg actttccatt 9600
   gacgtcaatg ggtggagtat ttacggtaaa ctgcccactt ggcagtacat caagtgtatc 9660
   atatgccaag tccgccccct attgacgtca atgacggtaa atggcccgcc tggcattatg 9720
   cccagtacat gaccttacgg gactttccta cttggcagta catctacgta ttagtcatcg 9780
   ctattaccat ggtgatgcgg ttttggcagt acaccaatgg gcgtggatag cggtttgact 9840
   cacggggatt tccaagtctc caccccattg acgtcaatgg gagtttgttt tggcaccaaa 9900
   atcaacggga ctttccaaaa tgtcgtaata accccgcccc gttgacgcaa atgggcggta 9960
   ggcgtgtacg gtgggaggtc tatataagca gagctcgttt agtgaaccgt cagaattgtt 10020
   tttattttta attttctttc aaatacttcc atcgaattca gatctggtac cacgcgtacc 10080
   atggtgccca agaagaagag gaaagtctcc aacctgctga ctgtgcacca aaacctgcct 10140
   gccctccctg tggatgccac ctctgatgaa gtcaggaaga acctgatgga catgttcagg 10200
   gacaggcagg ccttctctga acacacctgg aagatgctcc tgtctgtgtg cagatcctgg 10260
   gctgcctggt gcaagctgaa caacaggaaa tggttccctg ctgaacctga ggatgtgagg 10320
   gactacctcc tgtacctgca agccagaggc ctggctgtga agaccatcca acagcacctg 10380
   ggccagctca acatgctgca caggagatct ggcctgcctc gcccttctga ctccaatgct 10440
   gtgtccctgg tgatgaggag aatcagaaag gagaatgtgg atgctgggga gagagccaag 10500
   caggccctgg cctttgaacg cactgacttt gaccaagtca gatccctgat ggagaactct 10560
   gacagatgcc aggacatcag gaacctggcc ttcctgggca ttgcctacaa caccctgctg 10620
   cgcattgccg aaattgccag aatcagagtg aaggacatct cccgcaccga tggtgggaga 10680
   atgctgatcc acattggcag gaccaagacc ctggtgtcca cagctggtgt ggagaaggcc 10740
   ctgtccctgg gggttaccaa gctggtggag agatggatct ctgtgtctgg tgtggctgat 10800
   gaccccaaca actacctgtt ctgccgggtc agaaagaatg gtgtggctgc cccttctgcc 10860
   acctcccaac tgtccacccg ggccctggaa gggatctttg aggccaccca ccgcctgatc 10920
   tatggtgcca aggatgactc tgggcagaga tacctggcct ggtctggcca ctctgccaga 10980
   gtgggtgctg ccagggacat ggccagggct ggtgtgtcca tccctgaaat catgcaggct 11040
   ggtggctgga ccaatgtgaa catagtgatg aactacatca gaaacctgga ctctgagact 11100
   ggggccatgg tgaggctgct cgaggatggg gacggtggtg gtggcaagct ttgacgcatc 11160
   ccgggtagcc actttttaaa agaaaagggg ggactggaag ggctaattca ctcccaaaga 11220
   agacaagata tcccatccgg agtacttcaa gaactgctga catcgagctt gctacaaggg 11280
   actttccgct ggggactttc cagggaggcg tggcctgggc gggactgggg agtggcgagc 11340
   cctcagatgc tgcatataag cagctgcttt ttgcctgtac tgggtctctc tggttagacc 11400
   agatctgagc ctgggagctc tctggctaac tagggaaccc actgcttaag cctcaataaa 11460
   gcttgccttg agtgcttcaa gtagtgtgtg cccgtctgtt gtgtgactct ggtaactaga 11520
   gatccctcag acccttttag tcagtgtgga aaatctctag cagttctaga gcggccgctc 11580
   gcgaattctt gaagacgaaa gggcctcgtg atacgcctat ttttataggt taatgtcatg 11640
   ataataatgg tttcttagac gtcaggtggc acttttcggg gaaatgtgcg cggaacccct 11700
   atttgtttat ttttctaaat acattcaaat atgtatccgc tcatgagaca ataaccctga 11760
   taaatgcttc aataatattg aaaaaggaag agtatgagta ttcaacattt ccgtgtcgcc 11820
   cttattccct tttttgcggc attttgcctt cctgtttttg ctcacccaga aacgctggtg 11880
   aaagtaaaag atgctgaaga tcagttgggt gcacgagtgg gttacatcga actggatctc 11940
   aacagcggta agatccttga gagttttcgc cccgaagaac gttttccaat gatgagcact 12000
   tttaaagttc tgctatgtgg cgcggtatta tcccgtgttg acgccgggca agagcaactc 12060
   ggtcgccgca tacactattc tcagaatgac ttggttgagt actcaccagt cacagaaaag 12120
   catcttacgg atggcatgac agtaagagaa ttatgcagtg ctgccataac catgagtgat 12180
   aacactgcgg ccaacttact tctgacaacg atcggaggac cgaaggagct aaccgctttt 12240
   ttgcacaaca tgggggatca tgtaactcgc cttgatcgtt gggaaccgga gctgaatgaa 12300
   gccataccaa acgacgagcg tgacaccacg atgcctgcag caatggcaac aacgttgcgc 12360
   aaactattaa ctggcgaact acttactcta gcttcccggc aacaattaat agactggatg 12420
   gaggcggata aagttgcagg accacttctg cgctcggccc ttccggctgg ctggtttatt 12480
   gctgataaat ctggagccgg tgagcgtggg tctcgcggta tcattgcagc actggggcca 12540
   gatggtaagc cctcccgtat cgtagttatc tacacgacgg ggagtcaggc aactatggat 12600
   gaacgaaata gacagatcgc tgagataggt gcctcactga ttaagcattg gtaactgtca 12660
   gaccaagttt actcatatat actttagatt gatttaaaac ttcattttta atttaaaagg 12720
   atctaggtga agatcctttt tgataatctc atgaccaaaa tcccttaacg tgagttttcg 12780
   ttccactgag cgtcagaccc cgtagaaaag atcaaaggat cttcttgaga tccttttttt 12840
   ctgcgcgtaa tctgctgctt gcaaacaaaa aaaccaccgc taccagcggt ggtttgtttg 12900
   ccggatcaag agctaccaac tctttttccg aaggtaactg gcttcagcag agcgcagata 12960
   ccaaatactg tccttctagt gtagccgtag ttaggccacc acttcaagaa ctctgtagca 13020
   ccgcctacat acctcgctct gctaatcctg ttaccagtgg ctgctgccag tggcgataag 13080
   tcgtgtctta ccgggttgga ctcaagacga tagttaccgg ataaggcgca gcggtcgggc 13140
   tgaacggggg gttcgtgcac acagcccagc ttggagcgaa cgacctacac cgaactgaga 13200
   tacctacagc gtgagctatg agaaagcgcc acgcttcccg aagggagaaa ggcggacagg 13260
   tatccggtaa gcggcagggt cggaacagga gagcgcacga gggagcttcc agggggaaac 13320
   gcctggtatc tttatagtcc tgtcgggttt cgccacctct gacttgagcg tcgatttttg 13380
   tgatgctcgt caggggggcg gagcctatgg aaaaacgcca gcaacgcggc ctttttacgg 13440
   ttcctggcct tttgctggcc ttttgctcac atgttctttc ctgcgttatc ccctgattct 13500
   gtggataacc gtattaccgc ctttgagtga gctgataccg ctcgccgcag ccgaacgacc 13560
   gagcgcagcg agtcagtgag cgaggaagcg gaagagcgcc tgatgcggta ttttctcctt 13620
   acgcatctgt gcggtatttc acaccgcata tggtgcactc tcagtacaat ctgctctgat 13680
   gccgcatagt taagccagta tacactccgc tatcgctacg tgactgggtc atggctgcgc 13740
   cccgacaccc gccaacaccc gctgacgcgc cctgacgggc ttgtctgctc ccggcatccg 13800
   cttacagaca agctgtgacc gtctccggga gctgcatgtg tcagaggttt tcaccgtcat 13860
   caccgaaacg cgcgaggcag atcccgcaag aggcccggca gtaccggcat aaccaagcct 13920
   atgcctacag catccagggt gacggtgccg aggatgacga tgagcgcatt gttagatttc 13980
   atacacggtg cctgactgcg ttagcaattt aactgtgata aactaccgca ttaaagcttg 14040
   tcgacagcgc tacgcgc 14057
<210> 13
   <211> 14517
   <212> DNA
   <213> Artificial Sequence
<220>
   <223> pBR-SIVmac239-Nef-IRES-Cre
<400> 13
   tggaagggat ttattacagt gcaagaagac atagaatctt agacatatac ttagaaaagg        60
   aagaaggcat cataccagat tggcaggatt acacctcagg accaggaatt agatacccaa       120
   agacatttgg ctggctatgg aaattagtcc ctgtaaatgt atcagatgag gcacaggagg       180
   atgaggagca ttatttaatg catccagctc aaacttccca gtgggatgac ccttggggag       240
   aggttctagc atggaagttt gatccaactc tggcctacac ttatgaggca tatgttagat       300
   acccagaaga gtttggaagc aagtcaggcc tgtcagagga agaggttaga agaaggctaa       360
   ccgcaagagg ccttcttaac atggctgaca agaaggaaac tcgctgaaac agcagggact       420
   ttccacaagg ggatgttacg gggaggtact ggggaggagc cggtcgggaa cgcccacttt        480
   cttgatgtat aaatatcact gcatttcgct ctgtattcag tcgctctgcg gagaggctgg 540
   cagattgagc cctgggaggt tctctccagc actagcaggt agagcctggg tgttccctgc 600
   tagactctca ccagcacttg gccggtgctg ggcagagtga ctccacgctt gcttgcttaa 660
   agccctcttc aataaagctg ccattttagg aagtaagcta gtgtgtgttc ccatctctcc 720
   tagccgccgc ctggtcaact cggtacttca ataataagaa gactcctggt ctgttaggac 780
   cctttctgct ttgggaaacc gaagcaggaa aatccctagc agattggcgc ctgaacaggg 840
   acttgaagga gagtgagaga ctcctgagta cggctgagtg aaggcagtaa gggcggcagg 900
   aaccaaccac gacggagtgc tcctataaag gcgcgggtcg gtaccagacg gcgtgaggag 960
   cgggagagga agaggcctcc ggttgcaggt aagtgcaaca caaaaaagaa atagctgtct 1020
   tttatccagg aaggggtaat aagatagagt gggagatggg cgtgagaaac tccgtcttgt 1080
   cagggaagaa agcagatgaa ttagaaaaaa ttaggctacg acccaacgga aagaaaaagt 1140
   acatgttgaa gcatgtagta tgggcagcaa atgaattaga tagatttgga ttagcagaaa 1200
   gcctgttgga gaacaaagaa ggatgtcaaa aaatactttc ggtcttagct ccattagtgc 1260
   caacaggctc agaaaattta aaaagccttt ataatactgt ctgcgtcatc tggtgcattc 1320
   acgcagaaga gaaagtgaaa cacactgagg aagcaaaaca gatagtgcag agacacctag 1380
   tggtggaaac aggaacaaca gaaactatgc caaaaacaag tagaccaaca gcaccatcta 1440
   gcggcagagg aggaaattac ccagtacaac aaataggtgg taactatgtc cacctgccat 1500
   taagcccgag aacattaaat gcctgggtaa aattgataga ggaaaagaaa tttggagcag 1560
   aagtagtgcc aggatttcag gcactgtcag aaggttgcac cccctatgac attaatcaga 1620
   tgttaaattg tgtgggagac catcaagcgg ctatgcagat tatcagagat attataaacg 1680
   aggaggctgc agattgggac ttgcagcacc cacaaccagc tccacaacaa ggacaactta 1740
   gggagccgtc aggatcagat attgcaggaa caactagttc agtagatgaa caaatccagt 1800
   ggatgtacag acaacagaac cccataccag taggcaacat ttacaggaga tggatccaac 1860
   tggggttgca aaaatgtgtc agaatgtata acccaacaaa cattctagat gtaaaacaag 1920
   ggccaaaaga gccatttcag agctatgtag acaggttcta caaaagttta agagcagaac 1980
   agacagatgc agcagtaaag aattggatga ctcaaacact gctgattcaa aatgctaacc 2040
   cagattgcaa gctagtgctg aaggggctgg gtgtgaatcc caccctagaa gaaatgctga 2100
   cggcttgtca aggagtaggg gggccgggac agaaggctag attaatggca gaagccctga 2160
   aagaggccct cgcaccagtg ccaatccctt ttgcagcagc ccaacagagg ggaccaagaa 2220
   agccaattaa gtgttggaat tgtgggaaag agggacactc tgcaaggcaa tgcagagccc 2280
   caagaagaca gggatgctgg aaatgtggaa aaatggacca tgttatggcc aaatgcccag 2340
   acagacaggc gggtttttta ggccttggtc catggggaaa gaagccccgc aatttcccca 2400
   tggctcaagt gcatcagggg ctgatgccaa ctgctccccc agaggaccca gctgtggatc 2460
   tgctaaagaa ctacatgcag ttgggcaagc agcagagaga aaagcagaga gaaagcagag 2520
   agaagcctta caaggaggtg acagaggatt tgctgcacct caattctctc tttggaggag 2580
   accagtagtc actgctcata ttgaaggaca gcctgtagaa gtattactgg atacaggggc 2640
   tgatgattct attgtaacag gaatagagtt aggtccacat tataccccaa aaatagtagg 2700
   aggaatagga ggttttatta atactaaaga atacaaaaat gtagaaatag aagttttagg 2760
   caaaaggatt aaagggacaa tcatgacagg ggacaccccg attaacattt ttggtagaaa 2820
   tttgctaaca gctctgggga tgtctctaaa ttttcccata gctaaagtag agcctgtaaa 2880
   agtcgcctta aagccaggaa aggatggacc aaaattgaag cagtggccat tatcaaaaga 2940
   aaagatagtt gcattaagag aaatctgtga aaagatggaa aaggatggtc agttggagga 3000
   agctcccccg accaatccat acaacacccc cacatttgct ataaagaaaa aggataagaa 3060
   caaatggaga atgctgatag attttaggga actaaatagg gtcactcagg actttacgga 3120
   agtccaatta ggaataccac accctgcagg actagcaaaa aggaaaagaa ttacagtact 3180
   ggatataggt gatgcatatt tctccatacc tctagatgaa gaatttaggc agtacactgc 3240
   ctttacttta ccatcagtaa ataatgcaga gccaggaaaa cgatacattt ataaggttct 3300
   gcctcaggga tggaaggggt caccagccat cttccaatac actatgagac atgtgctaga 3360
   acccttcagg aaggcaaatc cagatgtgac cttagtccag tatatggatg acatcttaat 3420
   agctagtgac aggacagacc tggaacatga cagggtagtt ttacagtcaa aggaactctt 3480
   gaatagcata gggttttcta ccccagaaga gaaattccaa aaagatcccc catttcaatg 3540
   gatggggtac gaattgtggc caacaaaatg gaagttgcaa aagatagagt tgccacaaag 3600
   agagacctgg acagtgaatg atatacagaa gttagtagga gtattaaatt gggcagctca 3660
   aatttatcca ggtataaaaa ccaaacatct ctgtaggtta attagaggaa aaatgactct 3720
   aacagaggaa gttcagtgga ctgagatggc agaagcagaa tatgaggaaa ataaaataat 3780
   tctcagtcag gaacaagaag gatgttatta ccaagaaggc aagccattag aagccacggt 3840
   aataaagagt caggacaatc agtggtctta taaaattcac caagaagaca aaatactgaa 3900
   agtaggaaaa tttgcaaaga taaagaatac acataccaat ggagtgagac tattagcaca 3960
   tgtaatacag aaaataggaa aggaagcaat agtgatctgg ggacaggtcc caaaattcca 4020
   cttaccagtt gagaaggatg tatgggaaca gtggtggaca gactattggc aggtaacctg 4080
   gataccggaa tgggatttta tctcaacacc accgctagta agattagtct tcaatctagt 4140
   gaaggaccct atagagggag aagaaaccta ttatacagat ggatcatgta ataaacagtc 4200
   aaaagaaggg aaagcaggat atatcacaga taggggcaaa gacaaagtaa aagtgttaga 4260
   acagactact aatcaacaag cagaattgga agcatttctc atggcattga cagactcagg 4320
   gccaaaggca aatattatag tagattcaca atatgttatg ggaataataa caggatgccc 4380
   tacagaatca gagagcaggc tagttaatca aataatagaa gaaatgatta aaaagtcaga 4440
   aatttatgta gcatgggtac cagcacacaa aggtatagga ggaaaccaag aaatagacca 4500
   cctagttagt caagggatta gacaagttct cttcttggaa aagatagagc cagcacaaga 4560
   agaacatgat aaataccata gtaatgtaaa agaattggta ttcaaatttg gattacccag 4620
   aatagtggcc agacagatag tagacacctg tgataaatgt catcagaaag gagaggctat 4680
   acatgggcag gcaaattcag atctagggac ttggcaaatg gattgtaccc atctagaggg 4740
   aaaaataatc atagttgcag tacatgtagc tagtggattc atagaagcag aggtaattcc 4800
   acaagagaca ggaagacaga cagcactatt tctgttaaaa ttggcaggca gatggcctat 4860
   tacacatcta cacacagata atggtgctaa ctttgcttcg caagaagtaa agatggttgc 4920
   atggtgggca gggatagagc acacctttgg ggtaccatac aatccacaga gtcagggagt 4980
   agtggaagca atgaatcacc acctgaaaaa tcaaatagat agaatcaggg aacaagcaaa 5040
   ttcagtagaa accatagtat taatggcagt tcattgcatg aattttaaaa gaaggggagg 5100
   aataggggat atgactccag cagaaagatt aattaacatg atcactacag aacaagagat 5160
   acaatttcaa caatcaaaaa actcaaaatt taaaaatttt cgggtctatt acagagaagg 5220
   cagagatcaa ctgtggaagg gacccggtga gctattgtgg aaaggggaag gagcagtcat 5280
   cttaaaggta gggacagaca ttaaggtagt acccagaaga aaggctaaaa ttatcaaaga 5340
   ttatggagga ggaaaagagg tggatagcag ttcccacatg gaggataccg gagaggctag 5400
   agaggtggca tagcctcata aaatatctga aatataaaac taaagatcta caaaaggttt 5460
   gctatgtgcc ccattttaag gtcggatggg catggtggac ctgcagcaga gtaatcttcc 5520
   cactacagga aggaagccat ttagaagtac aagggtattg gcatttgaca ccagaaaaag 5580
   ggtggctcag tacttatgca gtgaggataa cctggtactc aaagaacttt tggacagatg 5640
   taacaccaaa ctatgcagac attttactgc atagcactta tttcccttgc tttacagcgg 5700
   gagaagtgag aagggccatc aggggagaac aactgctgtc ttgctgcagg ttcccgagag 5760
   ctcataagta ccaggtacca agcctacagt acttagcact gaaagtagta agcgatgtca 5820
   gatcccaggg agagaatccc acctggaaac agtggagaag agacaatagg agaggccttc 5880
   gaatggctaa acagaacagt agaggagata aacagagagg cggtaaacca cctaccaagg 5940
   gagctaattt tccaggtttg gcaaaggtct tgggaatact ggcatgatga acaagggatg 6000
   tcaccaagct atgtaaaata cagatacttg tgtttaatac aaaaggcttt atttatgcat 6060
   tgcaagaaag gctgtagatg tctaggggaa ggacatgggg cagggggatg gagaccagga 6120
   cctcctcctc ctccccctcc aggactagca taaatggaag aaagacctcc agaaaatgaa 6180
   ggaccacaaa gggaaccatg ggatgaatgg gtagtggagg ttctggaaga actgaaagaa 6240
   gaagctttaa aacattttga tcctcgcttg ctaactgcac ttggtaatca tatctataat 6300
   agacatggag acacccttga gggagcagga gaactcatta gaatcctcca acgagcgctc 6360
   ttcatgcatt tcagaggcgg atgcatccac tccagaatcg gccaacctgg gggaggaaat 6420
   cctctctcag ctataccgcc ctctagaagc atgctataac acatgctatt gtaaaaagtg 6480
   ttgctaccat tgccagtttt gttttcttaa aaaaggcttg gggatatgtt atgagcaatc 6540
   acgaaagaga agaagaactc cgaaaaaggc taaggctaat acatcttctg catcaaacaa 6600
   gtaagtatgg gatgtcttgg gaatcagctg cttatcgcca tcttgctttt aagtgtctat 6660
   gggatctatt gtactctata tgtcacagtc ttttatggtg taccagcttg gaggaatgcg 6720
   acaattcccc tcttttgtgc aaccaagaat agggatactt ggggaacaac tcagtgccta 6780
   ccagataatg gtgattattc agaagtggcc cttaatgtta cagaaagctt tgatgcctgg 6840
   aataatacag tcacagaaca ggcaatagag gatgtatggc aactctttga gacctcaata 6900
   aagccttgtg taaaattatc cccattatgc attactatga gatgcaataa aagtgagaca 6960
   gatagatggg gattgacaaa atcaataaca acaacagcat caacaacatc aacgacagca 7020
   tcagcaaaag tagacatggt caatgagact agttcttgta tagcccagga taattgcaca 7080
   ggcttggaac aagagcaaat gataagctgt aaattcaaca tgacagggtt aaaaagagac 7140
   aagaaaaaag agtacaatga aacttggtac tctgcagatt tggtatgtga acaagggaat 7200
   aacactggta atgaaagtag atgttacatg aaccactgta acacttctgt tatccaagag 7260
   tcttgtgaca aacattattg ggatgctatt agatttaggt attgtgcacc tccaggttat 7320
   gctttgctta gatgtaatga cacaaattat tcaggcttta tgcctaaatg ttctaaggtg 7380
   gtggtctctt catgcacaag gatgatggag acacagactt ctacttggtt tggctttaat 7440
   ggaactagag cagaaaatag aacttatatt tactggcatg gtagggataa taggactata 7500
   attagtttaa ataagtatta taatctaaca atgaaatgta gaagaccagg aaataagaca 7560
   gttttaccag tcaccattat gtctggattg gttttccact cacaaccaat caatgatagg 7620
   ccaaagcagg catggtgttg gtttggagga aaatggaagg atgcaataaa agaggtgaag 7680
   cagaccattg tcaaacatcc caggtatact ggaactaaca atactgataa aatcaatttg 7740
   acggctcctg gaggaggaga tccggaagtt accttcatgt ggacaaattg cagaggagag 7800
   ttcctctact gtaaaatgaa ttggtttcta aattgggtag aagataggaa tacagctaac 7860
   cagaagccaa aggaacagca taaaaggaat tacgtgccat gtcatattag acaaataatc 7920
   aacacttggc ataaagtagg caaaaatgtt tatttgcctc caagagaggg agacctcacg 7980
   tgtaactcca cagtgaccag tctcatagca aacatagatt ggattgatgg aaaccaaact 8040
   aatatcacca tgagtgcaga ggtggcagaa ctgtatcgat tggaattggg agattataaa 8100
   ttagtagaga tcactccaat tggcttggcc cccacagatg tgaagaggta cactactggt 8160
   ggcacctcaa gaaataaaag aggggtcttt gtgctagggt tcttgggttt tctcgcaacg 8220
   gcaggttctg caatgggcgc ggcgtcgttg acgctgaccg ctcagtcccg aactttattg 8280
   gctgggatag tgcagcaaca gcaacagctg ttggacgtgg tcaagagaca acaagaattg 8340
   ttgcgactga ccgtctgggg aacaaagaac ctccagacta gggtcactgc catcgagaag 8400
   tacttaaagg accaggcgca gctgaatgct tggggatgtg cgtttagaca agtctgccac 8460
   actactgtac catggccaaa tgcaagtcta acaccaaagt ggaacaatga gacttggcaa 8520
   gagtgggagc gaaaggttga cttcttggaa gaaaatataa cagccctcct agaggaggca 8580
   caaattcaac aagagaagaa catgtatgaa ttacaaaagt tgaatagctg ggatgtgttt 8640
   ggcaattggt ttgaccttgc ttcttggata aagtatatac aatatggagt ttatatagtt 8700
   gtaggagtaa tactgttaag aatagtgatc tatatagtac aaatgctagc taagttaagg 8760
   caggggtata ggccagtgtt ctcttcccca ccctcttatt tccagcagac ccatatccaa 8820
   caggacccgg cactgccaac cagagaaggc aaagaaagag acggtggaga aggcggtggc 8880
   aacagctcct ggccttggca gatagaatat attcatttcc tgatccgcca actgatacgc 8940
   ctcttgactt ggctattcag caactgcaga accttgctat cgagagtata ccagatcctc 9000
   caaccaatac tccagaggct ctctgcgacc ctacagagga ttcgagaagt cctcaggact 9060
   gaactgacct acctacaata tgggtggagc tatttccatg aggcggtcca ggccgtctgg 9120
   agatctgcga cagagactct tgcgggcgcg tggggagact tatgggagac tcttaggaga 9180
   ggtggaagat ggatactcgc aatccccagg aggattagac aagggcttga gctcactctc 9240
   ttgtgaggga cagaaataca atcagggaca gtatatgaat actccatgga gaaacccagc 9300
   tgaagagaga gaaaaattag catacagaaa acaaaatatg gatgatatag atgaggaaga 9360
   tgatgacttg gtaggggtat cagtgaggcc aaaagttccc ctaagaacaa tgagttacaa 9420
   attggcaata gacatgtctc attttataaa agaaaagggg ggactggaag ggatttatta 9480
   cagtgcaaga agacatagaa tcttagacat atacttagaa aaggaaggag gcatcatacc 9540
   agattggcag gattacacct caggaccagg aattagatac ccaaagacat ttggctggct 9600
   atggaaatta gtccctgtaa atgtatcaga tgaggcacag gaggatgagg agcattattt 9660
   aatgcatcca gctcaaactt cccagtggga tgacccttgg ggagaggttc tagcatggaa 9720
   gtttgatcca actctggcct acacttatga ggcatatgtt agatacccag aagagtttgg 9780
   aagcaagtca ggcctgtcag aggaagaggt tagaagaagg ctaaccgcaa gaggccttct 9840
   taacatggct gacaagaagg aaactcgctg aacgcgtcgg atcccggagt actacaaaga 9900
   ctgctgacgc gaattccgcc ccccccccta acgttactgg ccgaagccgc ttggaataag 9960
   gccggtgtgc gtttgtctat atgttatttt ccaccatatt gccgtctttt ggcaatgtga 10020
   gggcccggaa acctggccct gtcttcttga cgagcattcc taggggtctt tcccctctcg 10080
   ccaaaggaat gcaaggtctg ttgaatgtcg tgaaggaagc agttcctctg gaagcttctt 10140
   gaagacaaac aacgtctgta gcgacccttt gcaggcagcg gaacccccca cctggcgaca 10200
   ggtgcctctg cggccaaaag ccacgtgtat aagatacacc tgcaaaggcg gcacaacccc 10260
   agtgccacgt tgtgagttgg atagttgtgg aaagagtcaa atggctctcc tcaagcgtat 10320
   tcaacaaggg gctgaaggat gcccagaagg taccccattg tatgggatct gatctggggc 10380
   ctcggtgcac atgctttaca tgtgtttagt cgaggttaaa aaacgtctag gccccccgaa 10440
   ccacggggac gtggttttcc tttgaaaaac acgatgataa taccatggtg cccaagaaga 10500
   agaggaaagt ctccaacctg ctgactgtgc accaaaacct gcctgccctc cctgtggatg 10560
   ccacctctga tgaagtcagg aagaacctga tggacatgtt cagggacagg caggccttct 10620
   ctgaacacac ctggaagatg ctcctgtctg tgtgcagatc ctgggctgcc tggtgcaagc 10680
   tgaacaacag gaaatggttc cctgctgaac ctgaggatgt gagggactac ctcctgtacc 10740
   tgcaagccag aggcctggct gtgaagacca tccaacagca cctgggccag ctcaacatgc 10800
   tgcacaggag atctggcctg cctcgccctt ctgactccaa tgctgtgtcc ctggtgatga 10860
   ggagaatcag aaaggagaat gtggatgctg gggagagagc caagcaggcc ctggcctttg 10920
   aacgcactga ctttgaccaa gtcagatccc tgatggagaa ctctgacaga tgccaggaca 10980
   tcaggaacct ggccttcctg ggcattgcct acaacaccct gctgcgcatt gccgaaattg 11040
   ccagaatcag agtgaaggac atctcccgca ccgatggtgg gagaatgctg atccacattg 11100
   gcaggaccaa gaccctggtg tccacagctg gtgtggagaa ggccctgtcc ctgggggtta 11160
   ccaagctggt ggagagatgg atctctgtgt ctggtgtggc tgatgacccc aacaactacc 11220
   tgttctgccg ggtcagaaag aatggtgtgg ctgccccttc tgccacctcc caactgtcca 11280
   cccgggccct ggaagggatc tttgaggcca cccaccgcct gatctatggt gccaaggatg 11340
   actctgggca gagatacctg gcctggtctg gccactctgc cagagtgggt gctgccaggg 11400
   acatggccag ggctggtgtg tccatccctg aaatcatgca ggctggtggc tggaccaatg 11460
   tgaacatagt gatgaactac atcagaaacc tggactctga gactggggcc atggtgaggc 11520
   tgctcgagga tggggactga cccgggtctc attttataaa agaaaagggg ggactggaag 11580
   ggatttatcc gcggttcact cgagactcgc tgaaacagca gggactttcc acaaggggat 11640
   gttacgggga ggtactgggg aggagccggt cgggaacgcc cactttcttg atgtataaat 11700
   atcactgcat ttcgctctgt attcagtcgc tctgcggaga ggctggcaga ttgagccctg 11760
   ggaggttctc tccagcacta gcaggtagag cctgggtgtt ccctgctaga ctctcaccag 11820
   cacttggccg gtgctgggca gagtgactcc acgcttgctt gcttaaagcc ctcttcaata 11880
   aagctgccat tttagaagta agctagtgtg tgttcccatc tctcctagcc gccgcctggt 11940
   caactcggta ctcaataata agaagaccct ggtctgttag gaccctttct gctttgggaa 12000
   accgaagcag gaaaatccct agcagaattc ttgaagacga aagggcctcg tgatacgcct 12060
   atttttatag gttaatgtca tgataataat ggtttcttag acgtcaggtg gcacttttcg 12120
   gggaaatgtg cgcggaaccc ctatttgttt atttttctaa atacattcaa atatgtatcc 12180
   gctcatgaga caataaccct gataaatgct tcaataatat tgaaaaagga agagtatgag 12240
   tattcaacat ttccgtgtcg cccttattcc cttttttgcg gcattttgcc ttcctgtttt 12300
   tgctcaccca gaaacgctgg tgaaagtaaa agatgctgaa gatcagttgg gtgcacgagt 12360
   gggttacatc gaactggatc tcaacagcgg taagatcctt gagagttttc gccccgaaga 12420
   acgttttcca atgatgagca cttttaaagt tctgctatgt ggcgcggtat tatcccgtgt 12480
   tgacgccggg caagagcaac tcggtcgccg catacactat tctcagaatg acttggttga 12540
   gtactcacca gtcacagaaa agcatcttac ggatggcatg acagtaagag aattatgcag 12600
   tgctgccata accatgagtg ataacactgc ggccaactta cttctgacaa cgatcggagg 12660
   accgaaggag ctaaccgctt ttttgcacaa catgggggat catgtaactc gccttgatcg 12720
   ttgggaaccg gagctgaatg aagccatacc aaacgacgag cgtgacacca cgatgcctgc 12780
   agcaatggca acaacgttgc gcaaactatt aactggcgaa ctacttactc tagcttcccg 12840
   gcaacaatta atagactgga tggaggcgga taaagttgca ggaccacttc tgcgctcggc 12900
   ccttccggct ggctggttta ttgctgataa atctggagcc ggtgagcgtg ggtctcgcgg 12960
   tatcattgca gcactggggc cagatggtaa gccctcccgt atcgtagtta tctacacgac 13020
   ggggagtcag gcaactatgg atgaacgaaa tagacagatc gctgagatag gtgcctcact 13080
   gattaagcat tggtaactgt cagaccaagt ttactcatat atactttaga ttgatttaaa 13140
   acttcatttt taatttaaaa ggatctaggt gaagatcctt tttgataatc tcatgaccaa 13200
   aatcccttaa cgtgagtttt cgttccactg agcgtcagac cccgtagaaa agatcaaagg 13260
   atcttcttga gatccttttt ttctgcgcgt aatctgctgc ttgcaaacaa aaaaaccacc 13320
   gctaccagcg gtggtttgtt tgccggatca agagctacca actctttttc cgaaggtaac 13380
   tggcttcagc agagcgcaga taccaaatac tgtccttcta gtgtagccgt agttaggcca 13440
   ccacttcaag aactctgtag caccgcctac atacctcgct ctgctaatcc tgttaccagt 13500
   ggctgctgcc agtggcgata agtcgtgtct taccgggttg gactcaagac gatagttacc 13560
   ggataaggcg cagcggtcgg gctgaacggg gggttcgtgc acacagccca gcttggagcg 13620
   aacgacctac accgaactga gatacctaca gcgtgagcta tgagaaagcg ccacgcttcc 13680
   cgaagggaga aaggcggaca ggtatccggt aagcggcagg gtcggaacag gagagcgcac 13740
   gagggagctt ccagggggaa acgcctggta tctttatagt cctgtcgggt ttcgccacct 13800
   ctgacttgag cgtcgatttt tgtgatgctc gtcagggggg cggagcctat ggaaaaacgc 13860
   cagcaacgcg gcctttttac ggttcctggc cttttgctgg ccttttgctc acatgttctt 13920
   tcctgcgtta tcccctgatt ctgtggataa ccgtattacc gcctttgagt gagctgatac 13980
   cgctcgccgc agccgaacga ccgagcgcag cgagtcagtg agcgaggaag cggaagagcg 14040
   cctgatgcgg tattttctcc ttacgcatct gtgcggtatt tcacaccgca tatggtgcac 14100
   tctcagtaca atctgctctg atgccgcata gttaagccag tatacactcc gctatcgcta 14160
   cgtgactggg tcatggctgc gccccgacac ccgccaacac ccgctgacgc gccctgacgg 14220
   gcttgtctgc tcccggcatc cgcttacaga caagctgtga ccgtctccgg gagctgcatg 14280
   tgtcagaggt tttcaccgtc atcaccgaaa cgcgcgaggc agatcccgca agaggcccgg 14340
   cagtaccggc ataaccaagc ctatgcctac agcatccagg gtgacggtgc cgaggatgac 14400
   gatgagcgca ttgttagatt tcatacacgg tgcctgactg cgttagcaat ttaactgtga 14460
   taaactaccg cattaaagct tactgtaaat ttactggctg tcttccttgc aggtttc 14517
<210> 14
   <211> 14517
   <212> DNA
   <213> Artificial Sequence
<220>
   <223> pBR-SIVmac239-Nef IRES-Cre
<400> 14
   tggaagggat ttattacagt gcaagaagac atagaatctt agacatatac ttagaaaagg      60
   aagaaggcat cataccagat tggcaggatt acacctcagg accaggaatt agatacccaa     120
   agacatttgg ctggctatgg aaattagtcc ctgtaaatgt atcagatgag gcacaggagg     180
   atgaggagca ttatttaatg catccagctc aaacttccca gtgggatgac ccttggggag     240
   aggttctagc atggaagttt gatccaactc tggcctacac ttatgaggca tatgttagat     300
   acccagaaga gtttggaagc aagtcaggcc tgtcagagga agaggttaga agaaggctaa     360
   ccgcaagagg ccttcttaac atggctgaca agaaggaaac tcgctgaaac agcagggact     420
   ttccacaagg ggatgttacg gggaggtact ggggaggagc cggtcgggaa cgcccacttt     480
   cttgatgtat aaatatcact gcatttcgct ctgtattcag tcgctctgcg gagaggctgg     540
   cagattgagc cctgggaggt tctctccagc actagcaggt agagcctggg tgttccctgc     600
   tagactctca ccagcacttg gccggtgctg ggcagagtga ctccacgctt gcttgcttaa     660
   agccctcttc aataaagctg ccattttagg aagtaagcta gtgtgtgttc ccatctctcc     720
   tagccgccgc ctggtcaact cggtacttca ataataagaa gactcctggt ctgttaggac     780
   cctttctgct ttgggaaacc gaagcaggaa aatccctagc agattggcgc ctgaacaggg 840
   acttgaagga gagtgagaga ctcctgagta cggctgagtg aaggcagtaa gggcggcagg 900
   aaccaaccac gacggagtgc tcctataaag gcgcgggtcg gtaccagacg gcgtgaggag 960
   cgggagagga agaggcctcc ggttgcaggt aagtgcaaca caaaaaagaa atagctgtct 1020
   tttatccagg aaggggtaat aagatagagt gggagatggg cgtgagaaac tccgtcttgt 1080
   cagggaagaa agcagatgaa ttagaaaaaa ttaggctacg acccaacgga aagaaaaagt 1140
   acatgttgaa gcatgtagta tgggcagcaa atgaattaga tagatttgga ttagcagaaa 1200
   gcctgttgga gaacaaagaa ggatgtcaaa aaatactttc ggtcttagct ccattagtgc 1260
   caacaggctc agaaaattta aaaagccttt ataatactgt ctgcgtcatc tggtgcattc 1320
   acgcagaaga gaaagtgaaa cacactgagg aagcaaaaca gatagtgcag agacacctag 1380
   tggtggaaac aggaacaaca gaaactatgc caaaaacaag tagaccaaca gcaccatcta 1440
   gcggcagagg aggaaattac ccagtacaac aaataggtgg taactatgtc cacctgccat 1500
   taagcccgag aacattaaat gcctgggtaa aattgataga ggaaaagaaa tttggagcag 1560
   aagtagtgcc aggatttcag gcactgtcag aaggttgcac cccctatgac attaatcaga 1620
   tgttaaattg tgtgggagac catcaagcgg ctatgcagat tatcagagat attataaacg 1680
   aggaggctgc agattgggac ttgcagcacc cacaaccagc tccacaacaa ggacaactta 1740
   gggagccgtc aggatcagat attgcaggaa caactagttc agtagatgaa caaatccagt 1800
   ggatgtacag acaacagaac cccataccag taggcaacat ttacaggaga tggatccaac 1860
   tggggttgca aaaatgtgtc agaatgtata acccaacaaa cattctagat gtaaaacaag 1920
   ggccaaaaga gccatttcag agctatgtag acaggttcta caaaagttta agagcagaac 1980
   agacagatgc agcagtaaag aattggatga ctcaaacact gctgattcaa aatgctaacc 2040
   cagattgcaa gctagtgctg aaggggctgg gtgtgaatcc caccctagaa gaaatgctga 2100
   cggcttgtca aggagtaggg gggccgggac agaaggctag attaatggca gaagccctga 2160
   aagaggccct cgcaccagtg ccaatccctt ttgcagcagc ccaacagagg ggaccaagaa 2220
   agccaattaa gtgttggaat tgtgggaaag agggacactc tgcaaggcaa tgcagagccc 2280
   caagaagaca gggatgctgg aaatgtggaa aaatggacca tgttatggcc aaatgcccag 2340
   acagacaggc gggtttttta ggccttggtc catggggaaa gaagccccgc aatttcccca 2400
   tggctcaagt gcatcagggg ctgatgccaa ctgctccccc agaggaccca gctgtggatc 2460
   tgctaaagaa ctacatgcag ttgggcaagc agcagagaga aaagcagaga gaaagcagag 2520
   agaagcctta caaggaggtg acagaggatt tgctgcacct caattctctc tttggaggag 2580
   accagtagtc actgctcata ttgaaggaca gcctgtagaa gtattactgg atacaggggc 2640
   tgatgattct attgtaacag gaatagagtt aggtccacat tataccccaa aaatagtagg 2700
   aggaatagga ggttttatta atactaaaga atacaaaaat gtagaaatag aagttttagg 2760
   caaaaggatt aaagggacaa tcatgacagg ggacaccccg attaacattt ttggtagaaa 2820
   tttgctaaca gctctgggga tgtctctaaa ttttcccata gctaaagtag agcctgtaaa 2880
   agtcgcctta aagccaggaa aggatggacc aaaattgaag cagtggccat tatcaaaaga 2940
   aaagatagtt gcattaagag aaatctgtga aaagatggaa aaggatggtc agttggagga 3000
   agctcccccg accaatccat acaacacccc cacatttgct ataaagaaaa aggataagaa 3060
   caaatggaga atgctgatag attttaggga actaaatagg gtcactcagg actttacgga 3120
   agtccaatta ggaataccac accctgcagg actagcaaaa aggaaaagaa ttacagtact 3180
   ggatataggt gatgcatatt tctccatacc tctagatgaa gaatttaggc agtacactgc 3240
   ctttacttta ccatcagtaa ataatgcaga gccaggaaaa cgatacattt ataaggttct 3300
   gcctcaggga tggaaggggt caccagccat cttccaatac actatgagac atgtgctaga 3360
   acccttcagg aaggcaaatc cagatgtgac cttagtccag tatatggatg acatcttaat 3420
   agctagtgac aggacagacc tggaacatga cagggtagtt ttacagtcaa aggaactctt 3480
   gaatagcata gggttttcta ccccagaaga gaaattccaa aaagatcccc catttcaatg 3540
   gatggggtac gaattgtggc caacaaaatg gaagttgcaa aagatagagt tgccacaaag 3600
   agagacctgg acagtgaatg atatacagaa gttagtagga gtattaaatt gggcagctca 3660
   aatttatcca ggtataaaaa ccaaacatct ctgtaggtta attagaggaa aaatgactct 3720
   aacagaggaa gttcagtgga ctgagatggc agaagcagaa tatgaggaaa ataaaataat 3780
   tctcagtcag gaacaagaag gatgttatta ccaagaaggc aagccattag aagccacggt 3840
   aataaagagt caggacaatc agtggtctta taaaattcac caagaagaca aaatactgaa 3900
   agtaggaaaa tttgcaaaga taaagaatac acataccaat ggagtgagac tattagcaca 3960
   tgtaatacag aaaataggaa aggaagcaat agtgatctgg ggacaggtcc caaaattcca 4020
   cttaccagtt gagaaggatg tatgggaaca gtggtggaca gactattggc aggtaacctg 4080
   gataccggaa tgggatttta tctcaacacc accgctagta agattagtct tcaatctagt 4140
   gaaggaccct atagagggag aagaaaccta ttatacagat ggatcatgta ataaacagtc 4200
   aaaagaaggg aaagcaggat atatcacaga taggggcaaa gacaaagtaa aagtgttaga 4260
   acagactact aatcaacaag cagaattgga agcatttctc atggcattga cagactcagg 4320
   gccaaaggca aatattatag tagattcaca atatgttatg ggaataataa caggatgccc 4380
   tacagaatca gagagcaggc tagttaatca aataatagaa gaaatgatta aaaagtcaga 4440
   aatttatgta gcatgggtac cagcacacaa aggtatagga ggaaaccaag aaatagacca 4500
   cctagttagt caagggatta gacaagttct cttcttggaa aagatagagc cagcacaaga 4560
   agaacatgat aaataccata gtaatgtaaa agaattggta ttcaaatttg gattacccag 4620
   aatagtggcc agacagatag tagacacctg tgataaatgt catcagaaag gagaggctat 4680
   acatgggcag gcaaattcag atctagggac ttggcaaatg gattgtaccc atctagaggg 4740
   aaaaataatc atagttgcag tacatgtagc tagtggattc atagaagcag aggtaattcc 4800
   acaagagaca ggaagacaga cagcactatt tctgttaaaa ttggcaggca gatggcctat 4860
   tacacatcta cacacagata atggtgctaa ctttgcttcg caagaagtaa agatggttgc 4920
   atggtgggca gggatagagc acacctttgg ggtaccatac aatccacaga gtcagggagt 4980
   agtggaagca atgaatcacc acctgaaaaa tcaaatagat agaatcaggg aacaagcaaa 5040
   ttcagtagaa accatagtat taatggcagt tcattgcatg aattttaaaa gaaggggagg 5100
   aataggggat atgactccag cagaaagatt aattaacatg atcactacag aacaagagat 5160
   acaatttcaa caatcaaaaa actcaaaatt taaaaatttt cgggtctatt acagagaagg 5220
   cagagatcaa ctgtggaagg gacccggtga gctattgtgg aaaggggaag gagcagtcat 5280
   cttaaaggta gggacagaca ttaaggtagt acccagaaga aaggctaaaa ttatcaaaga 5340
   ttatggagga ggaaaagagg tggatagcag ttcccacatg gaggataccg gagaggctag 5400
   agaggtggca tagcctcata aaatatctga aatataaaac taaagatcta caaaaggttt 5460
   gctatgtgcc ccattttaag gtcggatggg catggtggac ctgcagcaga gtaatcttcc 5520
   cactacagga aggaagccat ttagaagtac aagggtattg gcatttgaca ccagaaaaag 5580
   ggtggctcag tacttatgca gtgaggataa cctggtactc aaagaacttt tggacagatg 5640
   taacaccaaa ctatgcagac attttactgc atagcactta tttcccttgc tttacagcgg 5700
   gagaagtgag aagggccatc aggggagaac aactgctgtc ttgctgcagg ttcccgagag 5760
   ctcataagta ccaggtacca agcctacagt acttagcact gaaagtagta agcgatgtca 5820
   gatcccaggg agagaatccc acctggaaac agtggagaag agacaatagg agaggccttc 5880
   gaatggctaa acagaacagt agaggagata aacagagagg cggtaaacca cctaccaagg 5940
   gagctaattt tccaggtttg gcaaaggtct tgggaatact ggcatgatga acaagggatg 6000
   tcaccaagct atgtaaaata cagatacttg tgtttaatac aaaaggcttt atttatgcat 6060
   tgcaagaaag gctgtagatg tctaggggaa ggacatgggg cagggggatg gagaccagga 6120
   cctcctcctc ctccccctcc aggactagca taaatggaag aaagacctcc agaaaatgaa 6180
   ggaccacaaa gggaaccatg ggatgaatgg gtagtggagg ttctggaaga actgaaagaa 6240
   gaagctttaa aacattttga tcctcgcttg ctaactgcac ttggtaatca tatctataat 6300
   agacatggag acacccttga gggagcagga gaactcatta gaatcctcca acgagcgctc 6360
   ttcatgcatt tcagaggcgg atgcatccac tccagaatcg gccaacctgg gggaggaaat 6420
   cctctctcag ctataccgcc ctctagaagc atgctataac acatgctatt gtaaaaagtg 6480
   ttgctaccat tgccagtttt gttttcttaa aaaaggcttg gggatatgtt atgagcaatc 6540
   acgaaagaga agaagaactc cgaaaaaggc taaggctaat acatcttctg catcaaacaa 6600
   gtaagtatgg gatgtcttgg gaatcagctg cttatcgcca tcttgctttt aagtgtctat 6660
   gggatctatt gtactctata tgtcacagtc ttttatggtg taccagcttg gaggaatgcg 6720
   acaattcccc tcttttgtgc aaccaagaat agggatactt ggggaacaac tcagtgccta 6780
   ccagataatg gtgattattc agaagtggcc cttaatgtta cagaaagctt tgatgcctgg 6840
   aataatacag tcacagaaca ggcaatagag gatgtatggc aactctttga gacctcaata 6900
   aagccttgtg taaaattatc cccattatgc attactatga gatgcaataa aagtgagaca 6960
   gatagatggg gattgacaaa atcaataaca acaacagcat caacaacatc aacgacagca 7020
   tcagcaaaag tagacatggt caatgagact agttcttgta tagcccagga taattgcaca 7080
   ggcttggaac aagagcaaat gataagctgt aaattcaaca tgacagggtt aaaaagagac 7140
   aagaaaaaag agtacaatga aacttggtac tctgcagatt tggtatgtga acaagggaat 7200
   aacactggta atgaaagtag atgttacatg aaccactgta acacttctgt tatccaagag 7260
   tcttgtgaca aacattattg ggatgctatt agatttaggt attgtgcacc tccaggttat 7320
   gctttgctta gatgtaatga cacaaattat tcaggcttta tgcctaaatg ttctaaggtg 7380
   gtggtctctt catgcacaag gatgatggag acacagactt ctacttggtt tggctttaat 7440
   ggaactagag cagaaaatag aacttatatt tactggcatg gtagggataa taggactata 7500
   attagtttaa ataagtatta taatctaaca atgaaatgta gaagaccagg aaataagaca 7560
   gttttaccag tcaccattat gtctggattg gttttccact cacaaccaat caatgatagg 7620
   ccaaagcagg catggtgttg gtttggagga aaatggaagg atgcaataaa agaggtgaag 7680
   cagaccattg tcaaacatcc caggtatact ggaactaaca atactgataa aatcaatttg 7740
   acggctcctg gaggaggaga tccggaagtt accttcatgt ggacaaattg cagaggagag 7800
   ttcctctact gtaaaatgaa ttggtttcta aattgggtag aagataggaa tacagctaac 7860
   cagaagccaa aggaacagca taaaaggaat tacgtgccat gtcatattag acaaataatc 7920
   aacacttggc ataaagtagg caaaaatgtt tatttgcctc caagagaggg agacctcacg 7980
   tgtaactcca cagtgaccag tctcatagca aacatagatt ggattgatgg aaaccaaact 8040
   aatatcacca tgagtgcaga ggtggcagaa ctgtatcgat tggaattggg agattataaa 8100
   ttagtagaga tcactccaat tggcttggcc cccacagatg tgaagaggta cactactggt 8160
   ggcacctcaa gaaataaaag aggggtcttt gtgctagggt tcttgggttt tctcgcaacg 8220
   gcaggttctg caatgggcgc ggcgtcgttg acgctgaccg ctcagtcccg aactttattg 8280
   gctgggatag tgcagcaaca gcaacagctg ttggacgtgg tcaagagaca acaagaattg 8340
   ttgcgactga ccgtctgggg aacaaagaac ctccagacta gggtcactgc catcgagaag 8400
   tacttaaagg accaggcgca gctgaatgct tggggatgtg cgtttagaca agtctgccac 8460
   actactgtac catggccaaa tgcaagtcta acaccaaagt ggaacaatga gacttggcaa 8520
   gagtgggagc gaaaggttga cttcttggaa gaaaatataa cagccctcct agaggaggca 8580
   caaattcaac aagagaagaa catgtatgaa ttacaaaagt tgaatagctg ggatgtgttt 8640
   ggcaattggt ttgaccttgc ttcttggata aagtatatac aatatggagt ttatatagtt 8700
   gtaggagtaa tactgttaag aatagtgatc tatatagtac aaatgctagc taagttaagg 8760
   caggggtata ggccagtgtt ctcttcccca ccctcttatt tccagcagac ccatatccaa 8820
   caggacccgg cactgccaac cagagaaggc aaagaaagag acggtggaga aggcggtggc 8880
   aacagctcct ggccttggca gatagaatat attcatttcc tgatccgcca actgatacgc 8940
   ctcttgactt ggctattcag caactgcaga accttgctat cgagagtata ccagatcctc 9000
   caaccaatac tccagaggct ctctgcgacc ctacagagga ttcgagaagt cctcaggact 9060
   gaactgacct acctacaata tgggtggagc tatttccatg aggcggtcca ggccgtctgg 9120
   agatctgcga cagagactct tgcgggcgcg tggggagact tatgggagac tcttaggaga 9180
   ggtggaagat ggatactcgc aatccccagg aggattagac aagggcttga gctcactctc 9240
   ttgtgaggga cagaaataca atcagggaca gtatatgaat actccatgga gaaacccagc 9300
   tgaagagaga gaaaaattag catacagaaa acaaaatatg gatgatatag atgaggaaga 9360
   tgatgacttg gtaggggtat cagtgaggcc aaaagttccc ctaagaacaa tgagttacaa 9420
   attggcaatt gacatgtcac atttcattaa ggagaagggc ggactggagg ggatatacta 9480
   ttctgccagg aggcatagaa tcttagacat atacttagaa aaggaaggag gcatcatacc 9540
   agattggcag gattacacct caggaccagg aattagatac ccaaagacat ttggctggct 9600
   atggaaatta gtccctgtaa atgtatcaga tgaggcacag gaggatgagg agcattattt 9660
   aatgcatcca gctcaaactt cccagtggga tgacccttgg ggagaggttc tagcatggaa 9720
   gtttgatcca actctggcct acacttatga ggcatatgtt agatacccag aagagtttgg 9780
   aagcaagtca ggcctgtcag aggaagaggt tagaagaagg ctaaccgcaa gaggccttct 9840
   taacatggct gacaagaagg aaactcgctg aacgcgtcgg atcccggagt actacaaaga 9900
   ctgctgacgc gaattccgcc ccccccccta acgttactgg ccgaagccgc ttggaataag 9960
   gccggtgtgc gtttgtctat atgttatttt ccaccatatt gccgtctttt ggcaatgtga 10020
   gggcccggaa acctggccct gtcttcttga cgagcattcc taggggtctt tcccctctcg 10080
   ccaaaggaat gcaaggtctg ttgaatgtcg tgaaggaagc agttcctctg gaagcttctt 10140
   gaagacaaac aacgtctgta gcgacccttt gcaggcagcg gaacccccca cctggcgaca 10200
   ggtgcctctg cggccaaaag ccacgtgtat aagatacacc tgcaaaggcg gcacaacccc 10260
   agtgccacgt tgtgagttgg atagttgtgg aaagagtcaa atggctctcc tcaagcgtat 10320
   tcaacaaggg gctgaaggat gcccagaagg taccccattg tatgggatct gatctggggc 10380
   ctcggtgcac atgctttaca tgtgtttagt cgaggttaaa aaacgtctag gccccccgaa 10440
   ccacggggac gtggttttcc tttgaaaaac acgatgataa taccatggtg cccaagaaga 10500
   agaggaaagt ctccaacctg ctgactgtgc accaaaacct gcctgccctc cctgtggatg 10560
   ccacctctga tgaagtcagg aagaacctga tggacatgtt cagggacagg caggccttct 10620
   ctgaacacac ctggaagatg ctcctgtctg tgtgcagatc ctgggctgcc tggtgcaagc 10680
   tgaacaacag gaaatggttc cctgctgaac ctgaggatgt gagggactac ctcctgtacc 10740
   tgcaagccag aggcctggct gtgaagacca tccaacagca cctgggccag ctcaacatgc 10800
   tgcacaggag atctggcctg cctcgccctt ctgactccaa tgctgtgtcc ctggtgatga 10860
   ggagaatcag aaaggagaat gtggatgctg gggagagagc caagcaggcc ctggcctttg 10920
   aacgcactga ctttgaccaa gtcagatccc tgatggagaa ctctgacaga tgccaggaca 10980
   tcaggaacct ggccttcctg ggcattgcct acaacaccct gctgcgcatt gccgaaattg 11040
   ccagaatcag agtgaaggac atctcccgca ccgatggtgg gagaatgctg atccacattg 11100
   gcaggaccaa gaccctggtg tccacagctg gtgtggagaa ggccctgtcc ctgggggtta 11160
   ccaagctggt ggagagatgg atctctgtgt ctggtgtggc tgatgacccc aacaactacc 11220
   tgttctgccg ggtcagaaag aatggtgtgg ctgccccttc tgccacctcc caactgtcca 11280
   cccgggccct ggaagggatc tttgaggcca cccaccgcct gatctatggt gccaaggatg 11340
   actctgggca gagatacctg gcctggtctg gccactctgc cagagtgggt gctgccaggg 11400
   acatggccag ggctggtgtg tccatccctg aaatcatgca ggctggtggc tggaccaatg 11460
   tgaacatagt gatgaactac atcagaaacc tggactctga gactggggcc atggtgaggc 11520
   tgctcgagga tggggactga cccgggtctc attttataaa agaaaagggg ggactggaag 11580
   ggatttatcc gcggttcact cgagactcgc tgaaacagca gggactttcc acaaggggat 11640
   gttacgggga ggtactgggg aggagccggt cgggaacgcc cactttcttg atgtataaat 11700
   atcactgcat ttcgctctgt attcagtcgc tctgcggaga ggctggcaga ttgagccctg 11760
   ggaggttctc tccagcacta gcaggtagag cctgggtgtt ccctgctaga ctctcaccag 11820
   cacttggccg gtgctgggca gagtgactcc acgcttgctt gcttaaagcc ctcttcaata 11880
   aagctgccat tttagaagta agctagtgtg tgttcccatc tctcctagcc gccgcctggt 11940
   caactcggta ctcaataata agaagaccct ggtctgttag gaccctttct gctttgggaa 12000
   accgaagcag gaaaatccct agcagaattc ttgaagacga aagggcctcg tgatacgcct 12060
   atttttatag gttaatgtca tgataataat ggtttcttag acgtcaggtg gcacttttcg 12120
   gggaaatgtg cgcggaaccc ctatttgttt atttttctaa atacattcaa atatgtatcc 12180
   gctcatgaga caataaccct gataaatgct tcaataatat tgaaaaagga agagtatgag 12240
   tattcaacat ttccgtgtcg cccttattcc cttttttgcg gcattttgcc ttcctgtttt 12300
   tgctcaccca gaaacgctgg tgaaagtaaa agatgctgaa gatcagttgg gtgcacgagt 12360
   gggttacatc gaactggatc tcaacagcgg taagatcctt gagagttttc gccccgaaga 12420
   acgttttcca atgatgagca cttttaaagt tctgctatgt ggcgcggtat tatcccgtgt 12480
   tgacgccggg caagagcaac tcggtcgccg catacactat tctcagaatg acttggttga 12540
   gtactcacca gtcacagaaa agcatcttac ggatggcatg acagtaagag aattatgcag 12600
   tgctgccata accatgagtg ataacactgc ggccaactta cttctgacaa cgatcggagg 12660
   accgaaggag ctaaccgctt ttttgcacaa catgggggat catgtaactc gccttgatcg 12720
   ttgggaaccg gagctgaatg aagccatacc aaacgacgag cgtgacacca cgatgcctgc 12780
   agcaatggca acaacgttgc gcaaactatt aactggcgaa ctacttactc tagcttcccg 12840
   gcaacaatta atagactgga tggaggcgga taaagttgca ggaccacttc tgcgctcggc 12900
   ccttccggct ggctggttta ttgctgataa atctggagcc ggtgagcgtg ggtctcgcgg 12960
   tatcattgca gcactggggc cagatggtaa gccctcccgt atcgtagtta tctacacgac 13020
   ggggagtcag gcaactatgg atgaacgaaa tagacagatc gctgagatag gtgcctcact 13080
   gattaagcat tggtaactgt cagaccaagt ttactcatat atactttaga ttgatttaaa 13140
   acttcatttt taatttaaaa ggatctaggt gaagatcctt tttgataatc tcatgaccaa 13200
   aatcccttaa cgtgagtttt cgttccactg agcgtcagac cccgtagaaa agatcaaagg 13260
   atcttcttga gatccttttt ttctgcgcgt aatctgctgc ttgcaaacaa aaaaaccacc 13320
   gctaccagcg gtggtttgtt tgccggatca agagctacca actctttttc cgaaggtaac 13380
   tggcttcagc agagcgcaga taccaaatac tgtccttcta gtgtagccgt agttaggcca 13440
   ccacttcaag aactctgtag caccgcctac atacctcgct ctgctaatcc tgttaccagt 13500
   ggctgctgcc agtggcgata agtcgtgtct taccgggttg gactcaagac gatagttacc 13560
   ggataaggcg cagcggtcgg gctgaacggg gggttcgtgc acacagccca gcttggagcg 13620
   aacgacctac accgaactga gatacctaca gcgtgagcta tgagaaagcg ccacgcttcc 13680
   cgaagggaga aaggcggaca ggtatccggt aagcggcagg gtcggaacag gagagcgcac 13740
   gagggagctt ccagggggaa acgcctggta tctttatagt cctgtcgggt ttcgccacct 13800
   ctgacttgag cgtcgatttt tgtgatgctc gtcagggggg cggagcctat ggaaaaacgc 13860
   cagcaacgcg gcctttttac ggttcctggc cttttgctgg ccttttgctc acatgttctt 13920
   tcctgcgtta tcccctgatt ctgtggataa ccgtattacc gcctttgagt gagctgatac 13980
   cgctcgccgc agccgaacga ccgagcgcag cgagtcagtg agcgaggaag cggaagagcg 14040
   cctgatgcgg tattttctcc ttacgcatct gtgcggtatt tcacaccgca tatggtgcac 14100
   tctcagtaca atctgctctg atgccgcata gttaagccag tatacactcc gctatcgcta 14160
   cgtgactggg tcatggctgc gccccgacac ccgccaacac ccgctgacgc gccctgacgg 14220
   gcttgtctgc tcccggcatc cgcttacaga caagctgtga ccgtctccgg gagctgcatg 14280
   tgtcagaggt tttcaccgtc atcaccgaaa cgcgcgaggc agatcccgca agaggcccgg 14340
   cagtaccggc ataaccaagc ctatgcctac agcatccagg gtgacggtgc cgaggatgac 14400
   gatgagcgca ttgttagatt tcatacacgg tgcctgactg cgttagcaat ttaactgtga 14460
   taaactaccg cattaaagct tactgtaaat ttactggctg tcttccttgc aggtttc 14517
<210> 15
   <211> 14596
   <212> DNA
   <213> Artificial Sequence
<220>
   <223> pBR-SIVmac239-Nef-CMV-Cre :
<400> 15
   tggaagggat ttattacagt gcaagaagac atagaatctt agacatatac ttagaaaagg      60
   aagaaggcat cataccagat tggcaggatt acacctcagg accaggaatt agatacccaa     120
   agacatttgg ctggctatgg aaattagtcc ctgtaaatgt atcagatgag gcacaggagg     180
   atgaggagca ttatttaatg catccagctc aaacttccca gtgggatgac ccttggggag     240
   aggttctagc atggaagttt gatccaactc tggcctacac ttatgaggca tatgttagat     300
   acccagaaga gtttggaagc aagtcaggcc tgtcagagga agaggttaga agaaggctaa     360
   ccgcaagagg ccttcttaac atggctgaca agaaggaaac tcgctgaaac agcagggact     420
   ttccacaagg ggatgttacg gggaggtact ggggaggagc cggtcgggaa cgcccacttt     480
   cttgatgtat aaatatcact gcatttcgct ctgtattcag tcgctctgcg gagaggctgg     540
   cagattgagc cctgggaggt tctctccagc actagcaggt agagcctggg tgttccctgc     600
   tagactctca ccagcacttg gccggtgctg ggcagagtga ctccacgctt gcttgcttaa     660
   agccctcttc aataaagctg ccattttagg aagtaagcta gtgtgtgttc ccatctctcc     720
   tagccgccgc ctggtcaact cggtacttca ataataagaa gactcctggt ctgttaggac     780
   cctttctgct ttgggaaacc gaagcaggaa aatccctagc agattggcgc ctgaacaggg     840
   acttgaagga gagtgagaga ctcctgagta cggctgagtg aaggcagtaa gggcggcagg     900
   aaccaaccac gacggagtgc tcctataaag gcgcgggtcg gtaccagacg gcgtgaggag     960
   cgggagagga agaggcctcc ggttgcaggt aagtgcaaca caaaaaagaa atagctgtct    1020
   tttatccagg aaggggtaat aagatagagt gggagatggg cgtgagaaac tccgtcttgt    1080
   cagggaagaa agcagatgaa ttagaaaaaa ttaggctacg acccaacgga aagaaaaagt     1140
   acatgttgaa gcatgtagta tgggcagcaa atgaattaga tagatttgga ttagcagaaa     1200
   gcctgttgga gaacaaagaa ggatgtcaaa aaatactttc ggtcttagct ccattagtgc     1260
   caacaggctc agaaaattta aaaagccttt ataatactgt ctgcgtcatc tggtgcattc     1320
   acgcagaaga gaaagtgaaa cacactgagg aagcaaaaca gatagtgcag agacacctag     1380
   tggtggaaac aggaacaaca gaaactatgc caaaaacaag tagaccaaca gcaccatcta     1440
   gcggcagagg aggaaattac ccagtacaac aaataggtgg taactatgtc cacctgccat     1500
   taagcccgag aacattaaat gcctgggtaa aattgataga ggaaaagaaa tttggagcag     1560
   aagtagtgcc aggatttcag gcactgtcag aaggttgcac cccctatgac attaatcaga     1620
   tgttaaattg tgtgggagac catcaagcgg ctatgcagat tatcagagat attataaacg     1680
   aggaggctgc agattgggac ttgcagcacc cacaaccagc tccacaacaa ggacaactta     1740
   gggagccgtc aggatcagat attgcaggaa caactagttc agtagatgaa caaatccagt     1800
   ggatgtacag acaacagaac cccataccag taggcaacat ttacaggaga tggatccaac     1860
   tggggttgca aaaatgtgtc agaatgtata acccaacaaa cattctagat gtaaaacaag     1920
   ggccaaaaga gccatttcag agctatgtag acaggttcta caaaagttta agagcagaac     1980
   agacagatgc agcagtaaag aattggatga ctcaaacact gctgattcaa aatgctaacc     2040
   cagattgcaa gctagtgctg aaggggctgg gtgtgaatcc caccctagaa gaaatgctga     2100
   cggcttgtca aggagtaggg gggccgggac agaaggctag attaatggca gaagccctga     2160
   aagaggccct cgcaccagtg ccaatccctt ttgcagcagc ccaacagagg ggaccaagaa     2220
   agccaattaa gtgttggaat tgtgggaaag agggacactc tgcaaggcaa tgcagagccc     2280
   caagaagaca gggatgctgg aaatgtggaa aaatggacca tgttatggcc aaatgcccag     2340
   acagacaggc gggtttttta ggccttggtc catggggaaa gaagccccgc aatttcccca     2400
   tggctcaagt gcatcagggg ctgatgccaa ctgctccccc agaggaccca gctgtggatc     2460
   tgctaaagaa ctacatgcag ttgggcaagc agcagagaga aaagcagaga gaaagcagag     2520
   agaagcctta caaggaggtg acagaggatt tgctgcacct caattctctc tttggaggag     2580
   accagtagtc actgctcata ttgaaggaca gcctgtagaa gtattactgg atacaggggc     2640
   tgatgattct attgtaacag gaatagagtt aggtccacat tataccccaa aaatagtagg     2700
   aggaatagga ggttttatta atactaaaga atacaaaaat gtagaaatag aagttttagg     2760
   caaaaggatt aaagggacaa tcatgacagg ggacaccccg attaacattt ttggtagaaa     2820
   tttgctaaca gctctgggga tgtctctaaa ttttcccata gctaaagtag agcctgtaaa     2880
   agtcgcctta aagccaggaa aggatggacc aaaattgaag cagtggccat tatcaaaaga     2940
   aaagatagtt gcattaagag aaatctgtga aaagatggaa aaggatggtc agttggagga 3000
   agctcccccg accaatccat acaacacccc cacatttgct ataaagaaaa aggataagaa 3060
   caaatggaga atgctgatag attttaggga actaaatagg gtcactcagg actttacgga 3120
   agtccaatta ggaataccac accctgcagg actagcaaaa aggaaaagaa ttacagtact 3180
   ggatataggt gatgcatatt tctccatacc tctagatgaa gaatttaggc agtacactgc 3240
   ctttacttta ccatcagtaa ataatgcaga gccaggaaaa cgatacattt ataaggttct 3300
   gcctcaggga tggaaggggt caccagccat cttccaatac actatgagac atgtgctaga 3360
   acccttcagg aaggcaaatc cagatgtgac cttagtccag tatatggatg acatcttaat 3420
   agctagtgac aggacagacc tggaacatga cagggtagtt ttacagtcaa aggaactctt 3480
   gaatagcata gggttttcta ccccagaaga gaaattccaa aaagatcccc catttcaatg 3540
   gatggggtac gaattgtggc caacaaaatg gaagttgcaa aagatagagt tgccacaaag 3600
   agagacctgg acagtgaatg atatacagaa gttagtagga gtattaaatt gggcagctca 3660
   aatttatcca ggtataaaaa ccaaacatct ctgtaggtta attagaggaa aaatgactct 3720
   aacagaggaa gttcagtgga ctgagatggc agaagcagaa tatgaggaaa ataaaataat 3780
   tctcagtcag gaacaagaag gatgttatta ccaagaaggc aagccattag aagccacggt 3840
   aataaagagt caggacaatc agtggtctta taaaattcac caagaagaca aaatactgaa 3900
   agtaggaaaa tttgcaaaga taaagaatac acataccaat ggagtgagac tattagcaca 3960
   tgtaatacag aaaataggaa aggaagcaat agtgatctgg ggacaggtcc caaaattcca 4020
   cttaccagtt gagaaggatg tatgggaaca gtggtggaca gactattggc aggtaacctg 4080
   gataccggaa tgggatttta tctcaacacc accgctagta agattagtct tcaatctagt 4140
   gaaggacccc atagagggag aagaaaccta ttatacagat ggatcatgta ataaacagtc 4200
   aaaagaaggg aaagcaggat atatcacaga taggggcaaa gacaaagtaa aagtgttaga 4260
   acagactact aatcaacaag cagaattgga agcatttctc atggcattga cagactcagg 4320
   gccaaaggca aatattatag tagattcaca atatgttatg ggaataataa caggatgccc 4380
   tacagaatca gagagcaggc tagttaatca aataatagaa gaaatgatta aaaagtcaga 4440
   aatttatgta gcatgggtac cagcacacaa aggtatagga ggaaaccaag aaatagacca 4500
   cctagttagt caagggatta gacaagttct cttcttggaa aagatagagc cagcacaaga 4560
   agaacatgat aaataccata gtaatgtaaa agaattggta ttcaaatttg gattacccag 4620
   aatagtggcc agacagatag tagacacctg tgataaatgt catcagaaag gagaggctat 4680
   acatgggcag gcaaattcag atctagggac ttggcaaatg gattgtaccc atctagaggg 4740
   aaaaataatc atagttgcag tacatgtagc tagtggattc atagaagcag aggtaattcc 4800
   acaagagaca ggaagacaga cagcactatt tctgttaaaa ttggcaggca gatggcctat 4860
   tacacatcta cacacagata atggtgctaa ctttgcttcg caagaagtaa agatggttgc 4920
   atggtgggca gggatagagc acacctttgg ggtaccatac aatccacaga gtcagggagt 4980
   agtggaagca atgaatcacc acctgaaaaa tcaaatagat agaatcaggg aacaagcaaa 5040
   ttcagtagaa accatagtat taatggcagt tcattgcatg aattttaaaa gaaggggagg 5100
   aataggggat atgactccag cagaaagatt aattaacatg atcactacag aacaagagat 5160
   acaatttcaa caatcaaaaa actcaaaatt taaaaatttt cgggtctatt acagagaagg 5220
   cagagatcaa ctgtggaagg gacccggtga gctattgtgg aaaggggaag gagcagtcat 5280
   cttaaaggta gggacagaca ttaaggtagt acccagaaga aaggctaaaa ttatcaaaga 5340
   ttatggagga ggaaaagagg tggatagcag ttcccacatg gaggataccg gagaggctag 5400
   agaggtggca tagcctcata aaatatctga aatataaaac taaagatcta caaaaggttt 5460
   gctatgtgcc ccattttaag gtcggatggg catggtggac ctgcagcaga gtaatcttcc 5520
   cactacagga aggaagccat ttagaagtac aagggtattg gcatttgaca ccagaaaaag 5580
   ggtggctcag tacttatgca gtgaggataa cctggtactc aaagaacttt tggacagatg 5640
   taacaccaaa ctatgcagac attttactgc atagcactta tttcccttgc tttacagcgg 5700
   gagaagtgag aagggccatc aggggagaac aactgctgtc ttgctgcagg ttcccgagag 5760
   ctcataagta ccaggtacca agcctacagt acttagcact gaaagtagta agcgatgtca 5820
   gatcccaggg agagaatccc acctggaaac agtggagaag agacaatagg agaggccttc 5880
   gaatggctaa acagaacagt agaggagata aacagagagg cggtaaacca cctaccaagg 5940
   gagctaattt tccaggtttg gcaaaggtct tgggaatact ggcatgatga acaagggatg 6000
   tcaccaagct atgtaaaata cagatacttg tgtttaatac aaaaggcttt atttatgcat 6060
   tgcaagaaag gctgtagatg tctaggggaa ggacatgggg cagggggatg gagaccagga 6120
   cctcctcctc ctccccctcc aggactagca taaatggaag aaagacctcc agaaaatgaa 6180
   ggaccacaaa gggaaccatg ggatgaatgg gtagtggagg ttctggaaga actgaaagaa 6240
   gaagctttaa aacattttga tcctcgcttg ctaactgcac ttggtaatca tatctataat 6300
   agacatggag acacccttga gggagcagga gaactcatta gaatcctcca acgagcgctc 6360
   ttcatgcatt tcagaggcgg atgcatccac tccagaatcg gccaacctgg gggaggaaat 6420
   cctctctcag ctataccgcc ctctagaagc atgctataac acatgctatt gtaaaaagtg 6480
   ttgctaccat tgccagtttt gttttcttaa aaaaggcttg gggatatgtt atgagcaatc 6540
   acgaaagaga agaagaactc cgaaaaaggc taaggctaat acatcttctg catcaaacaa 6600
   gtaagtatgg gatgtcttgg gaatcagctg cttatcgcca tcttgctttt aagtgtctat 6660
   gggatctatt gtactctata tgtcacagtc ttttatggtg taccagcttg gaggaatgcg 6720
   acaattcccc tcttttgtgc aaccaagaat agggatactt ggggaacaac tcagtgccta 6780
   ccagataatg gtgattattc agaagtggcc cttaatgtta cagaaagctt tgatgcctgg 6840
   aataatacag tcacagaaca ggcaatagag gatgtatggc aactctttga gacctcaata 6900
   aagccttgtg taaaattatc cccattatgc attactatga gatgcaataa aagtgagaca 6960
   gatagatggg gattgacaaa atcaataaca acaacagcat caacaacatc aacgacagca 7020
   tcagcaaaag tagacatggt caatgagact agttcttgta tagcccagga taattgcaca 7080
   ggcttggaac aagagcaaat gataagctgt aaattcaaca tgacagggtt aaaaagagac 7140
   aagaaaaaag agtacaatga aacttggtac tctgcagatt tggtatgtga acaagggaat 7200
   aacactggta atgaaagtag atgttacatg aaccactgta acacttctgt tatccaagag 7260
   tcttgtgaca aacattattg ggatgctatt agatttaggt attgtgcacc tccaggttat 7320
   gctttgctta gatgtaatga cacaaattat tcaggcttta tgcctaaatg ttctaaggtg 7380
   gtggtctctt catgcacaag gatgatggag acacagactt ctacttggtt tggctttaat 7440
   ggaactagag cagaaaatag aacttatatt tactggcatg gtagggataa taggactata 7500
   attagtttaa ataagtatta taatctaaca atgaaatgta gaagaccagg aaataagaca 7560
   gttttaccag tcaccattat gtctggattg gttttccact cacaaccaat caatgatagg 7620
   ccaaagcagg catggtgttg gtttggagga aaatggaagg atgcaataaa agaggtgaag 7680
   cagaccattg tcaaacatcc caggtatact ggaactaaca atactgataa aatcaatttg 7740
   acggctcctg gaggaggaga tccggaagtt accttcatgt ggacaaattg cagaggagag 7800
   ttcctctact gtaaaatgaa ttggtttcta aattgggtag aagataggaa tacagctaac 7860
   cagaagccaa aggaacagca taaaaggaat tacgtgccat gtcatattag acaaataatc 7920
   aacacttggc ataaagtagg caaaaatgtt tatttgcctc caagagaggg agacctcacg 7980
   tgtaactcca cagtgaccag tctcatagca aacatagatt ggattgatgg aaaccaaact 8040
   aatatcacca tgagtgcaga ggtggcagaa ctgtatcgat tggaattggg agattataaa 8100
   ttagtagaga tcactccaat tggcttggcc cccacagatg tgaagaggta cactactggt 8160
   ggcacctcaa gaaataaaag aggggtcttt gtgctagggt tcttgggttt tctcgcaacg 8220
   gcaggttctg caatgggcgc ggcgtcgttg acgctgaccg ctcagtcccg aactttattg 8280
   gctgggatag tgcagcaaca gcaacagctg ttggacgtgg tcaagagaca acaagaattg 8340
   ttgcgactga ccgtctgggg aacaaagaac ctccagacta gggtcactgc catcgagaag 8400
   tacttaaagg accaggcgca gctgaatgct tggggatgtg cgtttagaca agtctgccac 8460
   actactgtac catggccaaa tgcaagtcta acaccaaagt ggaacaatga gacttggcaa 8520
   gagtgggagc gaaaggttga cttcttggaa gaaaatataa cagccctcct agaggaggca 8580
   caaattcaac aagagaagaa catgtatgaa ttacaaaagt tgaatagctg ggatgtgttt 8640
   ggcaattggt ttgaccttgc ttcttggata aagtatatac aatatggagt ttatatagtt 8700
   gtaggagtaa tactgttaag aatagtgatc tatatagtac aaatgctagc taagttaagg 8760
   caggggtata ggccagtgtt ctcttcccca ccctcttatt tccagcagac ccatatccaa 8820
   caggacccgg cactgccaac cagagaaggc aaagaaagag acggtggaga aggcggtggc 8880
   aacagctcct ggccttggca gatagaatat attcatttcc tgatccgcca actgatacgc 8940
   ctcttgactt ggctattcag caactgcaga accttgctat cgagagtata ccagatcctc 9000
   caaccaatac tccagaggct ctctgcgacc ctacagagga ttcgagaagt cctcaggact 9060
   gaactgacct acctacaata tgggtggagc tatttccatg aggcggtcca ggccgtctgg 9120
   agatctgcga cagagactct tgcgggcgcg tggggagact tatgggagac tcttaggaga 9180
   ggtggaagat ggatactcgc aatccccagg aggattagac aagggcttga gctcactctc 9240
   ttgtgaggga cagaaataca atcagggaca gtatatgaat actccatgga gaaacccagc 9300
   tgaagagaga gaaaaattag catacagaaa acaaaatatg gatgatatag atgaggaaga 9360
   tgatgacttg gtaggggtat cagtgaggcc aaaagttccc ctaagaacaa tgagttacaa 9420
   attggcaata gacatgtctc attttataaa agaaaagggg ggactggaag ggatttatta 9480
   cagtgcaaga agacatagaa tcttagacat atacttagaa aaggaaggag gcatcatacc 9540
   agattggcag gattacacct caggaccagg aattagatac ccaaagacat ttggctggct 9600
   atggaaatta gtccctgtaa atgtatcaga tgaggcacag gaggatgagg agcattattt 9660
   aatgcatcca gctcaaactt cccagtggga tgacccttgg ggagaggttc tagcatggaa 9720
   gtttgatcca actctggcct acacttatga ggcatatgtt agatacccag aagagtttgg 9780
   aagcaagtca ggcctgtcag aggaagaggt tagaagaagg ctaaccgcaa gaggccttct 9840
   taacatggct gacaagaagg aaactcgctg aacgcgtatt gcattactag ttattaatag 9900
   taatcaatta cggggtcatt agttcatagc ccatatatgg agttccgcgt tacataactt 9960
   acggtaaatg gcccgcctgg ctgaccgccc aacgaccccc gcccattgac gtcaataatg 10020
   acgtatgttc ccatagtaac gccaataggg actttccatt gacgtcaatg ggtggagtat 10080
   ttacggtaaa ctgcccactt ggcagtacat caagtgtatc atatgccaag tccgccccct 10140
   attgacgtca atgacggtaa atggcccgcc tggcattatg cccagtacat gaccttacgg 10200
   gactttccta cttggcagta catctacgta ttagtcatcg ctattaccat ggtgatgcgg 10260
   ttttggcagt acaccaatgg gcgtggatag cggtttgact cacggggatt tccaagtctc 10320
   caccccattg acgtcaatgg gagtttgttt tggcaccaaa atcaacggga ctttccaaaa 10380
   tgtcgtaata accccgcccc gttgacgcaa atgggcggta ggcgtgtacg gtgggaggtc 10440
   tatataagca gagctcgttt agtgaaccgt cagaattgtt tttattttta attttctttc 10500
   aaatacttcc atcgaattca gatctggtac cacgcgtacc atggtgccca agaagaagag 10560
   gaaagtctcc aacctgctga ctgtgcacca aaacctgcct gccctccctg tggatgccac 10620
   ctctgatgaa gtcaggaaga acctgatgga catgttcagg gacaggcagg ccttctctga 10680
   acacacctgg aagatgctcc tgtctgtgtg cagatcctgg gctgcctggt gcaagctgaa 10740
   caacaggaaa tggttccctg ctgaacctga ggatgtgagg gactacctcc tgtacctgca 10800
   agccagaggc ctggctgtga agaccatcca acagcacctg ggccagctca acatgctgca 10860
   caggagatct ggcctgcctc gcccttctga ctccaatgct gtgtccctgg tgatgaggag 10920
   aatcagaaag gagaatgtgg atgctgggga gagagccaag caggccctgg cctttgaacg 10980
   cactgacttt gaccaagtca gatccctgat ggagaactct gacagatgcc aggacatcag 11040
   gaacctggcc ttcctgggca ttgcctacaa caccctgctg cgcattgccg aaattgccag 11100
   aatcagagtg aaggacatct cccgcaccga tggtgggaga atgctgatcc acattggcag 11160
   gaccaagacc ctggtgtcca cagctggtgt ggagaaggcc ctgtccctgg gggttaccaa 11220
   gctggtggag agatggatct ctgtgtctgg tgtggctgat gaccccaaca actacctgtt 11280
   ctgccgggtc agaaagaatg gtgtggctgc cccttctgcc acctcccaac tgtccacccg 11340
   ggccctggaa gggatctttg aggccaccca ccgcctgatc tatggtgcca aggatgactc 11400
   tgggcagaga tacctggcct ggtctggcca ctctgccaga gtgggtgctg ccagggacat 11460
   ggccagggct ggtgtgtcca tccctgaaat catgcaggct ggtggctgga ccaatgtgaa 11520
   catagtgatg aactacatca gaaacctgga ctctgagact ggggccatgg tgaggctgct 11580
   cgaggatggg gacggtggtg gtggcaagct ttgacgcatc ccgggtctca ttttataaaa 11640
   gaaaaggggg gactggaagg gatttatccg cggttcactc gagactcgct gaaacagcag 11700
   ggactttcca caaggggatg ttacggggag gtactgggga ggagccggtc gggaacgccc 11760
   actttcttga tgtataaata tcactgcatt tcgctctgta ttcagtcgct ctgcggagag 11820
   gctggcagat tgagccctgg gaggttctct ccagcactag caggtagagc ctgggtgttc 11880
   cctgctagac tctcaccagc acttggccgg tgctgggcag agtgactcca cgcttgcttg 11940
   cttaaagccc tcttcaataa agctgccatt ttagaagtaa gctagtgtgt gttcccatct 12000
   ctcctagccg ccgcctggtc aactcggtac tcaataataa gaagaccctg gtctgttagg 12060
   accctttctg ctttgggaaa ccgaagcagg aaaatcccta gcagaattct tgaagacgaa 12120
   agggcctcgt gatacgccta tttttatagg ttaatgtcat gataataatg gtttcttaga 12180
   cgtcaggtgg cacttttcgg ggaaatgtgc gcggaacccc tatttgttta tttttctaaa 12240
   tacattcaaa tatgtatccg ctcatgagac aataaccctg ataaatgctt caataatatt 12300
   gaaaaaggaa gagtatgagt attcaacatt tccgtgtcgc ccttattccc ttttttgcgg 12360
   cattttgcct tcctgttttt gctcacccag aaacgctggt gaaagtaaaa gatgctgaag 12420
   atcagttggg tgcacgagtg ggttacatcg aactggatct caacagcggt aagatccttg 12480
   agagttttcg ccccgaagaa cgttttccaa tgatgagcac ttttaaagtt ctgctatgtg 12540
   gcgcggtatt atcccgtgtt gacgccgggc aagagcaact cggtcgccgc atacactatt 12600
   ctcagaatga cttggttgag tactcaccag tcacagaaaa gcatcttacg gatggcatga 12660
   cagtaagaga attatgcagt gctgccataa ccatgagtga taacactgcg gccaacttac 12720
   ttctgacaac gatcggagga ccgaaggagc taaccgcttt tttgcacaac atgggggatc 12780
   atgtaactcg ccttgatcgt tgggaaccgg agctgaatga agccatacca aacgacgagc 12840
   gtgacaccac gatgcctgca gcaatggcaa caacgttgcg caaactatta actggcgaac 12900
   tacttactct agcttcccgg caacaattaa tagactggat ggaggcggat aaagttgcag 12960
   gaccacttct gcgctcggcc cttccggctg gctggtttat tgctgataaa tctggagccg 13020
   gtgagcgtgg gtctcgcggt atcattgcag cactggggcc agatggtaag ccctcccgta 13080
   tcgtagttat ctacacgacg gggagtcagg caactatgga tgaacgaaat agacagatcg 13140
   ctgagatagg tgcctcactg attaagcatt ggtaactgtc agaccaagtt tactcatata 13200
   tactttagat tgatttaaaa cttcattttt aatttaaaag gatctaggtg aagatccttt 13260
   ttgataatct catgaccaaa atcccttaac gtgagttttc gttccactga gcgtcagacc 13320
   ccgtagaaaa gatcaaagga tcttcttgag atcctttttt tctgcgcgta atctgctgct 13380
   tgcaaacaaa aaaaccaccg ctaccagcgg tggtttgttt gccggatcaa gagctaccaa 13440
   ctctttttcc gaaggtaact ggcttcagca gagcgcagat accaaatact gtccttctag 13500
   tgtagccgta gttaggccac cacttcaaga actctgtagc accgcctaca tacctcgctc 13560
   tgctaatcct gttaccagtg gctgctgcca gtggcgataa gtcgtgtctt accgggttgg 13620
   actcaagacg atagttaccg gataaggcgc agcggtcggg ctgaacgggg ggttcgtgca 13680
   cacagcccag cttggagcga acgacctaca ccgaactgag atacctacag cgtgagctat 13740
   gagaaagcgc cacgcttccc gaagggagaa aggcggacag gtatccggta agcggcaggg 13800
   tcggaacagg agagcgcacg agggagcttc cagggggaaa cgcctggtat ctttatagtc 13860
   ctgtcgggtt tcgccacctc tgacttgagc gtcgattttt gtgatgctcg tcaggggggc 13920
   ggagcctatg gaaaaacgcc agcaacgcgg cctttttacg gttcctggcc ttttgctggc 13980
   cttttgctca catgttcttt cctgcgttat cccctgattc tgtggataac cgtattaccg 14040
   cctttgagtg agctgatacc gctcgccgca gccgaacgac cgagcgcagc gagtcagtga 14100
   gcgaggaagc ggaagagcgc ctgatgcggt attttctcct tacgcatctg tgcggtattt 14160
   cacaccgcat atggtgcact ctcagtacaa tctgctctga tgccgcatag ttaagccagt 14220
   atacactccg ctatcgctac gtgactgggt catggctgcg ccccgacacc cgccaacacc 14280
   cgctgacgcg ccctgacggg cttgtctgct cccggcatcc gcttacagac aagctgtgac 14340
   cgtctccggg agctgcatgt gtcagaggtt ttcaccgtca tcaccgaaac gcgcgaggca 14400
   gatcccgcaa gaggcccggc agtaccggca taaccaagcc tatgcctaca gcatccaggg 14460
   tgacggtgcc gaggatgacg atgagcgcat tgttagattt catacacggt gcctgactgc 14520
   gttagcaatt taactgtgat aaactaccgc attaaagctt actgtaaatt tactggctgt 14580
   cttccttgca ggtttc 14596
<210> 16
   <211> 14596
   <212> DNA
   <213> Artificial Sequence
<220>
   <223> pBR-SIVmac239-Nef (codon opt)-CMV-Cre :
<400> 16
   tggaagggat ttattacagt gcaagaagac atagaatctt agacatatac ttagaaaagg 60
   aagaaggcat cataccagat tggcaggatt acacctcagg accaggaatt agatacccaa 120
   agacatttgg ctggctatgg aaattagtcc ctgtaaatgt atcagatgag gcacaggagg 180
   atgaggagca ttatttaatg catccagctc aaacttccca gtgggatgac ccttggggag 240
   aggttctagc atggaagttt gatccaactc tggcctacac ttatgaggca tatgttagat 300
   acccagaaga gtttggaagc aagtcaggcc tgtcagagga agaggttaga agaaggctaa 360
   ccgcaagagg ccttcttaac atggctgaca agaaggaaac tcgctgaaac agcagggact 420
   ttccacaagg ggatgttacg gggaggtact ggggaggagc cggtcgggaa cgcccacttt 480
   cttgatgtat aaatatcact gcatttcgct ctgtattcag tcgctctgcg gagaggctgg 540
   cagattgagc cctgggaggt tctctccagc actagcaggt agagcctggg tgttccctgc 600
   tagactctca ccagcacttg gccggtgctg ggcagagtga ctccacgctt gcttgcttaa 660
   agccctcttc aataaagctg ccattttagg aagtaagcta gtgtgtgttc ccatctctcc 720
   tagccgccgc ctggtcaact cggtacttca ataataagaa gactcctggt ctgttaggac 780
   cctttctgct ttgggaaacc gaagcaggaa aatccctagc agattggcgc ctgaacaggg 840
   acttgaagga gagtgagaga ctcctgagta cggctgagtg aaggcagtaa gggcggcagg 900
   aaccaaccac gacggagtgc tcctataaag gcgcgggtcg gtaccagacg gcgtgaggag 960
   cgggagagga agaggcctcc ggttgcaggt aagtgcaaca caaaaaagaa atagctgtct 1020
   tttatccagg aaggggtaat aagatagagt gggagatggg cgtgagaaac tccgtcttgt 1080
   cagggaagaa agcagatgaa ttagaaaaaa ttaggctacg acccaacgga aagaaaaagt 1140
   acatgttgaa gcatgtagta tgggcagcaa atgaattaga tagatttgga ttagcagaaa 1200
   gcctgttgga gaacaaagaa ggatgtcaaa aaatactttc ggtcttagct ccattagtgc 1260
   caacaggctc agaaaattta aaaagccttt ataatactgt ctgcgtcatc tggtgcattc 1320
   acgcagaaga gaaagtgaaa cacactgagg aagcaaaaca gatagtgcag agacacctag 1380
   tggtggaaac aggaacaaca gaaactatgc caaaaacaag tagaccaaca gcaccatcta 1440
   gcggcagagg aggaaattac ccagtacaac aaataggtgg taactatgtc cacctgccat 1500
   taagcccgag aacattaaat gcctgggtaa aattgataga ggaaaagaaa tttggagcag 1560
   aagtagtgcc aggatttcag gcactgtcag aaggttgcac cccctatgac attaatcaga 1620
   tgttaaattg tgtgggagac catcaagcgg ctatgcagat tatcagagat attataaacg 1680
   aggaggctgc agattgggac ttgcagcacc cacaaccagc tccacaacaa ggacaactta 1740
   gggagccgtc aggatcagat attgcaggaa caactagttc agtagatgaa caaatccagt 1800
   ggatgtacag acaacagaac cccataccag taggcaacat ttacaggaga tggatccaac 1860
   tggggttgca aaaatgtgtc agaatgtata acccaacaaa cattctagat gtaaaacaag 1920
   ggccaaaaga gccatttcag agctatgtag acaggttcta caaaagttta agagcagaac 1980
   agacagatgc agcagtaaag aattggatga ctcaaacact gctgattcaa aatgctaacc 2040
   cagattgcaa gctagtgctg aaggggctgg gtgtgaatcc caccctagaa gaaatgctga 2100
   cggcttgtca aggagtaggg gggccgggac agaaggctag attaatggca gaagccctga 2160
   aagaggccct cgcaccagtg ccaatccctt ttgcagcagc ccaacagagg ggaccaagaa 2220
   agccaattaa gtgttggaat tgtgggaaag agggacactc tgcaaggcaa tgcagagccc 2280
   caagaagaca gggatgctgg aaatgtggaa aaatggacca tgttatggcc aaatgcccag 2340
   acagacaggc gggtttttta ggccttggtc catggggaaa gaagccccgc aatttcccca 2400
   tggctcaagt gcatcagggg ctgatgccaa ctgctccccc agaggaccca gctgtggatc 2460
   tgctaaagaa ctacatgcag ttgggcaagc agcagagaga aaagcagaga gaaagcagag 2520
   agaagcctta caaggaggtg acagaggatt tgctgcacct caattctctc tttggaggag 2580
   accagtagtc actgctcata ttgaaggaca gcctgtagaa gtattactgg atacaggggc 2640
   tgatgattct attgtaacag gaatagagtt aggtccacat tataccccaa aaatagtagg 2700
   aggaatagga ggttttatta atactaaaga atacaaaaat gtagaaatag aagttttagg 2760
   caaaaggatt aaagggacaa tcatgacagg ggacaccccg attaacattt ttggtagaaa 2820
   tttgctaaca gctctgggga tgtctctaaa ttttcccata gctaaagtag agcctgtaaa 2880
   agtcgcctta aagccaggaa aggatggacc aaaattgaag cagtggccat tatcaaaaga 2940
   aaagatagtt gcattaagag aaatctgtga aaagatggaa aaggatggtc agttggagga 3000
   agctcccccg accaatccat acaacacccc cacatttgct ataaagaaaa aggataagaa 3060
   caaatggaga atgctgatag attttaggga actaaatagg gtcactcagg actttacgga 3120
   agtccaatta ggaataccac accctgcagg actagcaaaa aggaaaagaa ttacagtact 3180
   ggatataggt gatgcatatt tctccatacc tctagatgaa gaatttaggc agtacactgc 3240
   ctttacttta ccatcagtaa ataatgcaga gccaggaaaa cgatacattt ataaggttct 3300
   gcctcaggga tggaaggggt caccagccat cttccaatac actatgagac atgtgctaga 3360
   acccttcagg aaggcaaatc cagatgtgac cttagtccag tatatggatg acatcttaat 3420
   agctagtgac aggacagacc tggaacatga cagggtagtt ttacagtcaa aggaactctt 3480
   gaatagcata gggttttcta ccccagaaga gaaattccaa aaagatcccc catttcaatg 3540
   gatggggtac gaattgtggc caacaaaatg gaagttgcaa aagatagagt tgccacaaag 3600
   agagacctgg acagtgaatg atatacagaa gttagtagga gtattaaatt gggcagctca 3660
   aatttatcca ggtataaaaa ccaaacatct ctgtaggtta attagaggaa aaatgactct 3720
   aacagaggaa gttcagtgga ctgagatggc agaagcagaa tatgaggaaa ataaaataat 3780
   tctcagtcag gaacaagaag gatgttatta ccaagaaggc aagccattag aagccacggt 3840
   aataaagagt caggacaatc agtggtctta taaaattcac caagaagaca aaatactgaa 3900
   agtaggaaaa tttgcaaaga taaagaatac acataccaat ggagtgagac tattagcaca 3960
   tgtaatacag aaaataggaa aggaagcaat agtgatctgg ggacaggtcc caaaattcca 4020
   cttaccagtt gagaaggatg tatgggaaca gtggtggaca gactattggc aggtaacctg 4080
   gataccggaa tgggatttta tctcaacacc accgctagta agattagtct tcaatctagt 4140
   gaaggaccct atagagggag aagaaaccta ttatacagat ggatcatgta ataaacagtc 4200
   aaaagaaggg aaagcaggat atatcacaga taggggcaaa gacaaagtaa aagtgttaga 4260
   acagactact aatcaacaag cagaattgga agcatttctc atggcattga cagactcagg 4320
   gccaaaggca aatattatag tagattcaca atatgttatg ggaataataa caggatgccc 4380
   tacagaatca gagagcaggc tagttaatca aataatagaa gaaatgatta aaaagtcaga 4440
   aatttatgta gcatgggtac cagcacacaa aggtatagga ggaaaccaag aaatagacca 4500
   cctagttagt caagggatta gacaagttct cttcttggaa aagatagagc cagcacaaga 4560
   agaacatgat aaataccata gtaatgtaaa agaattggta ttcaaatttg gattacccag 4620
   aatagtggcc agacagatag tagacacctg tgataaatgt catcagaaag gagaggctat 4680
   acatgggcag gcaaattcag atctagggac ttggcaaatg gattgtaccc atctagaggg 4740
   aaaaataatc atagttgcag tacatgtagc tagtggattc atagaagcag aggtaattcc 4800
   acaagagaca ggaagacaga cagcactatt tctgttaaaa ttggcaggca gatggcctat 4860
   tacacatcta cacacagata atggtgctaa ctttgcttcg caagaagtaa agatggttgc 4920
   atggtgggca gggatagagc acacctttgg ggtaccatac aatccacaga gtcagggagt 4980
   agtggaagca atgaatcacc acctgaaaaa tcaaatagat agaatcaggg aacaagcaaa 5040
   ttcagtagaa accatagtat taatggcagt tcattgcatg aattttaaaa gaaggggagg 5100
   aataggggat atgactccag cagaaagatt aattaacatg atcactacag aacaagagat 5160
   acaatttcaa caatcaaaaa actcaaaatt taaaaatttt cgggtctatt acagagaagg 5220
   cagagatcaa ctgtggaagg gacccggtga gctattgtgg aaaggggaag gagcagtcat 5280
   cttaaaggta gggacagaca ttaaggtagt acccagaaga aaggctaaaa ttatcaaaga 5340
   ttatggagga ggaaaagagg tggatagcag ttcccacatg gaggataccg gagaggctag 5400
   agaggtggca tagcctcata aaatatctga aatataaaac taaagatcta caaaaggttt 5460
   gctatgtgcc ccattttaag gtcggatggg catggtggac ctgcagcaga gtaatcttcc 5520
   cactacagga aggaagccat ttagaagtac aagggtattg gcatttgaca ccagaaaaag 5580
   ggtggctcag tacttatgca gtgaggataa cctggtactc aaagaacttt tggacagatg 5640
   taacaccaaa ctatgcagac attttactgc atagcactta tttcccttgc tttacagcgg 5700
   gagaagtgag aagggccatc aggggagaac aactgctgtc ttgctgcagg ttcccgagag 5760
   ctcataagta ccaggtacca agcctacagt acttagcact gaaagtagta agcgatgtca 5820
   gatcccaggg agagaatccc acctggaaac agtggagaag agacaatagg agaggccttc 5880
   gaatggctaa acagaacagt agaggagata aacagagagg cggtaaacca cctaccaagg 5940
   gagctaattt tccaggtttg gcaaaggtct tgggaatact ggcatgatga acaagggatg 6000
   tcaccaagct atgtaaaata cagatacttg tgtttaatac aaaaggcttt atttatgcat 6060
   tgcaagaaag gctgtagatg tctaggggaa ggacatgggg cagggggatg gagaccagga 6120
   cctcctcctc ctccccctcc aggactagca taaatggaag aaagacctcc agaaaatgaa 6180
   ggaccacaaa gggaaccatg ggatgaatgg gtagtggagg ttctggaaga actgaaagaa 6240
   gaagctttaa aacattttga tcctcgcttg ctaactgcac ttggtaatca tatctataat 6300
   agacatggag acacccttga gggagcagga gaactcatta gaatcctcca acgagcgctc 6360
   ttcatgcatt tcagaggcgg atgcatccac tccagaatcg gccaacctgg gggaggaaat 6420
   cctctctcag ctataccgcc ctctagaagc atgctataac acatgctatt gtaaaaagtg 6480
   ttgctaccat tgccagtttt gttttcttaa aaaaggcttg gggatatgtt atgagcaatc 6540
   acgaaagaga agaagaactc cgaaaaaggc taaggctaat acatcttctg catcaaacaa 6600
   gtaagtatgg gatgtcttgg gaatcagctg cttatcgcca tcttgctttt aagtgtctat 6660
   gggatctatt gtactctata tgtcacagtc ttttatggtg taccagcttg gaggaatgcg 6720
   acaattcccc tcttttgtgc aaccaagaat agggatactt ggggaacaac tcagtgccta 6780
   ccagataatg gtgattattc agaagtggcc cttaatgtta cagaaagctt tgatgcctgg 6840
   aataatacag tcacagaaca ggcaatagag gatgtatggc aactctttga gacctcaata 6900
   aagccttgtg taaaattatc cccattatgc attactatga gatgcaataa aagtgagaca 6960
   gatagatggg gattgacaaa atcaataaca acaacagcat caacaacatc aacgacagca 7020
   tcagcaaaag tagacatggt caatgagact agttcttgta tagcccagga taattgcaca 7080
   ggcttggaac aagagcaaat gataagctgt aaattcaaca tgacagggtt aaaaagagac 7140
   aagaaaaaag agtacaatga aacttggtac tctgcagatt tggtatgtga acaagggaat 7200
   aacactggta atgaaagtag atgttacatg aaccactgta acacttctgt tatccaagag 7260
   tcttgtgaca aacattattg ggatgctatt agatttaggt attgtgcacc tccaggttat 7320
   gctttgctta gatgtaatga cacaaattat tcaggcttta tgcctaaatg ttctaaggtg 7380
   gtggtctctt catgcacaag gatgatggag acacagactt ctacttggtt tggctttaat 7440
   ggaactagag cagaaaatag aacttatatt tactggcatg gtagggataa taggactata 7500
   attagtttaa ataagtatta taatctaaca atgaaatgta gaagaccagg aaataagaca 7560
   gttttaccag tcaccattat gtctggattg gttttccact cacaaccaat caatgatagg 7620
   ccaaagcagg catggtgttg gtttggagga aaatggaagg atgcaataaa agaggtgaag 7680
   cagaccattg tcaaacatcc caggtatact ggaactaaca atactgataa aatcaatttg 7740
   acggctcctg gaggaggaga tccggaagtt accttcatgt ggacaaattg cagaggagag 7800
   ttcctctact gtaaaatgaa ttggtttcta aattgggtag aagataggaa tacagctaac 7860
   cagaagccaa aggaacagca taaaaggaat tacgtgccat gtcatattag acaaataatc 7920
   aacacttggc ataaagtagg caaaaatgtt tatttgcctc caagagaggg agacctcacg 7980
   tgtaactcca cagtgaccag tctcatagca aacatagatt ggattgatgg aaaccaaact 8040
   aatatcacca tgagtgcaga ggtggcagaa ctgtatcgat tggaattggg agattataaa 8100
   ttagtagaga tcactccaat tggcttggcc cccacagatg tgaagaggta cactactggt 8160
   ggcacctcaa gaaataaaag aggggtcttt gtgctagggt tcttgggttt tctcgcaacg 8220
   gcaggttctg caatgggcgc ggcgtcgttg acgctgaccg ctcagtcccg aactttattg 8280
   gctgggatag tgcagcaaca gcaacagctg ttggacgtgg tcaagagaca acaagaattg 8340
   ttgcgactga ccgtctgggg aacaaagaac ctccagacta gggtcactgc catcgagaag 8400
   tacttaaagg accaggcgca gctgaatgct tggggatgtg cgtttagaca agtctgccac 8460
   actactgtac catggccaaa tgcaagtcta acaccaaagt ggaacaatga gacttggcaa 8520
   gagtgggagc gaaaggttga cttcttggaa gaaaatataa cagccctcct agaggaggca 8580
   caaattcaac aagagaagaa catgtatgaa ttacaaaagt tgaatagctg ggatgtgttt 8640
   ggcaattggt ttgaccttgc ttcttggata aagtatatac aatatggagt ttatatagtt 8700
   gtaggagtaa tactgttaag aatagtgatc tatatagtac aaatgctagc taagttaagg 8760
   caggggtata ggccagtgtt ctcttcccca ccctcttatt tccagcagac ccatatccaa 8820
   caggacccgg cactgccaac cagagaaggc aaagaaagag acggtggaga aggcggtggc 8880
   aacagctcct ggccttggca gatagaatat attcatttcc tgatccgcca actgatacgc 8940
   ctcttgactt ggctattcag caactgcaga accttgctat cgagagtata ccagatcctc 9000
   caaccaatac tccagaggct ctctgcgacc ctacagagga ttcgagaagt cctcaggact 9060
   gaactgacct acctacaata tgggtggagc tatttccatg aggcggtcca ggccgtctgg 9120
   agatctgcga cagagactct tgcgggcgcg tggggagact tatgggagac tcttaggaga 9180
   ggtggaagat ggatactcgc aatccccagg aggattagac aagggcttga gctcactctc 9240
   ttgtgaggga cagaaataca atcagggaca gtatatgaat actccatgga gaaacccagc 9300
   tgaagagaga gaaaaattag catacagaaa acaaaatatg gatgatatag atgaggaaga 9360
   tgatgacttg gtaggggtat cagtgaggcc aaaagttccc ctaagaacaa tgagttacaa 9420
   attggcaatt gacatgtcac atttcattaa ggagaagggc ggactggagg ggatatacta 9480
   ttctgccagg aggcatagaa tcttagacat atacttagaa aaggaaggag gcatcatacc 9540
   agattggcag gattacacct caggaccagg aattagatac ccaaagacat ttggctggct 9600
   atggaaatta gtccctgtaa atgtatcaga tgaggcacag gaggatgagg agcattattt 9660
   aatgcatcca gctcaaactt cccagtggga tgacccttgg ggagaggttc tagcatggaa 9720
   gtttgatcca actctggcct acacttatga ggcatatgtt agatacccag aagagtttgg 9780
   aagcaagtca ggcctgtcag aggaagaggt tagaagaagg ctaaccgcaa gaggccttct 9840
   taacatggct gacaagaagg aaactcgctg aacgcgtatt gcattactag ttattaatag 9900
   taatcaatta cggggtcatt agttcatagc ccatatatgg agttccgcgt tacataactt 9960
   acggtaaatg gcccgcctgg ctgaccgccc aacgaccccc gcccattgac gtcaataatg 10020
   acgtatgttc ccatagtaac gccaataggg actttccatt gacgtcaatg ggtggagtat 10080
   ttacggtaaa ctgcccactt ggcagtacat caagtgtatc atatgccaag tccgccccct 10140
   attgacgtca atgacggtaa atggcccgcc tggcattatg cccagtacat gaccttacgg 10200
   gactttccta cttggcagta catctacgta ttagtcatcg ctattaccat ggtgatgcgg 10260
   ttttggcagt acaccaatgg gcgtggatag cggtttgact cacggggatt tccaagtctc 10320
   caccccattg acgtcaatgg gagtttgttt tggcaccaaa atcaacggga ctttccaaaa 10380
   tgtcgtaata accccgcccc gttgacgcaa atgggcggta ggcgtgtacg gtgggaggtc 10440
   tatataagca gagctcgttt agtgaaccgt cagaattgtt tttattttta attttctttc 10500
   aaatacttcc atcgaattca gatctggtac cacgcgtacc atggtgccca agaagaagag 10560
   gaaagtctcc aacctgctga ctgtgcacca aaacctgcct gccctccctg tggatgccac 10620
   ctctgatgaa gtcaggaaga acctgatgga catgttcagg gacaggcagg ccttctctga 10680
   acacacctgg aagatgctcc tgtctgtgtg cagatcctgg gctgcctggt gcaagctgaa 10740
   caacaggaaa tggttccctg ctgaacctga ggatgtgagg gactacctcc tgtacctgca 10800
   agccagaggc ctggctgtga agaccatcca acagcacctg ggccagctca acatgctgca 10860
   caggagatct ggcctgcctc gcccttctga ctccaatgct gtgtccctgg tgatgaggag 10920
   aatcagaaag gagaatgtgg atgctgggga gagagccaag caggccctgg cctttgaacg 10980
   cactgacttt gaccaagtca gatccctgat ggagaactct gacagatgcc aggacatcag 11040
   gaacctggcc ttcctgggca ttgcctacaa caccctgctg cgcattgccg aaattgccag 11100
   aatcagagtg aaggacatct cccgcaccga tggtgggaga atgctgatcc acattggcag 11160
   gaccaagacc ctggtgtcca cagctggtgt ggagaaggcc ctgtccctgg gggttaccaa 11220
   gctggtggag agatggatct ctgtgtctgg tgtggctgat gaccccaaca actacctgtt 11280
   ctgccgggtc agaaagaatg gtgtggctgc cccttctgcc acctcccaac tgtccacccg 11340
   ggccctggaa gggatctttg aggccaccca ccgcctgatc tatggtgcca aggatgactc 11400
   tgggcagaga tacctggcct ggtctggcca ctctgccaga gtgggtgctg ccagggacat 11460
   ggccagggct ggtgtgtcca tccctgaaat catgcaggct ggtggctgga ccaatgtgaa 11520
   catagtgatg aactacatca gaaacctgga ctctgagact ggggccatgg tgaggctgct 11580
   cgaggatggg gacggtggtg gtggcaagct ttgacgcatc ccgggtctca ttttataaaa 11640
   gaaaaggggg gactggaagg gatttatccg cggttcactc gagactcgct gaaacagcag 11700
   ggactttcca caaggggatg ttacggggag gtactgggga ggagccggtc gggaacgccc 11760
   actttcttga tgtataaata tcactgcatt tcgctctgta ttcagtcgct ctgcggagag 11820
   gctggcagat tgagccctgg gaggttctct ccagcactag caggtagagc ctgggtgttc 11880
   cctgctagac tctcaccagc acttggccgg tgctgggcag agtgactcca cgcttgcttg 11940
   cttaaagccc tcttcaataa agctgccatt ttagaagtaa gctagtgtgt gttcccatct 12000
   ctcctagccg ccgcctggtc aactcggtac tcaataataa gaagaccctg gtctgttagg 12060
   accctttctg ctttgggaaa ccgaagcagg aaaatcccta gcagaattct tgaagacgaa 12120
   agggcctcgt gatacgccta tttttatagg ttaatgtcat gataataatg gtttcttaga 12180
   cgtcaggtgg cacttttcgg ggaaatgtgc gcggaacccc tatttgttta tttttctaaa 12240
   tacattcaaa tatgtatccg ctcatgagac aataaccctg ataaatgctt caataatatt 12300
   gaaaaaggaa gagtatgagt attcaacatt tccgtgtcgc ccttattccc ttttttgcgg 12360
   cattttgcct tcctgttttt gctcacccag aaacgctggt gaaagtaaaa gatgctgaag 12420
   atcagttggg tgcacgagtg ggttacatcg aactggatct caacagcggt aagatccttg 12480
   agagttttcg ccccgaagaa cgttttccaa tgatgagcac ttttaaagtt ctgctatgtg 12540
   gcgcggtatt atcccgtgtt gacgccgggc aagagcaact cggtcgccgc atacactatt 12600
   ctcagaatga cttggttgag tactcaccag tcacagaaaa gcatcttacg gatggcatga 12660
   cagtaagaga attatgcagt gctgccataa ccatgagtga taacactgcg gccaacttac 12720
   ttctgacaac gatcggagga ccgaaggagc taaccgcttt tttgcacaac atgggggatc 12780
   atgtaactcg ccttgatcgt tgggaaccgg agctgaatga agccatacca aacgacgagc 12840
   gtgacaccac gatgcctgca gcaatggcaa caacgttgcg caaactatta actggcgaac 12900
   tacttactct agcttcccgg caacaattaa tagactggat ggaggcggat aaagttgcag 12960
   gaccacttct gcgctcggcc cttccggctg gctggtttat tgctgataaa tctggagccg 13020
   gtgagcgtgg gtctcgcggt atcattgcag cactggggcc agatggtaag ccctcccgta 13080
   tcgtagttat ctacacgacg gggagtcagg caactatgga tgaacgaaat agacagatcg 13140
   ctgagatagg tgcctcactg attaagcatt ggtaactgtc agaccaagtt tactcatata 13200
   tactttagat tgatttaaaa cttcattttt aatttaaaag gatctaggtg aagatccttt 13260
   ttgataatct catgaccaaa atcccttaac gtgagttttc gttccactga gcgtcagacc 13320
   ccgtagaaaa gatcaaagga tcttcttgag atcctttttt tctgcgcgta atctgctgct 13380
   tgcaaacaaa aaaaccaccg ctaccagcgg tggtttgttt gccggatcaa gagctaccaa 13440
   ctctttttcc gaaggtaact ggcttcagca gagcgcagat accaaatact gtccttctag 13500
   tgtagccgta gttaggccac cacttcaaga actctgtagc accgcctaca tacctcgctc 13560
   tgctaatcct gttaccagtg gctgctgcca gtggcgataa gtcgtgtctt accgggttgg 13620
   actcaagacg atagttaccg gataaggcgc agcggtcggg ctgaacgggg ggttcgtgca 13680
   cacagcccag cttggagcga acgacctaca ccgaactgag atacctacag cgtgagctat 13740
   gagaaagcgc cacgcttccc gaagggagaa aggcggacag gtatccggta agcggcaggg 13800
   tcggaacagg agagcgcacg agggagcttc cagggggaaa cgcctggtat ctttatagtc 13860
   ctgtcgggtt tcgccacctc tgacttgagc gtcgattttt gtgatgctcg tcaggggggc 13920
   ggagcctatg gaaaaacgcc agcaacgcgg cctttttacg gttcctggcc ttttgctggc 13980
   cttttgctca catgttcttt cctgcgttat cccctgattc tgtggataac cgtattaccg 14040
   cctttgagtg agctgatacc gctcgccgca gccgaacgac cgagcgcagc gagtcagtga 14100
   gcgaggaagc ggaagagcgc ctgatgcggt attttctcct tacgcatctg tgcggtattt 14160
   cacaccgcat atggtgcact ctcagtacaa tctgctctga tgccgcatag ttaagccagt 14220
   atacactccg ctatcgctac gtgactgggt catggctgcg ccccgacacc cgccaacacc 14280
   cgctgacgcg ccctgacggg cttgtctgct cccggcatcc gcttacagac aagctgtgac 14340
   cgtctccggg agctgcatgt gtcagaggtt ttcaccgtca tcaccgaaac gcgcgaggca 14400
   gatcccgcaa gaggcccggc agtaccggca taaccaagcc tatgcctaca gcatccaggg 14460
   tgacggtgcc gaggatgacg atgagcgcat tgttagattt catacacggt gcctgactgc 14520
   gttagcaatt taactgtgat aaactaccgc attaaagctt actgtaaatt tactggctgt 14580
   cttccttgca ggtttc 14596
<210> 17
   <211> 1056
   <212> DNA
   <213> Bacteriophage P1
<220>
   <221> CDS
   <222> (1)..(1056)
<400> 17
   atg gtg ccc aag aag aag agg aaa gtc tcc aac ctg ctg act gtg cac         48
   Met Val Pro Lys Lys Lys Arg Lys Val Ser Asn Leu Leu Thr Val His
   1               5                   10                  15
   caa aac ctg cct gcc ctc cct gtg gat gcc acc tct gat gaa gtc agg         96
   Gln Asn Leu Pro Ala Leu Pro Val Asp Ala Thr Ser Asp Glu Val Arg
               20                  25                  30
   aag aac ctg atg gac atg ttc agg gac agg cag gcc ttc tct gaa cac        144
   Lys Asn Leu Met Asp Met Phe Arg Asp Arg Gln Ala Phe Ser Glu His
           35                  40                  45
   acc tgg aag atg ctc ctg tct gtg tgc aga tcc tgg gct gcc tgg tgc        192
   Thr Trp Lys Met Leu Leu Ser Val Cys Arg Ser Trp Ala Ala Trp Cys
       50                  55                  60
   aag ctg aac aac agg aaa tgg ttc cct gct gaa cct gag gat gtg agg        240
   Lys Leu Asn Asn Arg Lys Trp Phe Pro Ala Glu Pro Glu Asp Val Arg
   65                  70                  75                  80
   gac tac ctc ctg tac ctg caa gcc aga ggc ctg gct gtg aag acc atc        288
   Asp Tyr Leu Leu Tyr Leu Gln Ala Arg Gly Leu Ala Val Lys Thr Ile
                   85                  90                  95
   caa cag cac ctg ggc cag ctc aac atg ctg cac agg aga tct ggc ctg        336
   Gln Gln His Leu Gly Gln Leu Asn Met Leu His Arg Arg Ser Gly Leu
               100                 105                 110
   cct cgc cct tct gac tcc aat gct gtg tcc ctg gtg atg agg aga atc        384
   Pro Arg Pro Ser Asp Ser Asn Ala Val Ser Leu Val Met Arg Arg Ile
           115                 120                 125
   aga aag gag aat gtg gat gct ggg gag aga gcc aag cag gcc ctg gcc        432
   Arg Lys Glu Asn Val Asp Ala Gly Glu Arg Ala Lys Gln Ala Leu Ala
       130                 135                 140
   ttt gaa cgc act gac ttt gac caa gtc aga tcc ctg atg gag aac tct        480
   Phe Glu Arg Thr Asp Phe Asp Gln Val Arg Ser Leu Met Glu Asn Ser
   145                 150                 155                 160
   gac aga tgc cag gac atc agg aac ctg gcc ttc ctg ggc att gcc tac        528
   Asp Arg Cys Gln Asp Ile Arg Asn Leu Ala Phe Leu Gly Ile Ala Tyr
                   165                 170                 175
   aac acc ctg ctg cgc att gcc gaa att gcc aga atc aga gtg aag gac        576
   Asn Thr Leu Leu Arg Ile Ala Glu Ile Ala Arg Ile Arg Val Lys Asp
               180                 185                 190
   atc tcc cgc acc gat ggt ggg aga atg ctg atc cac att ggc agg acc        624
   Ile Ser Arg Thr Asp Gly Gly Arg Met Leu Ile His Ile Gly Arg Thr
           195                 200                 205
   aag acc ctg gtg tcc aca gct ggt gtg gag aag gcc ctg tcc ctg ggg        672
   Lys Thr Leu Val Ser Thr Ala Gly Val Glu Lys Ala Leu Ser Leu Gly
       210                 215                 220
   gtt acc aag ctg gtg gag aga tgg atc tct gtg tct ggt gtg gct gat        720
   Val Thr Lys Leu Val Glu Arg Trp Ile Ser Val Ser Gly Val Ala Asp
   225                 230                 235                 240
   gac ccc aac aac tac ctg ttc tgc cgg gtc aga aag aat ggt gtg gct        768
   Asp Pro Asn Asn Tyr Leu Phe Cys Arg Val Arg Lys Asn Gly Val Ala
                   245                 250                 255
   gcc cct tct gcc acc tcc caa ctg tcc acc cgg gcc ctg gaa ggg atc        816
   Ala Pro Ser Ala Thr Ser Gln Leu Ser Thr Arg Ala Leu Glu Gly Ile
               260                 265                 270
   ttt gag gcc acc cac cgc ctg atc tat ggt gcc aag gat gac tct ggg        864
   Phe Glu Ala Thr His Arg Leu Ile Tyr Gly Ala Lys Asp Asp Ser Gly
           275                 280                 285
   cag aga tac ctg gcc tgg tct ggc cac tct gcc aga gtg ggt gct gcc        912
   Gln Arg Tyr Leu Ala Trp Ser Gly His Ser Ala Arg Val Gly Ala Ala
       290                 295                 300
   agg gac atg gcc agg gct ggt gtg tcc atc cct gaa atc atg cag gct        960
   Arg Asp Met Ala Arg Ala Gly Val Ser Ile Pro Glu Ile Met Gln Ala
   305                 310                 315                 320
   ggt ggc tgg acc aat gtg aac ata gtg atg aac tac atc aga aac ctg       1008
   Gly Gly Trp Thr Asn Val Asn Ile Val Met Asn Tyr Ile Arg Asn Leu
                   325                 330                 335
   gac tct gag act ggg gcc atg gtg agg ctg ctc gag gat ggg gac tga       1056
   Asp Ser Glu Thr Gly Ala Met Val Arg Leu Leu Glu Asp Gly Asp
               340                 345                 350
<210> 18
   <211> 351
   <212> PRT
   <213> Bacteriophage P1
<400> 18
   Met Val Pro Lys Lys Lys Arg Lys Val Ser Asn Leu Leu Thr Val His
   1               5                   10                  15
   Gln Asn Leu Pro Ala Leu Pro Val Asp Ala Thr Ser Asp Glu Val Arg
               20                  25                  30
   Lys Asn Leu Met Asp Met Phe Arg Asp Arg Gln Ala Phe Ser Glu His
           35                  40                  45
   Thr Trp Lys Met Leu Leu Ser Val Cys Arg Ser Trp Ala Ala Trp Cys
       50                  55                  60
   Lys Leu Asn Asn Arg Lys Trp Phe Pro Ala Glu Pro Glu Asp Val Arg
   65                  70                  75                  80
   Asp Tyr Leu Leu Tyr Leu Gln Ala Arg Gly Leu Ala Val Lys Thr Ile
                   85                  90                  95
   Gln Gln His Leu Gly Gln Leu Asn Met Leu His Arg Arg Ser Gly Leu
               100                 105                 110
   Pro Arg Pro Ser Asp Ser Asn Ala Val Ser Leu Val Met Arg Arg Ile
           115                 120                 125
   Arg Lys Glu Asn Val Asp Ala Gly Glu Arg Ala Lys Gln Ala Leu Ala
       130                 135                 140
   Phe Glu Arg Thr Asp Phe Asp Gln Val Arg Ser Leu Met Glu Asn Ser
   145                 150                 155                 160
   Asp Arg Cys Gln Asp Ile Arg Asn Leu Ala Phe Leu Gly Ile Ala Tyr
                   165                 170                 175
   Asn Thr Leu Leu Arg Ile Ala Glu Ile Ala Arg Ile Arg Val Lys Asp
               180                 185                 190
   Ile Ser Arg Thr Asp Gly Gly Arg Met Leu Ile His Ile Gly Arg Thr
           195                 200                 205
   Lys Thr Leu Val Ser Thr Ala Gly Val Glu Lys Ala Leu Ser Leu Gly
       210                 215                 220
   Val Thr Lys Leu Val Glu Arg Trp Ile Ser Val Ser Gly Val Ala Asp
   225                 230                 235                 240
   Asp Pro Asn Asn Tyr Leu Phe Cys Arg Val Arg Lys Asn Gly Val Ala
                   245                 250                 255
   Ala Pro Ser Ala Thr Ser Gln Leu Ser Thr Arg Ala Leu Glu Gly Ile
               260                 265                 270
   Phe Glu Ala Thr His Arg Leu Ile Tyr Gly Ala Lys Asp Asp Ser Gly
           275                 280                 285
   Gln Arg Tyr Leu Ala Trp Ser Gly His Ser Ala Arg Val Gly Ala Ala
       290                 295                 300
   Arg Asp Met Ala Arg Ala Gly Val Ser Ile Pro Glu Ile Met Gln Ala
   305 310 315 320
   Gly Gly Trp Thr Asn Val Asn Ile Val Met Asn Tyr Ile Arg Asn Leu
   325 330 335
   Asp Ser Glu Thr Gly Ala Met Val Arg Leu Leu Glu Asp Gly Asp
   340 345 350
<210> 19
   <211> 1074
   <212> DNA
   <213> Bacteriophage P1
<220>
   <221> CDS
   <222> (1)..(1074)
<400> 19
   atg gtg ccc aag aag aag agg aaa gtc tcc aac ctg ctg act gtg cac        48
   Met Val Pro Lys Lys Lys Arg Lys Val Ser Asn Leu Leu Thr Val His
   1               5                   10                  15
   caa aac ctg cct gcc ctc cct gtg gat gcc acc tct gat gaa gtc agg        96
   Gln Asn Leu Pro Ala Leu Pro Val Asp Ala Thr Ser Asp Glu Val Arg
               20                  25                  30
   aag aac ctg atg gac atg ttc agg gac agg cag gcc ttc tct gaa cac       144
   Lys Asn Leu Met Asp Met Phe Arg Asp Arg Gln Ala Phe Ser Glu His
           35                  40                  45
   acc tgg aag atg ctc ctg tct gtg tgc aga tcc tgg gct gcc tgg tgc       192
   Thr Trp Lys Met Leu Leu Ser Val Cys Arg Ser Trp Ala Ala Trp Cys
       50                  55                  60
   aag ctg aac aac agg aaa tgg ttc cct gct gaa cct gag gat gtg agg       240
   Lys Leu Asn Asn Arg Lys Trp Phe Pro Ala Glu Pro Glu Asp Val Arg
   65                  70                  75                  80
   gac tac ctc ctg tac ctg caa gcc aga ggc ctg gct gtg aag acc atc       288
   Asp Tyr Leu Leu Tyr Leu Gln Ala Arg Gly Leu Ala Val Lys Thr Ile
                   85                  90                  95
   caa cag cac ctg ggc cag ctc aac atg ctg cac agg aga tct ggc ctg       336
   Gln Gln His Leu Gly Gln Leu Asn Met Leu His Arg Arg Ser Gly Leu
               100                 105                 110
   cct cgc cct tct gac tcc aat gct gtg tcc ctg gtg atg agg aga atc       384
   Pro Arg Pro Ser Asp Ser Asn Ala Val Ser Leu Val Met Arg Arg Ile
           115                 120                 125
   aga aag gag aat gtg gat gct ggg gag aga gcc aag cag gcc ctg gcc       432
   Arg Lys Glu Asn Val Asp Ala Gly Glu Arg Ala Lys Gln Ala Leu Ala
       130                 135                 140
   ttt gaa cgc act gac ttt gac caa gtc aga tcc ctg atg gag aac tct       480
   Phe Glu Arg Thr Asp Phe Asp Gln Val Arg Ser Leu Met Glu Asn Ser
   145                 150                 155                 160
   gac aga tgc cag gac atc agg aac ctg gcc ttc ctg ggc att gcc tac        528
   Asp Arg Cys Gln Asp Ile Arg Asn Leu Ala Phe Leu Gly Ile Ala Tyr
                   165                 170                 175
   aac acc ctg ctg cgc att gcc gaa att gcc aga atc aga gtg aag gac        576
   Asn Thr Leu Leu Arg Ile Ala Glu Ile Ala Arg Ile Arg Val Lys Asp
               180                 185                 190
   atc tcc cgc acc gat ggt ggg aga atg ctg atc cac att ggc agg acc        624
   Ile Ser Arg Thr Asp Gly Gly Arg Met Leu Ile His Ile Gly Arg Thr
           195                 200                 205
   aag acc ctg gtg tcc aca gct ggt gtg gag aag gcc ctg tcc ctg ggg        672
   Lys Thr Leu Val Ser Thr Ala Gly Val Glu Lys Ala Leu Ser Leu Gly
       210                 215                 220
   gtt acc aag ctg gtg gag aga tgg atc tct gtg tct ggt gtg gct gat        720
   Val Thr Lys Leu Val Glu Arg Trp Ile Ser Val Ser Gly Val Ala Asp
   225                 230                 235                 240
   gac ccc aac aac tac ctg ttc tgc cgg gtc aga aag aat ggt gtg gct        768
   Asp Pro Asn Asn Tyr Leu Phe Cys Arg Val Arg Lys Asn Gly Val Ala
                   245                 250                 255
   gcc cct tct gcc acc tcc caa ctg tcc acc cgg gcc ctg gaa ggg atc        816
   Ala Pro Ser Ala Thr Ser Gln Leu Ser Thr Arg Ala Leu Glu Gly Ile
               260                 265                 270
   ttt gag gcc acc cac cgc ctg atc tat ggt gcc aag gat gac tct ggg        864
   Phe Glu Ala Thr His Arg Leu Ile Tyr Gly Ala Lys Asp Asp Ser Gly
           275                 280                 285
   cag aga tac ctg gcc tgg tct ggc cac tct gcc aga gtg ggt gct gcc        912
   Gln Arg Tyr Leu Ala Trp Ser Gly His Ser Ala Arg Val Gly Ala Ala
       290                 295                 300
   agg gac atg gcc agg gct ggt gtg tcc atc cct gaa atc atg cag gct        960
   Arg Asp Met Ala Arg Ala Gly Val Ser Ile Pro Glu Ile Met Gln Ala
   305                 310                 315                 320
   ggt ggc tgg acc aat gtg aac ata gtg atg aac tac atc aga aac ctg       1008
   Gly Gly Trp Thr Asn Val Asn Ile Val Met Asn Tyr Ile Arg Asn Leu
                   325                 330                 335
   gac tct gag act ggg gcc atg gtg agg ctg ctc gag gat ggg gac ggt       1056
   Asp Ser Glu Thr Gly Ala Met Val Arg Leu Leu Glu Asp Gly Asp Gly
               340                 345                 350
   ggt ggt ggc aag ctt tga                                               1074
   Gly Gly Gly Lys Leu
           355
<210> 20
   <211> 357
   <212> PRT
   <213> Bacteriophage P1
<400> 20
   Met Val Pro Lys Lys Lys Arg Lys Val Ser Asn Leu Leu Thr Val His
   1               5                   10                  15
   Gln Asn Leu Pro Ala Leu Pro Val Asp Ala Thr Ser Asp Glu Val Arg
   20 25 30
   Lys Asn Leu Met Asp Met Phe Arg Asp Arg Gln Ala Phe Ser Glu His
   35 40 45
   Thr Trp Lys Met Leu Leu Ser Val Cys Arg Ser Trp Ala Ala Trp Cys
   50 55 60
   Lys Leu Asn Asn Arg Lys Trp Phe Pro Ala Glu Pro Glu Asp Val Arg
   65 70 75 80
   Asp Tyr Leu Leu Tyr Leu Gln Ala Arg Gly Leu Ala Val Lys Thr Ile
   85 90 95
   Gln Gln His Leu Gly Gln Leu Asn Met Leu His Arg Arg Ser Gly Leu
   100 105 110
   Pro Arg Pro Ser Asp Ser Asn Ala Val Ser Leu Val Met Arg Arg Ile
   115 120 125
   Arg Lys Glu Asn Val Asp Ala Gly Glu Arg Ala Lys Gln Ala Leu Ala
   130 135 140
   Phe Glu Arg Thr Asp Phe Asp Gln Val Arg Ser Leu Met Glu Asn Ser
   145 150 155 160
   Asp Arg Cys Gln Asp Ile Arg Asn Leu Ala Phe Leu Gly Ile Ala Tyr
   165 170 175
   Asn Thr Leu Leu Arg Ile Ala Glu Ile Ala Arg Ile Arg Val Lys Asp
   180 185 190
   Ile Ser Arg Thr Asp Gly Gly Arg Met Leu Ile His Ile Gly Arg Thr
   195 200 205
   Lys Thr Leu Val Ser Thr Ala Gly Val Glu Lys Ala Leu Ser Leu Gly
   210 215 220
   Val Thr Lys Leu Val Glu Arg Trp Ile Ser Val Ser Gly Val Ala Asp
   225 230 235 240
   Asp Pro Asn Asn Tyr Leu Phe Cys Arg Val Arg Lys Asn Gly Val Ala
   245 250 255
   Ala Pro Ser Ala Thr Ser Gln Leu Ser Thr Arg Ala Leu Glu Gly Ile
                   260                 265                 270
   Phe Glu Ala Thr His Arg Leu Ile Tyr Gly Ala Lys Asp Asp Ser Gly
           275                 280                 285
   Gln Arg Tyr Leu Ala Trp Ser Gly His Ser Ala Arg Val Gly Ala Ala
       290                 295                 300
   Arg Asp Met Ala Arg Ala Gly Val Ser Ile Pro Glu Ile Met Gln Ala
   305                 310                 315                 320
   Gly Gly Trp Thr Asn Val Asn Ile Val Met Asn Tyr Ile Arg Asn Leu
                   325                 330                 335
   Asp Ser Glu Thr Gly Ala Met Val Arg Leu Leu Glu Asp Gly Asp Gly
               340                 345                 350
   Gly Gly Gly Lys Leu
           355
<210> 21
   <211> 11503
   <212> DNA
   <213> Artificial Sequence
<220>
   <223> HIV 1 strain NL4-3 Nef-IRES Cre
<400> 21
   gcgttggaag ggctaattca ctcccaaaga agacaagata tccttgatct gtggatctac      60
   cacacacaag gctacttccc tgattggcag aactacacac cagggccagg ggtcagatat     120
   ccactgacct ttggatggtg ctacaagcta gtaccagttg agccagataa ggtagaagag     180
   gccaataaag gagagaacac cagcttgtta caccctgtga gcctgcatgg aatggatgac     240
   cctgagagag aagtgttaga gtggaggttt gacagccgcc tagcatttca tcacgtggcc     300
   cgagagctgc atccggagta ctacaaagac tgctgacatc gagctttcta caagggactt     360
   tccgctgggg actttccagg gaggtgtggc ctgggcggga ctggggagtg gcgagccctc     420
   agatgctaca tataagcagc tgctttttgc ctgtactggg tctctctggt tagaccagat     480
   ctgagcctgg gagctctctg gctaactagg gaacccactg cttaagcctc aataaagctt     540
   gccttgagtg ctcaaagtag tgtgtgcccg tctgttgtgt gactctggta actagagatc     600
   cctcagaccc ttttagtcag tgtggaaaat ctctagcagt ggcgcccgaa cagggacttg     660
   aaagcgaaag taaagccaga ggagatctct cgacgcagga ctcggcttgc tgaagcgcgc     720
   acggcaagag gcgaggggcg gcgactggtg agtacgccaa aaattttgac tagcggaggc     780
   tagaaggaga gagatgggtg cgagagcgtc ggtattaagc gggggagaat tagataaatg     840
   ggaaaaaatt cggttaaggc cagggggaaa gaaacaatat aaactaaaac atatagtatg 900
   ggcaagcagg gagctagaac gattcgcagt taatcctggc cttttagaga catcagaagg 960
   ctgtagacaa atactgggac agctacaacc atcccttcag acaggatcag aagaacttag 1020
   atcattatat aatacaatag cagtcctcta ttgtgtgcat caaaggatag atgtaaaaga 1080
   caccaaggaa gccttagata agatagagga agagcaaaac aaaagtaaga aaaaggcaca 1140
   gcaagcagca gctgacacag gaaacaacag ccaggtcagc caaaattacc ctatagtgca 1200
   gaacctccag gggcaaatgg tacatcaggc catatcacct agaactttaa atgcatgggt 1260
   aaaagtagta gaagagaagg ctttcagccc agaagtaata cccatgtttt cagcattatc 1320
   agaaggagcc accccacaag atttaaatac catgctaaac acagtggggg gacatcaagc 1380
   agccatgcaa atgttaaaag agaccatcaa tgaggaagct gcagaatggg atagattgca 1440
   tccagtgcat gcagggccta ttgcaccagg ccagatgaga gaaccaaggg gaagtgacat 1500
   agcaggaact actagtaccc ttcaggaaca aataggatgg atgacacata atccacctat 1560
   cccagtagga gaaatctata aaagatggat aatcctggga ttaaataaaa tagtaagaat 1620
   gtatagccct accagcattc tggacataag acaaggacca aaggaaccct ttagagacta 1680
   tgtagaccga ttctataaaa ctctaagagc cgagcaagct tcacaagagg taaaaaattg 1740
   gatgacagaa accttgttgg tccaaaatgc gaacccagat tgtaagacta ttttaaaagc 1800
   attgggacca ggagcgacac tagaagaaat gatgacagca tgtcagggag tggggggacc 1860
   cggccataaa gcaagagttt tggctgaagc aatgagccaa gtaacaaatc cagctaccat 1920
   aatgatacag aaaggcaatt ttaggaacca aagaaagact gttaagtgtt tcaattgtgg 1980
   caaagaaggg cacatagcca aaaattgcag ggcccctagg aaaaagggct gttggaaatg 2040
   tggaaaggaa ggacaccaaa tgaaagattg tactgagaga caggctaatt ttttagggaa 2100
   gatctggcct tcccacaagg gaaggccagg gaattttctt cagagcagac cagagccaac 2160
   agccccacca gaagagagct tcaggtttgg ggaagagaca acaactccct ctcagaagca 2220
   ggagccgata gacaaggaac tgtatccttt agcttccctc agatcactct ttggcagcga 2280
   cccctcgtca caataaagat aggggggcaa ttaaaggaag ctctattaga tacaggagca 2340
   gatgatacag tattagaaga aatgaatttg ccaggaagat ggaaaccaaa aatgataggg 2400
   ggaattggag gttttatcaa agtaggacag tatgatcaga tactcataga aatctgcgga 2460
   cataaagcta taggtacagt attagtagga cctacacctg tcaacataat tggaagaaat 2520
   ctgttgactc agattggctg cactttaaat tttcccatta gtcctattga gactgtacca 2580
   gtaaaattaa agccaggaat ggatggccca aaagttaaac aatggccatt gacagaagaa 2640
   aaaataaaag cattagtaga aatttgtaca gaaatggaaa aggaaggaaa aatttcaaaa 2700
   attgggcctg aaaatccata caatactcca gtatttgcca taaagaaaaa agacagtact     2760
   aaatggagaa aattagtaga tttcagagaa cttaataaga gaactcaaga tttctgggaa     2820
   gttcaattag gaataccaca tcctgcaggg ttaaaacaga aaaaatcagt aacagtactg     2880
   gatgtgggcg atgcatattt ttcagttccc ttagataaag acttcaggaa gtatactgca     2940
   tttaccatac ctagtataaa caatgagaca ccagggatta gatatcagta caatgtgctt     3000
   ccacagggat ggaaaggatc accagcaata ttccagtgta gcatgacaaa aatcttagag     3060
   ccttttagaa aacaaaatcc agacatagtc atctatcaat acatggatga tttgtatgta     3120
   ggatctgact tagaaatagg gcagcataga acaaaaatag aggaactgag acaacatctg     3180
   ttgaggtggg gatttaccac accagacaaa aaacatcaga aagaacctcc attcctttgg     3240
   atgggttatg aactccatcc tgataaatgg acagtacagc ctatagtgct gccagaaaag     3300
   gacagctgga ctgtcaatga catacagaaa ttagtgggaa aattgaattg ggcaagtcag     3360
   atttatgcag ggattaaagt aaggcaatta tgtaaacttc ttaggggaac caaagcacta     3420
   acagaagtag taccactaac agaagaagca gagctagaac tggcagaaaa cagggagatt     3480
   ctaaaagaac cggtacatgg agtgtattat gacccatcaa aagacttaat agcagaaata     3540
   cagaagcagg ggcaaggcca atggacatat caaatttatc aagagccatt taaaaatctg     3600
   aaaacaggaa aatatgcaag aatgaagggt gcccacacta atgatgtgaa acaattaaca     3660
   gaggcagtac aaaaaatagc cacagaaagc atagtaatat ggggaaagac tcctaaattt     3720
   aaattaccca tacaaaagga aacatgggaa gcatggtgga cagagtattg gcaagccacc     3780
   tggattcctg agtgggagtt tgtcaatacc cctcccttag tgaagttatg gtaccagtta     3840
   gagaaagaac ccataatagg agcagaaact ttctatgtag atggggcagc caatagggaa     3900
   actaaattag gaaaagcagg atatgtaact gacagaggaa gacaaaaagt tgtcccccta     3960
   acggacacaa caaatcagaa gactgagtta caagcaattc atctagcttt gcaggattcg     4020
   ggattagaag taaacatagt gacagactca caatatgcat tgggaatcat tcaagcacaa     4080
   ccagataaga gtgaatcaga gttagtcagt caaataatag agcagttaat aaaaaaggaa     4140
   aaagtctacc tggcatgggt accagcacac aaaggaattg gaggaaatga acaagtagat     4200
   aaattggtca gtgctggaat caggaaagta ctatttttag atggaataga taaggcccaa     4260
   gaagaacatg agaaatatca cagtaattgg agagcaatgg ctagtgattt taacctacca     4320
   cctgtagtag caaaagaaat agtagccagc tgtgataaat gtcagctaaa aggggaagcc     4380
   atgcatggac aagtagactg tagcccagga atatggcagc tagattgtac acatttagaa     4440
   ggaaaagtta tcttggtagc agttcatgta gccagtggat atatagaagc agaagtaatt     4500
   ccagcagaga cagggcaaga aacagcatac ttcctcttaa aattagcagg aagatggcca     4560
   gtaaaaacag tacatacaga caatggcagc aatttcacca gtactacagt taaggccgcc     4620
   tgttggtggg cggggatcaa gcaggaattt ggcattccct acaatcccca aagtcaagga 4680
   gtaatagaat ctatgaataa agaattaaag aaaattatag gacaggtaag agatcaggct 4740
   gaacatctta agacagcagt acaaatggca gtattcatcc acaattttaa aagaaaaggg 4800
   gggattgggg ggtacagtgc aggggaaaga atagtagaca taatagcaac agacatacaa 4860
   actaaagaat tacaaaaaca aattacaaaa attcaaaatt ttcgggttta ttacagggac 4920
   agcagagatc cagtttggaa aggaccagca aagctcctct ggaaaggtga aggggcagta 4980
   gtaatacaag ataatagtga cataaaagta gtgccaagaa gaaaagcaaa gatcatcagg 5040
   gattatggaa aacagatggc aggtgatgat tgtgtggcaa gtagacagga tgaggattaa 5100
   cacatggaaa agattagtaa aacaccatat gtatatttca aggaaagcta aggactggtt 5160
   ttatagacat cactatgaaa gtactaatcc aaaaataagt tcagaagtac acatcccact 5220
   aggggatgct aaattagtaa taacaacata ttggggtctg catacaggag aaagagactg 5280
   gcatttgggt cagggagtct ccatagaatg gaggaaaaag agatatagca cacaagtaga 5340
   ccctgaccta gcagaccaac taattcatct gcactatttt gattgttttt cagaatctgc 5400
   tataagaaat accatattag gacgtatagt tagtcctagg tgtgaatatc aagcaggaca 5460
   taacaaggta ggatctctac agtacttggc actagcagca ttaataaaac caaaacagat 5520
   aaagccacct ttgcctagtg ttaggaaact gacagaggac agatggaaca agccccagaa 5580
   gaccaagggc cacagaggga gccatacaat gaatggacac tagagctttt agaggaactt 5640
   aagagtgaag ctgttagaca ttttcctagg atatggctcc ataacttagg acaacatatc 5700
   tatgaaactt acggggatac ttgggcagga gtggaagcca taataagaat tctgcaacaa 5760
   ctgctgttta tccatttcag aattgggtgt cgacatagca gaataggcgt tactcgacag 5820
   aggagagcaa gaaatggagc cagtagatcc tagactagag ccctggaagc atccaggaag 5880
   tcagcctaaa actgcttgta ccaattgcta ttgtaaaaag tgttgctttc attgccaagt 5940
   ttgtttcatg acaaaagcct taggcatctc ctatggcagg aagaagcgga gacagcgacg 6000
   aagagctcat cagaacagtc agactcatca agcttctcta tcaaagcagt aagtagtaca 6060
   tgtaatgcaa cctataatag tagcaatagt agcattagta gtagcaataa taatagcaat 6120
   agttgtgtgg tccatagtaa tcatagaata taggaaaata ttaagacaaa gaaaaataga 6180
   caggttaatt gatagactaa tagaaagagc agaagacagt ggcaatgaga gtgaaggaga 6240
   agtatcagca cttgtggaga tgggggtgga aatggggcac catgctcctt gggatattga 6300
   tgatctgtag tgctacagaa aaattgtggg tcacagtcta ttatggggta cctgtgtgga 6360
   aggaagcaac caccactcta ttttgtgcat cagatgctaa agcatatgat acagaggtac 6420
   ataatgtttg ggccacacat gcctgtgtac ccacagaccc caacccacaa gaagtagtat 6480
   tggtaaatgt gacagaaaat tttaacatgt ggaaaaatga catggtagaa cagatgcatg 6540
   aggatataat cagtttatgg gatcaaagcc taaagccatg tgtaaaatta accccactct 6600
   gtgttagttt aaagtgcact gatttgaaga atgatactaa taccaatagt agtagcggga 6660
   gaatgataat ggagaaagga gagataaaaa actgctcttt caatatcagc acaagcataa 6720
   gagataaggt gcagaaagaa tatgcattct tttataaact tgatatagta ccaatagata 6780
   ataccagcta taggttgata agttgtaaca cctcagtcat tacacaggcc tgtccaaagg 6840
   tatcctttga gccaattccc atacattatt gtgccccggc tggttttgcg attctaaaat 6900
   gtaataataa gacgttcaat ggaacaggac catgtacaaa tgtcagcaca gtacaatgta 6960
   cacatggaat caggccagta gtatcaactc aactgctgtt aaatggcagt ctagcagaag 7020
   aagatgtagt aattagatct gccaatttca cagacaatgc taaaaccata atagtacagc 7080
   tgaacacatc tgtagaaatt aattgtacaa gacccaacaa caatacaaga aaaagtatcc 7140
   gtatccagag gggaccaggg agagcatttg ttacaatagg aaaaatagga aatatgagac 7200
   aagcacattg taacattagt agagcaaaat ggaatgccac tttaaaacag atagctagca 7260
   aattaagaga acaatttgga aataataaaa caataatctt taagcaatcc tcaggagggg 7320
   acccagaaat tgtaacgcac agttttaatt gtggagggga atttttctac tgtaattcaa 7380
   cacaactgtt taatagtact tggtttaata gtacttggag tactgaaggg tcaaataaca 7440
   ctgaaggaag tgacacaatc acactcccat gcagaataaa acaatttata aacatgtggc 7500
   aggaagtagg aaaagcaatg tatgcccctc ccatcagtgg acaaattaga tgttcatcaa 7560
   atattactgg gctgctatta acaagagatg gtggtaataa caacaatggg tccgagatct 7620
   tcagacctgg aggaggcgat atgagggaca attggagaag tgaattatat aaatataaag 7680
   tagtaaaaat tgaaccatta ggagtagcac ccaccaaggc aaagagaaga gtggtgcaga 7740
   gagaaaaaag agcagtggga ataggagctt tgttccttgg gttcttggga gcagcaggaa 7800
   gcactatggg cgcagcgtca atgacgctga cggtacaggc cagacaatta ttgtctgata 7860
   tagtgcagca gcagaacaat ttgctgaggg ctattgaggc gcaacagcat ctgttgcaac 7920
   tcacagtctg gggcatcaaa cagctccagg caagaatcct ggctgtggaa agatacctaa 7980
   aggatcaaca gctcctgggg atttggggtt gctctggaaa actcatttgc accactgctg 8040
   tgccttggaa tgctagttgg agtaataaat ctctggaaca gatttggaat aacatgacct 8100
   ggatggagtg ggacagagaa attaacaatt acacaagctt aatacactcc ttaattgaag 8160
   aatcgcaaaa ccagcaagaa aagaatgaac aagaattatt ggaattagat aaatgggcaa 8220
   gtttgtggaa ttggtttaac ataacaaatt ggctgtggta tataaaatta ttcataatga 8280
   tagtaggagg cttggtaggt ttaagaatag tttttgctgt actttctata gtgaatagag 8340
   ttaggcaggg atattcacca ttatcgtttc agacccacct cccaatcccg aggggacccg 8400
   acaggcccga aggaatagaa gaagaaggtg gagagagaga cagagacaga tccattcgat 8460
   tagtgaacgg atccttagca cttatctggg acgatctgcg gagcctgtgc ctcttcagct 8520
   accaccgctt gagagactta ctcttgattg taacgaggat tgtggaactt ctgggacgca 8580
   gggggtggga agccctcaaa tattggtgga atctcctaca gtattggagt caggaactaa 8640
   agaatagtgc tgttaacttg ctcaatgcca cagccatagc agtagctgag gggacagata 8700
   gggttataga agtattacaa gcagcttata gagctattcg ccacatacct agaagaataa 8760
   gacagggctt ggaaaggatt ttgctataag atgggtggca agtggtcaaa aagtagtgtg 8820
   attggatggc ctgctgtaag ggaaagaatg agacgagctg agccagcagc agatggggtg 8880
   ggagcagtat ctcgagacct agaaaaacat ggagcaatca caagtagcaa tacagcagct 8940
   aacaatgctg cttgtgcctg gctagaagca caagaggagg aagaggtggg ttttccagtc 9000
   acacctcagg tacctttaag accaatgact tacaaggcag ctgtagatct tagccacttt 9060
   ttaaaagaaa aggggggact ggaagggcta attcactccc aaagaaggca agatatcctt 9120
   gatctgtgga tctaccacac acaaggctac ttccctgatt ggcagaacta cacaccaggg 9180
   ccaggggtca gatatccact gacctttgga tggtgctaca agctagtacc agctgagcca 9240
   gataaggtag aagaggccaa taaaggagag aacaccagct tgttacaccc tgtgagcctg 9300
   catggaatgg atgaccctga gagagaagtg ttagagtgga ggtttgacag ccgcctagca 9360
   tttcatcacg tggcccgaga gctgcatccg gagtacttca agaactgctg aacgcgtcgg 9420
   atcccggagt actacaaaga ctgctgacgc gaattccgcc ccccccccta acgttactgg 9480
   ccgaagccgc ttggaataag gccggtgtgc gtttgtctat atgttatttt ccaccatatt 9540
   gccgtctttt ggcaatgtga gggcccggaa acctggccct gtcttcttga cgagcattcc 9600
   taggggtctt tcccctctcg ccaaaggaat gcaaggtctg ttgaatgtcg tgaaggaagc 9660
   agttcctctg gaagcttctt gaagacaaac aacgtctgta gcgacccttt gcaggcagcg 9720
   gaacccccca cctggcgaca ggtgcctctg cggccaaaag ccacgtgtat aagatacacc 9780
   tgcaaaggcg gcacaacccc agtgccacgt tgtgagttgg atagttgtgg aaagagtcaa 9840
   atggctctcc tcaagcgtat tcaacaaggg gctgaaggat gcccagaagg taccccattg 9900
   tatgggatct gatctggggc ctcggtgcac atgctttaca tgtgtttagt cgaggttaaa 9960
   aaacgtctag gccccccgaa ccacggggac gtggttttcc tttgaaaaac acgatgataa 10020
   taccatggtg cccaagaaga agaggaaagt ctccaacctg ctgactgtgc accaaaacct 10080
   gcctgccctc cctgtggatg ccacctctga tgaagtcagg aagaacctga tggacatgtt 10140
   cagggacagg caggccttct ctgaacacac ctggaagatg ctcctgtctg tgtgcagatc 10200
   ctgggctgcc tggtgcaagc tgaacaacag gaaatggttc cctgctgaac ctgaggatgt 10260
   gagggactac ctcctgtacc tgcaagccag aggcctggct gtgaagacca tccaacagca 10320
   cctgggccag ctcaacatgc tgcacaggag atctggcctg cctcgccctt ctgactccaa 10380
   tgctgtgtcc ctggtgatga ggagaatcag aaaggagaat gtggatgctg gggagagagc 10440
   caagcaggcc ctggcctttg aacgcactga ctttgaccaa gtcagatccc tgatggagaa 10500
   ctctgacaga tgccaggaca tcaggaacct ggccttcctg ggcattgcct acaacaccct 10560
   gctgcgcatt gccgaaattg ccagaatcag agtgaaggac atctcccgca ccgatggtgg 10620
   gagaatgctg atccacattg gcaggaccaa gaccctggtg tccacagctg gtgtggagaa 10680
   ggccctgtcc ctgggggtta ccaagctggt ggagagatgg atctctgtgt ctggtgtggc 10740
   tgatgacccc aacaactacc tgttctgccg ggtcagaaag aatggtgtgg ctgccccttc 10800
   tgccacctcc caactgtcca cccgggccct ggaagggatc tttgaggcca cccaccgcct 10860
   gatctatggt gccaaggatg actctgggca gagatacctg gcctggtctg gccactctgc 10920
   cagagtgggt gctgccaggg acatggccag ggctggtgtg tccatccctg aaatcatgca 10980
   ggctggtggc tggaccaatg tgaacattgt gatgaactac atcagaaacc tggactctga 11040
   gactggggcc atggtgaggc tgctcgagga tggggactga cccgggtagc cactttttaa 11100
   aagaaaaggg gggactggaa gggctaattc actcccaaag aagacaagat atcccatccg 11160
   gagtacttca agaactgctg acatcgagct tgctacaagg gactttccgc tggggacttt 11220
   ccagggaggc gtggcctggg cgggactggg gagtggcgag ccctcagatg ctgcatataa 11280
   gcagctgctt tttgcctgta ctgggtctct ctggttagac cagatctgag cctgggagct 11340
   ctctggctaa ctagggaacc cactgcttaa gcctcaataa agcttgcctt gagtgcttca 11400
   agtagtgtgt gcccgtctgt tgtgtgactc tggtaactag agatccctca gaccctttta 11460
   gtcagtgtgg aaaatctcta gcagttctag agcggccgct cgc 11503
<210> 22
   <211> 11503
   <212> DNA
   <213> Artificial Sequence
<220>
   <223> HIV strain NL4-3 Nef IRES Cre with optimized codon
<400> 22
   gcgttggaag ggctaattca ctcccaaaga agacaagata tccttgatct gtggatctac 60
   cacacacaag gctacttccc tgattggcag aactacacac cagggccagg ggtcagatat 120
   ccactgacct ttggatggtg ctacaagcta gtaccagttg agccagataa ggtagaagag 180
   gccaataaag gagagaacac cagcttgtta caccctgtga gcctgcatgg aatggatgac 240
   cctgagagag aagtgttaga gtggaggttt gacagccgcc tagcatttca tcacgtggcc 300
   cgagagctgc atccggagta ctacaaagac tgctgacatc gagctttcta caagggactt 360
   tccgctgggg actttccagg gaggtgtggc ctgggcggga ctggggagtg gcgagccctc       420
   agatgctaca tataagcagc tgctttttgc ctgtactggg tctctctggt tagaccagat       480
   ctgagcctgg gagctctctg gctaactagg gaacccactg cttaagcctc aataaagctt       540
   gccttgagtg ctcaaagtag tgtgtgcccg tctgttgtgt gactctggta actagagatc       600
   cctcagaccc ttttagtcag tgtggaaaat ctctagcagt ggcgcccgaa cagggacttg       660
   aaagcgaaag taaagccaga ggagatctct cgacgcagga ctcggcttgc tgaagcgcgc       720
   acggcaagag gcgaggggcg gcgactggtg agtacgccaa aaattttgac tagcggaggc      780
   tagaaggaga gagatgggtg cgagagcgtc ggtattaagc gggggagaat tagataaatg       840
   ggaaaaaatt cggttaaggc cagggggaaa gaaacaatat aaactaaaac atatagtatg       900
   ggcaagcagg gagctagaac gattcgcagt taatcctggc cttttagaga catcagaagg       960
   ctgtagacaa atactgggac agctacaacc atcccttcag acaggatcag aagaacttag      1020
   atcattatat aatacaatag cagtcctcta ttgtgtgcat caaaggatag atgtaaaaga      1080
   caccaaggaa gccttagata agatagagga agagcaaaac aaaagtaaga aaaaggcaca      1140
   gcaagcagca gctgacacag gaaacaacag ccaggtcagc caaaattacc ctatagtgca      1200
   gaacctccag gggcaaatgg tacatcaggc catatcacct agaactttaa atgcatgggt      1260
   aaaagtagta gaagagaagg ctttcagccc agaagtaata cccatgtttt cagcattatc      1320
   agaaggagcc accccacaag atttaaatac catgctaaac acagtggggg gacatcaagc      1380
   agccatgcaa atgttaaaag agaccatcaa tgaggaagct gcagaatggg atagattgca      1440
   tccagtgcat gcagggccta ttgcaccagg ccagatgaga gaaccaaggg gaagtgacat      1500
   agcaggaact actagtaccc ttcaggaaca aataggatgg atgacacata atccacctat      1560
   cccagtagga gaaatctata aaagatggat aatcctggga ttaaataaaa tagtaagaat      1620
   gtatagccct accagcattc tggacataag acaaggacca aaggaaccct ttagagacta      1680
   tgtagaccga ttctataaaa ctctaagagc cgagcaagct tcacaagagg taaaaaattg      1740
   gatgacagaa accttgttgg tccaaaatgc gaacccagat tgtaagacta ttttaaaagc      1800
   attgggacca ggagcgacac tagaagaaat gatgacagca tgtcagggag tggggggacc      1860
   cggccataaa gcaagagttt tggctgaagc aatgagccaa gtaacaaatc cagctaccat      1920
   aatgatacag aaaggcaatt ttaggaacca aagaaagact gttaagtgtt tcaattgtgg      1980
   caaagaaggg cacatagcca aaaattgcag ggcccctagg aaaaagggct gttggaaatg      2040
   tggaaaggaa ggacaccaaa tgaaagattg tactgagaga caggctaatt ttttagggaa      2100
   gatctggcct tcccacaagg gaaggccagg gaattttctt cagagcagac cagagccaac      2160
   agccccacca gaagagagct tcaggtttgg ggaagagaca acaactccct ctcagaagca      2220
   ggagccgata gacaaggaac tgtatccttt agcttccctc agatcactct ttggcagcga 2280
   cccctcgtca caataaagat aggggggcaa ttaaaggaag ctctattaga tacaggagca 2340
   gatgatacag tattagaaga aatgaatttg ccaggaagat ggaaaccaaa aatgataggg 2400
   ggaattggag gttttatcaa agtaggacag tatgatcaga tactcataga aatctgcgga 2460
   cataaagcta taggtacagt attagtagga cctacacctg tcaacataat tggaagaaat 2520
   ctgttgactc agattggctg cactttaaat tttcccatta gtcctattga gactgtacca 2580
   gtaaaattaa agccaggaat ggatggccca aaagttaaac aatggccatt gacagaagaa 2640
   aaaataaaag cattagtaga aatttgtaca gaaatggaaa aggaaggaaa aatttcaaaa 2700
   attgggcctg aaaatccata caatactcca gtatttgcca taaagaaaaa agacagtact 2760
   aaatggagaa aattagtaga tttcagagaa cttaataaga gaactcaaga tttctgggaa 2820
   gttcaattag gaataccaca tcctgcaggg ttaaaacaga aaaaatcagt aacagtactg 2880
   gatgtgggcg atgcatattt ttcagttccc ttagataaag acttcaggaa gtatactgca 2940
   tttaccatac ctagtataaa caatgagaca ccagggatta gatatcagta caatgtgctt 3000
   ccacagggat ggaaaggatc accagcaata ttccagtgta gcatgacaaa aatcttagag 3060
   ccttttagaa aacaaaatcc agacatagtc atctatcaat acatggatga tttgtatgta 3120
   ggatctgact tagaaatagg gcagcataga acaaaaatag aggaactgag acaacatctg 3180
   ttgaggtggg gatttaccac accagacaaa aaacatcaga aagaacctcc attcctttgg 3240
   atgggttatg aactccatcc tgataaatgg acagtacagc ctatagtgct gccagaaaag 3300
   gacagctgga ctgtcaatga catacagaaa ttagtgggaa aattgaattg ggcaagtcag 3360
   atttatgcag ggattaaagt aaggcaatta tgtaaacttc ttaggggaac caaagcacta 3420
   acagaagtag taccactaac agaagaagca gagctagaac tggcagaaaa cagggagatt 3480
   ctaaaagaac cggtacatgg agtgtattat gacccatcaa aagacttaat agcagaaata 3540
   cagaagcagg ggcaaggcca atggacatat caaatttatc aagagccatt taaaaatctg 3600
   aaaacaggaa aatatgcaag aatgaagggt gcccacacta atgatgtgaa acaattaaca 3660
   gaggcagtac aaaaaatagc cacagaaagc atagtaatat ggggaaagac tcctaaattt 3720
   aaattaccca tacaaaagga aacatgggaa gcatggtgga cagagtattg gcaagccacc 3780
   tggattcctg agtgggagtt tgtcaatacc cctcccttag tgaagttatg gtaccagtta 3840
   gagaaagaac ccataatagg agcagaaact ttctatgtag atggggcagc caatagggaa 3900
   actaaattag gaaaagcagg atatgtaact gacagaggaa gacaaaaagt tgtcccccta 3960
   acggacacaa caaatcagaa gactgagtta caagcaattc atctagcttt gcaggattcg 4020
   ggattagaag taaacatagt gacagactca caatatgcat tgggaatcat tcaagcacaa 4080
   ccagataaga gtgaatcaga gttagtcagt caaataatag agcagttaat aaaaaaggaa 4140
   aaagtctacc tggcatgggt accagcacac aaaggaattg gaggaaatga acaagtagat 4200
   aaattggtca gtgctggaat caggaaagta ctatttttag atggaataga taaggcccaa 4260
   gaagaacatg agaaatatca cagtaattgg agagcaatgg ctagtgattt taacctacca 4320
   cctgtagtag caaaagaaat agtagccagc tgtgataaat gtcagctaaa aggggaagcc 4380
   atgcatggac aagtagactg tagcccagga atatggcagc tagattgtac acatttagaa 4440
   ggaaaagtta tcttggtagc agttcatgta gccagtggat atatagaagc agaagtaatt 4500
   ccagcagaga cagggcaaga aacagcatac ttcctcttaa aattagcagg aagatggcca 4560
   gtaaaaacag tacatacaga caatggcagc aatttcacca gtactacagt taaggccgcc 4620
   tgttggtggg cggggatcaa gcaggaattt ggcattccct acaatcccca aagtcaagga 4680
   gtaatagaat ctatgaataa agaattaaag aaaattatag gacaggtaag agatcaggct 4740
   gaacatctta agacagcagt acaaatggca gtattcatcc acaattttaa aagaaaaggg 4800
   gggattgggg ggtacagtgc aggggaaaga atagtagaca taatagcaac agacatacaa 4860
   actaaagaat tacaaaaaca aattacaaaa attcaaaatt ttcgggttta ttacagggac 4920
   agcagagatc cagtttggaa aggaccagca aagctcctct ggaaaggtga aggggcagta 4980
   gtaatacaag ataatagtga cataaaagta gtgccaagaa gaaaagcaaa gatcatcagg 5040
   gattatggaa aacagatggc aggtgatgat tgtgtggcaa gtagacagga tgaggattaa 5100
   cacatggaaa agattagtaa aacaccatat gtatatttca aggaaagcta aggactggtt 5160
   ttatagacat cactatgaaa gtactaatcc aaaaataagt tcagaagtac acatcccact 5220
   aggggatgct aaattagtaa taacaacata ttggggtctg catacaggag aaagagactg 5280
   gcatttgggt cagggagtct ccatagaatg gaggaaaaag agatatagca cacaagtaga 5340
   ccctgaccta gcagaccaac taattcatct gcactatttt gattgttttt cagaatctgc 5400
   tataagaaat accatattag gacgtatagt tagtcctagg tgtgaatatc aagcaggaca 5460
   taacaaggta ggatctctac agtacttggc actagcagca ttaataaaac caaaacagat 5520
   aaagccacct ttgcctagtg ttaggaaact gacagaggac agatggaaca agccccagaa 5580
   gaccaagggc cacagaggga gccatacaat gaatggacac tagagctttt agaggaactt 5640
   aagagtgaag ctgttagaca ttttcctagg atatggctcc ataacttagg acaacatatc 5700
   tatgaaactt acggggatac ttgggcagga gtggaagcca taataagaat tctgcaacaa 5760
   ctgctgttta tccatttcag aattgggtgt cgacatagca gaataggcgt tactcgacag 5820
   aggagagcaa gaaatggagc cagtagatcc tagactagag ccctggaagc atccaggaag 5880
   tcagcctaaa actgcttgta ccaattgcta ttgtaaaaag tgttgctttc attgccaagt 5940
   ttgtttcatg acaaaagcct taggcatctc ctatggcagg aagaagcgga gacagcgacg 6000
   aagagctcat cagaacagtc agactcatca agcttctcta tcaaagcagt aagtagtaca 6060
   tgtaatgcaa cctataatag tagcaatagt agcattagta gtagcaataa taatagcaat 6120
   agttgtgtgg tccatagtaa tcatagaata taggaaaata ttaagacaaa gaaaaataga 6180
   caggttaatt gatagactaa tagaaagagc agaagacagt ggcaatgaga gtgaaggaga 6240
   agtatcagca cttgtggaga tgggggtgga aatggggcac catgctcctt gggatattga 6300
   tgatctgtag tgctacagaa aaattgtggg tcacagtcta ttatggggta cctgtgtgga 6360
   aggaagcaac caccactcta ttttgtgcat cagatgctaa agcatatgat acagaggtac 6420
   ataatgtttg ggccacacat gcctgtgtac ccacagaccc caacccacaa gaagtagtat 6480
   tggtaaatgt gacagaaaat tttaacatgt ggaaaaatga catggtagaa cagatgcatg 6540
   aggatataat cagtttatgg gatcaaagcc taaagccatg tgtaaaatta accccactct 6600
   gtgttagttt aaagtgcact gatttgaaga atgatactaa taccaatagt agtagcggga 6660
   gaatgataat ggagaaagga gagataaaaa actgctcttt caatatcagc acaagcataa 6720
   gagataaggt gcagaaagaa tatgcattct tttataaact tgatatagta ccaatagata 6780
   ataccagcta taggttgata agttgtaaca cctcagtcat tacacaggcc tgtccaaagg 6840
   tatcctttga gccaattccc atacattatt gtgccccggc tggttttgcg attctaaaat 6900
   gtaataataa gacgttcaat ggaacaggac catgtacaaa tgtcagcaca gtacaatgta 6960
   cacatggaat caggccagta gtatcaactc aactgctgtt aaatggcagt ctagcagaag 7020
   aagatgtagt aattagatct gccaatttca cagacaatgc taaaaccata atagtacagc 7080
   tgaacacatc tgtagaaatt aattgtacaa gacccaacaa caatacaaga aaaagtatcc 7140
   gtatccagag gggaccaggg agagcatttg ttacaatagg aaaaatagga aatatgagac 7200
   aagcacattg taacattagt agagcaaaat ggaatgccac tttaaaacag atagctagca 7260
   aattaagaga acaatttgga aataataaaa caataatctt taagcaatcc tcaggagggg 7320
   acccagaaat tgtaacgcac agttttaatt gtggagggga atttttctac tgtaattcaa 7380
   cacaactgtt taatagtact tggtttaata gtacttggag tactgaaggg tcaaataaca 7440
   ctgaaggaag tgacacaatc acactcccat gcagaataaa acaatttata aacatgtggc 7500
   aggaagtagg aaaagcaatg tatgcccctc ccatcagtgg acaaattaga tgttcatcaa 7560
   atattactgg gctgctatta acaagagatg gtggtaataa caacaatggg tccgagatct 7620
   tcagacctgg aggaggcgat atgagggaca attggagaag tgaattatat aaatataaag 7680
   tagtaaaaat tgaaccatta ggagtagcac ccaccaaggc aaagagaaga gtggtgcaga 7740
   gagaaaaaag agcagtggga ataggagctt tgttccttgg gttcttggga gcagcaggaa 7800
   gcactatggg cgcagcgtca atgacgctga cggtacaggc cagacaatta ttgtctgata 7860
   tagtgcagca gcagaacaat ttgctgaggg ctattgaggc gcaacagcat ctgttgcaac 7920
   tcacagtctg gggcatcaaa cagctccagg caagaatcct ggctgtggaa agatacctaa 7980
   aggatcaaca gctcctgggg atttggggtt gctctggaaa actcatttgc accactgctg 8040
   tgccttggaa tgctagttgg agtaataaat ctctggaaca gatttggaat aacatgacct 8100
   ggatggagtg ggacagagaa attaacaatt acacaagctt aatacactcc ttaattgaag 8160
   aatcgcaaaa ccagcaagaa aagaatgaac aagaattatt ggaattagat aaatgggcaa 8220
   gtttgtggaa ttggtttaac ataacaaatt ggctgtggta tataaaatta ttcataatga 8280
   tagtaggagg cttggtaggt ttaagaatag tttttgctgt actttctata gtgaatagag 8340
   ttaggcaggg atattcacca ttatcgtttc agacccacct cccaatcccg aggggacccg 8400
   acaggcccga aggaatagaa gaagaaggtg gagagagaga cagagacaga tccattcgat 8460
   tagtgaacgg atccttagca cttatctggg acgatctgcg gagcctgtgc ctcttcagct 8520
   accaccgctt gagagactta ctcttgattg taacgaggat tgtggaactt ctgggacgca 8580
   gggggtggga agccctcaaa tattggtgga atctcctaca gtattggagt caggaactaa 8640
   agaatagtgc tgttaacttg ctcaatgcca cagccatagc agtagctgag gggacagata 8700
   gggttataga agtattacaa gcagcttata gagctattcg ccacatacct agaagaataa 8760
   gacagggctt ggaaaggatt ttgctataag atgggtggca agtggtcaaa aagtagtgtg 8820
   attggatggc ctgctgtaag ggaaagaatg agacgagctg agccagcagc agatggggtg 8880
   ggagcagtat ctcgagacct agaaaaacat ggagcaatca caagtagcaa tacagcagct 8940
   aacaatgctg cttgtgcctg gctagaagca caagaggagg aagaggtggg ttttccagtc 9000
   acacctcagg tacctttaag accaatgact tacaaggcag ctgtagatct gagccatttc 9060
   ctgaaggaga agggaggcct tgagggcctg attcacagcc agcggcgcca agacatcctt 9120
   gacttgtgga tttaccatac acaggggtac ttccccgact ggcagaacta tacaccagga 9180
   cccggagtga gataccccct gaccttcgga tggtgttaca aactggttcc agtggagcct 9240
   gataaggtcg aagaggcaaa caaaggcgag aatacatctt tgctgcatcc tgtgtcactg 9300
   cacgggatgg acgaccctga gcgggaggtg cttgagtgga ggttcgactc tcgactggcc 9360
   tttcaccacg tagcaaggga gctgcaccct gagtatttta aaaattgttg aacgcgtcgg 9420
   atcccggagt actacaaaga ctgctgacgc gaattccgcc ccccccccta acgttactgg 9480
   ccgaagccgc ttggaataag gccggtgtgc gtttgtctat atgttatttt ccaccatatt 9540
   gccgtctttt ggcaatgtga gggcccggaa acctggccct gtcttcttga cgagcattcc 9600
   taggggtctt tcccctctcg ccaaaggaat gcaaggtctg ttgaatgtcg tgaaggaagc 9660
   agttcctctg gaagcttctt gaagacaaac aacgtctgta gcgacccttt gcaggcagcg 9720
   gaacccccca cctggcgaca ggtgcctctg cggccaaaag ccacgtgtat aagatacacc 9780
   tgcaaaggcg gcacaacccc agtgccacgt tgtgagttgg atagttgtgg aaagagtcaa      9840
   atggctctcc tcaagcgtat tcaacaaggg gctgaaggat gcccagaagg taccccattg      9900
   tatgggatct gatctggggc ctcggtgcac atgctttaca tgtgtttagt cgaggttaaa      9960
   aaacgtctag gccccccgaa ccacggggac gtggttttcc tttgaaaaac acgatgataa     10020
   taccatggtg cccaagaaga agaggaaagt ctccaacctg ctgactgtgc accaaaacct     10080
   gcctgccctc cctgtggatg ccacctctga tgaagtcagg aagaacctga tggacatgtt     10140
   cagggacagg caggccttct ctgaacacac ctggaagatg ctcctgtctg tgtgcagatc     10200
   ctgggctgcc tggtgcaagc tgaacaacag gaaatggttc cctgctgaac ctgaggatgt     10260
   gagggactac ctcctgtacc tgcaagccag aggcctggct gtgaagacca tccaacagca     10320
   cctgggccag ctcaacatgc tgcacaggag atctggcctg cctcgccctt ctgactccaa     10380
   tgctgtgtcc ctggtgatga ggagaatcag aaaggagaat gtggatgctg gggagagagc      10440
   caagcaggcc ctggcctttg aacgcactga ctttgaccaa gtcagatccc tgatggagaa     10500
   ctctgacaga tgccaggaca tcaggaacct ggccttcctg ggcattgcct acaacaccct     10560
   gctgcgcatt gccgaaattg ccagaatcag agtgaaggac atctcccgca ccgatggtgg     10620
   gagaatgctg atccacattg gcaggaccaa gaccctggtg tccacagctg gtgtggagaa     10680
   ggccctgtcc ctgggggtta ccaagctggt ggagagatgg atctctgtgt ctggtgtggc     10740
   tgatgacccc aacaactacc tgttctgccg ggtcagaaag aatggtgtgg ctgccccttc     10800
   tgccacctcc caactgtcca cccgggccct ggaagggatc tttgaggcca cccaccgcct     10860
   gatctatggt gccaaggatg actctgggca gagatacctg gcctggtctg gccactctgc     10920
   cagagtgggt gctgccaggg acatggccag ggctggtgtg tccatccctg aaatcatgca     10980
   ggctggtggc tggaccaatg tgaacattgt gatgaactac atcagaaacc tggactctga     11040
   gactggggcc atggtgaggc tgctcgagga tggggactga cccgggtagc cactttttaa     11100
   aagaaaaggg gggactggaa gggctaattc actcccaaag aagacaagat atcccatccg     11160
   gagtacttca agaactgctg acatcgagct tgctacaagg gactttccgc tggggacttt     11220
   ccagggaggc gtggcctggg cgggactggg gagtggcgag ccctcagatg ctgcatataa     11280
   gcagctgctt tttgcctgta ctgggtctct ctggttagac cagatctgag cctgggagct     11340
   ctctggctaa ctagggaacc cactgcttaa gcctcaataa agcttgcctt gagtgcttca     11400
   agtagtgtgt gcccgtctgt tgtgtgactc tggtaactag agatccctca gaccctttta      11460
   gtcagtgtgg aaaatctcta gcagttctag agcggccgct cgc                       11503
<210> 23
   <211> 11582
   <212> DNA
   <213> Artificial Sequence
<220>
   <223> HIV strain NL4-3 CMV Cre
<400> 23
   gcgttggaag ggctaattca ctcccaaaga agacaagata tccttgatct gtggatctac 60
   cacacacaag gctacttccc tgattggcag aactacacac cagggccagg ggtcagatat 120
   ccactgacct ttggatggtg ctacaagcta gtaccagttg agccagataa ggtagaagag 180
   gccaataaag gagagaacac cagcttgtta caccctgtga gcctgcatgg aatggatgac 240
   cctgagagag aagtgttaga gtggaggttt gacagccgcc tagcatttca tcacgtggcc 300
   cgagagctgc atccggagta ctacaaagac tgctgacatc gagctttcta caagggactt 360
   tccgctgggg actttccagg gaggtgtggc ctgggcggga ctggggagtg gcgagccctc 420
   agatgctaca tataagcagc tgctttttgc ctgtactggg tctctctggt tagaccagat 480
   ctgagcctgg gagctctctg gctaactagg gaacccactg cttaagcctc aataaagctt 540
   gccttgagtg ctcaaagtag tgtgtgcccg tctgttgtgt gactctggta actagagatc 600
   cctcagaccc ttttagtcag tgtggaaaat ctctagcagt ggcgcccgaa cagggacttg 660
   aaagcgaaag taaagccaga ggagatctct cgacgcagga ctcggcttgc tgaagcgcgc 720
   acggcaagag gcgaggggcg gcgactggtg agtacgccaa aaattttgac tagcggaggc 780
   tagaaggaga gagatgggtg cgagagcgtc ggtattaagc gggggagaat tagataaatg 840
   ggaaaaaatt cggttaaggc cagggggaaa gaaacaatat aaactaaaac atatagtatg 900
   ggcaagcagg gagctagaac gattcgcagt taatcctggc cttttagaga catcagaagg 960
   ctgtagacaa atactgggac agctacaacc atcccttcag acaggatcag aagaacttag 1020
   atcattatat aatacaatag cagtcctcta ttgtgtgcat caaaggatag atgtaaaaga 1080
   caccaaggaa gccttagata agatagagga agagcaaaac aaaagtaaga aaaaggcaca 1140
   gcaagcagca gctgacacag gaaacaacag ccaggtcagc caaaattacc ctatagtgca 1200
   gaacctccag gggcaaatgg tacatcaggc catatcacct agaactttaa atgcatgggt 1260
   aaaagtagta gaagagaagg ctttcagccc agaagtaata cccatgtttt cagcattatc 1320
   agaaggagcc accccacaag atttaaatac catgctaaac acagtggggg gacatcaagc 1380
   agccatgcaa atgttaaaag agaccatcaa tgaggaagct gcagaatggg atagattgca 1440
   tccagtgcat gcagggccta ttgcaccagg ccagatgaga gaaccaaggg gaagtgacat 1500
   agcaggaact actagtaccc ttcaggaaca aataggatgg atgacacata atccacctat 1560
   cccagtagga gaaatctata aaagatggat aatcctggga ttaaataaaa tagtaagaat 1620
   gtatagccct accagcattc tggacataag acaaggacca aaggaaccct ttagagacta 1680
   tgtagaccga ttctataaaa ctctaagagc cgagcaagct tcacaagagg taaaaaattg 1740
   gatgacagaa accttgttgg tccaaaatgc gaacccagat tgtaagacta ttttaaaagc     1800
   attgggacca ggagcgacac tagaagaaat gatgacagca tgtcagggag tggggggacc    1860
   cggccataaa gcaagagttt tggctgaagc aatgagccaa gtaacaaatc cagctaccat     1920
   aatgatacag aaaggcaatt ttaggaacca aagaaagact gttaagtgtt tcaattgtgg     1980
   caaagaaggg cacatagcca aaaattgcag ggcccctagg aaaaagggct gttggaaatg     2040
   tggaaaggaa ggacaccaaa tgaaagattg tactgagaga caggctaatt ttttagggaa     2100
   gatctggcct tcccacaagg gaaggccagg gaattttctt cagagcagac cagagccaac     2160
   agccccacca gaagagagct tcaggtttgg ggaagagaca acaactccct ctcagaagca     2220
   ggagccgata gacaaggaac tgtatccttt agcttccctc agatcactct ttggcagcga     2280
   cccctcgtca caataaagat aggggggcaa ttaaaggaag ctctattaga tacaggagca     2340
   gatgatacag tattagaaga aatgaatttg ccaggaagat ggaaaccaaa aatgataggg     2400
   ggaattggag gttttatcaa agtaggacag tatgatcaga tactcataga aatctgcgga     2460
   cataaagcta taggtacagt attagtagga cctacacctg tcaacataat tggaagaaat     2520
   ctgttgactc agattggctg cactttaaat tttcccatta gtcctattga gactgtacca     2580
   gtaaaattaa agccaggaat ggatggccca aaagttaaac aatggccatt gacagaagaa     2640
   aaaataaaag cattagtaga aatttgtaca gaaatggaaa aggaaggaaa aatttcaaaa     2700
   attgggcctg aaaatccata caatactcca gtatttgcca taaagaaaaa agacagtact     2760
   aaatggagaa aattagtaga tttcagagaa cttaataaga gaactcaaga tttctgggaa     2820
   gttcaattag gaataccaca tcctgcaggg ttaaaacaga aaaaatcagt aacagtactg     2880
   gatgtgggcg atgcatattt ttcagttccc ttagataaag acttcaggaa gtatactgca     2940
   tttaccatac ctagtataaa caatgagaca ccagggatta gatatcagta caatgtgctt     3000
   ccacagggat ggaaaggatc accagcaata ttccagtgta gcatgacaaa aatcttagag     3060
   ccttttagaa aacaaaatcc agacatagtc atctatcaat acatggatga tttgtatgta     3120
   ggatctgact tagaaatagg gcagcataga acaaaaatag aggaactgag acaacatctg     3180
   ttgaggtggg gatttaccac accagacaaa aaacatcaga aagaacctcc attcctttgg     3240
   atgggttatg aactccatcc tgataaatgg acagtacagc ctatagtgct gccagaaaag     3300
   gacagctgga ctgtcaatga catacagaaa ttagtgggaa aattgaattg ggcaagtcag     3360
   atttatgcag ggattaaagt aaggcaatta tgtaaacttc ttaggggaac caaagcacta     3420
   acagaagtag taccactaac agaagaagca gagctagaac tggcagaaaa cagggagatt     3480
   ctaaaagaac cggtacatgg agtgtattat gacccatcaa aagacttaat agcagaaata     3540
   cagaagcagg ggcaaggcca atggacatat caaatttatc aagagccatt taaaaatctg     3600
   aaaacaggaa aatatgcaag aatgaagggt gcccacacta atgatgtgaa acaattaaca     3660
   gaggcagtac aaaaaatagc cacagaaagc atagtaatat ggggaaagac tcctaaattt 3720
   aaattaccca tacaaaagga aacatgggaa gcatggtgga cagagtattg gcaagccacc 3780
   tggattcctg agtgggagtt tgtcaatacc cctcccttag tgaagttatg gtaccagtta 3840
   gagaaagaac ccataatagg agcagaaact ttctatgtag atggggcagc caatagggaa 3900
   actaaattag gaaaagcagg atatgtaact gacagaggaa gacaaaaagt tgtcccccta 3960
   acggacacaa caaatcagaa gactgagtta caagcaattc atctagcttt gcaggattcg 4020
   ggattagaag taaacatagt gacagactca caatatgcat tgggaatcat tcaagcacaa 4080
   ccagataaga gtgaatcaga gttagtcagt caaataatag agcagttaat aaaaaaggaa 4140
   aaagtctacc tggcatgggt accagcacac aaaggaattg gaggaaatga acaagtagat 4200
   aaattggtca gtgctggaat caggaaagta ctatttttag atggaataga taaggcccaa 4260
   gaagaacatg agaaatatca cagtaattgg agagcaatgg ctagtgattt taacctacca 4320
   cctgtagtag caaaagaaat agtagccagc tgtgataaat gtcagctaaa aggggaagcc 4380
   atgcatggac aagtagactg tagcccagga atatggcagc tagattgtac acatttagaa 4440
   ggaaaagtta tcttggtagc agttcatgta gccagtggat atatagaagc agaagtaatt 4500
   ccagcagaga cagggcaaga aacagcatac ttcctcttaa aattagcagg aagatggcca 4560
   gtaaaaacag tacatacaga caatggcagc aatttcacca gtactacagt taaggccgcc 4620
   tgttggtggg cggggatcaa gcaggaattt ggcattccct acaatcccca aagtcaagga 4680
   gtaatagaat ctatgaataa agaattaaag aaaattatag gacaggtaag agatcaggct 4740
   gaacatctta agacagcagt acaaatggca gtattcatcc acaattttaa aagaaaaggg 4800
   gggattgggg ggtacagtgc aggggaaaga atagtagaca taatagcaac agacatacaa 4860
   actaaagaat tacaaaaaca aattacaaaa attcaaaatt ttcgggttta ttacagggac 4920
   agcagagatc cagtttggaa aggaccagca aagctcctct ggaaaggtga aggggcagta 4980
   gtaatacaag ataatagtga cataaaagta gtgccaagaa gaaaagcaaa gatcatcagg 5040
   gattatggaa aacagatggc aggtgatgat tgtgtggcaa gtagacagga tgaggattaa 5100
   cacatggaaa agattagtaa aacaccatat gtatatttca aggaaagcta aggactggtt 5160
   ttatagacat cactatgaaa gtactaatcc aaaaataagt tcagaagtac acatcccact 5220
   aggggatgct aaattagtaa taacaacata ttggggtctg catacaggag aaagagactg 5280
   gcatttgggt cagggagtct ccatagaatg gaggaaaaag agatatagca cacaagtaga 5340
   ccctgaccta gcagaccaac taattcatct gcactatttt gattgttttt cagaatctgc 5400
   tataagaaat accatattag gacgtatagt tagtcctagg tgtgaatatc aagcaggaca 5460
   taacaaggta ggatctctac agtacttggc actagcagca ttaataaaac caaaacagat 5520
   aaagccacct ttgcctagtg ttaggaaact gacagaggac agatggaaca agccccagaa 5580
   gaccaagggc cacagaggga gccatacaat gaatggacac tagagctttt agaggaactt 5640
   aagagtgaag ctgttagaca ttttcctagg atatggctcc ataacttagg acaacatatc 5700
   tatgaaactt acggggatac ttgggcagga gtggaagcca taataagaat tctgcaacaa 5760
   ctgctgttta tccatttcag aattgggtgt cgacatagca gaataggcgt tactcgacag 5820
   aggagagcaa gaaatggagc cagtagatcc tagactagag ccctggaagc atccaggaag 5880
   tcagcctaaa actgcttgta ccaattgcta ttgtaaaaag tgttgctttc attgccaagt 5940
   ttgtttcatg acaaaagcct taggcatctc ctatggcagg aagaagcgga gacagcgacg 6000
   aagagctcat cagaacagtc agactcatca agcttctcta tcaaagcagt aagtagtaca 6060
   tgtaatgcaa cctataatag tagcaatagt agcattagta gtagcaataa taatagcaat 6120
   agttgtgtgg tccatagtaa tcatagaata taggaaaata ttaagacaaa gaaaaataga 6180
   caggttaatt gatagactaa tagaaagagc agaagacagt ggcaatgaga gtgaaggaga 6240
   agtatcagca cttgtggaga tgggggtgga aatggggcac catgctcctt gggatattga 6300
   tgatctgtag tgctacagaa aaattgtggg tcacagtcta ttatggggta cctgtgtgga 6360
   aggaagcaac caccactcta ttttgtgcat cagatgctaa agcatatgat acagaggtac 6420
   ataatgtttg ggccacacat gcctgtgtac ccacagaccc caacccacaa gaagtagtat 6480
   tggtaaatgt gacagaaaat tttaacatgt ggaaaaatga catggtagaa cagatgcatg 6540
   aggatataat cagtttatgg gatcaaagcc taaagccatg tgtaaaatta accccactct 6600
   gtgttagttt aaagtgcact gatttgaaga atgatactaa taccaatagt agtagcggga 6660
   gaatgataat ggagaaagga gagataaaaa actgctcttt caatatcagc acaagcataa 6720
   gagataaggt gcagaaagaa tatgcattct tttataaact tgatatagta ccaatagata 6780
   ataccagcta taggttgata agttgtaaca cctcagtcat tacacaggcc tgtccaaagg 6840
   tatcctttga gccaattccc atacattatt gtgccccggc tggttttgcg attctaaaat 6900
   gtaataataa gacgttcaat ggaacaggac catgtacaaa tgtcagcaca gtacaatgta 6960
   cacatggaat caggccagta gtatcaactc aactgctgtt aaatggcagt ctagcagaag 7020
   aagatgtagt aattagatct gccaatttca cagacaatgc taaaaccata atagtacagc 7080
   tgaacacatc tgtagaaatt aattgtacaa gacccaacaa caatacaaga aaaagtatcc 7140
   gtatccagag gggaccaggg agagcatttg ttacaatagg aaaaatagga aatatgagac 7200
   aagcacattg taacattagt agagcaaaat ggaatgccac tttaaaacag atagctagca 7260
   aattaagaga acaatttgga aataataaaa caataatctt taagcaatcc tcaggagggg 7320
   acccagaaat tgtaacgcac agttttaatt gtggagggga atttttctac tgtaattcaa 7380
   cacaactgtt taatagtact tggtttaata gtacttggag tactgaaggg tcaaataaca 7440
   ctgaaggaag tgacacaatc acactcccat gcagaataaa acaatttata aacatgtggc 7500
   aggaagtagg aaaagcaatg tatgcccctc ccatcagtgg acaaattaga tgttcatcaa 7560
   atattactgg gctgctatta acaagagatg gtggtaataa caacaatggg tccgagatct 7620
   tcagacctgg aggaggcgat atgagggaca attggagaag tgaattatat aaatataaag 7680
   tagtaaaaat tgaaccatta ggagtagcac ccaccaaggc aaagagaaga gtggtgcaga 7740
   gagaaaaaag agcagtggga ataggagctt tgttccttgg gttcttggga gcagcaggaa 7800
   gcactatggg cgcagcgtca atgacgctga cggtacaggc cagacaatta ttgtctgata 7860
   tagtgcagca gcagaacaat ttgctgaggg ctattgaggc gcaacagcat ctgttgcaac 7920
   tcacagtctg gggcatcaaa cagctccagg caagaatcct ggctgtggaa agatacctaa 7980
   aggatcaaca gctcctgggg atttggggtt gctctggaaa actcatttgc accactgctg 8040
   tgccttggaa tgctagttgg agtaataaat ctctggaaca gatttggaat aacatgacct 8100
   ggatggagtg ggacagagaa attaacaatt acacaagctt aatacactcc ttaattgaag 8160
   aatcgcaaaa ccagcaagaa aagaatgaac aagaattatt ggaattagat aaatgggcaa 8220
   gtttgtggaa ttggtttaac ataacaaatt ggctgtggta tataaaatta ttcataatga 8280
   tagtaggagg cttggtaggt ttaagaatag tttttgctgt actttctata gtgaatagag 8340
   ttaggcaggg atattcacca ttatcgtttc agacccacct cccaatcccg aggggacccg 8400
   acaggcccga aggaatagaa gaagaaggtg gagagagaga cagagacaga tccattcgat 8460
   tagtgaacgg atccttagca cttatctggg acgatctgcg gagcctgtgc ctcttcagct 8520
   accaccgctt gagagactta ctcttgattg taacgaggat tgtggaactt ctgggacgca 8580
   gggggtggga agccctcaaa tattggtgga atctcctaca gtattggagt caggaactaa 8640
   agaatagtgc tgttaacttg ctcaatgcca cagccatagc agtagctgag gggacagata 8700
   gggttataga agtattacaa gcagcttata gagctattcg ccacatacct agaagaataa 8760
   gacagggctt ggaaaggatt ttgctataag atgggtggca agtggtcaaa aagtagtgtg 8820
   attggatggc ctgctgtaag ggaaagaatg agacgagctg agccagcagc agatggggtg 8880
   ggagcagtat ctcgagacct agaaaaacat ggagcaatca caagtagcaa tacagcagct 8940
   aacaatgctg cttgtgcctg gctagaagca caagaggagg aagaggtggg ttttccagtc 9000
   acacctcagg tacctttaag accaatgact tacaaggcag ctgtagatct tagccacttt 9060
   ttaaaagaaa aggggggact ggaagggcta attcactccc aaagaaggca agatatcctt 9120
   gatctgtgga tctaccacac acaaggctac ttccctgatt ggcagaacta cacaccaggg 9180
   ccaggggtca gatatccact gacctttgga tggtgctaca agctagtacc agctgagcca 9240
   gataaggtag aagaggccaa taaaggagag aacaccagct tgttacaccc tgtgagcctg 9300
   catggaatgg atgaccctga gagagaagtg ttagagtgga ggtttgacag ccgcctagca 9360
   tttcatcacg tggcccgaga gctgcatccg gagtacttca agaactgctg aacgcgtatt 9420
   gcattactag ttattaatag taatcaatta cggggtcatt agttcatagc ccatatatgg 9480
   agttccgcgt tacataactt acggtaaatg gcccgcctgg ctgaccgccc aacgaccccc 9540
   gcccattgac gtcaataatg acgtatgttc ccatagtaac gccaataggg actttccatt 9600
   gacgtcaatg ggtggagtat ttacggtaaa ctgcccactt ggcagtacat caagtgtatc 9660
   atatgccaag tccgccccct attgacgtca atgacggtaa atggcccgcc tggcattatg 9720
   cccagtacat gaccttacgg gactttccta cttggcagta catctacgta ttagtcatcg 9780
   ctattaccat ggtgatgcgg ttttggcagt acaccaatgg gcgtggatag cggtttgact 9840
   cacggggatt tccaagtctc caccccattg acgtcaatgg gagtttgttt tggcaccaaa 9900
   atcaacggga ctttccaaaa tgtcgtaata accccgcccc gttgacgcaa atgggcggta 9960
   ggcgtgtacg gtgggaggtc tatataagca gagctcgttt agtgaaccgt cagaattgtt 10020
   tttattttta attttctttc aaatacttcc atcgaattca gatctggtac cacgcgtacc 10080
   atggtgccca agaagaagag gaaagtctcc aacctgctga ctgtgcacca aaacctgcct 10140
   gccctccctg tggatgccac ctctgatgaa gtcaggaaga acctgatgga catgttcagg 10200
   gacaggcagg ccttctctga acacacctgg aagatgctcc tgtctgtgtg cagatcctgg 10260
   gctgcctggt gcaagctgaa caacaggaaa tggttccctg ctgaacctga ggatgtgagg 10320
   gactacctcc tgtacctgca agccagaggc ctggctgtga agaccatcca acagcacctg 10380
   ggccagctca acatgctgca caggagatct ggcctgcctc gcccttctga ctccaatgct 10440
   gtgtccctgg tgatgaggag aatcagaaag gagaatgtgg atgctgggga gagagccaag 10500
   caggccctgg cctttgaacg cactgacttt gaccaagtca gatccctgat ggagaactct 10560
   gacagatgcc aggacatcag gaacctggcc ttcctgggca ttgcctacaa caccctgctg 10620
   cgcattgccg aaattgccag aatcagagtg aaggacatct cccgcaccga tggtgggaga 10680
   atgctgatcc acattggcag gaccaagacc ctggtgtcca cagctggtgt ggagaaggcc 10740
   ctgtccctgg gggttaccaa gctggtggag agatggatct ctgtgtctgg tgtggctgat 10800
   gaccccaaca actacctgtt ctgccgggtc agaaagaatg gtgtggctgc cccttctgcc 10860
   acctcccaac tgtccacccg ggccctggaa gggatctttg aggccaccca ccgcctgatc 10920
   tatggtgcca aggatgactc tgggcagaga tacctggcct ggtctggcca ctctgccaga 10980
   gtgggtgctg ccagggacat ggccagggct ggtgtgtcca tccctgaaat catgcaggct 11040
   ggtggctgga ccaatgtgaa catagtgatg aactacatca gaaacctgga ctctgagact 11100
   ggggccatgg tgaggctgct cgaggatggg gacggtggtg gtggcaagct ttgacgcatc 11160
   ccgggtagcc actttttaaa agaaaagggg ggactggaag ggctaattca ctcccaaaga 11220
   agacaagata tcccatccgg agtacttcaa gaactgctga catcgagctt gctacaaggg    11280
   actttccgct ggggactttc cagggaggcg tggcctgggc gggactgggg agtggcgagc    11340
   cctcagatgc tgcatataag cagctgcttt ttgcctgtac tgggtctctc tggttagacc    11400
   agatctgagc ctgggagctc tctggctaac tagggaaccc actgcttaag cctcaataaa    11460
   gcttgccttg agtgcttcaa gtagtgtgtg cccgtctgtt gtgtgactct ggtaactaga    11520
   gatccctcag acccttttag tcagtgtgga aaatctctag cagttctaga gcggccgctc    11580
   gc                                                                   11582
<210> 24
   <211> 11582
   <212> DNA
   <213> Artificial Sequence
<220>
   <223> HIV strain NL4-3 CMV Cre optimized codon
<400> 24
   gcgttggaag ggctaattca ctcccaaaga agacaagata tccttgatct gtggatctac      60
   cacacacaag gctacttccc tgattggcag aactacacac cagggccagg ggtcagatat     120
   ccactgacct ttggatggtg ctacaagcta gtaccagttg agccagataa ggtagaagag     180
   gccaataaag gagagaacac cagcttgtta caccctgtga gcctgcatgg aatggatgac     240
   cctgagagag aagtgttaga gtggaggttt gacagccgcc tagcatttca tcacgtggcc     300
   cgagagctgc atccggagta ctacaaagac tgctgacatc gagctttcta caagggactt     360
   tccgctgggg actttccagg gaggtgtggc ctgggcggga ctggggagtg gcgagccctc     420
   agatgctaca tataagcagc tgctttttgc ctgtactggg tctctctggt tagaccagat     480
   ctgagcctgg gagctctctg gctaactagg gaacccactg cttaagcctc aataaagctt     540
   gccttgagtg ctcaaagtag tgtgtgcccg tctgttgtgt gactctggta actagagatc     600
   cctcagaccc ttttagtcag tgtggaaaat ctctagcagt ggcgcccgaa cagggacttg     660
   aaagcgaaag taaagccaga ggagatctct cgacgcagga ctcggcttgc tgaagcgcgc     720
   acggcaagag gcgaggggcg gcgactggtg agtacgccaa aaattttgac tagcggaggc     780
   tagaaggaga gagatgggtg cgagagcgtc ggtattaagc gggggagaat tagataaatg     840
   ggaaaaaatt cggttaaggc cagggggaaa gaaacaatat aaactaaaac atatagtatg     900
   ggcaagcagg gagctagaac gattcgcagt taatcctggc cttttagaga catcagaagg     960
   ctgtagacaa atactgggac agctacaacc atcccttcag acaggatcag aagaacttag    1020
   atcattatat aatacaatag cagtcctcta ttgtgtgcat caaaggatag atgtaaaaga    1080
   caccaaggaa gccttagata agatagagga agagcaaaac aaaagtaaga aaaaggcaca    1140
   gcaagcagca gctgacacag gaaacaacag ccaggtcagc caaaattacc ctatagtgca 1200
   gaacctccag gggcaaatgg tacatcaggc catatcacct agaactttaa atgcatgggt 1260
   aaaagtagta gaagagaagg ctttcagccc agaagtaata cccatgtttt cagcattatc 1320
   agaaggagcc accccacaag atttaaatac catgctaaac acagtggggg gacatcaagc 1380
   agccatgcaa atgttaaaag agaccatcaa tgaggaagct gcagaatggg atagattgca 1440
   tccagtgcat gcagggccta ttgcaccagg ccagatgaga gaaccaaggg gaagtgacat 1500
   agcaggaact actagtaccc ttcaggaaca aataggatgg atgacacata atccacctat 1560
   cccagtagga gaaatctata aaagatggat aatcctggga ttaaataaaa tagtaagaat 1620
   gtatagccct accagcattc tggacataag acaaggacca aaggaaccct ttagagacta 1680
   tgtagaccga ttctataaaa ctctaagagc cgagcaagct tcacaagagg taaaaaattg 1740
   gatgacagaa accttgttgg tccaaaatgc gaacccagat tgtaagacta ttttaaaagc 1800
   attgggacca ggagcgacac tagaagaaat gatgacagca tgtcagggag tggggggacc 1860
   cggccataaa gcaagagttt tggctgaagc aatgagccaa gtaacaaatc cagctaccat 1920
   aatgatacag aaaggcaatt ttaggaacca aagaaagact gttaagtgtt tcaattgtgg 1980
   caaagaaggg cacatagcca aaaattgcag ggcccctagg aaaaagggct gttggaaatg 2040
   tggaaaggaa ggacaccaaa tgaaagattg tactgagaga caggctaatt ttttagggaa 2100
   gatctggcct tcccacaagg gaaggccagg gaattttctt cagagcagac cagagccaac 2160
   agccccacca gaagagagct tcaggtttgg ggaagagaca acaactccct ctcagaagca 2220
   ggagccgata gacaaggaac tgtatccttt agcttccctc agatcactct ttggcagcga 2280
   cccctcgtca caataaagat aggggggcaa ttaaaggaag ctctattaga tacaggagca 2340
   gatgatacag tattagaaga aatgaatttg ccaggaagat ggaaaccaaa aatgataggg 2400
   ggaattggag gttttatcaa agtaggacag tatgatcaga tactcataga aatctgcgga 2460
   cataaagcta taggtacagt attagtagga cctacacctg tcaacataat tggaagaaat 2520
   ctgttgactc agattggctg cactttaaat tttcccatta gtcctattga gactgtacca 2580
   gtaaaattaa agccaggaat ggatggccca aaagttaaac aatggccatt gacagaagaa 2640
   aaaataaaag cattagtaga aatttgtaca gaaatggaaa aggaaggaaa aatttcaaaa 2700
   attgggcctg aaaatccata caatactcca gtatttgcca taaagaaaaa agacagtact 2760
   aaatggagaa aattagtaga tttcagagaa cttaataaga gaactcaaga tttctgggaa 2820
   gttcaattag gaataccaca tcctgcaggg ttaaaacaga aaaaatcagt aacagtactg 2880
   gatgtgggcg atgcatattt ttcagttccc ttagataaag acttcaggaa gtatactgca 2940
   tttaccatac ctagtataaa caatgagaca ccagggatta gatatcagta caatgtgctt 3000
   ccacagggat ggaaaggatc accagcaata ttccagtgta gcatgacaaa aatcttagag 3060
   ccttttagaa aacaaaatcc agacatagtc atctatcaat acatggatga tttgtatgta 3120
   ggatctgact tagaaatagg gcagcataga acaaaaatag aggaactgag acaacatctg 3180
   ttgaggtggg gatttaccac accagacaaa aaacatcaga aagaacctcc attcctttgg 3240
   atgggttatg aactccatcc tgataaatgg acagtacagc ctatagtgct gccagaaaag 3300
   gacagctgga ctgtcaatga catacagaaa ttagtgggaa aattgaattg ggcaagtcag 3360
   atttatgcag ggattaaagt aaggcaatta tgtaaacttc ttaggggaac caaagcacta 3420
   acagaagtag taccactaac agaagaagca gagctagaac tggcagaaaa cagggagatt 3480
   ctaaaagaac cggtacatgg agtgtattat gacccatcaa aagacttaat agcagaaata 3540
   cagaagcagg ggcaaggcca atggacatat caaatttatc aagagccatt taaaaatctg 3600
   aaaacaggaa aatatgcaag aatgaagggt gcccacacta atgatgtgaa acaattaaca 3660
   gaggcagtac aaaaaatagc cacagaaagc atagtaatat ggggaaagac tcctaaattt 3720
   aaattaccca tacaaaagga aacatgggaa gcatggtgga cagagtattg gcaagccacc 3780
   tggattcctg agtgggagtt tgtcaatacc cctcccttag tgaagttatg gtaccagtta 3840
   gagaaagaac ccataatagg agcagaaact ttctatgtag atggggcagc caatagggaa 3900
   actaaattag gaaaagcagg atatgtaact gacagaggaa gacaaaaagt tgtcccccta 3960
   acggacacaa caaatcagaa gactgagtta caagcaattc atctagcttt gcaggattcg 4020
   ggattagaag taaacatagt gacagactca caatatgcat tgggaatcat tcaagcacaa 4080
   ccagataaga gtgaatcaga gttagtcagt caaataatag agcagttaat aaaaaaggaa 4140
   aaagtctacc tggcatgggt accagcacac aaaggaattg gaggaaatga acaagtagat 4200
   aaattggtca gtgctggaat caggaaagta ctatttttag atggaataga taaggcccaa 4260
   gaagaacatg agaaatatca cagtaattgg agagcaatgg ctagtgattt taacctacca 4320
   cctgtagtag caaaagaaat agtagccagc tgtgataaat gtcagctaaa aggggaagcc 4380
   atgcatggac aagtagactg tagcccagga atatggcagc tagattgtac acatttagaa 4440
   ggaaaagtta tcttggtagc agttcatgta gccagtggat atatagaagc agaagtaatt 4500
   ccagcagaga cagggcaaga aacagcatac ttcctcttaa aattagcagg aagatggcca 4560
   gtaaaaacag tacatacaga caatggcagc aatttcacca gtactacagt taaggccgcc 4620
   tgttggtggg cggggatcaa gcaggaattt ggcattccct acaatcccca aagtcaagga 4680
   gtaatagaat ctatgaataa agaattaaag aaaattatag gacaggtaag agatcaggct 4740
   gaacatctta agacagcagt acaaatggca gtattcatcc acaattttaa aagaaaaggg 4800
   gggattgggg ggtacagtgc aggggaaaga atagtagaca taatagcaac agacatacaa 4860
   actaaagaat tacaaaaaca aattacaaaa attcaaaatt ttcgggttta ttacagggac 4920
   agcagagatc cagtttggaa aggaccagca aagctcctct ggaaaggtga aggggcagta 4980
   gtaatacaag ataatagtga cataaaagta gtgccaagaa gaaaagcaaa gatcatcagg 5040
   gattatggaa aacagatggc aggtgatgat tgtgtggcaa gtagacagga tgaggattaa 5100
   cacatggaaa agattagtaa aacaccatat gtatatttca aggaaagcta aggactggtt 5160
   ttatagacat cactatgaaa gtactaatcc aaaaataagt tcagaagtac acatcccact 5220
   aggggatgct aaattagtaa taacaacata ttggggtctg catacaggag aaagagactg 5280
   gcatttgggt cagggagtct ccatagaatg gaggaaaaag agatatagca cacaagtaga 5340
   ccctgaccta gcagaccaac taattcatct gcactatttt gattgttttt cagaatctgc 5400
   tataagaaat accatattag gacgtatagt tagtcctagg tgtgaatatc aagcaggaca 5460
   taacaaggta ggatctctac agtacttggc actagcagca ttaataaaac caaaacagat 5520
   aaagccacct ttgcctagtg ttaggaaact gacagaggac agatggaaca agccccagaa 5580
   gaccaagggc cacagaggga gccatacaat gaatggacac tagagctttt agaggaactt 5640
   aagagtgaag ctgttagaca ttttcctagg atatggctcc ataacttagg acaacatatc 5700
   tatgaaactt acggggatac ttgggcagga gtggaagcca taataagaat tctgcaacaa 5760
   ctgctgttta tccatttcag aattgggtgt cgacatagca gaataggcgt tactcgacag 5820
   aggagagcaa gaaatggagc cagtagatcc tagactagag ccctggaagc atccaggaag 5880
   tcagcctaaa actgcttgta ccaattgcta ttgtaaaaag tgttgctttc attgccaagt 5940
   ttgtttcatg acaaaagcct taggcatctc ctatggcagg aagaagcgga gacagcgacg 6000
   aagagctcat cagaacagtc agactcatca agcttctcta tcaaagcagt aagtagtaca 6060
   tgtaatgcaa cctataatag tagcaatagt agcattagta gtagcaataa taatagcaat 6120
   agttgtgtgg tccatagtaa tcatagaata taggaaaata ttaagacaaa gaaaaataga 6180
   caggttaatt gatagactaa tagaaagagc agaagacagt ggcaatgaga gtgaaggaga 6240
   agtatcagca cttgtggaga tgggggtgga aatggggcac catgctcctt gggatattga 6300
   tgatctgtag tgctacagaa aaattgtggg tcacagtcta ttatggggta cctgtgtgga 6360
   aggaagcaac caccactcta ttttgtgcat cagatgctaa agcatatgat acagaggtac 6420
   ataatgtttg ggccacacat gcctgtgtac ccacagaccc caacccacaa gaagtagtat 6480
   tggtaaatgt gacagaaaat tttaacatgt ggaaaaatga catggtagaa cagatgcatg 6540
   aggatataat cagtttatgg gatcaaagcc taaagccatg tgtaaaatta accccactct 6600
   gtgttagttt aaagtgcact gatttgaaga atgatactaa taccaatagt agtagcggga 6660
   gaatgataat ggagaaagga gagataaaaa actgctcttt caatatcagc acaagcataa 6720
   gagataaggt gcagaaagaa tatgcattct tttataaact tgatatagta ccaatagata 6780
   ataccagcta taggttgata agttgtaaca cctcagtcat tacacaggcc tgtccaaagg 6840
   tatcctttga gccaattccc atacattatt gtgccccggc tggttttgcg attctaaaat 6900
   gtaataataa gacgttcaat ggaacaggac catgtacaaa tgtcagcaca gtacaatgta 6960
   cacatggaat caggccagta gtatcaactc aactgctgtt aaatggcagt ctagcagaag 7020
   aagatgtagt aattagatct gccaatttca cagacaatgc taaaaccata atagtacagc 7080
   tgaacacatc tgtagaaatt aattgtacaa gacccaacaa caatacaaga aaaagtatcc 7140
   gtatccagag gggaccaggg agagcatttg ttacaatagg aaaaatagga aatatgagac 7200
   aagcacattg taacattagt agagcaaaat ggaatgccac tttaaaacag atagctagca 7260
   aattaagaga acaatttgga aataataaaa caataatctt taagcaatcc tcaggagggg 7320
   acccagaaat tgtaacgcac agttttaatt gtggagggga atttttctac tgtaattcaa 7380
   cacaactgtt taatagtact tggtttaata gtacttggag tactgaaggg tcaaataaca 7440
   ctgaaggaag tgacacaatc acactcccat gcagaataaa acaatttata aacatgtggc 7500
   aggaagtagg aaaagcaatg tatgcccctc ccatcagtgg acaaattaga tgttcatcaa 7560
   atattactgg gctgctatta acaagagatg gtggtaataa caacaatggg tccgagatct 7620
   tcagacctgg aggaggcgat atgagggaca attggagaag tgaattatat aaatataaag 7680
   tagtaaaaat tgaaccatta ggagtagcac ccaccaaggc aaagagaaga gtggtgcaga 7740
   gagaaaaaag agcagtggga ataggagctt tgttccttgg gttcttggga gcagcaggaa 7800
   gcactatggg cgcagcgtca atgacgctga cggtacaggc cagacaatta ttgtctgata 7860
   tagtgcagca gcagaacaat ttgctgaggg ctattgaggc gcaacagcat ctgttgcaac 7920
   tcacagtctg gggcatcaaa cagctccagg caagaatcct ggctgtggaa agatacctaa 7980
   aggatcaaca gctcctgggg atttggggtt gctctggaaa actcatttgc accactgctg 8040
   tgccttggaa tgctagttgg agtaataaat ctctggaaca gatttggaat aacatgacct 8100
   ggatggagtg ggacagagaa attaacaatt acacaagctt aatacactcc ttaattgaag 8160
   aatcgcaaaa ccagcaagaa aagaatgaac aagaattatt ggaattagat aaatgggcaa 8220
   gtttgtggaa ttggtttaac ataacaaatt ggctgtggta tataaaatta ttcataatga 8280
   tagtaggagg cttggtaggt ttaagaatag tttttgctgt actttctata gtgaatagag 8340
   ttaggcaggg atattcacca ttatcgtttc agacccacct cccaatcccg aggggacccg 8400
   acaggcccga aggaatagaa gaagaaggtg gagagagaga cagagacaga tccattcgat 8460
   tagtgaacgg atccttagca cttatctggg acgatctgcg gagcctgtgc ctcttcagct 8520
   accaccgctt gagagactta ctcttgattg taacgaggat tgtggaactt ctgggacgca 8580
   gggggtggga agccctcaaa tattggtgga atctcctaca gtattggagt caggaactaa 8640
   agaatagtgc tgttaacttg ctcaatgcca cagccatagc agtagctgag gggacagata 8700
   gggttataga agtattacaa gcagcttata gagctattcg ccacatacct agaagaataa 8760
   gacagggctt ggaaaggatt ttgctataag atgggtggca agtggtcaaa aagtagtgtg 8820
   attggatggc ctgctgtaag ggaaagaatg agacgagctg agccagcagc agatggggtg 8880
   ggagcagtat ctcgagacct agaaaaacat ggagcaatca caagtagcaa tacagcagct 8940
   aacaatgctg cttgtgcctg gctagaagca caagaggagg aagaggtggg ttttccagtc 9000
   acacctcagg tacctttaag accaatgact tacaaggcag ctgtagatct gagccatttc 9060
   ctgaaggaga agggaggcct tgagggcctg attcacagcc agcggcgcca agacatcctt 9120
   gacttgtgga tttaccatac acaggggtac ttccccgact ggcagaacta tacaccagga 9180
   cccggagtga gataccccct gaccttcgga tggtgttaca aactggttcc agtggagcct 9240
   gataaggtcg aagaggcaaa caaaggcgag aatacatctt tgctgcatcc tgtgtcactg 9300
   cacgggatgg acgaccctga gcgggaggtg cttgagtgga ggttcgactc tcgactggcc 9360
   tttcaccacg tagcaaggga gctgcaccct gagtatttta aaaattgttg aacgcgtatt 9420
   gcattactag ttattaatag taatcaatta cggggtcatt agttcatagc ccatatatgg 9480
   agttccgcgt tacataactt acggtaaatg gcccgcctgg ctgaccgccc aacgaccccc 9540
   gcccattgac gtcaataatg acgtatgttc ccatagtaac gccaataggg actttccatt 9600
   gacgtcaatg ggtggagtat ttacggtaaa ctgcccactt ggcagtacat caagtgtatc 9660
   atatgccaag tccgccccct attgacgtca atgacggtaa atggcccgcc tggcattatg 9720
   cccagtacat gaccttacgg gactttccta cttggcagta catctacgta ttagtcatcg 9780
   ctattaccat ggtgatgcgg ttttggcagt acaccaatgg gcgtggatag cggtttgact 9840
   cacggggatt tccaagtctc caccccattg acgtcaatgg gagtttgttt tggcaccaaa 9900
   atcaacggga ctttccaaaa tgtcgtaata accccgcccc gttgacgcaa atgggcggta 9960
   ggcgtgtacg gtgggaggtc tatataagca gagctcgttt agtgaaccgt cagaattgtt 10020
   tttattttta attttctttc aaatacttcc atcgaattca gatctggtac cacgcgtacc 10080
   atggtgccca agaagaagag gaaagtctcc aacctgctga ctgtgcacca aaacctgcct 10140
   gccctccctg tggatgccac ctctgatgaa gtcaggaaga acctgatgga catgttcagg 10200
   gacaggcagg ccttctctga acacacctgg aagatgctcc tgtctgtgtg cagatcctgg 10260
   gctgcctggt gcaagctgaa caacaggaaa tggttccctg ctgaacctga ggatgtgagg 10320
   gactacctcc tgtacctgca agccagaggc ctggctgtga agaccatcca acagcacctg 10380
   ggccagctca acatgctgca caggagatct ggcctgcctc gcccttctga ctccaatgct 10440
   gtgtccctgg tgatgaggag aatcagaaag gagaatgtgg atgctgggga gagagccaag 10500
   caggccctgg cctttgaacg cactgacttt gaccaagtca gatccctgat ggagaactct 10560
   gacagatgcc aggacatcag gaacctggcc ttcctgggca ttgcctacaa caccctgctg 10620
   cgcattgccg aaattgccag aatcagagtg aaggacatct cccgcaccga tggtgggaga 10680
   atgctgatcc acattggcag gaccaagacc ctggtgtcca cagctggtgt ggagaaggcc 10740
   ctgtccctgg gggttaccaa gctggtggag agatggatct ctgtgtctgg tgtggctgat 10800
   gaccccaaca actacctgtt ctgccgggtc agaaagaatg gtgtggctgc cccttctgcc 10860
   acctcccaac tgtccacccg ggccctggaa gggatctttg aggccaccca ccgcctgatc 10920
   tatggtgcca aggatgactc tgggcagaga tacctggcct ggtctggcca ctctgccaga 10980
   gtgggtgctg ccagggacat ggccagggct ggtgtgtcca tccctgaaat catgcaggct 11040
   ggtggctgga ccaatgtgaa catagtgatg aactacatca gaaacctgga ctctgagact 11100
   ggggccatgg tgaggctgct cgaggatggg gacggtggtg gtggcaagct ttgacgcatc 11160
   ccgggtagcc actttttaaa agaaaagggg ggactggaag ggctaattca ctcccaaaga 11220
   agacaagata tcccatccgg agtacttcaa gaactgctga catcgagctt gctacaaggg 11280
   actttccgct ggggactttc cagggaggcg tggcctgggc gggactgggg agtggcgagc 11340
   cctcagatgc tgcatataag cagctgcttt ttgcctgtac tgggtctctc tggttagacc 11400
   agatctgagc ctgggagctc tctggctaac tagggaaccc actgcttaag cctcaataaa 11460
   gcttgccttg agtgcttcaa gtagtgtgtg cccgtctgtt gtgtgactct ggtaactaga 11520
   gatccctcag acccttttag tcagtgtgga aaatctctag cagttctaga gcggccgctc 11580
   gc 11582
<210> 25
   <211> 12024
   <212> DNA
   <213> Artificial Sequence
<220>
   <223> SIVmac239-Nef-IRES-Cre
<400> 25
   tggaagggat ttattacagt gcaagaagac atagaatctt agacatatac ttagaaaagg        60
   aagaaggcat cataccagat tggcaggatt acacctcagg accaggaatt agatacccaa       120
   agacatttgg ctggctatgg aaattagtcc ctgtaaatgt atcagatgag gcacaggagg       180
   atgaggagca ttatttaatg catccagctc aaacttccca gtgggatgac ccttggggag       240
   aggttctagc atggaagttt gatccaactc tggcctacac ttatgaggca tatgttagat       300
   acccagaaga gtttggaagc aagtcaggcc tgtcagagga agaggttaga agaaggctaa       360
   ccgcaagagg ccttcttaac atggctgaca agaaggaaac tcgctgaaac agcagggact       420
   ttccacaagg ggatgttacg gggaggtact ggggaggagc cggtcgggaa cgcccacttt       480
   cttgatgtat aaatatcact gcatttcgct ctgtattcag tcgctctgcg gagaggctgg       540
   cagattgagc cctgggaggt tctctccagc actagcaggt agagcctggg tgttccctgc 600
   tagactctca ccagcacttg gccggtgctg ggcagagtga ctccacgctt gcttgcttaa 660
   agccctcttc aataaagctg ccattttagg aagtaagcta gtgtgtgttc ccatctctcc 720
   tagccgccgc ctggtcaact cggtacttca ataataagaa gactcctggt ctgttaggac 780
   cctttctgct ttgggaaacc gaagcaggaa aatccctagc agattggcgc ctgaacaggg 840
   acttgaagga gagtgagaga ctcctgagta cggctgagtg aaggcagtaa gggcggcagg 900
   aaccaaccac gacggagtgc tcctataaag gcgcgggtcg gtaccagacg gcgtgaggag 960
   cgggagagga agaggcctcc ggttgcaggt aagtgcaaca caaaaaagaa atagctgtct 1020
   tttatccagg aaggggtaat aagatagagt gggagatggg cgtgagaaac tccgtcttgt 1080
   cagggaagaa agcagatgaa ttagaaaaaa ttaggctacg acccaacgga aagaaaaagt 1140
   acatgttgaa gcatgtagta tgggcagcaa atgaattaga tagatttgga ttagcagaaa 1200
   gcctgttgga gaacaaagaa ggatgtcaaa aaatactttc ggtcttagct ccattagtgc 1260
   caacaggctc agaaaattta aaaagccttt ataatactgt ctgcgtcatc tggtgcattc 1320
   acgcagaaga gaaagtgaaa cacactgagg aagcaaaaca gatagtgcag agacacctag 1380
   tggtggaaac aggaacaaca gaaactatgc caaaaacaag tagaccaaca gcaccatcta 1440
   gcggcagagg aggaaattac ccagtacaac aaataggtgg taactatgtc cacctgccat 1500
   taagcccgag aacattaaat gcctgggtaa aattgataga ggaaaagaaa tttggagcag 1560
   aagtagtgcc aggatttcag gcactgtcag aaggttgcac cccctatgac attaatcaga 1620
   tgttaaattg tgtgggagac catcaagcgg ctatgcagat tatcagagat attataaacg 1680
   aggaggctgc agattgggac ttgcagcacc cacaaccagc tccacaacaa ggacaactta 1740
   gggagccgtc aggatcagat attgcaggaa caactagttc agtagatgaa caaatccagt 1800
   ggatgtacag acaacagaac cccataccag taggcaacat ttacaggaga tggatccaac 1860
   tggggttgca aaaatgtgtc agaatgtata acccaacaaa cattctagat gtaaaacaag 1920
   ggccaaaaga gccatttcag agctatgtag acaggttcta caaaagttta agagcagaac 1980
   agacagatgc agcagtaaag aattggatga ctcaaacact gctgattcaa aatgctaacc 2040
   cagattgcaa gctagtgctg aaggggctgg gtgtgaatcc caccctagaa gaaatgctga 2100
   cggcttgtca aggagtaggg gggccgggac agaaggctag attaatggca gaagccctga 2160
   aagaggccct cgcaccagtg ccaatccctt ttgcagcagc ccaacagagg ggaccaagaa 2220
   agccaattaa gtgttggaat tgtgggaaag agggacactc tgcaaggcaa tgcagagccc 2280
   caagaagaca gggatgctgg aaatgtggaa aaatggacca tgttatggcc aaatgcccag 2340
   acagacaggc gggtttttta ggccttggtc catggggaaa gaagccccgc aatttcccca 2400
   tggctcaagt gcatcagggg ctgatgccaa ctgctccccc agaggaccca gctgtggatc 2460
   tgctaaagaa ctacatgcag ttgggcaagc agcagagaga aaagcagaga gaaagcagag 2520
   agaagcctta caaggaggtg acagaggatt tgctgcacct caattctctc tttggaggag 2580
   accagtagtc actgctcata ttgaaggaca gcctgtagaa gtattactgg atacaggggc 2640
   tgatgattct attgtaacag gaatagagtt aggtccacat tataccccaa aaatagtagg 2700
   aggaatagga ggttttatta atactaaaga atacaaaaat gtagaaatag aagttttagg 2760
   caaaaggatt aaagggacaa tcatgacagg ggacaccccg attaacattt ttggtagaaa 2820
   tttgctaaca gctctgggga tgtctctaaa ttttcccata gctaaagtag agcctgtaaa 2880
   agtcgcctta aagccaggaa aggatggacc aaaattgaag cagtggccat tatcaaaaga 2940
   aaagatagtt gcattaagag aaatctgtga aaagatggaa aaggatggtc agttggagga 3000
   agctcccccg accaatccat acaacacccc cacatttgct ataaagaaaa aggataagaa 3060
   caaatggaga atgctgatag attttaggga actaaatagg gtcactcagg actttacgga 3120
   agtccaatta ggaataccac accctgcagg actagcaaaa aggaaaagaa ttacagtact 3180
   ggatataggt gatgcatatt tctccatacc tctagatgaa gaatttaggc agtacactgc 3240
   ctttacttta ccatcagtaa ataatgcaga gccaggaaaa cgatacattt ataaggttct 3300
   gcctcaggga tggaaggggt caccagccat cttccaatac actatgagac atgtgctaga 3360
   acccttcagg aaggcaaatc cagatgtgac cttagtccag tatatggatg acatcttaat 3420
   agctagtgac aggacagacc tggaacatga cagggtagtt ttacagtcaa aggaactctt 3480
   gaatagcata gggttttcta ccccagaaga gaaattccaa aaagatcccc catttcaatg 3540
   gatggggtac gaattgtggc caacaaaatg gaagttgcaa aagatagagt tgccacaaag 3600
   agagacctgg acagtgaatg atatacagaa gttagtagga gtattaaatt gggcagctca 3660
   aatttatcca ggtataaaaa ccaaacatct ctgtaggtta attagaggaa aaatgactct 3720
   aacagaggaa gttcagtgga ctgagatggc agaagcagaa tatgaggaaa ataaaataat 3780
   tctcagtcag gaacaagaag gatgttatta ccaagaaggc aagccattag aagccacggt 3840
   aataaagagt caggacaatc agtggtctta taaaattcac caagaagaca aaatactgaa 3900
   agtaggaaaa tttgcaaaga taaagaatac acataccaat ggagtgagac tattagcaca 3960
   tgtaatacag aaaataggaa aggaagcaat agtgatctgg ggacaggtcc caaaattcca 4020
   cttaccagtt gagaaggatg tatgggaaca gtggtggaca gactattggc aggtaacctg 4080
   gataccggaa tgggatttta tctcaacacc accgctagta agattagtct tcaatctagt 4140
   gaaggaccct atagagggag aagaaaccta ttatacagat ggatcatgta ataaacagtc 4200
   aaaagaaggg aaagcaggat atatcacaga taggggcaaa gacaaagtaa aagtgttaga 4260
   acagactact aatcaacaag cagaattgga agcatttctc atggcattga cagactcagg 4320
   gccaaaggca aatattatag tagattcaca atatgttatg ggaataataa caggatgccc 4380
   tacagaatca gagagcaggc tagttaatca aataatagaa gaaatgatta aaaagtcaga 4440
   aatttatgta gcatgggtac cagcacacaa aggtatagga ggaaaccaag aaatagacca 4500
   cctagttagt caagggatta gacaagttct cttcttggaa aagatagagc cagcacaaga 4560
   agaacatgat aaataccata gtaatgtaaa agaattggta ttcaaatttg gattacccag 4620
   aatagtggcc agacagatag tagacacctg tgataaatgt catcagaaag gagaggctat 4680
   acatgggcag gcaaattcag atctagggac ttggcaaatg gattgtaccc atctagaggg 4740
   aaaaataatc atagttgcag tacatgtagc tagtggattc atagaagcag aggtaattcc 4800
   acaagagaca ggaagacaga cagcactatt tctgttaaaa ttggcaggca gatggcctat 4860
   tacacatcta cacacagata atggtgctaa ctttgcttcg caagaagtaa agatggttgc 4920
   atggtgggca gggatagagc acacctttgg ggtaccatac aatccacaga gtcagggagt 4980
   agtggaagca atgaatcacc acctgaaaaa tcaaatagat agaatcaggg aacaagcaaa 5040
   ttcagtagaa accatagtat taatggcagt tcattgcatg aattttaaaa gaaggggagg 5100
   aataggggat atgactccag cagaaagatt aattaacatg atcactacag aacaagagat 5160
   acaatttcaa caatcaaaaa actcaaaatt taaaaatttt cgggtctatt acagagaagg 5220
   cagagatcaa ctgtggaagg gacccggtga gctattgtgg aaaggggaag gagcagtcat 5280
   cttaaaggta gggacagaca ttaaggtagt acccagaaga aaggctaaaa ttatcaaaga 5340
   ttatggagga ggaaaagagg tggatagcag ttcccacatg gaggataccg gagaggctag 5400
   agaggtggca tagcctcata aaatatctga aatataaaac taaagatcta caaaaggttt 5460
   gctatgtgcc ccattttaag gtcggatggg catggtggac ctgcagcaga gtaatcttcc 5520
   cactacagga aggaagccat ttagaagtac aagggtattg gcatttgaca ccagaaaaag 5580
   ggtggctcag tacttatgca gtgaggataa cctggtactc aaagaacttt tggacagatg 5640
   taacaccaaa ctatgcagac attttactgc atagcactta tttcccttgc tttacagcgg 5700
   gagaagtgag aagggccatc aggggagaac aactgctgtc ttgctgcagg ttcccgagag 5760
   ctcataagta ccaggtacca agcctacagt acttagcact gaaagtagta agcgatgtca 5820
   gatcccaggg agagaatccc acctggaaac agtggagaag agacaatagg agaggccttc 5880
   gaatggctaa acagaacagt agaggagata aacagagagg cggtaaacca cctaccaagg 5940
   gagctaattt tccaggtttg gcaaaggtct tgggaatact ggcatgatga acaagggatg 6000
   tcaccaagct atgtaaaata cagatacttg tgtttaatac aaaaggcttt atttatgcat 6060
   tgcaagaaag gctgtagatg tctaggggaa ggacatgggg cagggggatg gagaccagga 6120
   cctcctcctc ctccccctcc aggactagca taaatggaag aaagacctcc agaaaatgaa 6180
   ggaccacaaa gggaaccatg ggatgaatgg gtagtggagg ttctggaaga actgaaagaa 6240
   gaagctttaa aacattttga tcctcgcttg ctaactgcac ttggtaatca tatctataat 6300
   agacatggag acacccttga gggagcagga gaactcatta gaatcctcca acgagcgctc 6360
   ttcatgcatt tcagaggcgg atgcatccac tccagaatcg gccaacctgg gggaggaaat 6420
   cctctctcag ctataccgcc ctctagaagc atgctataac acatgctatt gtaaaaagtg 6480
   ttgctaccat tgccagtttt gttttcttaa aaaaggcttg gggatatgtt atgagcaatc 6540
   acgaaagaga agaagaactc cgaaaaaggc taaggctaat acatcttctg catcaaacaa 6600
   gtaagtatgg gatgtcttgg gaatcagctg cttatcgcca tcttgctttt aagtgtctat 6660
   gggatctatt gtactctata tgtcacagtc ttttatggtg taccagcttg gaggaatgcg 6720
   acaattcccc tcttttgtgc aaccaagaat agggatactt ggggaacaac tcagtgccta 6780
   ccagataatg gtgattattc agaagtggcc cttaatgtta cagaaagctt tgatgcctgg 6840
   aataatacag tcacagaaca ggcaatagag gatgtatggc aactctttga gacctcaata 6900
   aagccttgtg taaaattatc cccattatgc attactatga gatgcaataa aagtgagaca 6960
   gatagatggg gattgacaaa atcaataaca acaacagcat caacaacatc aacgacagca 7020
   tcagcaaaag tagacatggt caatgagact agttcttgta tagcccagga taattgcaca 7080
   ggcttggaac aagagcaaat gataagctgt aaattcaaca tgacagggtt aaaaagagac 7140
   aagaaaaaag agtacaatga aacttggtac tctgcagatt tggtatgtga acaagggaat 7200
   aacactggta atgaaagtag atgttacatg aaccactgta acacttctgt tatccaagag 7260
   tcttgtgaca aacattattg ggatgctatt agatttaggt attgtgcacc tccaggttat 7320
   gctttgctta gatgtaatga cacaaattat tcaggcttta tgcctaaatg ttctaaggtg 7380
   gtggtctctt catgcacaag gatgatggag acacagactt ctacttggtt tggctttaat 7440
   ggaactagag cagaaaatag aacttatatt tactggcatg gtagggataa taggactata 7500
   attagtttaa ataagtatta taatctaaca atgaaatgta gaagaccagg aaataagaca 7560
   gttttaccag tcaccattat gtctggattg gttttccact cacaaccaat caatgatagg 7620
   ccaaagcagg catggtgttg gtttggagga aaatggaagg atgcaataaa agaggtgaag 7680
   cagaccattg tcaaacatcc caggtatact ggaactaaca atactgataa aatcaatttg 7740
   acggctcctg gaggaggaga tccggaagtt accttcatgt ggacaaattg cagaggagag 7800
   ttcctctact gtaaaatgaa ttggtttcta aattgggtag aagataggaa tacagctaac 7860
   cagaagccaa aggaacagca taaaaggaat tacgtgccat gtcatattag acaaataatc 7920
   aacacttggc ataaagtagg caaaaatgtt tatttgcctc caagagaggg agacctcacg 7980
   tgtaactcca cagtgaccag tctcatagca aacatagatt ggattgatgg aaaccaaact 8040
   aatatcacca tgagtgcaga ggtggcagaa ctgtatcgat tggaattggg agattataaa 8100
   ttagtagaga tcactccaat tggcttggcc cccacagatg tgaagaggta cactactggt 8160
   ggcacctcaa gaaataaaag aggggtcttt gtgctagggt tcttgggttt tctcgcaacg 8220
   gcaggttctg caatgggcgc ggcgtcgttg acgctgaccg ctcagtcccg aactttattg 8280
   gctgggatag tgcagcaaca gcaacagctg ttggacgtgg tcaagagaca acaagaattg 8340
   ttgcgactga ccgtctgggg aacaaagaac ctccagacta gggtcactgc catcgagaag 8400
   tacttaaagg accaggcgca gctgaatgct tggggatgtg cgtttagaca agtctgccac 8460
   actactgtac catggccaaa tgcaagtcta acaccaaagt ggaacaatga gacttggcaa 8520
   gagtgggagc gaaaggttga cttcttggaa gaaaatataa cagccctcct agaggaggca 8580
   caaattcaac aagagaagaa catgtatgaa ttacaaaagt tgaatagctg ggatgtgttt 8640
   ggcaattggt ttgaccttgc ttcttggata aagtatatac aatatggagt ttatatagtt 8700
   gtaggagtaa tactgttaag aatagtgatc tatatagtac aaatgctagc taagttaagg 8760
   caggggtata ggccagtgtt ctcttcccca ccctcttatt tccagcagac ccatatccaa 8820
   caggacccgg cactgccaac cagagaaggc aaagaaagag acggtggaga aggcggtggc 8880
   aacagctcct ggccttggca gatagaatat attcatttcc tgatccgcca actgatacgc 8940
   ctcttgactt ggctattcag caactgcaga accttgctat cgagagtata ccagatcctc 9000
   caaccaatac tccagaggct ctctgcgacc ctacagagga ttcgagaagt cctcaggact 9060
   gaactgacct acctacaata tgggtggagc tatttccatg aggcggtcca ggccgtctgg 9120
   agatctgcga cagagactct tgcgggcgcg tggggagact tatgggagac tcttaggaga 9180
   ggtggaagat ggatactcgc aatccccagg aggattagac aagggcttga gctcactctc 9240
   ttgtgaggga cagaaataca atcagggaca gtatatgaat actccatgga gaaacccagc 9300
   tgaagagaga gaaaaattag catacagaaa acaaaatatg gatgatatag atgaggaaga 9360
   tgatgacttg gtaggggtat cagtgaggcc aaaagttccc ctaagaacaa tgagttacaa 9420
   attggcaata gacatgtctc attttataaa agaaaagggg ggactggaag ggatttatta 9480
   cagtgcaaga agacatagaa tcttagacat atacttagaa aaggaaggag gcatcatacc 9540
   agattggcag gattacacct caggaccagg aattagatac ccaaagacat ttggctggct 9600
   atggaaatta gtccctgtaa atgtatcaga tgaggcacag gaggatgagg agcattattt 9660
   aatgcatcca gctcaaactt cccagtggga tgacccttgg ggagaggttc tagcatggaa 9720
   gtttgatcca actctggcct acacttatga ggcatatgtt agatacccag aagagtttgg 9780
   aagcaagtca ggcctgtcag aggaagaggt tagaagaagg ctaaccgcaa gaggccttct 9840
   taacatggct gacaagaagg aaactcgctg aacgcgtcgg atcccggagt actacaaaga 9900
   ctgctgacgc gaattccgcc ccccccccta acgttactgg ccgaagccgc ttggaataag 9960
   gccggtgtgc gtttgtctat atgttatttt ccaccatatt gccgtctttt ggcaatgtga 10020
   gggcccggaa acctggccct gtcttcttga cgagcattcc taggggtctt tcccctctcg 10080
   ccaaaggaat gcaaggtctg ttgaatgtcg tgaaggaagc agttcctctg gaagcttctt 10140
   gaagacaaac aacgtctgta gcgacccttt gcaggcagcg gaacccccca cctggcgaca 10200
   ggtgcctctg cggccaaaag ccacgtgtat aagatacacc tgcaaaggcg gcacaacccc 10260
   agtgccacgt tgtgagttgg atagttgtgg aaagagtcaa atggctctcc tcaagcgtat 10320
   tcaacaaggg gctgaaggat gcccagaagg taccccattg tatgggatct gatctggggc 10380
   ctcggtgcac atgctttaca tgtgtttagt cgaggttaaa aaacgtctag gccccccgaa 10440
   ccacggggac gtggttttcc tttgaaaaac acgatgataa taccatggtg cccaagaaga 10500
   agaggaaagt ctccaacctg ctgactgtgc accaaaacct gcctgccctc cctgtggatg 10560
   ccacctctga tgaagtcagg aagaacctga tggacatgtt cagggacagg caggccttct 10620
   ctgaacacac ctggaagatg ctcctgtctg tgtgcagatc ctgggctgcc tggtgcaagc 10680
   tgaacaacag gaaatggttc cctgctgaac ctgaggatgt gagggactac ctcctgtacc 10740
   tgcaagccag aggcctggct gtgaagacca tccaacagca cctgggccag ctcaacatgc 10800
   tgcacaggag atctggcctg cctcgccctt ctgactccaa tgctgtgtcc ctggtgatga 10860
   ggagaatcag aaaggagaat gtggatgctg gggagagagc caagcaggcc ctggcctttg 10920
   aacgcactga ctttgaccaa gtcagatccc tgatggagaa ctctgacaga tgccaggaca 10980
   tcaggaacct ggccttcctg ggcattgcct acaacaccct gctgcgcatt gccgaaattg 11040
   ccagaatcag agtgaaggac atctcccgca ccgatggtgg gagaatgctg atccacattg 11100
   gcaggaccaa gaccctggtg tccacagctg gtgtggagaa ggccctgtcc ctgggggtta 11160
   ccaagctggt ggagagatgg atctctgtgt ctggtgtggc tgatgacccc aacaactacc 11220
   tgttctgccg ggtcagaaag aatggtgtgg ctgccccttc tgccacctcc caactgtcca 11280
   cccgggccct ggaagggatc tttgaggcca cccaccgcct gatctatggt gccaaggatg 11340
   actctgggca gagatacctg gcctggtctg gccactctgc cagagtgggt gctgccaggg 11400
   acatggccag ggctggtgtg tccatccctg aaatcatgca ggctggtggc tggaccaatg 11460
   tgaacatagt gatgaactac atcagaaacc tggactctga gactggggcc atggtgaggc 11520
   tgctcgagga tggggactga cccgggtctc attttataaa agaaaagggg ggactggaag 11580
   ggatttatcc gcggttcact cgagactcgc tgaaacagca gggactttcc acaaggggat 11640
   gttacgggga ggtactgggg aggagccggt cgggaacgcc cactttcttg atgtataaat 11700
   atcactgcat ttcgctctgt attcagtcgc tctgcggaga ggctggcaga ttgagccctg 11760
   ggaggttctc tccagcacta gcaggtagag cctgggtgtt ccctgctaga ctctcaccag 11820
   cacttggccg gtgctgggca gagtgactcc acgcttgctt gcttaaagcc ctcttcaata 11880
   aagctgccat tttagaagta agctagtgtg tgttcccatc tctcctagcc gccgcctggt 11940
   caactcggta ctcaataata agaagaccct ggtctgttag gaccctttct gctttgggaa 12000
   accgaagcag gaaaatccct agca 12024
<210> 26
   <211> 12024
   <212> DNA
   <213> Artificial Sequence
<220>
   <223> SIVmac239-Nef-IRES-Cre optimized codon
<400> 26
   tggaagggat ttattacagt gcaagaagac atagaatctt agacatatac ttagaaaagg      60
   aagaaggcat cataccagat tggcaggatt acacctcagg accaggaatt agatacccaa     120
   agacatttgg ctggctatgg aaattagtcc ctgtaaatgt atcagatgag gcacaggagg     180
   atgaggagca ttatttaatg catccagctc aaacttccca gtgggatgac ccttggggag     240
   aggttctagc atggaagttt gatccaactc tggcctacac ttatgaggca tatgttagat     300
   acccagaaga gtttggaagc aagtcaggcc tgtcagagga agaggttaga agaaggctaa     360
   ccgcaagagg ccttcttaac atggctgaca agaaggaaac tcgctgaaac agcagggact     420
   ttccacaagg ggatgttacg gggaggtact ggggaggagc cggtcgggaa cgcccacttt      480
   cttgatgtat aaatatcact gcatttcgct ctgtattcag tcgctctgcg gagaggctgg     540
   cagattgagc cctgggaggt tctctccagc actagcaggt agagcctggg tgttccctgc     600
   tagactctca ccagcacttg gccggtgctg ggcagagtga ctccacgctt gcttgcttaa     660
   agccctcttc aataaagctg ccattttagg aagtaagcta gtgtgtgttc ccatctctcc     720
   tagccgccgc ctggtcaact cggtacttca ataataagaa gactcctggt ctgttaggac     780
   cctttctgct ttgggaaacc gaagcaggaa aatccctagc agattggcgc ctgaacaggg     840
   acttgaagga gagtgagaga ctcctgagta cggctgagtg aaggcagtaa gggcggcagg     900
   aaccaaccac gacggagtgc tcctataaag gcgcgggtcg gtaccagacg gcgtgaggag     960
   cgggagagga agaggcctcc ggttgcaggt aagtgcaaca caaaaaagaa atagctgtct    1020
   tttatccagg aaggggtaat aagatagagt gggagatggg cgtgagaaac tccgtcttgt    1080
   cagggaagaa agcagatgaa ttagaaaaaa ttaggctacg acccaacgga aagaaaaagt    1140
   acatgttgaa gcatgtagta tgggcagcaa atgaattaga tagatttgga ttagcagaaa    1200
   gcctgttgga gaacaaagaa ggatgtcaaa aaatactttc ggtcttagct ccattagtgc    1260
   caacaggctc agaaaattta aaaagccttt ataatactgt ctgcgtcatc tggtgcattc    1320
   acgcagaaga gaaagtgaaa cacactgagg aagcaaaaca gatagtgcag agacacctag    1380
   tggtggaaac aggaacaaca gaaactatgc caaaaacaag tagaccaaca gcaccatcta    1440
   gcggcagagg aggaaattac ccagtacaac aaataggtgg taactatgtc cacctgccat 1500
   taagcccgag aacattaaat gcctgggtaa aattgataga ggaaaagaaa tttggagcag 1560
   aagtagtgcc aggatttcag gcactgtcag aaggttgcac cccctatgac attaatcaga 1620
   tgttaaattg tgtgggagac catcaagcgg ctatgcagat tatcagagat attataaacg 1680
   aggaggctgc agattgggac ttgcagcacc cacaaccagc tccacaacaa ggacaactta 1740
   gggagccgtc aggatcagat attgcaggaa caactagttc agtagatgaa caaatccagt 1800
   ggatgtacag acaacagaac cccataccag taggcaacat ttacaggaga tggatccaac 1860
   tggggttgca aaaatgtgtc agaatgtata acccaacaaa cattctagat gtaaaacaag 1920
   ggccaaaaga gccatttcag agctatgtag acaggttcta caaaagttta agagcagaac 1980
   agacagatgc agcagtaaag aattggatga ctcaaacact gctgattcaa aatgctaacc 2040
   cagattgcaa gctagtgctg aaggggctgg gtgtgaatcc caccctagaa gaaatgctga 2100
   cggcttgtca aggagtaggg gggccgggac agaaggctag attaatggca gaagccctga 2160
   aagaggccct cgcaccagtg ccaatccctt ttgcagcagc ccaacagagg ggaccaagaa 2220
   agccaattaa gtgttggaat tgtgggaaag agggacactc tgcaaggcaa tgcagagccc 2280
   caagaagaca gggatgctgg aaatgtggaa aaatggacca tgttatggcc aaatgcccag 2340
   acagacaggc gggtttttta ggccttggtc catggggaaa gaagccccgc aatttcccca 2400
   tggctcaagt gcatcagggg ctgatgccaa ctgctccccc agaggaccca gctgtggatc 2460
   tgctaaagaa ctacatgcag ttgggcaagc agcagagaga aaagcagaga gaaagcagag 2520
   agaagcctta caaggaggtg acagaggatt tgctgcacct caattctctc tttggaggag 2580
   accagtagtc actgctcata ttgaaggaca gcctgtagaa gtattactgg atacaggggc 2640
   tgatgattct attgtaacag gaatagagtt aggtccacat tataccccaa aaatagtagg 2700
   aggaatagga ggttttatta atactaaaga atacaaaaat gtagaaatag aagttttagg 2760
   caaaaggatt aaagggacaa tcatgacagg ggacaccccg attaacattt ttggtagaaa 2820
   tttgctaaca gctctgggga tgtctctaaa ttttcccata gctaaagtag agcctgtaaa 2880
   agtcgcctta aagccaggaa aggatggacc aaaattgaag cagtggccat tatcaaaaga 2940
   aaagatagtt gcattaagag aaatctgtga aaagatggaa aaggatggtc agttggagga 3000
   agctcccccg accaatccat acaacacccc cacatttgct ataaagaaaa aggataagaa 3060
   caaatggaga atgctgatag attttaggga actaaatagg gtcactcagg actttacgga 3120
   agtccaatta ggaataccac accctgcagg actagcaaaa aggaaaagaa ttacagtact 3180
   ggatataggt gatgcatatt tctccatacc tctagatgaa gaatttaggc agtacactgc 3240
   ctttacttta ccatcagtaa ataatgcaga gccaggaaaa cgatacattt ataaggttct 3300
   gcctcaggga tggaaggggt caccagccat cttccaatac actatgagac atgtgctaga 3360
   acccttcagg aaggcaaatc cagatgtgac cttagtccag tatatggatg acatcttaat 3420
   agctagtgac aggacagacc tggaacatga cagggtagtt ttacagtcaa aggaactctt 3480
   gaatagcata gggttttcta ccccagaaga gaaattccaa aaagatcccc catttcaatg 3540
   gatggggtac gaattgtggc caacaaaatg gaagttgcaa aagatagagt tgccacaaag 3600
   agagacctgg acagtgaatg atatacagaa gttagtagga gtattaaatt gggcagctca 3660
   aatttatcca ggtataaaaa ccaaacatct ctgtaggtta attagaggaa aaatgactct 3720
   aacagaggaa gttcagtgga ctgagatggc agaagcagaa tatgaggaaa ataaaataat 3780
   tctcagtcag gaacaagaag gatgttatta ccaagaaggc aagccattag aagccacggt 3840
   aataaagagt caggacaatc agtggtctta taaaattcac caagaagaca aaatactgaa 3900
   agtaggaaaa tttgcaaaga taaagaatac acataccaat ggagtgagac tattagcaca 3960
   tgtaatacag aaaataggaa aggaagcaat agtgatctgg ggacaggtcc caaaattcca 4020
   cttaccagtt gagaaggatg tatgggaaca gtggtggaca gactattggc aggtaacctg 4080
   gataccggaa tgggatttta tctcaacacc accgctagta agattagtct tcaatctagt 4140
   gaaggaccct atagagggag aagaaaccta ttatacagat ggatcatgta ataaacagtc 4200
   aaaagaaggg aaagcaggat atatcacaga taggggcaaa gacaaagtaa aagtgttaga 4260
   acagactact aatcaacaag cagaattgga agcatttctc atggcattga cagactcagg 4320
   gccaaaggca aatattatag tagattcaca atatgttatg ggaataataa caggatgccc 4380
   tacagaatca gagagcaggc tagttaatca aataatagaa gaaatgatta aaaagtcaga 4440
   aatttatgta gcatgggtac cagcacacaa aggtatagga ggaaaccaag aaatagacca 4500
   cctagttagt caagggatta gacaagttct cttcttggaa aagatagagc cagcacaaga 4560
   agaacatgat aaataccata gtaatgtaaa agaattggta ttcaaatttg gattacccag 4620
   aatagtggcc agacagatag tagacacctg tgataaatgt catcagaaag gagaggctat 4680
   acatgggcag gcaaattcag atctagggac ttggcaaatg gattgtaccc atctagaggg 4740
   aaaaataatc atagttgcag tacatgtagc tagtggattc atagaagcag aggtaattcc 4800
   acaagagaca ggaagacaga cagcactatt tctgttaaaa ttggcaggca gatggcctat 4860
   tacacatcta cacacagata atggtgctaa ctttgcttcg caagaagtaa agatggttgc 4920
   atggtgggca gggatagagc acacctttgg ggtaccatac aatccacaga gtcagggagt 4980
   agtggaagca atgaatcacc acctgaaaaa tcaaatagat agaatcaggg aacaagcaaa 5040
   ttcagtagaa accatagtat taatggcagt tcattgcatg aattttaaaa gaaggggagg 5100
   aataggggat atgactccag cagaaagatt aattaacatg atcactacag aacaagagat 5160
   acaatttcaa caatcaaaaa actcaaaatt taaaaatttt cgggtctatt acagagaagg 5220
   cagagatcaa ctgtggaagg gacccggtga gctattgtgg aaaggggaag gagcagtcat 5280
   cttaaaggta gggacagaca ttaaggtagt acccagaaga aaggctaaaa ttatcaaaga 5340
   ttatggagga ggaaaagagg tggatagcag ttcccacatg gaggataccg gagaggctag 5400
   agaggtggca tagcctcata aaatatctga aatataaaac taaagatcta caaaaggttt 5460
   gctatgtgcc ccattttaag gtcggatggg catggtggac ctgcagcaga gtaatcttcc 5520
   cactacagga aggaagccat ttagaagtac aagggtattg gcatttgaca ccagaaaaag 5580
   ggtggctcag tacttatgca gtgaggataa cctggtactc aaagaacttt tggacagatg 5640
   taacaccaaa ctatgcagac attttactgc atagcactta tttcccttgc tttacagcgg 5700
   gagaagtgag aagggccatc aggggagaac aactgctgtc ttgctgcagg ttcccgagag 5760
   ctcataagta ccaggtacca agcctacagt acttagcact gaaagtagta agcgatgtca 5820
   gatcccaggg agagaatccc acctggaaac agtggagaag agacaatagg agaggccttc 5880
   gaatggctaa acagaacagt agaggagata aacagagagg cggtaaacca cctaccaagg 5940
   gagctaattt tccaggtttg gcaaaggtct tgggaatact ggcatgatga acaagggatg 6000
   tcaccaagct atgtaaaata cagatacttg tgtttaatac aaaaggcttt atttatgcat 6060
   tgcaagaaag gctgtagatg tctaggggaa ggacatgggg cagggggatg gagaccagga 6120
   cctcctcctc ctccccctcc aggactagca taaatggaag aaagacctcc agaaaatgaa 6180
   ggaccacaaa gggaaccatg ggatgaatgg gtagtggagg ttctggaaga actgaaagaa 6240
   gaagctttaa aacattttga tcctcgcttg ctaactgcac ttggtaatca tatctataat 6300
   agacatggag acacccttga gggagcagga gaactcatta gaatcctcca acgagcgctc 6360
   ttcatgcatt tcagaggcgg atgcatccac tccagaatcg gccaacctgg gggaggaaat 6420
   cctctctcag ctataccgcc ctctagaagc atgctataac acatgctatt gtaaaaagtg 6480
   ttgctaccat tgccagtttt gttttcttaa aaaaggcttg gggatatgtt atgagcaatc 6540
   acgaaagaga agaagaactc cgaaaaaggc taaggctaat acatcttctg catcaaacaa 6600
   gtaagtatgg gatgtcttgg gaatcagctg cttatcgcca tcttgctttt aagtgtctat 6660
   gggatctatt gtactctata tgtcacagtc ttttatggtg taccagcttg gaggaatgcg 6720
   acaattcccc tcttttgtgc aaccaagaat agggatactt ggggaacaac tcagtgccta 6780
   ccagataatg gtgattattc agaagtggcc cttaatgtta cagaaagctt tgatgcctgg 6840
   aataatacag tcacagaaca ggcaatagag gatgtatggc aactctttga gacctcaata 6900
   aagccttgtg taaaattatc cccattatgc attactatga gatgcaataa aagtgagaca 6960
   gatagatggg gattgacaaa atcaataaca acaacagcat caacaacatc aacgacagca 7020
   tcagcaaaag tagacatggt caatgagact agttcttgta tagcccagga taattgcaca 7080
   ggcttggaac aagagcaaat gataagctgt aaattcaaca tgacagggtt aaaaagagac 7140
   aagaaaaaag agtacaatga aacttggtac tctgcagatt tggtatgtga acaagggaat 7200
   aacactggta atgaaagtag atgttacatg aaccactgta acacttctgt tatccaagag 7260
   tcttgtgaca aacattattg ggatgctatt agatttaggt attgtgcacc tccaggttat 7320
   gctttgctta gatgtaatga cacaaattat tcaggcttta tgcctaaatg ttctaaggtg 7380
   gtggtctctt catgcacaag gatgatggag acacagactt ctacttggtt tggctttaat 7440
   ggaactagag cagaaaatag aacttatatt tactggcatg gtagggataa taggactata 7500
   attagtttaa ataagtatta taatctaaca atgaaatgta gaagaccagg aaataagaca 7560
   gttttaccag tcaccattat gtctggattg gttttccact cacaaccaat caatgatagg 7620
   ccaaagcagg catggtgttg gtttggagga aaatggaagg atgcaataaa agaggtgaag 7680
   cagaccattg tcaaacatcc caggtatact ggaactaaca atactgataa aatcaatttg 7740
   acggctcctg gaggaggaga tccggaagtt accttcatgt ggacaaattg cagaggagag 7800
   ttcctctact gtaaaatgaa ttggtttcta aattgggtag aagataggaa tacagctaac 7860
   cagaagccaa aggaacagca taaaaggaat tacgtgccat gtcatattag acaaataatc 7920
   aacacttggc ataaagtagg caaaaatgtt tatttgcctc caagagaggg agacctcacg 7980
   tgtaactcca cagtgaccag tctcatagca aacatagatt ggattgatgg aaaccaaact 8040
   aatatcacca tgagtgcaga ggtggcagaa ctgtatcgat tggaattggg agattataaa 8100
   ttagtagaga tcactccaat tggcttggcc cccacagatg tgaagaggta cactactggt 8160
   ggcacctcaa gaaataaaag aggggtcttt gtgctagggt tcttgggttt tctcgcaacg 8220
   gcaggttctg caatgggcgc ggcgtcgttg acgctgaccg ctcagtcccg aactttattg 8280
   gctgggatag tgcagcaaca gcaacagctg ttggacgtgg tcaagagaca acaagaattg 8340
   ttgcgactga ccgtctgggg aacaaagaac ctccagacta gggtcactgc catcgagaag 8400
   tacttaaagg accaggcgca gctgaatgct tggggatgtg cgtttagaca agtctgccac 8460
   actactgtac catggccaaa tgcaagtcta acaccaaagt ggaacaatga gacttggcaa 8520
   gagtgggagc gaaaggttga cttcttggaa gaaaatataa cagccctcct agaggaggca 8580
   caaattcaac aagagaagaa catgtatgaa ttacaaaagt tgaatagctg ggatgtgttt 8640
   ggcaattggt ttgaccttgc ttcttggata aagtatatac aatatggagt ttatatagtt 8700
   gtaggagtaa tactgttaag aatagtgatc tatatagtac aaatgctagc taagttaagg 8760
   caggggtata ggccagtgtt ctcttcccca ccctcttatt tccagcagac ccatatccaa 8820
   caggacccgg cactgccaac cagagaaggc aaagaaagag acggtggaga aggcggtggc 8880
   aacagctcct ggccttggca gatagaatat attcatttcc tgatccgcca actgatacgc 8940
   ctcttgactt ggctattcag caactgcaga accttgctat cgagagtata ccagatcctc 9000
   caaccaatac tccagaggct ctctgcgacc ctacagagga ttcgagaagt cctcaggact 9060
   gaactgacct acctacaata tgggtggagc tatttccatg aggcggtcca ggccgtctgg 9120
   agatctgcga cagagactct tgcgggcgcg tggggagact tatgggagac tcttaggaga 9180
   ggtggaagat ggatactcgc aatccccagg aggattagac aagggcttga gctcactctc 9240
   ttgtgaggga cagaaataca atcagggaca gtatatgaat actccatgga gaaacccagc 9300
   tgaagagaga gaaaaattag catacagaaa acaaaatatg gatgatatag atgaggaaga 9360
   tgatgacttg gtaggggtat cagtgaggcc aaaagttccc ctaagaacaa tgagttacaa 9420
   attggcaatt gacatgtcac atttcattaa ggagaagggc ggactggagg ggatatacta 9480
   ttctgccagg aggcatagaa tcttagacat atacttagaa aaggaaggag gcatcatacc 9540
   agattggcag gattacacct caggaccagg aattagatac ccaaagacat ttggctggct 9600
   atggaaatta gtccctgtaa atgtatcaga tgaggcacag gaggatgagg agcattattt 9660
   aatgcatcca gctcaaactt cccagtggga tgacccttgg ggagaggttc tagcatggaa 9720
   gtttgatcca actctggcct acacttatga ggcatatgtt agatacccag aagagtttgg 9780
   aagcaagtca ggcctgtcag aggaagaggt tagaagaagg ctaaccgcaa gaggccttct 9840
   taacatggct gacaagaagg aaactcgctg aacgcgtcgg atcccggagt actacaaaga 9900
   ctgctgacgc gaattccgcc ccccccccta acgttactgg ccgaagccgc ttggaataag 9960
   gccggtgtgc gtttgtctat atgttatttt ccaccatatt gccgtctttt ggcaatgtga 10020
   gggcccggaa acctggccct gtcttcttga cgagcattcc taggggtctt tcccctctcg 10080
   ccaaaggaat gcaaggtctg ttgaatgtcg tgaaggaagc agttcctctg gaagcttctt 10140
   gaagacaaac aacgtctgta gcgacccttt gcaggcagcg gaacccccca cctggcgaca 10200
   ggtgcctctg cggccaaaag ccacgtgtat aagatacacc tgcaaaggcg gcacaacccc 10260
   agtgccacgt tgtgagttgg atagttgtgg aaagagtcaa atggctctcc tcaagcgtat 10320
   tcaacaaggg gctgaaggat gcccagaagg taccccattg tatgggatct gatctggggc 10380
   ctcggtgcac atgctttaca tgtgtttagt cgaggttaaa aaacgtctag gccccccgaa 10440
   ccacggggac gtggttttcc tttgaaaaac acgatgataa taccatggtg cccaagaaga 10500
   agaggaaagt ctccaacctg ctgactgtgc accaaaacct gcctgccctc cctgtggatg 10560
   ccacctctga tgaagtcagg aagaacctga tggacatgtt cagggacagg caggccttct 10620
   ctgaacacac ctggaagatg ctcctgtctg tgtgcagatc ctgggctgcc tggtgcaagc 10680
   tgaacaacag gaaatggttc cctgctgaac ctgaggatgt gagggactac ctcctgtacc 10740
   tgcaagccag aggcctggct gtgaagacca tccaacagca cctgggccag ctcaacatgc 10800
   tgcacaggag atctggcctg cctcgccctt ctgactccaa tgctgtgtcc ctggtgatga 10860
   ggagaatcag aaaggagaat gtggatgctg gggagagagc caagcaggcc ctggcctttg 10920
   aacgcactga ctttgaccaa gtcagatccc tgatggagaa ctctgacaga tgccaggaca 10980
   tcaggaacct ggccttcctg ggcattgcct acaacaccct gctgcgcatt gccgaaattg 11040
   ccagaatcag agtgaaggac atctcccgca ccgatggtgg gagaatgctg atccacattg 11100
   gcaggaccaa gaccctggtg tccacagctg gtgtggagaa ggccctgtcc ctgggggtta 11160
   ccaagctggt ggagagatgg atctctgtgt ctggtgtggc tgatgacccc aacaactacc 11220
   tgttctgccg ggtcagaaag aatggtgtgg ctgccccttc tgccacctcc caactgtcca 11280
   cccgggccct ggaagggatc tttgaggcca cccaccgcct gatctatggt gccaaggatg 11340
   actctgggca gagatacctg gcctggtctg gccactctgc cagagtgggt gctgccaggg 11400
   acatggccag ggctggtgtg tccatccctg aaatcatgca ggctggtggc tggaccaatg 11460
   tgaacatagt gatgaactac atcagaaacc tggactctga gactggggcc atggtgaggc 11520
   tgctcgagga tggggactga cccgggtctc attttataaa agaaaagggg ggactggaag 11580
   ggatttatcc gcggttcact cgagactcgc tgaaacagca gggactttcc acaaggggat 11640
   gttacgggga ggtactgggg aggagccggt cgggaacgcc cactttcttg atgtataaat 11700
   atcactgcat ttcgctctgt attcagtcgc tctgcggaga ggctggcaga ttgagccctg 11760
   ggaggttctc tccagcacta gcaggtagag cctgggtgtt ccctgctaga ctctcaccag 11820
   cacttggccg gtgctgggca gagtgactcc acgcttgctt gcttaaagcc ctcttcaata 11880
   aagctgccat tttagaagta agctagtgtg tgttcccatc tctcctagcc gccgcctggt 11940
   caactcggta ctcaataata agaagaccct ggtctgttag gaccctttct gctttgggaa 12000
   accgaagcag gaaaatccct agca 12024
<210> 27
   <211> 12103
   <212> DNA
   <213> Artificial Sequence
<220>
   <223> SIVmac239-Nef-CMV-Cre
<400> 27
   tggaagggat ttattacagt gcaagaagac atagaatctt agacatatac ttagaaaagg       60
   aagaaggcat cataccagat tggcaggatt acacctcagg accaggaatt agatacccaa      120
   agacatttgg ctggctatgg aaattagtcc ctgtaaatgt atcagatgag gcacaggagg      180
   atgaggagca ttatttaatg catccagctc aaacttccca gtgggatgac ccttggggag      240
   aggttctagc atggaagttt gatccaactc tggcctacac ttatgaggca tatgttagat      300
   acccagaaga gtttggaagc aagtcaggcc tgtcagagga agaggttaga agaaggctaa      360
   ccgcaagagg ccttcttaac atggctgaca agaaggaaac tcgctgaaac agcagggact      420
   ttccacaagg ggatgttacg gggaggtact ggggaggagc cggtcgggaa cgcccacttt 480
   cttgatgtat aaatatcact gcatttcgct ctgtattcag tcgctctgcg gagaggctgg 540
   cagattgagc cctgggaggt tctctccagc actagcaggt agagcctggg tgttccctgc 600
   tagactctca ccagcacttg gccggtgctg ggcagagtga ctccacgctt gcttgcttaa 660
   agccctcttc aataaagctg ccattttagg aagtaagcta gtgtgtgttc ccatctctcc 720
   tagccgccgc ctggtcaact cggtacttca ataataagaa gactcctggt ctgttaggac 780
   cctttctgct ttgggaaacc gaagcaggaa aatccctagc agattggcgc ctgaacaggg 840
   acttgaagga gagtgagaga ctcctgagta cggctgagtg aaggcagtaa gggcggcagg 900
   aaccaaccac gacggagtgc tcctataaag gcgcgggtcg gtaccagacg gcgtgaggag 960
   cgggagagga agaggcctcc ggttgcaggt aagtgcaaca caaaaaagaa atagctgtct 1020
   tttatccagg aaggggtaat aagatagagt gggagatggg cgtgagaaac tccgtcttgt 1080
   cagggaagaa agcagatgaa ttagaaaaaa ttaggctacg acccaacgga aagaaaaagt 1140
   acatgttgaa gcatgtagta tgggcagcaa atgaattaga tagatttgga ttagcagaaa 1200
   gcctgttgga gaacaaagaa ggatgtcaaa aaatactttc ggtcttagct ccattagtgc 1260
   caacaggctc agaaaattta aaaagccttt ataatactgt ctgcgtcatc tggtgcattc 1320
   acgcagaaga gaaagtgaaa cacactgagg aagcaaaaca gatagtgcag agacacctag 1380
   tggtggaaac aggaacaaca gaaactatgc caaaaacaag tagaccaaca gcaccatcta 1440
   gcggcagagg aggaaattac ccagtacaac aaataggtgg taactatgtc cacctgccat 1500
   taagcccgag aacattaaat gcctgggtaa aattgataga ggaaaagaaa tttggagcag 1560
   aagtagtgcc aggatttcag gcactgtcag aaggttgcac cccctatgac attaatcaga 1620
   tgttaaattg tgtgggagac catcaagcgg ctatgcagat tatcagagat attataaacg 1680
   aggaggctgc agattgggac ttgcagcacc cacaaccagc tccacaacaa ggacaactta 1740
   gggagccgtc aggatcagat attgcaggaa caactagttc agtagatgaa caaatccagt 1800
   ggatgtacag acaacagaac cccataccag taggcaacat ttacaggaga tggatccaac 1860
   tggggttgca aaaatgtgtc agaatgtata acccaacaaa cattctagat gtaaaacaag 1920
   ggccaaaaga gccatttcag agctatgtag acaggttcta caaaagttta agagcagaac 1980
   agacagatgc agcagtaaag aattggatga ctcaaacact gctgattcaa aatgctaacc 2040
   cagattgcaa gctagtgctg aaggggctgg gtgtgaatcc caccctagaa gaaatgctga 2100
   cggcttgtca aggagtaggg gggccgggac agaaggctag attaatggca gaagccctga 2160
   aagaggccct cgcaccagtg ccaatccctt ttgcagcagc ccaacagagg ggaccaagaa 2220
   agccaattaa gtgttggaat tgtgggaaag agggacactc tgcaaggcaa tgcagagccc 2280
   caagaagaca gggatgctgg aaatgtggaa aaatggacca tgttatggcc aaatgcccag 2340
   acagacaggc gggtttttta ggccttggtc catggggaaa gaagccccgc aatttcccca 2400
   tggctcaagt gcatcagggg ctgatgccaa ctgctccccc agaggaccca gctgtggatc 2460
   tgctaaagaa ctacatgcag ttgggcaagc agcagagaga aaagcagaga gaaagcagag 2520
   agaagcctta caaggaggtg acagaggatt tgctgcacct caattctctc tttggaggag 2580
   accagtagtc actgctcata ttgaaggaca gcctgtagaa gtattactgg atacaggggc 2640
   tgatgattct attgtaacag gaatagagtt aggtccacat tataccccaa aaatagtagg 2700
   aggaatagga ggttttatta atactaaaga atacaaaaat gtagaaatag aagttttagg 2760
   caaaaggatt aaagggacaa tcatgacagg ggacaccccg attaacattt ttggtagaaa 2820
   tttgctaaca gctctgggga tgtctctaaa ttttcccata gctaaagtag agcctgtaaa 2880
   agtcgcctta aagccaggaa aggatggacc aaaattgaag cagtggccat tatcaaaaga 2940
   aaagatagtt gcattaagag aaatctgtga aaagatggaa aaggatggtc agttggagga 3000
   agctcccccg accaatccat acaacacccc cacatttgct ataaagaaaa aggataagaa 3060
   caaatggaga atgctgatag attttaggga actaaatagg gtcactcagg actttacgga 3120
   agtccaatta ggaataccac accctgcagg actagcaaaa aggaaaagaa ttacagtact 3180
   ggatataggt gatgcatatt tctccatacc tctagatgaa gaatttaggc agtacactgc 3240
   ctttacttta ccatcagtaa ataatgcaga gccaggaaaa cgatacattt ataaggttct 3300
   gcctcaggga tggaaggggt caccagccat cttccaatac actatgagac atgtgctaga 3360
   acccttcagg aaggcaaatc cagatgtgac cttagtccag tatatggatg acatcttaat 3420
   agctagtgac aggacagacc tggaacatga cagggtagtt ttacagtcaa aggaactctt 3480
   gaatagcata gggttttcta ccccagaaga gaaattccaa aaagatcccc catttcaatg 3540
   gatggggtac gaattgtggc caacaaaatg gaagttgcaa aagatagagt tgccacaaag 3600
   agagacctgg acagtgaatg atatacagaa gttagtagga gtattaaatt gggcagctca 3660
   aatttatcca ggtataaaaa ccaaacatct ctgtaggtta attagaggaa aaatgactct 3720
   aacagaggaa gttcagtgga ctgagatggc agaagcagaa tatgaggaaa ataaaataat 3780
   tctcagtcag gaacaagaag gatgttatta ccaagaaggc aagccattag aagccacggt 3840
   aataaagagt caggacaatc agtggtctta taaaattcac caagaagaca aaatactgaa 3900
   agtaggaaaa tttgcaaaga taaagaatac acataccaat ggagtgagac tattagcaca 3960
   tgtaatacag aaaataggaa aggaagcaat agtgatctgg ggacaggtcc caaaattcca 4020
   cttaccagtt gagaaggatg tatgggaaca gtggtggaca gactattggc aggtaacctg 4080
   gataccggaa tgggatttta tctcaacacc accgctagta agattagtct tcaatctagt 4140
   gaaggaccct atagagggag aagaaaccta ttatacagat ggatcatgta ataaacagtc 4200
   aaaagaaggg aaagcaggat atatcacaga taggggcaaa gacaaagtaa aagtgttaga 4260
   acagactact aatcaacaag cagaattgga agcatttctc atggcattga cagactcagg 4320
   gccaaaggca aatattatag tagattcaca atatgttatg ggaataataa caggatgccc 4380
   tacagaatca gagagcaggc tagttaatca aataatagaa gaaatgatta aaaagtcaga 4440
   aatttatgta gcatgggtac cagcacacaa aggtatagga ggaaaccaag aaatagacca 4500
   cctagttagt caagggatta gacaagttct cttcttggaa aagatagagc cagcacaaga 4560
   agaacatgat aaataccata gtaatgtaaa agaattggta ttcaaatttg gattacccag 4620
   aatagtggcc agacagatag tagacacctg tgataaatgt catcagaaag gagaggctat 4680
   acatgggcag gcaaattcag atctagggac ttggcaaatg gattgtaccc atctagaggg 4740
   aaaaataatc atagttgcag tacatgtagc tagtggattc atagaagcag aggtaattcc 4800
   acaagagaca ggaagacaga cagcactatt tctgttaaaa ttggcaggca gatggcctat 4860
   tacacatcta cacacagata atggtgctaa ctttgcttcg caagaagtaa agatggttgc 4920
   atggtgggca gggatagagc acacctttgg ggtaccatac aatccacaga gtcagggagt 4980
   agtggaagca atgaatcacc acctgaaaaa tcaaatagat agaatcaggg aacaagcaaa 5040
   ttcagtagaa accatagtat taatggcagt tcattgcatg aattttaaaa gaaggggagg 5100
   aataggggat atgactccag cagaaagatt aattaacatg atcactacag aacaagagat 5160
   acaatttcaa caatcaaaaa actcaaaatt taaaaatttt cgggtctatt acagagaagg 5220
   cagagatcaa ctgtggaagg gacccggtga gctattgtgg aaaggggaag gagcagtcat 5280
   cttaaaggta gggacagaca ttaaggtagt acccagaaga aaggctaaaa ttatcaaaga 5340
   ttatggagga ggaaaagagg tggatagcag ttcccacatg gaggataccg gagaggctag 5400
   agaggtggca tagcctcata aaatatctga aatataaaac taaagatcta caaaaggttt 5460
   gctatgtgcc ccattttaag gtcggatggg catggtggac ctgcagcaga gtaatcttcc 5520
   cactacagga aggaagccat ttagaagtac aagggtattg gcatttgaca ccagaaaaag 5580
   ggtggctcag tacttatgca gtgaggataa cctggtactc aaagaacttt tggacagatg 5640
   taacaccaaa ctatgcagac attttactgc atagcactta tttcccttgc tttacagcgg 5700
   gagaagtgag aagggccatc aggggagaac aactgctgtc ttgctgcagg ttcccgagag 5760
   ctcataagta ccaggtacca agcctacagt acttagcact gaaagtagta agcgatgtca 5820
   gatcccaggg agagaatccc acctggaaac agtggagaag agacaatagg agaggccttc 5880
   gaatggctaa acagaacagt agaggagata aacagagagg cggtaaacca cctaccaagg 5940
   gagctaattt tccaggtttg gcaaaggtct tgggaatact ggcatgatga acaagggatg 6000
   tcaccaagct atgtaaaata cagatacttg tgtttaatac aaaaggcttt atttatgcat 6060
   tgcaagaaag gctgtagatg tctaggggaa ggacatgggg cagggggatg gagaccagga 6120
   cctcctcctc ctccccctcc aggactagca taaatggaag aaagacctcc agaaaatgaa 6180
   ggaccacaaa gggaaccatg ggatgaatgg gtagtggagg ttctggaaga actgaaagaa 6240
   gaagctttaa aacattttga tcctcgcttg ctaactgcac ttggtaatca tatctataat 6300
   agacatggag acacccttga gggagcagga gaactcatta gaatcctcca acgagcgctc 6360
   ttcatgcatt tcagaggcgg atgcatccac tccagaatcg gccaacctgg gggaggaaat 6420
   cctctctcag ctataccgcc ctctagaagc atgctataac acatgctatt gtaaaaagtg 6480
   ttgctaccat tgccagtttt gttttcttaa aaaaggcttg gggatatgtt atgagcaatc 6540
   acgaaagaga agaagaactc cgaaaaaggc taaggctaat acatcttctg catcaaacaa 6600
   gtaagtatgg gatgtcttgg gaatcagctg cttatcgcca tcttgctttt aagtgtctat 6660
   gggatctatt gtactctata tgtcacagtc ttttatggtg taccagcttg gaggaatgcg 6720
   acaattcccc tcttttgtgc aaccaagaat agggatactt ggggaacaac tcagtgccta 6780
   ccagataatg gtgattattc agaagtggcc cttaatgtta cagaaagctt tgatgcctgg 6840
   aataatacag tcacagaaca ggcaatagag gatgtatggc aactctttga gacctcaata 6900
   aagccttgtg taaaattatc cccattatgc attactatga gatgcaataa aagtgagaca 6960
   gatagatggg gattgacaaa atcaataaca acaacagcat caacaacatc aacgacagca 7020
   tcagcaaaag tagacatggt caatgagact agttcttgta tagcccagga taattgcaca 7080
   ggcttggaac aagagcaaat gataagctgt aaattcaaca tgacagggtt aaaaagagac 7140
   aagaaaaaag agtacaatga aacttggtac tctgcagatt tggtatgtga acaagggaat 7200
   aacactggta atgaaagtag atgttacatg aaccactgta acacttctgt tatccaagag 7260
   tcttgtgaca aacattattg ggatgctatt agatttaggt attgtgcacc tccaggttat 7320
   gctttgctta gatgtaatga cacaaattat tcaggcttta tgcctaaatg ttctaaggtg 7380
   gtggtctctt catgcacaag gatgatggag acacagactt ctacttggtt tggctttaat 7440
   ggaactagag cagaaaatag aacttatatt tactggcatg gtagggataa taggactata 7500
   attagtttaa ataagtatta taatctaaca atgaaatgta gaagaccagg aaataagaca 7560
   gttttaccag tcaccattat gtctggattg gttttccact cacaaccaat caatgatagg 7620
   ccaaagcagg catggtgttg gtttggagga aaatggaagg atgcaataaa agaggtgaag 7680
   cagaccattg tcaaacatcc caggtatact ggaactaaca atactgataa aatcaatttg 7740
   acggctcctg gaggaggaga tccggaagtt accttcatgt ggacaaattg cagaggagag 7800
   ttcctctact gtaaaatgaa ttggtttcta aattgggtag aagataggaa tacagctaac 7860
   cagaagccaa aggaacagca taaaaggaat tacgtgccat gtcatattag acaaataatc 7920
   aacacttggc ataaagtagg caaaaatgtt tatttgcctc caagagaggg agacctcacg 7980
   tgtaactcca cagtgaccag tctcatagca aacatagatt ggattgatgg aaaccaaact 8040
   aatatcacca tgagtgcaga ggtggcagaa ctgtatcgat tggaattggg agattataaa 8100
   ttagtagaga tcactccaat tggcttggcc cccacagatg tgaagaggta cactactggt 8160
   ggcacctcaa gaaataaaag aggggtcttt gtgctagggt tcttgggttt tctcgcaacg 8220
   gcaggttctg caatgggcgc ggcgtcgttg acgctgaccg ctcagtcccg aactttattg 8280
   gctgggatag tgcagcaaca gcaacagctg ttggacgtgg tcaagagaca acaagaattg 8340
   ttgcgactga ccgtctgggg aacaaagaac ctccagacta gggtcactgc catcgagaag 8400
   tacttaaagg accaggcgca gctgaatgct tggggatgtg cgtttagaca agtctgccac 8460
   actactgtac catggccaaa tgcaagtcta acaccaaagt ggaacaatga gacttggcaa 8520
   gagtgggagc gaaaggttga cttcttggaa gaaaatataa cagccctcct agaggaggca 8580
   caaattcaac aagagaagaa catgtatgaa ttacaaaagt tgaatagctg ggatgtgttt 8640
   ggcaattggt ttgaccttgc ttcttggata aagtatatac aatatggagt ttatatagtt 8700
   gtaggagtaa tactgttaag aatagtgatc tatatagtac aaatgctagc taagttaagg 8760
   caggggtata ggccagtgtt ctcttcccca ccctcttatt tccagcagac ccatatccaa 8820
   caggacccgg cactgccaac cagagaaggc aaagaaagag acggtggaga aggcggtggc 8880
   aacagctcct ggccttggca gatagaatat attcatttcc tgatccgcca actgatacgc 8940
   ctcttgactt ggctattcag caactgcaga accttgctat cgagagtata ccagatcctc 9000
   caaccaatac tccagaggct ctctgcgacc ctacagagga ttcgagaagt cctcaggact 9060
   gaactgacct acctacaata tgggtggagc tatttccatg aggcggtcca ggccgtctgg 9120
   agatctgcga cagagactct tgcgggcgcg tggggagact tatgggagac tcttaggaga 9180
   ggtggaagat ggatactcgc aatccccagg aggattagac aagggcttga gctcactctc 9240
   ttgtgaggga cagaaataca atcagggaca gtatatgaat actccatgga gaaacccagc 9300
   tgaagagaga gaaaaattag catacagaaa acaaaatatg gatgatatag atgaggaaga 9360
   tgatgacttg gtaggggtat cagtgaggcc aaaagttccc ctaagaacaa tgagttacaa 9420
   attggcaata gacatgtctc attttataaa agaaaagggg ggactggaag ggatttatta 9480
   cagtgcaaga agacatagaa tcttagacat atacttagaa aaggaaggag gcatcatacc 9540
   agattggcag gattacacct caggaccagg aattagatac ccaaagacat ttggctggct 9600
   atggaaatta gtccctgtaa atgtatcaga tgaggcacag gaggatgagg agcattattt 9660
   aatgcatcca gctcaaactt cccagtggga tgacccttgg ggagaggttc tagcatggaa 9720
   gtttgatcca actctggcct acacttatga ggcatatgtt agatacccag aagagtttgg 9780
   aagcaagtca ggcctgtcag aggaagaggt tagaagaagg ctaaccgcaa gaggccttct 9840
   taacatggct gacaagaagg aaactcgctg aacgcgtatt gcattactag ttattaatag 9900
   taatcaatta cggggtcatt agttcatagc ccatatatgg agttccgcgt tacataactt 9960
   acggtaaatg gcccgcctgg ctgaccgccc aacgaccccc gcccattgac gtcaataatg 10020
   acgtatgttc ccatagtaac gccaataggg actttccatt gacgtcaatg ggtggagtat 10080
   ttacggtaaa ctgcccactt ggcagtacat caagtgtatc atatgccaag tccgccccct 10140
   attgacgtca atgacggtaa atggcccgcc tggcattatg cccagtacat gaccttacgg 10200
   gactttccta cttggcagta catctacgta ttagtcatcg ctattaccat ggtgatgcgg 10260
   ttttggcagt acaccaatgg gcgtggatag cggtttgact cacggggatt tccaagtctc 10320
   caccccattg acgtcaatgg gagtttgttt tggcaccaaa atcaacggga ctttccaaaa 10380
   tgtcgtaata accccgcccc gttgacgcaa atgggcggta ggcgtgtacg gtgggaggtc 10440
   tatataagca gagctcgttt agtgaaccgt cagaattgtt tttattttta attttctttc 10500
   aaatacttcc atcgaattca gatctggtac cacgcgtacc atggtgccca agaagaagag 10560
   gaaagtctcc aacctgctga ctgtgcacca aaacctgcct gccctccctg tggatgccac 10620
   ctctgatgaa gtcaggaaga acctgatgga catgttcagg gacaggcagg ccttctctga 10680
   acacacctgg aagatgctcc tgtctgtgtg cagatcctgg gctgcctggt gcaagctgaa 10740
   caacaggaaa tggttccctg ctgaacctga ggatgtgagg gactacctcc tgtacctgca 10800
   agccagaggc ctggctgtga agaccatcca acagcacctg ggccagctca acatgctgca 10860
   caggagatct ggcctgcctc gcccttctga ctccaatgct gtgtccctgg tgatgaggag 10920
   aatcagaaag gagaatgtgg atgctgggga gagagccaag caggccctgg cctttgaacg 10980
   cactgacttt gaccaagtca gatccctgat ggagaactct gacagatgcc aggacatcag 11040
   gaacctggcc ttcctgggca ttgcctacaa caccctgctg cgcattgccg aaattgccag 11100
   aatcagagtg aaggacatct cccgcaccga tggtgggaga atgctgatcc acattggcag 11160
   gaccaagacc ctggtgtcca cagctggtgt ggagaaggcc ctgtccctgg gggttaccaa 11220
   gctggtggag agatggatct ctgtgtctgg tgtggctgat gaccccaaca actacctgtt 11280
   ctgccgggtc agaaagaatg gtgtggctgc cccttctgcc acctcccaac tgtccacccg 11340
   ggccctggaa gggatctttg aggccaccca ccgcctgatc tatggtgcca aggatgactc 11400
   tgggcagaga tacctggcct ggtctggcca ctctgccaga gtgggtgctg ccagggacat 11460
   ggccagggct ggtgtgtcca tccctgaaat catgcaggct ggtggctgga ccaatgtgaa 11520
   catagtgatg aactacatca gaaacctgga ctctgagact ggggccatgg tgaggctgct 11580
   cgaggatggg gacggtggtg gtggcaagct ttgacgcatc ccgggtctca ttttataaaa 11640
   gaaaaggggg gactggaagg gatttatccg cggttcactc gagactcgct gaaacagcag 11700
   ggactttcca caaggggatg ttacggggag gtactgggga ggagccggtc gggaacgccc 11760
   actttcttga tgtataaata tcactgcatt tcgctctgta ttcagtcgct ctgcggagag    11820
   gctggcagat tgagccctgg gaggttctct ccagcactag caggtagagc ctgggtgttc    11880
   cctgctagac tctcaccagc acttggccgg tgctgggcag agtgactcca cgcttgcttg    11940
   cttaaagccc tcttcaataa agctgccatt ttagaagtaa gctagtgtgt gttcccatct    12000
   ctcctagccg ccgcctggtc aactcggtac tcaataataa gaagaccctg gtctgttagg    12060
   accctttctg ctttgggaaa ccgaagcagg aaaatcccta gca                      12103
<210> 28
   <211> 12103
   <212> DNA
   <213> Artificial Sequence
<220>
   <223> SIVmac239-Nef-CMV-Cre optimized codon
<400> 28
   tggaagggat ttattacagt gcaagaagac atagaatctt agacatatac ttagaaaagg        60
   aagaaggcat cataccagat tggcaggatt acacctcagg accaggaatt agatacccaa       120
   agacatttgg ctggctatgg aaattagtcc ctgtaaatgt atcagatgag gcacaggagg       180
   atgaggagca ttatttaatg catccagctc aaacttccca gtgggatgac ccttggggag       240
   aggttctagc atggaagttt gatccaactc tggcctacac ttatgaggca tatgttagat       300
   acccagaaga gtttggaagc aagtcaggcc tgtcagagga agaggttaga agaaggctaa       360
   ccgcaagagg ccttcttaac atggctgaca agaaggaaac tcgctgaaac agcagggact       420
   ttccacaagg ggatgttacg gggaggtact ggggaggagc cggtcgggaa cgcccacttt       480
   cttgatgtat aaatatcact gcatttcgct ctgtattcag tcgctctgcg gagaggctgg       540
   cagattgagc cctgggaggt tctctccagc actagcaggt agagcctggg tgttccctgc       600
   tagactctca ccagcacttg gccggtgctg ggcagagtga ctccacgctt gcttgcttaa       660
   agccctcttc aataaagctg ccattttagg aagtaagcta gtgtgtgttc ccatctctcc       720
   tagccgccgc ctggtcaact cggtacttca ataataagaa gactcctggt ctgttaggac       780
   cctttctgct ttgggaaacc gaagcaggaa aatccctagc agattggcgc ctgaacaggg       840
   acttgaagga gagtgagaga ctcctgagta cggctgagtg aaggcagtaa gggcggcagg       900
   aaccaaccac gacggagtgc tcctataaag gcgcgggtcg gtaccagacg gcgtgaggag       960
   cgggagagga agaggcctcc ggttgcaggt aagtgcaaca caaaaaagaa atagctgtct      1020
   tttatccagg aaggggtaat aagatagagt gggagatggg cgtgagaaac tccgtcttgt      1080
   cagggaagaa agcagatgaa ttagaaaaaa ttaggctacg acccaacgga aagaaaaagt      1140
   acatgttgaa gcatgtagta tgggcagcaa atgaattaga tagatttgga ttagcagaaa      1200
   gcctgttgga gaacaaagaa ggatgtcaaa aaatactttc ggtcttagct ccattagtgc 1260
   caacaggctc agaaaattta aaaagccttt ataatactgt ctgcgtcatc tggtgcattc 1320
   acgcagaaga gaaagtgaaa cacactgagg aagcaaaaca gatagtgcag agacacctag 1380
   tggtggaaac aggaacaaca gaaactatgc caaaaacaag tagaccaaca gcaccatcta 1440
   gcggcagagg aggaaattac ccagtacaac aaataggtgg taactatgtc cacctgccat 1500
   taagcccgag aacattaaat gcctgggtaa aattgataga ggaaaagaaa tttggagcag 1560
   aagtagtgcc aggatttcag gcactgtcag aaggttgcac cccctatgac attaatcaga 1620
   tgttaaattg tgtgggagac catcaagcgg ctatgcagat tatcagagat attataaacg 1680
   aggaggctgc agattgggac ttgcagcacc cacaaccagc tccacaacaa ggacaactta 1740
   gggagccgtc aggatcagat attgcaggaa caactagttc agtagatgaa caaatccagt 1800
   ggatgtacag acaacagaac cccataccag taggcaacat ttacaggaga tggatccaac 1860
   tggggttgca aaaatgtgtc agaatgtata acccaacaaa cattctagat gtaaaacaag 1920
   ggccaaaaga gccatttcag agctatgtag acaggttcta caaaagttta agagcagaac 1980
   agacagatgc agcagtaaag aattggatga ctcaaacact gctgattcaa aatgctaacc 2040
   cagattgcaa gctagtgctg aaggggctgg gtgtgaatcc caccctagaa gaaatgctga 2100
   cggcttgtca aggagtaggg gggccgggac agaaggctag attaatggca gaagccctga 2160
   aagaggccct cgcaccagtg ccaatccctt ttgcagcagc ccaacagagg ggaccaagaa 2220
   agccaattaa gtgttggaat tgtgggaaag agggacactc tgcaaggcaa tgcagagccc 2280
   caagaagaca gggatgctgg aaatgtggaa aaatggacca tgttatggcc aaatgcccag 2340
   acagacaggc gggtttttta ggccttggtc catggggaaa gaagccccgc aatttcccca 2400
   tggctcaagt gcatcagggg ctgatgccaa ctgctccccc agaggaccca gctgtggatc 2460
   tgctaaagaa ctacatgcag ttgggcaagc agcagagaga aaagcagaga gaaagcagag 2520
   agaagcctta caaggaggtg acagaggatt tgctgcacct caattctctc tttggaggag 2580
   accagtagtc actgctcata ttgaaggaca gcctgtagaa gtattactgg atacaggggc 2640
   tgatgattct attgtaacag gaatagagtt aggtccacat tataccccaa aaatagtagg 2700
   aggaatagga ggttttatta atactaaaga atacaaaaat gtagaaatag aagttttagg 2760
   caaaaggatt aaagggacaa tcatgacagg ggacaccccg attaacattt ttggtagaaa 2820
   tttgctaaca gctctgggga tgtctctaaa ttttcccata gctaaagtag agcctgtaaa 2880
   agtcgcctta aagccaggaa aggatggacc aaaattgaag cagtggccat tatcaaaaga 2940
   aaagatagtt gcattaagag aaatctgtga aaagatggaa aaggatggtc agttggagga 3000
   agctcccccg accaatccat acaacacccc cacatttgct ataaagaaaa aggataagaa 3060
   caaatggaga atgctgatag attttaggga actaaatagg gtcactcagg actttacgga 3120
   agtccaatta ggaataccac accctgcagg actagcaaaa aggaaaagaa ttacagtact 3180
   ggatataggt gatgcatatt tctccatacc tctagatgaa gaatttaggc agtacactgc 3240
   ctttacttta ccatcagtaa ataatgcaga gccaggaaaa cgatacattt ataaggttct 3300
   gcctcaggga tggaaggggt caccagccat cttccaatac actatgagac atgtgctaga 3360
   acccttcagg aaggcaaatc cagatgtgac cttagtccag tatatggatg acatcttaat 3420
   agctagtgac aggacagacc tggaacatga cagggtagtt ttacagtcaa aggaactctt 3480
   gaatagcata gggttttcta ccccagaaga gaaattccaa aaagatcccc catttcaatg 3540
   gatggggtac gaattgtggc caacaaaatg gaagttgcaa aagatagagt tgccacaaag 3600
   agagacctgg acagtgaatg atatacagaa gttagtagga gtattaaatt gggcagctca 3660
   aatttatcca ggtataaaaa ccaaacatct ctgtaggtta attagaggaa aaatgactct 3720
   aacagaggaa gttcagtgga ctgagatggc agaagcagaa tatgaggaaa ataaaataat 3780
   tctcagtcag gaacaagaag gatgttatta ccaagaaggc aagccattag aagccacggt 3840
   aataaagagt caggacaatc agtggtctta taaaattcac caagaagaca aaatactgaa 3900
   agtaggaaaa tttgcaaaga taaagaatac acataccaat ggagtgagac tattagcaca 3960
   tgtaatacag aaaataggaa aggaagcaat agtgatctgg ggacaggtcc caaaattcca 4020
   cttaccagtt gagaaggatg tatgggaaca gtggtggaca gactattggc aggtaacctg 4080
   gataccggaa tgggatttta tctcaacacc accgctagta agattagtct tcaatctagt 4140
   gaaggaccct atagagggag aagaaaccta ttatacagat ggatcatgta ataaacagtc 4200
   aaaagaaggg aaagcaggat atatcacaga taggggcaaa gacaaagtaa aagtgttaga 4260
   acagactact aatcaacaag cagaattgga agcatttctc atggcattga cagactcagg 4320
   gccaaaggca aatattatag tagattcaca atatgttatg ggaataataa caggatgccc 4380
   tacagaatca gagagcaggc tagttaatca aataatagaa gaaatgatta aaaagtcaga 4440
   aatttatgta gcatgggtac cagcacacaa aggtatagga ggaaaccaag aaatagacca 4500
   cctagttagt caagggatta gacaagttct cttcttggaa aagatagagc cagcacaaga 4560
   agaacatgat aaataccata gtaatgtaaa agaattggta ttcaaatttg gattacccag 4620
   aatagtggcc agacagatag tagacacctg tgataaatgt catcagaaag gagaggctat 4680
   acatgggcag gcaaattcag atctagggac ttggcaaatg gattgtaccc atctagaggg 4740
   aaaaataatc atagttgcag tacatgtagc tagtggattc atagaagcag aggtaattcc 4800
   acaagagaca ggaagacaga cagcactatt tctgttaaaa ttggcaggca gatggcctat 4860
   tacacatcta cacacagata atggtgctaa ctttgcttcg caagaagtaa agatggttgc 4920
   atggtgggca gggatagagc acacctttgg ggtaccatac aatccacaga gtcagggagt 4980
   agtggaagca atgaatcacc acctgaaaaa tcaaatagat agaatcaggg aacaagcaaa 5040
   ttcagtagaa accatagtat taatggcagt tcattgcatg aattttaaaa gaaggggagg 5100
   aataggggat atgactccag cagaaagatt aattaacatg atcactacag aacaagagat 5160
   acaatttcaa caatcaaaaa actcaaaatt taaaaatttt cgggtctatt acagagaagg 5220
   cagagatcaa ctgtggaagg gacccggtga gctattgtgg aaaggggaag gagcagtcat 5280
   cttaaaggta gggacagaca ttaaggtagt acccagaaga aaggctaaaa ttatcaaaga 5340
   ttatggagga ggaaaagagg tggatagcag ttcccacatg gaggataccg gagaggctag 5400
   agaggtggca tagcctcata aaatatctga aatataaaac taaagatcta caaaaggttt 5460
   gctatgtgcc ccattttaag gtcggatggg catggtggac ctgcagcaga gtaatcttcc 5520
   cactacagga aggaagccat ttagaagtac aagggtattg gcatttgaca ccagaaaaag 5580
   ggtggctcag tacttatgca gtgaggataa cctggtactc aaagaacttt tggacagatg 5640
   taacaccaaa ctatgcagac attttactgc atagcactta tttcccttgc tttacagcgg 5700
   gagaagtgag aagggccatc aggggagaac aactgctgtc ttgctgcagg ttcccgagag 5760
   ctcataagta ccaggtacca agcctacagt acttagcact gaaagtagta agcgatgtca 5820
   gatcccaggg agagaatccc acctggaaac agtggagaag agacaatagg agaggccttc 5880
   gaatggctaa acagaacagt agaggagata aacagagagg cggtaaacca cctaccaagg 5940
   gagctaattt tccaggtttg gcaaaggtct tgggaatact ggcatgatga acaagggatg 6000
   tcaccaagct atgtaaaata cagatacttg tgtttaatac aaaaggcttt atttatgcat 6060
   tgcaagaaag gctgtagatg tctaggggaa ggacatgggg cagggggatg gagaccagga 6120
   cctcctcctc ctccccctcc aggactagca taaatggaag aaagacctcc agaaaatgaa 6180
   ggaccacaaa gggaaccatg ggatgaatgg gtagtggagg ttctggaaga actgaaagaa 6240
   gaagctttaa aacattttga tcctcgcttg ctaactgcac ttggtaatca tatctataat 6300
   agacatggag acacccttga gggagcagga gaactcatta gaatcctcca acgagcgctc 6360
   ttcatgcatt tcagaggcgg atgcatccac tccagaatcg gccaacctgg gggaggaaat 6420
   cctctctcag ctataccgcc ctctagaagc atgctataac acatgctatt gtaaaaagtg 6480
   ttgctaccat tgccagtttt gttttcttaa aaaaggcttg gggatatgtt atgagcaatc 6540
   acgaaagaga agaagaactc cgaaaaaggc taaggctaat acatcttctg catcaaacaa 6600
   gtaagtatgg gatgtcttgg gaatcagctg cttatcgcca tcttgctttt aagtgtctat 6660
   gggatctatt gtactctata tgtcacagtc ttttatggtg taccagcttg gaggaatgcg 6720
   acaattcccc tcttttgtgc aaccaagaat agggatactt ggggaacaac tcagtgccta 6780
   ccagataatg gtgattattc agaagtggcc cttaatgtta cagaaagctt tgatgcctgg 6840
   aataatacag tcacagaaca ggcaatagag gatgtatggc aactctttga gacctcaata 6900
   aagccttgtg taaaattatc cccattatgc attactatga gatgcaataa aagtgagaca 6960
   gatagatggg gattgacaaa atcaataaca acaacagcat caacaacatc aacgacagca 7020
   tcagcaaaag tagacatggt caatgagact agttcttgta tagcccagga taattgcaca 7080
   ggcttggaac aagagcaaat gataagctgt aaattcaaca tgacagggtt aaaaagagac 7140
   aagaaaaaag agtacaatga aacttggtac tctgcagatt tggtatgtga acaagggaat 7200
   aacactggta atgaaagtag atgttacatg aaccactgta acacttctgt tatccaagag 7260
   tcttgtgaca aacattattg ggatgctatt agatttaggt attgtgcacc tccaggttat 7320
   gctttgctta gatgtaatga cacaaattat tcaggcttta tgcctaaatg ttctaaggtg 7380
   gtggtctctt catgcacaag gatgatggag acacagactt ctacttggtt tggctttaat 7440
   ggaactagag cagaaaatag aacttatatt tactggcatg gtagggataa taggactata 7500
   attagtttaa ataagtatta taatctaaca atgaaatgta gaagaccagg aaataagaca 7560
   gttttaccag tcaccattat gtctggattg gttttccact cacaaccaat caatgatagg 7620
   ccaaagcagg catggtgttg gtttggagga aaatggaagg atgcaataaa agaggtgaag 7680
   cagaccattg tcaaacatcc caggtatact ggaactaaca atactgataa aatcaatttg 7740
   acggctcctg gaggaggaga tccggaagtt accttcatgt ggacaaattg cagaggagag 7800
   ttcctctact gtaaaatgaa ttggtttcta aattgggtag aagataggaa tacagctaac 7860
   cagaagccaa aggaacagca taaaaggaat tacgtgccat gtcatattag acaaataatc 7920
   aacacttggc ataaagtagg caaaaatgtt tatttgcctc caagagaggg agacctcacg 7980
   tgtaactcca cagtgaccag tctcatagca aacatagatt ggattgatgg aaaccaaact 8040
   aatatcacca tgagtgcaga ggtggcagaa ctgtatcgat tggaattggg agattataaa 8100
   ttagtagaga tcactccaat tggcttggcc cccacagatg tgaagaggta cactactggt 8160
   ggcacctcaa gaaataaaag aggggtcttt gtgctagggt tcttgggttt tctcgcaacg 8220
   gcaggttctg caatgggcgc ggcgtcgttg acgctgaccg ctcagtcccg aactttattg 8280
   gctgggatag tgcagcaaca gcaacagctg ttggacgtgg tcaagagaca acaagaattg 8340
   ttgcgactga ccgtctgggg aacaaagaac ctccagacta gggtcactgc catcgagaag 8400
   tacttaaagg accaggcgca gctgaatgct tggggatgtg cgtttagaca agtctgccac 8460
   actactgtac catggccaaa tgcaagtcta acaccaaagt ggaacaatga gacttggcaa 8520
   gagtgggagc gaaaggttga cttcttggaa gaaaatataa cagccctcct agaggaggca 8580
   caaattcaac aagagaagaa catgtatgaa ttacaaaagt tgaatagctg ggatgtgttt 8640
   ggcaattggt ttgaccttgc ttcttggata aagtatatac aatatggagt ttatatagtt 8700
   gtaggagtaa tactgttaag aatagtgatc tatatagtac aaatgctagc taagttaagg 8760
   caggggtata ggccagtgtt ctcttcccca ccctcttatt tccagcagac ccatatccaa 8820
   caggacccgg cactgccaac cagagaaggc aaagaaagag acggtggaga aggcggtggc 8880
   aacagctcct ggccttggca gatagaatat attcatttcc tgatccgcca actgatacgc 8940
   ctcttgactt ggctattcag caactgcaga accttgctat cgagagtata ccagatcctc 9000
   caaccaatac tccagaggct ctctgcgacc ctacagagga ttcgagaagt cctcaggact 9060
   gaactgacct acctacaata tgggtggagc tatttccatg aggcggtcca ggccgtctgg 9120
   agatctgcga cagagactct tgcgggcgcg tggggagact tatgggagac tcttaggaga 9180
   ggtggaagat ggatactcgc aatccccagg aggattagac aagggcttga gctcactctc 9240
   ttgtgaggga cagaaataca atcagggaca gtatatgaat actccatgga gaaacccagc 9300
   tgaagagaga gaaaaattag catacagaaa acaaaatatg gatgatatag atgaggaaga 9360
   tgatgacttg gtaggggtat cagtgaggcc aaaagttccc ctaagaacaa tgagttacaa 9420
   attggcaatt gacatgtcac atttcattaa ggagaagggc ggactggagg ggatatacta 9480
   ttctgccagg aggcatagaa tcttagacat atacttagaa aaggaaggag gcatcatacc 9540
   agattggcag gattacacct caggaccagg aattagatac ccaaagacat ttggctggct 9600
   atggaaatta gtccctgtaa atgtatcaga tgaggcacag gaggatgagg agcattattt 9660
   aatgcatcca gctcaaactt cccagtggga tgacccttgg ggagaggttc tagcatggaa 9720
   gtttgatcca actctggcct acacttatga ggcatatgtt agatacccag aagagtttgg 9780
   aagcaagtca ggcctgtcag aggaagaggt tagaagaagg ctaaccgcaa gaggccttct 9840
   taacatggct gacaagaagg aaactcgctg aacgcgtatt gcattactag ttattaatag 9900
   taatcaatta cggggtcatt agttcatagc ccatatatgg agttccgcgt tacataactt 9960
   acggtaaatg gcccgcctgg ctgaccgccc aacgaccccc gcccattgac gtcaataatg 10020
   acgtatgttc ccatagtaac gccaataggg actttccatt gacgtcaatg ggtggagtat 10080
   ttacggtaaa ctgcccactt ggcagtacat caagtgtatc atatgccaag tccgccccct 10140
   attgacgtca atgacggtaa atggcccgcc tggcattatg cccagtacat gaccttacgg 10200
   gactttccta cttggcagta catctacgta ttagtcatcg ctattaccat ggtgatgcgg 10260
   ttttggcagt acaccaatgg gcgtggatag cggtttgact cacggggatt tccaagtctc 10320
   caccccattg acgtcaatgg gagtttgttt tggcaccaaa atcaacggga ctttccaaaa 10380
   tgtcgtaata accccgcccc gttgacgcaa atgggcggta ggcgtgtacg gtgggaggtc 10440
   tatataagca gagctcgttt agtgaaccgt cagaattgtt tttattttta attttctttc 10500
   aaatacttcc atcgaattca gatctggtac cacgcgtacc atggtgccca agaagaagag 10560
   gaaagtctcc aacctgctga ctgtgcacca aaacctgcct gccctccctg tggatgccac 10620
   ctctgatgaa gtcaggaaga acctgatgga catgttcagg gacaggcagg ccttctctga 10680
   acacacctgg aagatgctcc tgtctgtgtg cagatcctgg gctgcctggt gcaagctgaa 10740
   caacaggaaa tggttccctg ctgaacctga ggatgtgagg gactacctcc tgtacctgca 10800
   agccagaggc ctggctgtga agaccatcca acagcacctg ggccagctca acatgctgca 10860
   caggagatct ggcctgcctc gcccttctga ctccaatgct gtgtccctgg tgatgaggag 10920
   aatcagaaag gagaatgtgg atgctgggga gagagccaag caggccctgg cctttgaacg 10980
   cactgacttt gaccaagtca gatccctgat ggagaactct gacagatgcc aggacatcag 11040
   gaacctggcc ttcctgggca ttgcctacaa caccctgctg cgcattgccg aaattgccag 11100
   aatcagagtg aaggacatct cccgcaccga tggtgggaga atgctgatcc acattggcag 11160
   gaccaagacc ctggtgtcca cagctggtgt ggagaaggcc ctgtccctgg gggttaccaa 11220
   gctggtggag agatggatct ctgtgtctgg tgtggctgat gaccccaaca actacctgtt 11280
   ctgccgggtc agaaagaatg gtgtggctgc cccttctgcc acctcccaac tgtccacccg 11340
   ggccctggaa gggatctttg aggccaccca ccgcctgatc tatggtgcca aggatgactc 11400
   tgggcagaga tacctggcct ggtctggcca ctctgccaga gtgggtgctg ccagggacat 11460
   ggccagggct ggtgtgtcca tccctgaaat catgcaggct ggtggctgga ccaatgtgaa 11520
   catagtgatg aactacatca gaaacctgga ctctgagact ggggccatgg tgaggctgct 11580
   cgaggatggg gacggtggtg gtggcaagct ttgacgcatc ccgggtctca ttttataaaa 11640
   gaaaaggggg gactggaagg gatttatccg cggttcactc gagactcgct gaaacagcag 11700
   ggactttcca caaggggatg ttacggggag gtactgggga ggagccggtc gggaacgccc 11760
   actttcttga tgtataaata tcactgcatt tcgctctgta ttcagtcgct ctgcggagag 11820
   gctggcagat tgagccctgg gaggttctct ccagcactag caggtagagc ctgggtgttc 11880
   cctgctagac tctcaccagc acttggccgg tgctgggcag agtgactcca cgcttgcttg 11940
   cttaaagccc tcttcaataa agctgccatt ttagaagtaa gctagtgtgt gttcccatct 12000
   ctcctagccg ccgcctggtc aactcggtac tcaataataa gaagaccctg gtctgttagg 12060
   accctttctg ctttgggaaa ccgaagcagg aaaatcccta gca 12103
<210> 29
   <211> 11151
   <212> DNA
   <213> Artificial Sequence
<220>
   <223> pHR-41ox-RFP/GFP flox
<400> 29
   ttaattccgt gtattctata gtgtcaccta aatcgtatgt gtatgataca taaggttatg 60
   tattaattgt agccgcgttc taacgacaat atgtacaagc ctaattgtgt agcatctggc 120
   ttactgaagc agaccctatc atctctctcg taaactgccg tcagagtcgg tttggttgga 180
   cgaaccttct gagtttctgg taacgccgtc ccgcacccgg aaatggtcag cgaaccaatc 240
   agcagggtca tcgctagcca gatcctctac gccggacgca tcgtggccgg catcaccggc 300
   gccacaggtg cggttgctgg cgcctatatc gccgacatca ccgatgggga agatcgggct 360
   cgccacttcg ggctcatgag cgcttgtttc ggcgtgggta tggtggcagg ccccgtggcc 420
   gggggactgt tgggcgccat ctccttgcat gcaccattcc ttgcggcggc ggtgctcaac 480
   ggcctcaacc tactactggg ctgcttccta atgcaggagt cgcataaggg agagcgtcga 540
   atggtgcact ctcagtacaa tctgctctga tgccgcatag ttaagccagc cccgacaccc 600
   gccaacaccc gctgacgcgc cctgacgggc ttgtctgctc ccggcatccg cttacagaca 660
   agctgtgacc gtctccggga gctgcatgtg tcagaggttt tcaccgtcat caccgaaacg 720
   cgcgagacga aagggcctcg tgatacgcct atttttatag gttaatgtca tgataataat 780
   ggtttcttag acgtcaggtg gcacttttcg gggaaatgtg cgcggaaccc ctatttgttt 840
   atttttctaa atacattcaa atatgtatcc gctcatgaga caataaccct gataaatgct 900
   tcaataatat tgaaaaagga agagtatgag tattcaacat ttccgtgtcg cccttattcc 960
   cttttttgcg gcattttgcc ttcctgtttt tgctcaccca gaaacgctgg tgaaagtaaa 1020
   agatgctgaa gatcagttgg gtgcacgagt gggttacatc gaactggatc tcaacagcgg 1080
   taagatcctt gagagttttc gccccgaaga acgttttcca atgatgagca cttttaaagt 1140
   tctgctatgt ggcgcggtat tatcccgtat tgacgccggg caagagcaac tcggtcgccg 1200
   catacactat tctcagaatg acttggttga gtactcacca gtcacagaaa agcatcttac 1260
   ggatggcatg acagtaagag aattatgcag tgctgccata accatgagtg ataacactgc 1320
   ggccaactta cttctgacaa cgatcggagg accgaaggag ctaaccgctt ttttgcacaa 1380
   catgggggat catgtaactc gccttgatcg ttgggaaccg gagctgaatg aagccatacc 1440
   aaacgacgag cgtgacacca cgatgcctgt agcaatggca acaacgttgc gcaaactatt 1500
   aactggcgaa ctacttactc tagcttcccg gcaacaatta atagactgga tggaggcgga 1560
   taaagttgca ggaccacttc tgcgctcggc ccttccggct ggctggttta ttgctgataa 1620
   atctggagcc ggtgagcgtg ggtctcgcgg tatcattgca gcactggggc cagatggtaa 1680
   gccctcccgt atcgtagtta tctacacgac ggggagtcag gcaactatgg atgaacgaaa 1740
   tagacagatc gctgagatag gtgcctcact gattaagcat tggtaactgt cagaccaagt 1800
   ttactcatat atactttaga ttgatttaaa acttcatttt taatttaaaa ggatctaggt 1860
   gaagatcctt tttgataatc tcatgaccaa aatcccttaa cgtgagtttt cgttccactg 1920
   agcgtcagac cccgtagaaa agatcaaagg atcttcttga gatccttttt ttctgcgcgt 1980
   aatctgctgc ttgcaaacaa aaaaaccacc gctaccagcg gtggtttgtt tgccggatca 2040
   agagctacca actctttttc cgaaggtaac tggcttcagc agagcgcaga taccaaatac 2100
   tgtccttcta gtgtagccgt agttaggcca ccacttcaag aactctgtag caccgcctac 2160
   atacctcgct ctgctaatcc tgttaccagt ggctgctgcc agtggcgata agtcgtgtct 2220
   taccgggttg gactcaagac gatagttacc ggataaggcg cagcggtcgg gctgaacggg 2280
   gggttcgtgc acacagccca gcttggagcg aacgacctac accgaactga gatacctaca 2340
   gcgtgagcat tgagaaagcg ccacgcttcc cgaagggaga aaggcggaca ggtatccggt 2400
   aagcggcagg gtcggaacag gagagcgcac gagggagctt ccagggggaa acgcctggta 2460
   tctttatagt cctgtcgggt ttcgccacct ctgacttgag cgtcgatttt tgtgatgctc 2520
   gtcagggggg cggagcctat ggaaaaacgc cagcaacgcg gcctttttac ggttcctggc 2580
   cttttgctgg ccttttgctc acatgttctt tcctgcgtta tcccctgatt ctgtggataa 2640
   ccgtattacc gcctttgagt gagctgatac cgctcgccgc agccgaacga ccgagcgcag 2700
   cgagtcagtg agcgaggaag cggaagagcg cccaatacgc aaaccgcctc tccccgcgcg 2760
   ttggccgatt cattaatgca gctgtggaat gtgtgtcagt tagggtgtgg aaagtcccca 2820
   ggctccccag caggcagaag tatgcaaagc atgcatctca attagtcagc aaccaggtgt 2880
   ggaaagtccc caggctcccc agcaggcaga agtatgcaaa gcatgcatct caattagtca 2940
   gcaaccatag tcccgcccct aactccgccc atcccgcccc taactccgcc cagttccgcc 3000
   cattctccgc cccatggctg actaattttt tttatttatg cagaggccga ggccgcctcg 3060
   gcctctgagc tattccagaa gtagtgagga ggcttttttg gaggcctagg cttttgcaaa 3120
   aagcttggac acaagacagg cttgcgagat atgtttgaga ataccacttt atcccgcgtc 3180
   agggagaggc agtgcgtaaa aagacgcgga ctcatgtgaa atactggttt ttagtgcgcc 3240
   agatctctat aatctcgcgc aacctatttt cccctcgaac actttttaag ccgtagataa 3300
   acaggctggg acacttcaca tgagcgaaaa atacatcgtc acctgggaca tgttgcagat 3360
   ccatgcacgt aaactcgcaa gccgactgat gccttctgaa caatggaaag gcattattgc 3420
   cgtaagccgt ggcggtctgt accgggtgcg ttactggcgc gtgaactggg tattcgtcat 3480
   gtcgataccg tttgtatttc cagctacgat cacgacaacc agcgcgagct taaagtgctg 3540
   aaacgcgcag aaggcgatgg cgaaggcttc atcgttattg atgacctggt ggataccggt 3600
   ggtactgcgg ttgcgattcg tgaaatgtat ccaaaagcgc actttgtcac catcttcgca 3660
   aaaccggctg gtcgtccgct ggttgatgac tatgttgttg atatcccgca agatacctgg 3720
   attgaacagc cgtgggatat gggcgtcgta ttcgtcccgc caatctccgg tcgctaatct 3780
   tttcaacgcc tggcactgcc gggcgttgtt ctttttaact tcaggcgggt tacaatagtt 3840
   tccagtaagt attctggagg ctgcatccat gacacaggca aacctgagcg aaaccctgtt 3900
   caaaccccgc tttaaacatc ctgaaacctc gacgctagtc cgccgcttta atcacggcgc 3960
   acaaccgcct gtgcagtcgg cccttgatgg taaaaccatc cctcactggt atcgcatgat 4020
   taaccgtctg atgtggatct ggcgcggcat tgacccacgc gaaatcctcg acgtccaggc 4080
   acgtattgtg atgagcgatg ccgaacgtac cgacgatgat ttatacgata cggtgattgg 4140
   ctaccgtggc ggcaactgga tttatgagtg ggccccggat ctttgtgaag gaaccttact 4200
   tctgtggtgt gacataattg gacaaactac ctacagagat ttaaagctct aaggtaaata 4260
   taaaattttt aagtgtataa tgtgttaaac tactgattct aattgtttgt gtattttaga 4320
   ttccaaccta tggaactgat gaatgggagc agtggtggaa tgcctttaat gaggaaaacc 4380
   tgttttgctc agaagaaatg ccatctagtg atgatgaggc tactgctgac tctcaacatt 4440
   ctactcctcc aaaaaagaag agaaaggtag aagaccccaa ggactttcct tcagaattgc 4500
   taagtttttt gagtcatgct gtgtttagta atagaactct tgcttgcttt gctatttaca 4560
   ccacaaagga aaaagctgca ctgctataca agaaaattat ggaaaaatat tctgtaacct 4620
   ttataagtag gcataacagt tataatcata acatactgtt ttttcttact ccacacaggc 4680
   atagagtgtc tgctattaat aactatgctc aaaaattgtg tacctttagc tttttaattt 4740
   gtaaaggggt taataaggaa tatttgatgt atagtgcctt gactagagat cataatcagc 4800
   cataccacat ttgtagaggt tttacttgct ttaaaaaacc tcccacacct ccccctgaac 4860
   ctgaaacata aaatgaatgc aattgttgtt gttaacttgt ttattgcagc ttataatggt 4920
   tacaaataaa gcaatagcat cacaaatttc acaaataaag catttttttc actgcattct 4980
   agttgtggtt tgtccaaact catcaatgta tcttatcatg tctggatcaa ctggataact 5040
   caagctaacc aaaatcatcc caaacttccc accccatacc ctattaccac tgccaattac 5100
   ctagtggttt catttactct aaacctgtga ttcctctgaa ttattttcat tttaaagaaa 5160
   ttgtatttgt taaatatgta ctacaaactt agtagttgga agggctaatt cactcccaaa 5220
   gaagacaaga tatccttgat ctgtggatct accacacaca aggctacttc cctgattagc 5280
   agaactacac accagggcca ggggtcagat atccactgac ctttggatgg tgctacaagc 5340
   tagtaccagt tgagccagat aaggtagaag aggccaataa aggagagaac accagcttgt 5400
   tacaccctgt gagcctgcat gggatggatg acccggagag agaagtgtta gagtggaggt 5460
   ttgacagccg cctagcattt catcacgtgg cccgagagct gcatccggag tacttcaaga 5520
   actgctgata tcgagcttgc tacaagggac tttccgctgg ggactttcca gggaggcgtg 5580
   gcctgggcgg gactggggag tggcgagccc tcagatcctg catataagca gctgcttttt 5640
   gcctgtactg ggtctctctg gttagaccag atctgagcct gggagctctc tggctaacta 5700
   gggaacccac tgcttaagcc tcaataaagc ttgccttgag tgcttcaagt agtgtgtgcc 5760
   cgtctgttgt gtgactctgg taactagaga tccctcagac ccttttagtc agtgtggaaa 5820
   atctctagca gtggcgcccg aacagggact tgaaagcgaa agggaaacca gaggagctct 5880
   ctcgacgcag gactcggctt gctgaagcgc gcacggcaag aggcgagggg cggcgactgg 5940
   tgagtacgcc aaaaattttg actagcggag gctagaagga gagagatggg tgcgagagcg 6000
   tcagtattaa gcgggggaga attagatcgc gatgggaaaa aattcggtta aggccagggg 6060
   gaaagaaaaa atataaatta aaacatatag tatgggcaag cagggagcta gaacgattcg 6120
   cagttaatcc tggcctgtta gaaacatcag aaggctgtag acaaatactg ggacagctac 6180
   aaccatccct tcagacagga tcagaagaac ttagatcatt atataataca gtagcaaccc 6240
   tctattgtgt gcatcaaagg atagagataa aagacaccaa ggaagcttta gacaagatag 6300
   aggaagagca aaacaaaagt aagaccaccg cacagcaagc ggccgctgat cttcagacct 6360
   ggaggaggag atatgaggga caattggaga agtgaattat ataaatataa agtagtaaaa 6420
   attgaaccat taggagtagc acccaccaag gcaaagagaa gagtggtgca gagagaaaaa 6480
   agagcagtgg gaataggagc tttgttcctt gggttcttgg gagcagcagg aagcactatg 6540
   ggcgcagcgt caatgacgct gacggtacag gccagacaat tattgtctgg tatagtgcag 6600
   cagcagaaca atttgctgag ggctattgag gcgcaacagc atctgttgca actcacagtc 6660
   tggggcatca agcagctcca ggcaagaatc ctggctgtgg aaagatacct aaaggatcaa 6720
   cagctcctgg ggatttgggg ttgctctgga aaactcattt gcaccactgc tgtgccttgg 6780
   aatgctagtt ggagtaataa atctctggaa cagatttgga atcacacgac ctggatggag 6840
   tgggacagag aaattaacaa ttacacaagc ttaatacact ccttaattga agaatcgcaa 6900
   aaccagcaag aaaagaatga acaagaatta ttggaattag ataaatgggc aagtttgtgg 6960
   aattggttta acataacaaa ttggctgtgg tatataaaat tattcataat gatagtagga 7020
   ggcttggtag gtttaagaat agtttttgct gtactttcta tagtgaatag agttaggcag 7080
   ggatattcac cattatcgtt tcagacccac ctcccaaccc cgaggggacc cgacaggccc 7140
   gaaggaatag aagaagaagg tggagagaga gacagagaca gatccattcg attagtgaac 7200
   ggatctcgac ggtatcgccg aattcacaaa tggcagtatt catccacaat tttaaaagaa 7260
   aaggggggat tggggggtac agtgcagggg aaagaatagt agacataata gcaacagaca 7320
   tacaaactaa agaattacaa aaacaaatta caaaaattca aaattttcgg gtttattaca 7380
   gggacagcag agatccactt tatcgataag cttgggagtt ccgcgttaca taacttacgg 7440
   taaatggccc gcctggctga ccgcccaacg acccccgccc attgacgtca ataatgacgt 7500
   atgttcccat agtaacgcca atagggactt tccattgacg tcaatgggtg gagtatttac 7560
   ggtaaactgc ccacttggca gtacatcaag tgtatcatat gccaagtacg ccccctattg 7620
   acgtcaatga cggtaaatgg cccgcctggc attatgccca gtacatgacc ttacgggact 7680
   ttcctacttg gcagtacatc tacgtattag tcatcgctat taccatggtg atgcggtttt 7740
   ggcagtacac caatgggcgt ggatagcggt ttgactcacg gggatttcca agtctccacc 7800
   ccattgacgt caatgggagt ttgttttggc accaaaatca acgggacttt ccaaaatgtc 7860
   gtaacaactc cgccccattg acgcaaatgg gcggtaggcg tgtacggtgg gaggtctata 7920
   taagcagagc tcgtttagtg aaccgtcaga tcgcctggag acgccatcca cgctgttttg 7980
   acctccatag aagacaccga ctctagctag aggatccgga ctagtaactc gaggatgggg 8040
   actgacccgg gctgcaggaa ttcataactt cgtataatgt atgctatacg aagttatcat 8100
   atgccaccat gagcgagctg atcaaggaga acatgcacat gaagctgtac atggagggca 8160
   ccgtgaacaa ccaccacttc aagtgcacat ccgagggcga aggcaagccc tacgagggca 8220
   cccagaccat gaagatcaag gtggtcgagg gcggccctct ccccttcgcc ttcgacatcc 8280
   tggctaccag cttcatgtac ggcagcaaag ccttcatcaa ccacacccag ggcatccccg 8340
   acttctttaa gcagtccttc cctgagggct tcacatggga gagaatcacc acatacgaag 8400
   acgggggcgt gctgaccgct acccaggaca ccagcttcca gaacggctgc atcatctaca 8460
   acgtcaagat caacggggtg aacttcccat ccaacggccc tgtgatgcag aagaaaacac 8520
   gcggctggga ggccaacacc gagatgctgt accccgctga cggcggcctg agaggccaca 8580
   gccagatggc cctgaagctc gtgggcgggg gctacctgca ctgctccttc aagaccacat 8640
   acagatccaa gaaacccgct aagaacctca agatgcccgg cttccacttc gtggaccaca 8700
   gactggaaag aatcaaggag gccgacaaag agacctacgt cgagcagcac gagatggctg 8760
   tggccaagta ctgcgacctc cctagcaaac tggggcacag ataaataact tcgtataaag 8820
   tatcctatac gaagttatac tttggccgcg gctcgagggg gttggggttg cgccttttcc 8880
   aaggcagccc tgggtttgcg cagggacgcg gctgctctgg gcgtggttcc gggaaacgca 8940
   gcggcgccga ccctgggtct cgcacattct tcacgtccgt tcgcagcgtc acccggatct 9000
   tcgccgctac ccttgtgggc cccccggcga cgcttcctgc tccgccccta agtcgggaag 9060
   gttccttgcg gttcgcggcg tgccggacgt gacaaacgga agccgcacgt ctcactagta 9120
   ccctcgcaga cggacagcgc cagggagcaa tggcagcgcg ccgaccgcga tgggctgtgg 9180
   ccaatagcgg ctgctcagca gggcgcgccg agagcagcgg ccgggaaggg gcggtgcggg 9240
   aggcggggtg tggggcggta gtgtgggccc tgttcctgcc cgcgcggtgt tccgcattct 9300
   gcaagcctcc ggagcgcacg tcggcagtcg gctccctcgt tgaccgaatc accgacctct 9360
   ctccccaggg ggatccaccg gtcgccacca tggtgagcaa gggcgaggag ctgttcaccg 9420
   gggtggtgcc catcctggtc gagctggacg gcgacgtaaa cggccacaag ttcagcgtgt 9480
   ccggcgaggg cgagggcgat gccacctacg gcaagctgac cctgaagttc atctgcacca 9540
   ccggcaagct gcccgtgccc tggcccaccc tcgtgaccac cctgacctac ggcgtgcagt 9600
   gcttcagccg ctaccccgac cacatgaagc agcacgactt cttcaagtcc gccatgcccg 9660
   aaggctacgt ccaggagcgc accatcttct tcaaggacga cggcaactac aagacccgcg 9720
   ccgaggtgaa gttcgagggc gacaccctgg tgaaccgcat cgagctgaag ggcatcgact 9780
   tcaaggagga cggcaacatc ctggggcaca agctggagta caactacaac agccacaacg 9840
   tctatatcat ggccgacaag cagaagaacg gcatcaaggt gaacttcaag atccgccaca 9900
   acatcgagga cggcagcgtg cagctcgccg accactacca gcagaacacc cccatcggcg 9960
   acggccccgt gctgctgccc gacaaccact acctgagcac ccagtccgcc ctgagcaaag 10020
   accccaacga gaagcgcgat cacatggtcc tgctggagtt cgtgaccgcc gccgggatca 10080
   ctctcggcat ggacgagctg tacaagtaag atatcaagct tatcgataat caacctctgg 10140
   attacaaaat ttgtgaaaga ttgactggta ttcttaacta tgttgctcct tttacgctat 10200
   gtggatacgc tgctttaatg cctttgtatc atgctattgc ttcccgtatg gctttcattt 10260
   tctcctcctt gtataaatcc tggttgctgt ctctttatga ggagttgtgg cccgttgtca 10320
   ggcaacgtgg cgtggtgtgc actgtgtttg ctgacgcaac ccccactggt tggggcattg 10380
   ccaccacctg tcagctcctt tccgggactt tcgctttccc cctccctatt gccacggcgg 10440
   aactcatcgc cgcctgcctt gcccgctgct ggacaggggc tcggctgttg ggcactgaca 10500
   attccgtggt gttgtcgggg aaatcatcgt cctttccttg gctgctcgcc tgtgttgcca 10560
   cctggattct gcgcgggacg tccttctgct acgtcccttc ggccctcaat ccagcggacc 10620
   ttccttcccg cggcctgctg ccggctctgc ggcctcttcc gcgtcttcgc cttcgccctc 10680
   agacgagtcg gatctccctt tgggccgcct ccccgcatcg ataccgtcga cctcgagaac 10740
   ctagaaaaac atggagcaat cacaagtagc aatacagcag ctaccaatgc tgattgtgcc 10800
   tggctagaag cacaagagga ggaggaggtg ggttttccag tcacacctca ggtaccttta 10860
   agaccaatga cttacaaggc agctgtagat cttagccact ttttaaaaga aaagggggga 10920
   ctggaagggc taattcactc ccaacgaaga caagatcttt ttgcttgtac tgggtctctc 10980
   tggttagacc agatctgagc ctgggagctc tctggctaac tagggaaccc actgcttaag 11040
   cctcaataaa gcttgccttg agtgcttcaa gtagtgtgtg cccgtctgtt gtgtgactct 11100
   ggtaactaga gatccctcag acccttttag tcagtgtgga aaatctctag a 11151
<210> 30
   <211> 9118
   <212> DNA
   <213> Artificial Sequence
<220>
   <223> pSDT-41ox-RFP/GFP-WPRE-flox
<400> 30
   ggaaattgta aacgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcg cgccattcgc cattcaggct 480
   gcgcaactgt tgggaagggc gatcggtgcg ggcctcttcg ctattacgcc agctggcgaa 540
   agggggatgt gctgcaaggc gattaagttg ggtaacgcca gggttttccc agtcacgacg 600
   ttgtaaaacg acggccagtg aattgtaata cgactcacta tagggcgaat tggagctcca 660
   ccgcggtggc ggccgctcta gaattcccat tgcatacgtt gtatccatat cataatatgt 720
   acatttatat tggctcatgt ccaacattac cgccatgttg acattgatta ttgactagtt 780
   attaatagta atcaattacg gggtcattag ttcatagccc atatatggag ttccgcgtta 840
   cataacttac ggtaaatggc ccgcctggct gaccgcccaa cgacccccgc ccattgacgt 900
   caataatgac gtatgttccc atagtaacgc caatagggac tttccattga cgtcaatggg 960
   tggagtattt acggtaaact gcccacttgg cagtacatca agtgtatcat atgccaagta 1020
   cgccccctat tgacgtcaat gacggtaaat ggcccgcctg gcattatgcc cagtacatga 1080
   ccttatggga ctttcctact tggcagtaca tctacgtatt agtcatcgct attaccatgg 1140
   tgatgcggtt ttggcagtac atcaatgggc gtggatagcg gtttgactca cggggatttc 1200
   caagtctcca ccccattgac gtcaatggga gtttgttttg gcaccaaaat caacgggact 1260
   ttccaaaatg tcgtaacaac tccgccccat tgacgcaaat gggcggtagg cgtgtacggt 1320
   gggaggtcta tataagcaga gctcgtttag tgaaccgcag tcgctctgcg gagaggctgg 1380
   cagattgagc cctgggaggt tctctccagc actagcaggt agagcctggg tgttccctgc 1440
   tagactctca ccagcacttg gccagtgctg ggcagagtgg ctccacgctt gcttgcttaa 1500
   agacctcttc aataaagctg ccattttaga agtaagccag tgtgtgttcc catctctcct 1560
   agtcgccgcc tggtcaactc ggtactcggt aataagaaga ccctggtctg ttaggaccct 1620
   ttctgctttg agaaaccgaa gcaggaaaat ccctagcaga ttggcgcccg aacagggact 1680
   tgaaggagag tgagagactc ctgagtacgg ctgagtgaag gcagtaaggg cggcaggaac 1740
   caaccacgac ggagtgctcc tataaaggcg cgggtcggta ccagacggcg tgaggagcgg 1800
   gagaggagga ggcctccggt tgcagtaagt gcaacacaaa aaagaaatag ctgtcttgtt 1860
   atccaggaag ggataataag atagagtggg agatgggcgc gagaaactcc gtcttgtcag 1920
   ggaagaaagc agatgaattg aaaaaattat taatcgcatg aattttaaaa gaaggggagg 1980
   aataggggat atgactccag cagaaagatt aattaacatg atcactacag aacaagaaat 2040
   acaatttcaa caatcaaaaa actcaaaatt taaaaatttt cgggtctatt acagagctca 2100
   cgcgtgattg gagttgggag attataaatt agtagagatc actccgattg gcttggcccc 2160
   cacagatgtg aagaggtaca ctactggtgg cacctcaaga aataaaagag gggtctttgt 2220
   gctagggttc ttgggttttc tcgcaacggc aggttctgca atgggcgcgg cgtcgttgac 2280
   gctgaccgct cagtcccgga ctttattggc tgggatagtg cagcaacagc aacagctgtt 2340
   ggacgtggtc aagagacaac aagaattgtt gcgactgacc gtctggggaa caaagaacct 2400
   ccagactagg gtcactgcca tcgagaagta cttaaaggac caggcgcagc taaatgcttg 2460
   gggatgtgcg tttagacaag tctgccacac tactgtacca tggccaaatg caagtctaac 2520
   accagactgg aacaatgata cttggcaaga gtgggagcga aaggttgact tcttggagga 2580
   aaatataaca gccctcctag aagaggcaca aattcaacaa gagaagaaca tgtatgaatt 2640
   acaaaagttg aatagctggg atgtgtttgg caattggttt gaccttgctt cttggataaa 2700
   gtatatacaa tatggaattt atgtagttgt aggagtaata ctgttaagaa tagtgatcta 2760
   tatagtacaa atgctagcta agttaaggca ggggtatagg ccagtgttct cttccccacc 2820
   ctcttatttc cagtagactc atacccaaca ggacccggca ctgccaacca gagaaggcaa 2880
   agaaggagac ggtggagaag gcggtggatc ctattatcga attcctgcag ccccgataaa 2940
   ataaaagatt ttatttagtc tccagaaaaa ggggggaatg aaagacccca cctgtaggtt 3000
   tggcaagcta gctgcagtaa cgccattttg caaggcatgg aaaaatacca aaccaagaat 3060
   agagaagttc agatcaaggg cgggtacatg aaaatagcta acgttgggcc aaacaggata 3120
   tctgcggtga gcagtttcgg ccccggcccg gggccaagaa cagatggtca ccgcagtttc 3180
   ggccccggcc cgaggccaag aacagatggt ccccagatat ggcccaaccc tcagcagttt 3240
   cttaagaccc atcagatgtt tccaggctcc cccaaggacc tgaaatgacc ctgcgcctta 3300
   tttgaattaa ccaatcagcc tgcttctcgc ttctgttcgc gcgcttctgc ttcccgagct 3360
   ctataaaaga gctcacaacc cctcactcgg cgcgccagtc ctccgacaga ctgagtcgcc 3420
   cgggggggat ccaccgggct gcaggaattc gatatcaagc ttatcgatga ttctagacat 3480
   gcatatctag cgtcccgggc tgcaggaatt cataacttcg tataatgtat gctatacgaa 3540
   gttatcatat gccaccatga gcgagctgat caaggagaac atgcacatga agctgtacat 3600
   ggagggcacc gtgaacaacc accacttcaa gtgcacatcc gagggcgaag gcaagcccta 3660
   cgagggcacc cagaccatga agatcaaggt ggtcgagggc ggccctctcc ccttcgcctt 3720
   cgacatcctg gctaccagct tcatgtacgg cagcaaagcc ttcatcaacc acacccaggg 3780
   catccccgac ttctttaagc agtccttccc tgagggcttc acatgggaga gaatcaccac 3840
   atacgaagac gggggcgtgc tgaccgctac ccaggacacc agcttccaga acggctgcat 3900
   catctacaac gtcaagatca acggggtgaa cttcccatcc aacggccctg tgatgcagaa 3960
   gaaaacacgc ggctgggagg ccaacaccga gatgctgtac cccgctgacg gcggcctgag 4020
   aggccacagc cagatggccc tgaagctcgt gggcgggggc tacctgcact gctccttcaa 4080
   gaccacatac agatccaaga aacccgctaa gaacctcaag atgcccggct tccacttcgt 4140
   ggaccacaga ctggaaagaa tcaaggaggc cgacaaagag acctacgtcg agcagcacga 4200
   gatggctgtg gccaagtact gcgacctccc tagcaaactg gggcacagat aaataacttc 4260
   gtataaagta tcctatacga agttatactt tggccgcggc tcgagggggt tggggttgcg 4320
   ccttttccaa ggcagccctg ggtttgcgca gggacgcggc tgctctgggc gtggttccgg 4380
   gaaacgcagc ggcgccgacc ctgggtctcg cacattcttc acgtccgttc gcagcgtcac 4440
   ccggatcttc gccgctaccc ttgtgggccc cccggcgacg cttcctgctc cgcccctaag 4500
   tcgggaaggt tccttgcggt tcgcggcgtg ccggacgtga caaacggaag ccgcacgtct 4560
   cactagtacc ctcgcagacg gacagcgcca gggagcaatg gcagcgcgcc gaccgcgatg 4620
   ggctgtggcc aatagcggct gctcagcagg gcgcgccgag agcagcggcc gggaaggggc 4680
   ggtgcgggag gcggggtgtg gggcggtagt gtgggccctg ttcctgcccg cgcggtgttc 4740
   cgcattctgc aagcctccgg agcgcacgtc ggcagtcggc tccctcgttg accgaatcac 4800
   cgacctctct ccccaggggg atccaccggt cgccaccatg gtgagcaagg gcgaggagct 4860
   gttcaccggg gtggtgccca tcctggtcga gctggacggc gacgtaaacg gccacaagtt 4920
   cagcgtgtcc ggcgagggcg agggcgatgc cacctacggc aagctgaccc tgaagttcat 4980
   ctgcaccacc ggcaagctgc ccgtgccctg gcccaccctc gtgaccaccc tgacctacgg 5040
   cgtgcagtgc ttcagccgct accccgacca catgaagcag cacgacttct tcaagtccgc 5100
   catgcccgaa ggctacgtcc aggagcgcac catcttcttc aaggacgacg gcaactacaa 5160
   gacccgcgcc gaggtgaagt tcgagggcga caccctggtg aaccgcatcg agctgaaggg 5220
   catcgacttc aaggaggacg gcaacatcct ggggcacaag ctggagtaca actacaacag 5280
   ccacaacgtc tatatcatgg ccgacaagca gaagaacggc atcaaggtga acttcaagat 5340
   ccgccacaac atcgaggacg gcagcgtgca gctcgccgac cactaccagc agaacacccc 5400
   catcggcgac ggccccgtgc tgctgcccga caaccactac ctgagcaccc agtccgccct 5460
   gagcaaagac cccaacgaga agcgcgatca catggtcctg ctggagttcg tgaccgccgc 5520
   cgggatcact ctcggcatgg acgagctgta caagtaaagc ggccgcgact ctagagtcga 5580
   cctgcaggca tgcaagcttg atatcaagct tatcgataat caacctctgg attacaaaat 5640
   ttgtgaaaga ttgactggta ttcttaacta tgttgctcct tttacgctat gtggatacgc 5700
   tgctttaatg cctttgtatc atgctattgc ttcccgtatg gctttcattt tctcctcctt 5760
   gtataaatcc tggttgctgt ctctttatga ggagttgtgg cccgttgtca ggcaacgtgg 5820
   cgtggtgtgc actgtgtttg ctgacgcaac ccccactggt tggggcattg ccaccacctg 5880
   tcagctcctt tccgggactt tcgctttccc cctccctatt gccacggcgg aactcatcgc 5940
   cgcctgcctt gcccgctgct ggacaggggc tcggctgttg ggcactgaca attccgtggt 6000
   gttgtcgggg aaatcatcgt cctttccttg gctgctcgcc tgtgttgcca cctggattct 6060
   gcgcgggacg tccttctgct acgtcccttc ggccctcaat ccagcggacc ttccttcccg 6120
   cggcctgctg ccggctctgc ggcctcttcc gcgtcttcgc cttcgccctc agacgagtcg 6180
   gatctccctt tgggccgcct ccccgcatcg ataccgtcga cctcgagttt tataaaagaa 6240
   aaggggggac tggaagggat ttattacagt gcaagaagac atagaatctt agacatgtac 6300
   ttagaaaagg aagaaggcat cataccagat tggcaggatt acacctcagg accaggaatt 6360
   agatacccaa agacatttgg ctggctatgg aaattagtcc ctgtaaatgt atcagatgag 6420
   gcacaggagg atgagaggca ttatttaatg cagccagctc aaacttccaa gtgggatgac 6480
   ccttggggag aggttctagc gtggaagttt gatccaactc tagcctacac ttatgaggca 6540
   tatgctagat acccagaaga gttggaagca agtcaggcct gtcagaactg catttcgctc 6600
   tgtattcagt cgctctgcgg agaggctggc agattgagcc ctgggaggtt ctctccagca 6660
   ctagcaggta gagcctgggt gttccctgct agactctcac cagcacttgg ccagtgctgg 6720
   gcagagtggc tccacgcttg cttgcttaaa gacctcttca ataaagctgc cattttagaa 6780
   gtaagccagt gtgtgttccc atctctccta gtcgccgcct ggtcaactcg gtactcggta 6840
   ataagaagac cctggtctgt taggaccctt tctgctttga gaaaccgaag caggaaaatc 6900
   cctagcatgg tacccagctt ttgttccctt tagtgagggt taattccgag cttggcgtaa 6960
   tcatggtcat agctgtttcc tgtgtgaaat tgttatccgc tcacaattcc acacaacata 7020
   cgagccggaa gcataaagtg taaagcctgg ggtgcctaat gagtgagcta actcacatta 7080
   attgcgttgc gctcactgcc cgctttccag tcgggaaacc tgtcgtgcca gctgcattaa 7140
   tgaatcggcc aacgcgcggg gagaggcggt ttgcgtattg ggcgctcttc cgcttcctcg 7200
   ctcactgact cgctgcgctc ggtcgttcgg ctgcggcgag cggtatcagc tcactcaaag 7260
   gcggtaatac ggttatccac agaatcaggg gataacgcag gaaagaacat gtgagcaaaa 7320
   ggccagcaaa aggccaggaa ccgtaaaaag gccgcgttgc tggcgttttt ccataggctc 7380
   cgcccccctg acgagcatca caaaaatcga cgctcaagtc agaggtggcg aaacccgaca 7440
   ggactataaa gataccaggc gtttccccct ggaagctccc tcgtgcgctc tcctgttccg 7500
   accctgccgc ttaccggata cctgtccgcc tttctccctt cgggaagcgt ggcgctttct 7560
   catagctcac gctgtaggta tctcagttcg gtgtaggtcg ttcgctccaa gctgggctgt 7620
   gtgcacgaac cccccgttca gcccgaccgc tgcgccttat ccggtaacta tcgtcttgag 7680
   tccaacccgg taagacacga cttatcgcca ctggcagcag ccactggtaa caggattagc 7740
   agagcgaggt atgtaggcgg tgctacagag ttcttgaagt ggtggcctaa ctacggctac 7800
   actagaagga cagtatttgg tatctgcgct ctgctgaagc cagttacctt cggaaaaaga 7860
   gttggtagct cttgatccgg caaacaaacc accgctggta gcggtggttt ttttgtttgc 7920
   aagcagcaga ttacgcgcag aaaaaaagga tctcaagaag atcctttgat cttttctacg 7980
   gggtctgacg ctcagtggaa cgaaaactca cgttaaggga ttttggtcat gagattatca 8040
   aaaaggatct tcacctagat ccttttaaat taaaaatgaa gttttaaatc aatctaaagt 8100
   atatatgagt aaacttggtc tgacagttac caatgcttaa tcagtgaggc acctatctca 8160
   gcgatctgtc tatttcgttc atccatagtt gcctgactcc ccgtcgtgta gataactacg 8220
   atacgggagg gcttaccatc tggccccagt gctgcaatga taccgcgaga cccacgctca 8280
   ccggctccag atttatcagc aataaaccag ccagccggaa gggccgagcg cagaagtggt 8340
   cctgcaactt tatccgcctc catccagtct attaattgtt gccgggaagc tagagtaagt 8400
   agttcgccag ttaatagttt gcgcaacgtt gttgccattg ctacaggcat cgtggtgtca 8460
   cgctcgtcgt ttggtatggc ttcattcagc tccggttccc aacgatcaag gcgagttaca 8520
   tgatccccca tgttgtgcaa aaaagcggtt agctccttcg gtcctccgat cgttgtcaga 8580
   agtaagttgg ccgcagtgtt atcactcatg gttatggcag cactgcataa ttctcttact 8640
   gtcatgccat ccgtaagatg cttttctgtg actggtgagt actcaaccaa gtcattctga 8700
   gaatagtgta tgcggcgacc gagttgctct tgcccggcgt caatacggga taataccgcg 8760
   ccacatagca gaactttaaa agtgctcatc attggaaaac gttcttcggg gcgaaaactc 8820
   tcaaggatct taccgctgtt gagatccagt tcgatgtaac ccactcgtgc acccaactga 8880
   tcttcagcat cttttacttt caccagcgtt tctgggtgag caaaaacagg aaggcaaaat 8940
   gccgcaaaaa agggaataag ggcgacacgg aaatgttgaa tactcatact cttccttttt 9000
   caatattatt gaagcattta tcagggttat tgtctcatga gcggatacat atttgaatgt 9060
   atttagaaaa ataaacaaat aggggttccg cgcacatttc cccgaaaagt gccacctg 9118
<210> 31
   <211> 1653
   <212> DNA
   <213> Artificial Sequence
<220>
   <223> reverse luciferase
<400> 31
   ttacacggcg atctttccgc ccttcttggc ctttatgagg atctctctga tttttcttgc        60
   gtcgagtttt ccggtaagac ctttcggtac ttcgtccaca aacacaactc ctccgcgcaa       120
   ctttttcgcg gttgttactt gactggcgac gtaatccacg atctcttttt ccgtcatcgt       180
   ctttccgtgc tccaaaacaa caacggcggc gggaagttca ccggcgtcat cgtcgggaag       240
   acctgcgaca cctgcgtcga agatgttggg gtgttggagc aagatggatt ccaattcagc       300
   gggagccacc tgatagcctt tgtacttaat cagagacttc aggcggtcaa cgatgaagaa       360
   gtgttcgtct tcgtcccagt aagctatgtc tccagaatgt agccatccat ccttgtcaat       420
   caaggcgttg gtcgcttccg gattgtttac ataaccggac ataatcatag gacctctcac       480
   acacagttcg cctctttgat taacgcccag cgttttcccg gtatccagat ccacaacctt       540
   cgcttcaaaa aatggaacaa ctttaccgac cgcgcccggt ttatcatccc cctcgggtgt       600
   aatcagaata gctgatgtag tctcagtgag cccatatcct tgcctgatac ctggcagatg       660
   gaacctcttg gcaaccgctt ccccgacttc cttagagagg ggagcgccac cagaagcaat       720
   ttcgtgtaaa ttagataaat cgtatttgtc aatcagagtg cttttggcga agaaggagaa       780
   tagggttggc accagcagcg cactttgaat cttgtaatcc tgaaggctcc tcagaaacag       840
   ctcttcttca aatctataca ttaagacgac tcgaaatcca catatcaaat atccgagtgt       900
   agtaaacatt ccaaaaccgt gatggaatgg aacaacactt aaaatcgcag tatccggaat       960
   gatttgattg ccaaaaatag gatctctggc atgcgagaat ctcacgcagg cagttctatg      1020
   aggcagagcg acacctttag gcagaccagt agatccagag gagttcatga tcagtgcaat      1080
   tgtcttgtcc ctatcgaagg actctggcac aaaatcgtat tcattaaaac cgggaggtag      1140
   atgagatgtg acgaacgtgt acatcgactg aaatccctgg taatccgttt tagaatccat      1200
   gataataatt ttttggatga ttgggagctt tttttgcacg ttcaaaattt tttgcaaccc      1260
   ctttttggaa acgaacacca cggtaggctg cgaaatgccc atactgttga gcaattcacg      1320
   ttcattataa atgtcgttcg cgggcgcaac tgcaactccg ataaataacg cgcccaacac      1380
   cggcataaag aattgaagag agttttcact gcatacgacg attctgtgat ttgtattcag      1440
   cccatatcgt ttcatagctt ctgccaaccg aacggacatt tcgaagtact cagcgtaagt      1500
   gatgtccacc tcgatatgtg catctgtaaa agcaattgtt ccaggaacca gggcgtatct      1560
   cttcatagcc ttatgcagtt gctctccagc ggttccatct tccagcggat agaatggcgc      1620
   cgggcctttc tttatgtttt tggcgtcttc cat                                   1653
<210> 32
   <211> 11336
   <212> DNA
   <213> Artificial Sequence
<220>
   <223> sequence for screening human cells
<400> 32
   ttaattccgt gtattctata gtgtcaccta aatcgtatgt gtatgataca taaggttatg      60
   tattaattgt agccgcgttc taacgacaat atgtacaagc ctaattgtgt agcatctggc     120
   ttactgaagc agaccctatc atctctctcg taaactgccg tcagagtcgg tttggttgga     180
   cgaaccttct gagtttctgg taacgccgtc ccgcacccgg aaatggtcag cgaaccaatc     240
   agcagggtca tcgctagcca gatcctctac gccggacgca tcgtggccgg catcaccggc     300
   gccacaggtg cggttgctgg cgcctatatc gccgacatca ccgatgggga agatcgggct     360
   cgccacttcg ggctcatgag cgcttgtttc ggcgtgggta tggtggcagg ccccgtggcc     420
   gggggactgt tgggcgccat ctccttgcat gcaccattcc ttgcggcggc ggtgctcaac     480
   ggcctcaacc tactactggg ctgcttccta atgcaggagt cgcataaggg agagcgtcga     540
   atggtgcact ctcagtacaa tctgctctga tgccgcatag ttaagccagc cccgacaccc     600
   gccaacaccc gctgacgcgc cctgacgggc ttgtctgctc ccggcatccg cttacagaca     660
   agctgtgacc gtctccggga gctgcatgtg tcagaggttt tcaccgtcat caccgaaacg     720
   cgcgagacga aagggcctcg tgatacgcct atttttatag gttaatgtca tgataataat     780
   ggtttcttag acgtcaggtg gcacttttcg gggaaatgtg cgcggaaccc ctatttgttt     840
   atttttctaa atacattcaa atatgtatcc gctcatgaga caataaccct gataaatgct     900
   tcaataatat tgaaaaagga agagtatgag tattcaacat ttccgtgtcg cccttattcc     960
   cttttttgcg gcattttgcc ttcctgtttt tgctcaccca gaaacgctgg tgaaagtaaa    1020
   agatgctgaa gatcagttgg gtgcacgagt gggttacatc gaactggatc tcaacagcgg    1080
   taagatcctt gagagttttc gccccgaaga acgttttcca atgatgagca cttttaaagt    1140
   tctgctatgt ggcgcggtat tatcccgtat tgacgccggg caagagcaac tcggtcgccg    1200
   catacactat tctcagaatg acttggttga gtactcacca gtcacagaaa agcatcttac    1260
   ggatggcatg acagtaagag aattatgcag tgctgccata accatgagtg ataacactgc    1320
   ggccaactta cttctgacaa cgatcggagg accgaaggag ctaaccgctt ttttgcacaa    1380
   catgggggat catgtaactc gccttgatcg ttgggaaccg gagctgaatg aagccatacc    1440
   aaacgacgag cgtgacacca cgatgcctgt agcaatggca acaacgttgc gcaaactatt    1500
   aactggcgaa ctacttactc tagcttcccg gcaacaatta atagactgga tggaggcgga    1560
   taaagttgca ggaccacttc tgcgctcggc ccttccggct ggctggttta ttgctgataa    1620
   atctggagcc ggtgagcgtg ggtctcgcgg tatcattgca gcactggggc cagatggtaa    1680
   gccctcccgt atcgtagtta tctacacgac ggggagtcag gcaactatgg atgaacgaaa    1740
   tagacagatc gctgagatag gtgcctcact gattaagcat tggtaactgt cagaccaagt    1800
   ttactcatat atactttaga ttgatttaaa acttcatttt taatttaaaa ggatctaggt    1860
   gaagatcctt tttgataatc tcatgaccaa aatcccttaa cgtgagtttt cgttccactg 1920
   agcgtcagac cccgtagaaa agatcaaagg atcttcttga gatccttttt ttctgcgcgt 1980
   aatctgctgc ttgcaaacaa aaaaaccacc gctaccagcg gtggtttgtt tgccggatca 2040
   agagctacca actctttttc cgaaggtaac tggcttcagc agagcgcaga taccaaatac 2100
   tgtccttcta gtgtagccgt agttaggcca ccacttcaag aactctgtag caccgcctac 2160
   atacctcgct ctgctaatcc tgttaccagt ggctgctgcc agtggcgata agtcgtgtct 2220
   taccgggttg gactcaagac gatagttacc ggataaggcg cagcggtcgg gctgaacggg 2280
   gggttcgtgc acacagccca gcttggagcg aacgacctac accgaactga gatacctaca 2340
   gcgtgagcat tgagaaagcg ccacgcttcc cgaagggaga aaggcggaca ggtatccggt 2400
   aagcggcagg gtcggaacag gagagcgcac gagggagctt ccagggggaa acgcctggta 2460
   tctttatagt cctgtcgggt ttcgccacct ctgacttgag cgtcgatttt tgtgatgctc 2520
   gtcagggggg cggagcctat ggaaaaacgc cagcaacgcg gcctttttac ggttcctggc 2580
   cttttgctgg ccttttgctc acatgttctt tcctgcgtta tcccctgatt ctgtggataa 2640
   ccgtattacc gcctttgagt gagctgatac cgctcgccgc agccgaacga ccgagcgcag 2700
   cgagtcagtg agcgaggaag cggaagagcg cccaatacgc aaaccgcctc tccccgcgcg 2760
   ttggccgatt cattaatgca gctgtggaat gtgtgtcagt tagggtgtgg aaagtcccca 2820
   ggctccccag caggcagaag tatgcaaagc atgcatctca attagtcagc aaccaggtgt 2880
   ggaaagtccc caggctcccc agcaggcaga agtatgcaaa gcatgcatct caattagtca 2940
   gcaaccatag tcccgcccct aactccgccc atcccgcccc taactccgcc cagttccgcc 3000
   cattctccgc cccatggctg actaattttt tttatttatg cagaggccga ggccgcctcg 3060
   gcctctgagc tattccagaa gtagtgagga ggcttttttg gaggcctagg cttttgcaaa 3120
   aagcttggac acaagacagg cttgcgagat atgtttgaga ataccacttt atcccgcgtc 3180
   agggagaggc agtgcgtaaa aagacgcgga ctcatgtgaa atactggttt ttagtgcgcc 3240
   agatctctat aatctcgcgc aacctatttt cccctcgaac actttttaag ccgtagataa 3300
   acaggctggg acacttcaca tgagcgaaaa atacatcgtc acctgggaca tgttgcagat 3360
   ccatgcacgt aaactcgcaa gccgactgat gccttctgaa caatggaaag gcattattgc 3420
   cgtaagccgt ggcggtctgt accgggtgcg ttactggcgc gtgaactggg tattcgtcat 3480
   gtcgataccg tttgtatttc cagctacgat cacgacaacc agcgcgagct taaagtgctg 3540
   aaacgcgcag aaggcgatgg cgaaggcttc atcgttattg atgacctggt ggataccggt 3600
   ggtactgcgg ttgcgattcg tgaaatgtat ccaaaagcgc actttgtcac catcttcgca 3660
   aaaccggctg gtcgtccgct ggttgatgac tatgttgttg atatcccgca agatacctgg 3720
   attgaacagc cgtgggatat gggcgtcgta ttcgtcccgc caatctccgg tcgctaatct 3780
   tttcaacgcc tggcactgcc gggcgttgtt ctttttaact tcaggcgggt tacaatagtt 3840
   tccagtaagt attctggagg ctgcatccat gacacaggca aacctgagcg aaaccctgtt 3900
   caaaccccgc tttaaacatc ctgaaacctc gacgctagtc cgccgcttta atcacggcgc 3960
   acaaccgcct gtgcagtcgg cccttgatgg taaaaccatc cctcactggt atcgcatgat 4020
   taaccgtctg atgtggatct ggcgcggcat tgacccacgc gaaatcctcg acgtccaggc 4080
   acgtattgtg atgagcgatg ccgaacgtac cgacgatgat ttatacgata cggtgattgg 4140
   ctaccgtggc ggcaactgga tttatgagtg ggccccggat ctttgtgaag gaaccttact 4200
   tctgtggtgt gacataattg gacaaactac ctacagagat ttaaagctct aaggtaaata 4260
   taaaattttt aagtgtataa tgtgttaaac tactgattct aattgtttgt gtattttaga 4320
   ttccaaccta tggaactgat gaatgggagc agtggtggaa tgcctttaat gaggaaaacc 4380
   tgttttgctc agaagaaatg ccatctagtg atgatgaggc tactgctgac tctcaacatt 4440
   ctactcctcc aaaaaagaag agaaaggtag aagaccccaa ggactttcct tcagaattgc 4500
   taagtttttt gagtcatgct gtgtttagta atagaactct tgcttgcttt gctatttaca 4560
   ccacaaagga aaaagctgca ctgctataca agaaaattat ggaaaaatat tctgtaacct 4620
   ttataagtag gcataacagt tataatcata acatactgtt ttttcttact ccacacaggc 4680
   atagagtgtc tgctattaat aactatgctc aaaaattgtg tacctttagc tttttaattt 4740
   gtaaaggggt taataaggaa tatttgatgt atagtgcctt gactagagat cataatcagc 4800
   cataccacat ttgtagaggt tttacttgct ttaaaaaacc tcccacacct ccccctgaac 4860
   ctgaaacata aaatgaatgc aattgttgtt gttaacttgt ttattgcagc ttataatggt 4920
   tacaaataaa gcaatagcat cacaaatttc acaaataaag catttttttc actgcattct 4980
   agttgtggtt tgtccaaact catcaatgta tcttatcatg tctggatcaa ctggataact 5040
   caagctaacc aaaatcatcc caaacttccc accccatacc ctattaccac tgccaattac 5100
   ctagtggttt catttactct aaacctgtga ttcctctgaa ttattttcat tttaaagaaa 5160
   ttgtatttgt taaatatgta ctacaaactt agtagttgga agggctaatt cactcccaaa 5220
   gaagacaaga tatccttgat ctgtggatct accacacaca aggctacttc cctgattagc 5280
   agaactacac accagggcca ggggtcagat atccactgac ctttggatgg tgctacaagc 5340
   tagtaccagt tgagccagat aaggtagaag aggccaataa aggagagaac accagcttgt 5400
   tacaccctgt gagcctgcat gggatggatg acccggagag agaagtgtta gagtggaggt 5460
   ttgacagccg cctagcattt catcacgtgg cccgagagct gcatccggag tacttcaaga 5520
   actgctgata tcgagcttgc tacaagggac tttccgctgg ggactttcca gggaggcgtg 5580
   gcctgggcgg gactggggag tggcgagccc tcagatcctg catataagca gctgcttttt 5640
   gcctgtactg ggtctctctg gttagaccag atctgagcct gggagctctc tggctaacta 5700
   gggaacccac tgcttaagcc tcaataaagc ttgccttgag tgcttcaagt agtgtgtgcc 5760
   cgtctgttgt gtgactctgg taactagaga tccctcagac ccttttagtc agtgtggaaa 5820
   atctctagca gtggcgcccg aacagggact tgaaagcgaa agggaaacca gaggagctct 5880
   ctcgacgcag gactcggctt gctgaagcgc gcacggcaag aggcgagggg cggcgactgg 5940
   tgagtacgcc aaaaattttg actagcggag gctagaagga gagagatggg tgcgagagcg 6000
   tcagtattaa gcgggggaga attagatcgc gatgggaaaa aattcggtta aggccagggg 6060
   gaaagaaaaa atataaatta aaacatatag tatgggcaag cagggagcta gaacgattcg 6120
   cagttaatcc tggcctgtta gaaacatcag aaggctgtag acaaatactg ggacagctac 6180
   aaccatccct tcagacagga tcagaagaac ttagatcatt atataataca gtagcaaccc 6240
   tctattgtgt gcatcaaagg atagagataa aagacaccaa ggaagcttta gacaagatag 6300
   aggaagagca aaacaaaagt aagaccaccg cacagcaagc ggccgctgat cttcagacct 6360
   ggaggaggag atatgaggga caattggaga agtgaattat ataaatataa agtagtaaaa 6420
   attgaaccat taggagtagc acccaccaag gcaaagagaa gagtggtgca gagagaaaaa 6480
   agagcagtgg gaataggagc tttgttcctt gggttcttgg gagcagcagg aagcactatg 6540
   ggcgcagcgt caatgacgct gacggtacag gccagacaat tattgtctgg tatagtgcag 6600
   cagcagaaca atttgctgag ggctattgag gcgcaacagc atctgttgca actcacagtc 6660
   tggggcatca agcagctcca ggcaagaatc ctggctgtgg aaagatacct aaaggatcaa 6720
   cagctcctgg ggatttgggg ttgctctgga aaactcattt gcaccactgc tgtgccttgg 6780
   aatgctagtt ggagtaataa atctctggaa cagatttgga atcacacgac ctggatggag 6840
   tgggacagag aaattaacaa ttacacaagc ttaatacact ccttaattga agaatcgcaa 6900
   aaccagcaag aaaagaatga acaagaatta ttggaattag ataaatgggc aagtttgtgg 6960
   aattggttta acataacaaa ttggctgtgg tatataaaat tattcataat gatagtagga 7020
   ggcttggtag gtttaagaat agtttttgct gtactttcta tagtgaatag agttaggcag 7080
   ggatattcac cattatcgtt tcagacccac ctcccaaccc cgaggggacc cgacaggccc 7140
   gaaggaatag aagaagaagg tggagagaga gacagagaca gatccattcg attagtgaac 7200
   ggatctcgac ggtatcgccg aattcacaaa tggcagtatt catccacaat tttaaaagaa 7260
   aaggggggat tggggggtac agtgcagggg aaagaatagt agacataata gcaacagaca 7320
   tacaaactaa agaattacaa aaacaaatta caaaaattca aaattttcgg gtttattaca 7380
   gggacagcag agatccactt tatcgataag cttgggagtt ccgcgttaca taacttacgg 7440
   taaatggccc gcctggctga ccgcccaacg acccccgccc attgacgtca ataatgacgt 7500
   atgttcccat agtaacgcca atagggactt tccattgacg tcaatgggtg gagtatttac 7560
   ggtaaactgc ccacttggca gtacatcaag tgtatcatat gccaagtacg ccccctattg 7620
   acgtcaatga cggtaaatgg cccgcctggc attatgccca gtacatgacc ttacgggact 7680
   ttcctacttg gcagtacatc tacgtattag tcatcgctat taccatggtg atgcggtttt 7740
   ggcagtacac caatgggcgt ggatagcggt ttgactcacg gggatttcca agtctccacc 7800
   ccattgacgt caatgggagt ttgttttggc accaaaatca acgggacttt ccaaaatgtc 7860
   gtaacaactc cgccccattg acgcaaatgg gcggtaggcg tgtacggtgg gaggtctata 7920
   taagcagagc tcgtttagtg aaccgtcaga tcgcctggag acgccatcca cgctgttttg 7980
   acctccatag aagacaccga ctctagctag aggatccgga ctagtaactc gaggatgggg 8040
   actgacccgg gctgcaggaa ttcataactt cgtataatgt atgctatacg aagttatagt 8100
   taagccttat tgtttatcat cctcattcaa cgacgcgaag gtgtaccttg gcgagaataa 8160
   cttcgtatag gatactttat acgaagttat ttatctgtgc cccagtttgc tagggaggtc 8220
   gcagtacttg gccacagcca tctcgtgctg ctcgacgtag gtctctttgt cggcctcctt 8280
   gattctttcc agtctgtggt ccacgaagtg gaagccgggc atcttgaggt tcttagcggg 8340
   tttcttggat ctgtatgtgg tcttgaagga gcagtgcagg tagcccccgc ccacgagctt 8400
   cagggccatc tggctgtggc ctctcaggcc gccgtcagcg gggtacagca tctcggtgtt 8460
   ggcctcccag ccgcgtgttt tcttctgcat cacagggccg ttggatggga agttcacccc 8520
   gttgatcttg acgttgtaga tgatgcagcc gttctggaag ctggtgtcct gggtagcggt 8580
   cagcacgccc ccgtcttcgt atgtggtgat tctctcccat gtgaagccct cagggaagga 8640
   ctgcttaaag aagtcgggga tgccctgggt gtggttgatg aaggctttgc tgccgtacat 8700
   gaagctggta gccaggatgt cgaaggcgaa ggggagaggg ccgccctcga ccaccttgat 8760
   cttcatggtc tgggtgccct cgtagggctt gccttcgccc tcggatgtgc acttgaagtg 8820
   gtggttgttc acggtgccct ccatgtacag cttcatgtgc atgttctcct tgatcagctc 8880
   gctcatggtg gcatatgata acttcgtata gcatacatta tacgaagtta tattaagggt 8940
   tattgaatat gatcggaagt caacgggtcg atggtgatgc ttggctcgaa taacttcgta 9000
   taaagtatcc tatacgaagt tatactttgg ccgcggctcg agggggttgg ggttgcgcct 9060
   tttccaaggc agccctgggt ttgcgcaggg acgcggctgc tctgggcgtg gttccgggaa 9120
   acgcagcggc gccgaccctg ggtctcgcac attcttcacg tccgttcgca gcgtcacccg 9180
   gatcttcgcc gctacccttg tgggcccccc ggcgacgctt cctgctccgc ccctaagtcg 9240
   ggaaggttcc ttgcggttcg cggcgtgccg gacgtgacaa acggaagccg cacgtctcac 9300
   tagtaccctc gcagacggac agcgccaggg agcaatggca gcgcgccgac cgcgatgggc 9360
   tgtggccaat agcggctgct cagcagggcg cgccgagagc agcggccggg aaggggcggt 9420
   gcgggaggcg gggtgtgggg cggtagtgtg ggccctgttc ctgcccgcgc ggtgttccgc 9480
   attctgcaag cctccggagc gcacgtcggc agtcggctcc ctcgttgacc gaatcaccga 9540
   cctctctccc cagggggatc caccggtcgc caccatggtg agcaagggcg aggagctgtt 9600
   caccggggtg gtgcccatcc tggtcgagct ggacggcgac gtaaacggcc acaagttcag 9660
   cgtgtccggc gagggcgagg gcgatgccac ctacggcaag ctgaccctga agttcatctg 9720
   caccaccggc aagctgcccg tgccctggcc caccctcgtg accaccctga cctacggcgt 9780
   gcagtgcttc agccgctacc ccgaccacat gaagcagcac gacttcttca agtccgccat 9840
   gcccgaaggc tacgtccagg agcgcaccat cttcttcaag gacgacggca actacaagac 9900
   ccgcgccgag gtgaagttcg agggcgacac cctggtgaac cgcatcgagc tgaagggcat 9960
   cgacttcaag gaggacggca acatcctggg gcacaagctg gagtacaact acaacagcca 10020
   caacgtctat atcatggccg acaagcagaa gaacggcatc aaggtgaact tcaagatccg 10080
   ccacaacatc gaggacggca gcgtgcagct cgccgaccac taccagcaga acacccccat 10140
   cggcgacggc cccgtgctgc tgcccgacaa ccactacctg agcacccagt ccgccctgag 10200
   caaagacccc aacgagaagc gcgatcacat ggtcctgctg gagttcgtga ccgccgccgg 10260
   gatcactctc ggcatggacg agctgtacaa gtaagatatc aagcttatcg ataatcaacc 10320
   tctggattac aaaatttgtg aaagattgac tggtattctt aactatgttg ctccttttac 10380
   gctatgtgga tacgctgctt taatgccttt gtatcatgct attgcttccc gtatggcttt 10440
   cattttctcc tccttgtata aatcctggtt gctgtctctt tatgaggagt tgtggcccgt 10500
   tgtcaggcaa cgtggcgtgg tgtgcactgt gtttgctgac gcaaccccca ctggttgggg 10560
   cattgccacc acctgtcagc tcctttccgg gactttcgct ttccccctcc ctattgccac 10620
   ggcggaactc atcgccgcct gccttgcccg ctgctggaca ggggctcggc tgttgggcac 10680
   tgacaattcc gtggtgttgt cggggaaatc atcgtccttt ccttggctgc tcgcctgtgt 10740
   tgccacctgg attctgcgcg ggacgtcctt ctgctacgtc ccttcggccc tcaatccagc 10800
   ggaccttcct tcccgcggcc tgctgccggc tctgcggcct cttccgcgtc ttcgccttcg 10860
   ccctcagacg agtcggatct ccctttgggc cgcctccccg catcgatacc gtcgacctcg 10920
   agaacctaga aaaacatgga gcaatcacaa gtagcaatac agcagctacc aatgctgatt 10980
   gtgcctggct agaagcacaa gaggaggagg aggtgggttt tccagtcaca cctcaggtac 11040
   ctttaagacc aatgacttac aaggcagctg tagatcttag ccacttttta aaagaaaagg 11100
   ggggactgga agggctaatt cactcccaac gaagacaaga tctttttgct tgtactgggt 11160
   ctctctggtt agaccagatc tgagcctggg agctctctgg ctaactaggg aacccactgc 11220
   ttaagcctca ataaagcttg ccttgagtgc ttcaagtagt gtgtgcccgt ctgttgtgtg 11280
   actctggtaa ctagagatcc ctcagaccct tttagtcagt gtggaaaatc tctaga 11336
<210> 33
   <211> 11223
   <212> DNA
   <213> Artificial Sequence
<220>
   <223> sequence for screening human cells
<400> 33
   ttaattccgt gtattctata gtgtcaccta aatcgtatgt gtatgataca taaggttatg 60
   tattaattgt agccgcgttc taacgacaat atgtacaagc ctaattgtgt agcatctggc 120
   ttactgaagc agaccctatc atctctctcg taaactgccg tcagagtcgg tttggttgga 180
   cgaaccttct gagtttctgg taacgccgtc ccgcacccgg aaatggtcag cgaaccaatc 240
   agcagggtca tcgctagcca gatcctctac gccggacgca tcgtggccgg catcaccggc 300
   gccacaggtg cggttgctgg cgcctatatc gccgacatca ccgatgggga agatcgggct 360
   cgccacttcg ggctcatgag cgcttgtttc ggcgtgggta tggtggcagg ccccgtggcc 420
   gggggactgt tgggcgccat ctccttgcat gcaccattcc ttgcggcggc ggtgctcaac 480
   ggcctcaacc tactactggg ctgcttccta atgcaggagt cgcataaggg agagcgtcga 540
   atggtgcact ctcagtacaa tctgctctga tgccgcatag ttaagccagc cccgacaccc 600
   gccaacaccc gctgacgcgc cctgacgggc ttgtctgctc ccggcatccg cttacagaca 660
   agctgtgacc gtctccggga gctgcatgtg tcagaggttt tcaccgtcat caccgaaacg 720
   cgcgagacga aagggcctcg tgatacgcct atttttatag gttaatgtca tgataataat 780
   ggtttcttag acgtcaggtg gcacttttcg gggaaatgtg cgcggaaccc ctatttgttt 840
   atttttctaa atacattcaa atatgtatcc gctcatgaga caataaccct gataaatgct 900
   tcaataatat tgaaaaagga agagtatgag tattcaacat ttccgtgtcg cccttattcc 960
   cttttttgcg gcattttgcc ttcctgtttt tgctcaccca gaaacgctgg tgaaagtaaa 1020
   agatgctgaa gatcagttgg gtgcacgagt gggttacatc gaactggatc tcaacagcgg 1080
   taagatcctt gagagttttc gccccgaaga acgttttcca atgatgagca cttttaaagt 1140
   tctgctatgt ggcgcggtat tatcccgtat tgacgccggg caagagcaac tcggtcgccg 1200
   catacactat tctcagaatg acttggttga gtactcacca gtcacagaaa agcatcttac 1260
   ggatggcatg acagtaagag aattatgcag tgctgccata accatgagtg ataacactgc 1320
   ggccaactta cttctgacaa cgatcggagg accgaaggag ctaaccgctt ttttgcacaa 1380
   catgggggat catgtaactc gccttgatcg ttgggaaccg gagctgaatg aagccatacc 1440
   aaacgacgag cgtgacacca cgatgcctgt agcaatggca acaacgttgc gcaaactatt 1500
   aactggcgaa ctacttactc tagcttcccg gcaacaatta atagactgga tggaggcgga 1560
   taaagttgca ggaccacttc tgcgctcggc ccttccggct ggctggttta ttgctgataa 1620
   atctggagcc ggtgagcgtg ggtctcgcgg tatcattgca gcactggggc cagatggtaa 1680
   gccctcccgt atcgtagtta tctacacgac ggggagtcag gcaactatgg atgaacgaaa 1740
   tagacagatc gctgagatag gtgcctcact gattaagcat tggtaactgt cagaccaagt 1800
   ttactcatat atactttaga ttgatttaaa acttcatttt taatttaaaa ggatctaggt 1860
   gaagatcctt tttgataatc tcatgaccaa aatcccttaa cgtgagtttt cgttccactg 1920
   agcgtcagac cccgtagaaa agatcaaagg atcttcttga gatccttttt ttctgcgcgt 1980
   aatctgctgc ttgcaaacaa aaaaaccacc gctaccagcg gtggtttgtt tgccggatca 2040
   agagctacca actctttttc cgaaggtaac tggcttcagc agagcgcaga taccaaatac 2100
   tgtccttcta gtgtagccgt agttaggcca ccacttcaag aactctgtag caccgcctac 2160
   atacctcgct ctgctaatcc tgttaccagt ggctgctgcc agtggcgata agtcgtgtct 2220
   taccgggttg gactcaagac gatagttacc ggataaggcg cagcggtcgg gctgaacggg 2280
   gggttcgtgc acacagccca gcttggagcg aacgacctac accgaactga gatacctaca 2340
   gcgtgagcat tgagaaagcg ccacgcttcc cgaagggaga aaggcggaca ggtatccggt 2400
   aagcggcagg gtcggaacag gagagcgcac gagggagctt ccagggggaa acgcctggta 2460
   tctttatagt cctgtcgggt ttcgccacct ctgacttgag cgtcgatttt tgtgatgctc 2520
   gtcagggggg cggagcctat ggaaaaacgc cagcaacgcg gcctttttac ggttcctggc 2580
   cttttgctgg ccttttgctc acatgttctt tcctgcgtta tcccctgatt ctgtggataa 2640
   ccgtattacc gcctttgagt gagctgatac cgctcgccgc agccgaacga ccgagcgcag 2700
   cgagtcagtg agcgaggaag cggaagagcg cccaatacgc aaaccgcctc tccccgcgcg 2760
   ttggccgatt cattaatgca gctgtggaat gtgtgtcagt tagggtgtgg aaagtcccca 2820
   ggctccccag caggcagaag tatgcaaagc atgcatctca attagtcagc aaccaggtgt 2880
   ggaaagtccc caggctcccc agcaggcaga agtatgcaaa gcatgcatct caattagtca 2940
   gcaaccatag tcccgcccct aactccgccc atcccgcccc taactccgcc cagttccgcc 3000
   cattctccgc cccatggctg actaattttt tttatttatg cagaggccga ggccgcctcg 3060
   gcctctgagc tattccagaa gtagtgagga ggcttttttg gaggcctagg cttttgcaaa 3120
   aagcttggac acaagacagg cttgcgagat atgtttgaga ataccacttt atcccgcgtc 3180
   agggagaggc agtgcgtaaa aagacgcgga ctcatgtgaa atactggttt ttagtgcgcc 3240
   agatctctat aatctcgcgc aacctatttt cccctcgaac actttttaag ccgtagataa 3300
   acaggctggg acacttcaca tgagcgaaaa atacatcgtc acctgggaca tgttgcagat 3360
   ccatgcacgt aaactcgcaa gccgactgat gccttctgaa caatggaaag gcattattgc 3420
   cgtaagccgt ggcggtctgt accgggtgcg ttactggcgc gtgaactggg tattcgtcat 3480
   gtcgataccg tttgtatttc cagctacgat cacgacaacc agcgcgagct taaagtgctg 3540
   aaacgcgcag aaggcgatgg cgaaggcttc atcgttattg atgacctggt ggataccggt 3600
   ggtactgcgg ttgcgattcg tgaaatgtat ccaaaagcgc actttgtcac catcttcgca 3660
   aaaccggctg gtcgtccgct ggttgatgac tatgttgttg atatcccgca agatacctgg 3720
   attgaacagc cgtgggatat gggcgtcgta ttcgtcccgc caatctccgg tcgctaatct 3780
   tttcaacgcc tggcactgcc gggcgttgtt ctttttaact tcaggcgggt tacaatagtt 3840
   tccagtaagt attctggagg ctgcatccat gacacaggca aacctgagcg aaaccctgtt 3900
   caaaccccgc tttaaacatc ctgaaacctc gacgctagtc cgccgcttta atcacggcgc 3960
   acaaccgcct gtgcagtcgg cccttgatgg taaaaccatc cctcactggt atcgcatgat 4020
   taaccgtctg atgtggatct ggcgcggcat tgacccacgc gaaatcctcg acgtccaggc 4080
   acgtattgtg atgagcgatg ccgaacgtac cgacgatgat ttatacgata cggtgattgg 4140
   ctaccgtggc ggcaactgga tttatgagtg ggccccggat ctttgtgaag gaaccttact 4200
   tctgtggtgt gacataattg gacaaactac ctacagagat ttaaagctct aaggtaaata 4260
   taaaattttt aagtgtataa tgtgttaaac tactgattct aattgtttgt gtattttaga 4320
   ttccaaccta tggaactgat gaatgggagc agtggtggaa tgcctttaat gaggaaaacc 4380
   tgttttgctc agaagaaatg ccatctagtg atgatgaggc tactgctgac tctcaacatt 4440
   ctactcctcc aaaaaagaag agaaaggtag aagaccccaa ggactttcct tcagaattgc 4500
   taagtttttt gagtcatgct gtgtttagta atagaactct tgcttgcttt gctatttaca 4560
   ccacaaagga aaaagctgca ctgctataca agaaaattat ggaaaaatat tctgtaacct 4620
   ttataagtag gcataacagt tataatcata acatactgtt ttttcttact ccacacaggc 4680
   atagagtgtc tgctattaat aactatgctc aaaaattgtg tacctttagc tttttaattt 4740
   gtaaaggggt taataaggaa tatttgatgt atagtgcctt gactagagat cataatcagc 4800
   cataccacat ttgtagaggt tttacttgct ttaaaaaacc tcccacacct ccccctgaac 4860
   ctgaaacata aaatgaatgc aattgttgtt gttaacttgt ttattgcagc ttataatggt 4920
   tacaaataaa gcaatagcat cacaaatttc acaaataaag catttttttc actgcattct 4980
   agttgtggtt tgtccaaact catcaatgta tcttatcatg tctggatcaa ctggataact 5040
   caagctaacc aaaatcatcc caaacttccc accccatacc ctattaccac tgccaattac 5100
   ctagtggttt catttactct aaacctgtga ttcctctgaa ttattttcat tttaaagaaa 5160
   ttgtatttgt taaatatgta ctacaaactt agtagttgga agggctaatt cactcccaaa 5220
   gaagacaaga tatccttgat ctgtggatct accacacaca aggctacttc cctgattagc 5280
   agaactacac accagggcca ggggtcagat atccactgac ctttggatgg tgctacaagc 5340
   tagtaccagt tgagccagat aaggtagaag aggccaataa aggagagaac accagcttgt 5400
   tacaccctgt gagcctgcat gggatggatg acccggagag agaagtgtta gagtggaggt 5460
   ttgacagccg cctagcattt catcacgtgg cccgagagct gcatccggag tacttcaaga 5520
   actgctgata tcgagcttgc tacaagggac tttccgctgg ggactttcca gggaggcgtg 5580
   gcctgggcgg gactggggag tggcgagccc tcagatcctg catataagca gctgcttttt 5640
   gcctgtactg ggtctctctg gttagaccag atctgagcct gggagctctc tggctaacta 5700
   gggaacccac tgcttaagcc tcaataaagc ttgccttgag tgcttcaagt agtgtgtgcc 5760
   cgtctgttgt gtgactctgg taactagaga tccctcagac ccttttagtc agtgtggaaa 5820
   atctctagca gtggcgcccg aacagggact tgaaagcgaa agggaaacca gaggagctct 5880
   ctcgacgcag gactcggctt gctgaagcgc gcacggcaag aggcgagggg cggcgactgg 5940
   tgagtacgcc aaaaattttg actagcggag gctagaagga gagagatggg tgcgagagcg 6000
   tcagtattaa gcgggggaga attagatcgc gatgggaaaa aattcggtta aggccagggg 6060
   gaaagaaaaa atataaatta aaacatatag tatgggcaag cagggagcta gaacgattcg 6120
   cagttaatcc tggcctgtta gaaacatcag aaggctgtag acaaatactg ggacagctac 6180
   aaccatccct tcagacagga tcagaagaac ttagatcatt atataataca gtagcaaccc 6240
   tctattgtgt gcatcaaagg atagagataa aagacaccaa ggaagcttta gacaagatag 6300
   aggaagagca aaacaaaagt aagaccaccg cacagcaagc ggccgctgat cttcagacct 6360
   ggaggaggag atatgaggga caattggaga agtgaattat ataaatataa agtagtaaaa 6420
   attgaaccat taggagtagc acccaccaag gcaaagagaa gagtggtgca gagagaaaaa 6480
   agagcagtgg gaataggagc tttgttcctt gggttcttgg gagcagcagg aagcactatg 6540
   ggcgcagcgt caatgacgct gacggtacag gccagacaat tattgtctgg tatagtgcag 6600
   cagcagaaca atttgctgag ggctattgag gcgcaacagc atctgttgca actcacagtc 6660
   tggggcatca agcagctcca ggcaagaatc ctggctgtgg aaagatacct aaaggatcaa 6720
   cagctcctgg ggatttgggg ttgctctgga aaactcattt gcaccactgc tgtgccttgg 6780
   aatgctagtt ggagtaataa atctctggaa cagatttgga atcacacgac ctggatggag 6840
   tgggacagag aaattaacaa ttacacaagc ttaatacact ccttaattga agaatcgcaa 6900
   aaccagcaag aaaagaatga acaagaatta ttggaattag ataaatgggc aagtttgtgg 6960
   aattggttta acataacaaa ttggctgtgg tatataaaat tattcataat gatagtagga 7020
   ggcttggtag gtttaagaat agtttttgct gtactttcta tagtgaatag agttaggcag 7080
   ggatattcac cattatcgtt tcagacccac ctcccaaccc cgaggggacc cgacaggccc 7140
   gaaggaatag aagaagaagg tggagagaga gacagagaca gatccattcg attagtgaac 7200
   ggatctcgac ggtatcgccg aattcacaaa tggcagtatt catccacaat tttaaaagaa 7260
   aaggggggat tggggggtac agtgcagggg aaagaatagt agacataata gcaacagaca 7320
   tacaaactaa agaattacaa aaacaaatta caaaaattca aaattttcgg gtttattaca 7380
   gggacagcag agatccactt tatcgatggt ccggtccgga attcctgcag ccccgataaa 7440
   ataaaagatt ttatttagtc tccagaaaaa ggggggaatg aaagacccca cctgtaggtt 7500
   tggcaagcta gctgcagtaa cgccattttg caaggcatgg aaaaatacca aaccaagaat 7560
   agagaagttc agatcaaggg cgggtacatg aaaatagcta acgttgggcc aaacaggata 7620
   tctgcggtga gcagtttcgg ccccggcccg gggccaagaa cagatggtca ccgcagtttc 7680
   ggccccggcc cgaggccaag aacagatggt ccccagatat ggcccaaccc tcagcagttt 7740
   cttaagaccc atcagatgtt tccaggctcc cccaaggacc tgaaatgacc ctgcgcctta 7800
   tttgaattaa ccaatcagcc tgcttctcgc ttctgttcgc gcgcttctgc ttcccgagct 7860
   ctataaaaga gctcacaacc cctcactcgg cgcgccagtc ctccgacaga ctgagtcgcc 7920
   cgggggggat ccaccgggct gcaggaattc ataacttcgt ataatgtatg ctatacgaag 7980
   ttatagttaa gccttattgt ttatcatcct cattcaacga cgcgaaggtg taccttggcg 8040
   agaataactt cgtataggat actttatacg aagttattta tctgtgcccc agtttgctag 8100
   ggaggtcgca gtacttggcc acagccatct cgtgctgctc gacgtaggtc tctttgtcgg 8160
   cctccttgat tctttccagt ctgtggtcca cgaagtggaa gccgggcatc ttgaggttct 8220
   tagcgggttt cttggatctg tatgtggtct tgaaggagca gtgcaggtag cccccgccca 8280
   cgagcttcag ggccatctgg ctgtggcctc tcaggccgcc gtcagcgggg tacagcatct 8340
   cggtgttggc ctcccagccg cgtgttttct tctgcatcac agggccgttg gatgggaagt 8400
   tcaccccgtt gatcttgacg ttgtagatga tgcagccgtt ctggaagctg gtgtcctggg 8460
   tagcggtcag cacgcccccg tcttcgtatg tggtgattct ctcccatgtg aagccctcag 8520
   ggaaggactg cttaaagaag tcggggatgc cctgggtgtg gttgatgaag gctttgctgc 8580
   cgtacatgaa gctggtagcc aggatgtcga aggcgaaggg gagagggccg ccctcgacca 8640
   ccttgatctt catggtctgg gtgccctcgt agggcttgcc ttcgccctcg gatgtgcact 8700
   tgaagtggtg gttgttcacg gtgccctcca tgtacagctt catgtgcatg ttctccttga 8760
   tcagctcgct catggtggca tatgataact tcgtatagca tacattatac gaagttatat 8820
   taagggttat tgaatatgat cggaagtcaa cgggtcgatg gtgatgcttg gctcgaataa 8880
   cttcgtataa agtatcctat acgaagttat actttggccg cggctcgagg gggttggggt 8940
   tgcgcctttt ccaaggcagc cctgggtttg cgcagggacg cggctgctct gggcgtggtt 9000
   ccgggaaacg cagcggcgcc gaccctgggt ctcgcacatt cttcacgtcc gttcgcagcg 9060
   tcacccggat cttcgccgct acccttgtgg gccccccggc gacgcttcct gctccgcccc 9120
   taagtcggga aggttccttg cggttcgcgg cgtgccggac gtgacaaacg gaagccgcac 9180
   gtctcactag taccctcgca gacggacagc gccagggagc aatggcagcg cgccgaccgc 9240
   gatgggctgt ggccaatagc ggctgctcag cagggcgcgc cgagagcagc ggccgggaag 9300
   gggcggtgcg ggaggcgggg tgtggggcgg tagtgtgggc cctgttcctg cccgcgcggt 9360
   gttccgcatt ctgcaagcct ccggagcgca cgtcggcagt cggctccctc gttgaccgaa 9420
   tcaccgacct ctctccccag ggggatccac cggtcgccac catggtgagc aagggcgagg 9480
   agctgttcac cggggtggtg cccatcctgg tcgagctgga cggcgacgta aacggccaca 9540
   agttcagcgt gtccggcgag ggcgagggcg atgccaccta cggcaagctg accctgaagt 9600
   tcatctgcac caccggcaag ctgcccgtgc cctggcccac cctcgtgacc accctgacct 9660
   acggcgtgca gtgcttcagc cgctaccccg accacatgaa gcagcacgac ttcttcaagt 9720
   ccgccatgcc cgaaggctac gtccaggagc gcaccatctt cttcaaggac gacggcaact 9780
   acaagacccg cgccgaggtg aagttcgagg gcgacaccct ggtgaaccgc atcgagctga 9840
   agggcatcga cttcaaggag gacggcaaca tcctggggca caagctggag tacaactaca 9900
   acagccacaa cgtctatatc atggccgaca agcagaagaa cggcatcaag gtgaacttca 9960
   agatccgcca caacatcgag gacggcagcg tgcagctcgc cgaccactac cagcagaaca 10020
   cccccatcgg cgacggcccc gtgctgctgc ccgacaacca ctacctgagc acccagtccg 10080
   ccctgagcaa agaccccaac gagaagcgcg atcacatggt cctgctggag ttcgtgaccg 10140
   ccgccgggat cactctcggc atggacgagc tgtacaagta agatatcaag cttatcgata 10200
   atcaacctct ggattacaaa atttgtgaaa gattgactgg tattcttaac tatgttgctc 10260
   cttttacgct atgtggatac gctgctttaa tgcctttgta tcatgctatt gcttcccgta 10320
   tggctttcat tttctcctcc ttgtataaat cctggttgct gtctctttat gaggagttgt 10380
   ggcccgttgt caggcaacgt ggcgtggtgt gcactgtgtt tgctgacgca acccccactg 10440
   gttggggcat tgccaccacc tgtcagctcc tttccgggac tttcgctttc cccctcccta 10500
   ttgccacggc ggaactcatc gccgcctgcc ttgcccgctg ctggacaggg gctcggctgt 10560
   tgggcactga caattccgtg gtgttgtcgg ggaaatcatc gtcctttcct tggctgctcg 10620
   cctgtgttgc cacctggatt ctgcgcggga cgtccttctg ctacgtccct tcggccctca 10680
   atccagcgga ccttccttcc cgcggcctgc tgccggctct gcggcctctt ccgcgtcttc 10740
   gccttcgccc tcagacgagt cggatctccc tttgggccgc ctccccgcat cgataccgtc 10800
   gacctcgaga acctagaaaa acatggagca atcacaagta gcaatacagc agctaccaat 10860
   gctgattgtg cctggctaga agcacaagag gaggaggagg tgggttttcc agtcacacct 10920
   caggtacctt taagaccaat gacttacaag gcagctgtag atcttagcca ctttttaaaa 10980
   gaaaaggggg gactggaagg gctaattcac tcccaacgaa gacaagatct ttttgcttgt 11040
   actgggtctc tctggttaga ccagatctga gcctgggagc tctctggcta actagggaac 11100
   ccactgctta agcctcaata aagcttgcct tgagtgcttc aagtagtgtg tgcccgtctg 11160
   ttgtgtgact ctggtaacta gagatccctc agaccctttt agtcagtgtg gaaaatctct 11220
   aga 11223
<210> 34
   <211> 11357
   <212> DNA
   <213> Artificial Sequence
<220>
   <223> sequence for screeining human cells
<400> 34
   ttaattccgt gtattctata gtgtcaccta aatcgtatgt gtatgataca taaggttatg 60
   tattaattgt agccgcgttc taacgacaat atgtacaagc ctaattgtgt agcatctggc 120
   ttactgaagc agaccctatc atctctctcg taaactgccg tcagagtcgg tttggttgga 180
   cgaaccttct gagtttctgg taacgccgtc ccgcacccgg aaatggtcag cgaaccaatc 240
   agcagggtca tcgctagcca gatcctctac gccggacgca tcgtggccgg catcaccggc 300
   gccacaggtg cggttgctgg cgcctatatc gccgacatca ccgatgggga agatcgggct 360
   cgccacttcg ggctcatgag cgcttgtttc ggcgtgggta tggtggcagg ccccgtggcc 420
   gggggactgt tgggcgccat ctccttgcat gcaccattcc ttgcggcggc ggtgctcaac 480
   ggcctcaacc tactactggg ctgcttccta atgcaggagt cgcataaggg agagcgtcga 540
   atggtgcact ctcagtacaa tctgctctga tgccgcatag ttaagccagc cccgacaccc 600
   gccaacaccc gctgacgcgc cctgacgggc ttgtctgctc ccggcatccg cttacagaca 660
   agctgtgacc gtctccggga gctgcatgtg tcagaggttt tcaccgtcat caccgaaacg 720
   cgcgagacga aagggcctcg tgatacgcct atttttatag gttaatgtca tgataataat 780
   ggtttcttag acgtcaggtg gcacttttcg gggaaatgtg cgcggaaccc ctatttgttt 840
   atttttctaa atacattcaa atatgtatcc gctcatgaga caataaccct gataaatgct 900
   tcaataatat tgaaaaagga agagtatgag tattcaacat ttccgtgtcg cccttattcc 960
   cttttttgcg gcattttgcc ttcctgtttt tgctcaccca gaaacgctgg tgaaagtaaa 1020
   agatgctgaa gatcagttgg gtgcacgagt gggttacatc gaactggatc tcaacagcgg 1080
   taagatcctt gagagttttc gccccgaaga acgttttcca atgatgagca cttttaaagt 1140
   tctgctatgt ggcgcggtat tatcccgtat tgacgccggg caagagcaac tcggtcgccg 1200
   catacactat tctcagaatg acttggttga gtactcacca gtcacagaaa agcatcttac 1260
   ggatggcatg acagtaagag aattatgcag tgctgccata accatgagtg ataacactgc 1320
   ggccaactta cttctgacaa cgatcggagg accgaaggag ctaaccgctt ttttgcacaa 1380
   catgggggat catgtaactc gccttgatcg ttgggaaccg gagctgaatg aagccatacc 1440
   aaacgacgag cgtgacacca cgatgcctgt agcaatggca acaacgttgc gcaaactatt 1500
   aactggcgaa ctacttactc tagcttcccg gcaacaatta atagactgga tggaggcgga 1560
   taaagttgca ggaccacttc tgcgctcggc ccttccggct ggctggttta ttgctgataa 1620
   atctggagcc ggtgagcgtg ggtctcgcgg tatcattgca gcactggggc cagatggtaa 1680
   gccctcccgt atcgtagtta tctacacgac ggggagtcag gcaactatgg atgaacgaaa 1740
   tagacagatc gctgagatag gtgcctcact gattaagcat tggtaactgt cagaccaagt 1800
   ttactcatat atactttaga ttgatttaaa acttcatttt taatttaaaa ggatctaggt 1860
   gaagatcctt tttgataatc tcatgaccaa aatcccttaa cgtgagtttt cgttccactg 1920
   agcgtcagac cccgtagaaa agatcaaagg atcttcttga gatccttttt ttctgcgcgt 1980
   aatctgctgc ttgcaaacaa aaaaaccacc gctaccagcg gtggtttgtt tgccggatca 2040
   agagctacca actctttttc cgaaggtaac tggcttcagc agagcgcaga taccaaatac 2100
   tgtccttcta gtgtagccgt agttaggcca ccacttcaag aactctgtag caccgcctac 2160
   atacctcgct ctgctaatcc tgttaccagt ggctgctgcc agtggcgata agtcgtgtct 2220
   taccgggttg gactcaagac gatagttacc ggataaggcg cagcggtcgg gctgaacggg 2280
   gggttcgtgc acacagccca gcttggagcg aacgacctac accgaactga gatacctaca 2340
   gcgtgagcat tgagaaagcg ccacgcttcc cgaagggaga aaggcggaca ggtatccggt 2400
   aagcggcagg gtcggaacag gagagcgcac gagggagctt ccagggggaa acgcctggta 2460
   tctttatagt cctgtcgggt ttcgccacct ctgacttgag cgtcgatttt tgtgatgctc 2520
   gtcagggggg cggagcctat ggaaaaacgc cagcaacgcg gcctttttac ggttcctggc 2580
   cttttgctgg ccttttgctc acatgttctt tcctgcgtta tcccctgatt ctgtggataa 2640
   ccgtattacc gcctttgagt gagctgatac cgctcgccgc agccgaacga ccgagcgcag 2700
   cgagtcagtg agcgaggaag cggaagagcg cccaatacgc aaaccgcctc tccccgcgcg 2760
   ttggccgatt cattaatgca gctgtggaat gtgtgtcagt tagggtgtgg aaagtcccca 2820
   ggctccccag caggcagaag tatgcaaagc atgcatctca attagtcagc aaccaggtgt 2880
   ggaaagtccc caggctcccc agcaggcaga agtatgcaaa gcatgcatct caattagtca 2940
   gcaaccatag tcccgcccct aactccgccc atcccgcccc taactccgcc cagttccgcc 3000
   cattctccgc cccatggctg actaattttt tttatttatg cagaggccga ggccgcctcg 3060
   gcctctgagc tattccagaa gtagtgagga ggcttttttg gaggcctagg cttttgcaaa 3120
   aagcttggac acaagacagg cttgcgagat atgtttgaga ataccacttt atcccgcgtc 3180
   agggagaggc agtgcgtaaa aagacgcgga ctcatgtgaa atactggttt ttagtgcgcc 3240
   agatctctat aatctcgcgc aacctatttt cccctcgaac actttttaag ccgtagataa 3300
   acaggctggg acacttcaca tgagcgaaaa atacatcgtc acctgggaca tgttgcagat 3360
   ccatgcacgt aaactcgcaa gccgactgat gccttctgaa caatggaaag gcattattgc 3420
   cgtaagccgt ggcggtctgt accgggtgcg ttactggcgc gtgaactggg tattcgtcat 3480
   gtcgataccg tttgtatttc cagctacgat cacgacaacc agcgcgagct taaagtgctg 3540
   aaacgcgcag aaggcgatgg cgaaggcttc atcgttattg atgacctggt ggataccggt 3600
   ggtactgcgg ttgcgattcg tgaaatgtat ccaaaagcgc actttgtcac catcttcgca 3660
   aaaccggctg gtcgtccgct ggttgatgac tatgttgttg atatcccgca agatacctgg 3720
   attgaacagc cgtgggatat gggcgtcgta ttcgtcccgc caatctccgg tcgctaatct 3780
   tttcaacgcc tggcactgcc gggcgttgtt ctttttaact tcaggcgggt tacaatagtt 3840
   tccagtaagt attctggagg ctgcatccat gacacaggca aacctgagcg aaaccctgtt 3900
   caaaccccgc tttaaacatc ctgaaacctc gacgctagtc cgccgcttta atcacggcgc 3960
   acaaccgcct gtgcagtcgg cccttgatgg taaaaccatc cctcactggt atcgcatgat 4020
   taaccgtctg atgtggatct ggcgcggcat tgacccacgc gaaatcctcg acgtccaggc 4080
   acgtattgtg atgagcgatg ccgaacgtac cgacgatgat ttatacgata cggtgattgg 4140
   ctaccgtggc ggcaactgga tttatgagtg ggccccggat ctttgtgaag gaaccttact 4200
   tctgtggtgt gacataattg gacaaactac ctacagagat ttaaagctct aaggtaaata 4260
   taaaattttt aagtgtataa tgtgttaaac tactgattct aattgtttgt gtattttaga 4320
   ttccaaccta tggaactgat gaatgggagc agtggtggaa tgcctttaat gaggaaaacc 4380
   tgttttgctc agaagaaatg ccatctagtg atgatgaggc tactgctgac tctcaacatt 4440
   ctactcctcc aaaaaagaag agaaaggtag aagaccccaa ggactttcct tcagaattgc 4500
   taagtttttt gagtcatgct gtgtttagta atagaactct tgcttgcttt gctatttaca 4560
   ccacaaagga aaaagctgca ctgctataca agaaaattat ggaaaaatat tctgtaacct 4620
   ttataagtag gcataacagt tataatcata acatactgtt ttttcttact ccacacaggc 4680
   atagagtgtc tgctattaat aactatgctc aaaaattgtg tacctttagc tttttaattt 4740
   gtaaaggggt taataaggaa tatttgatgt atagtgcctt gactagagat cataatcagc 4800
   cataccacat ttgtagaggt tttacttgct ttaaaaaacc tcccacacct ccccctgaac 4860
   ctgaaacata aaatgaatgc aattgttgtt gttaacttgt ttattgcagc ttataatggt 4920
   tacaaataaa gcaatagcat cacaaatttc acaaataaag catttttttc actgcattct 4980
   agttgtggtt tgtccaaact catcaatgta tcttatcatg tctggatcaa ctggataact 5040
   caagctaacc aaaatcatcc caaacttccc accccatacc ctattaccac tgccaattac 5100
   ctagtggttt catttactct aaacctgtga ttcctctgaa ttattttcat tttaaagaaa 5160
   ttgtatttgt taaatatgta ctacaaactt agtagttgga agggctaatt cactcccaaa 5220
   gaagacaaga tatccttgat ctgtggatct accacacaca aggctacttc cctgattagc 5280
   agaactacac accagggcca ggggtcagat atccactgac ctttggatgg tgctacaagc 5340
   tagtaccagt tgagccagat aaggtagaag aggccaataa aggagagaac accagcttgt 5400
   tacaccctgt gagcctgcat gggatggatg acccggagag agaagtgtta gagtggaggt 5460
   ttgacagccg cctagcattt catcacgtgg cccgagagct gcatccggag tacttcaaga 5520
   actgctgata tcgagcttgc tacaagggac tttccgctgg ggactttcca gggaggcgtg 5580
   gcctgggcgg gactggggag tggcgagccc tcagatcctg catataagca gctgcttttt 5640
   gcctgtactg ggtctctctg gttagaccag atctgagcct gggagctctc tggctaacta 5700
   gggaacccac tgcttaagcc tcaataaagc ttgccttgag tgcttcaagt agtgtgtgcc 5760
   cgtctgttgt gtgactctgg taactagaga tccctcagac ccttttagtc agtgtggaaa 5820
   atctctagca gtggcgcccg aacagggact tgaaagcgaa agggaaacca gaggagctct 5880
   ctcgacgcag gactcggctt gctgaagcgc gcacggcaag aggcgagggg cggcgactgg 5940
   tgagtacgcc aaaaattttg actagcggag gctagaagga gagagatggg tgcgagagcg 6000
   tcagtattaa gcgggggaga attagatcgc gatgggaaaa aattcggtta aggccagggg 6060
   gaaagaaaaa atataaatta aaacatatag tatgggcaag cagggagcta gaacgattcg 6120
   cagttaatcc tggcctgtta gaaacatcag aaggctgtag acaaatactg ggacagctac 6180
   aaccatccct tcagacagga tcagaagaac ttagatcatt atataataca gtagcaaccc 6240
   tctattgtgt gcatcaaagg atagagataa aagacaccaa ggaagcttta gacaagatag 6300
   aggaagagca aaacaaaagt aagaccaccg cacagcaagc ggccgctgat cttcagacct 6360
   ggaggaggag atatgaggga caattggaga agtgaattat ataaatataa agtagtaaaa 6420
   attgaaccat taggagtagc acccaccaag gcaaagagaa gagtggtgca gagagaaaaa 6480
   agagcagtgg gaataggagc tttgttcctt gggttcttgg gagcagcagg aagcactatg 6540
   ggcgcagcgt caatgacgct gacggtacag gccagacaat tattgtctgg tatagtgcag 6600
   cagcagaaca atttgctgag ggctattgag gcgcaacagc atctgttgca actcacagtc 6660
   tggggcatca agcagctcca ggcaagaatc ctggctgtgg aaagatacct aaaggatcaa 6720
   cagctcctgg ggatttgggg ttgctctgga aaactcattt gcaccactgc tgtgccttgg 6780
   aatgctagtt ggagtaataa atctctggaa cagatttgga atcacacgac ctggatggag 6840
   tgggacagag aaattaacaa ttacacaagc ttaatacact ccttaattga agaatcgcaa 6900
   aaccagcaag aaaagaatga acaagaatta ttggaattag ataaatgggc aagtttgtgg 6960
   aattggttta acataacaaa ttggctgtgg tatataaaat tattcataat gatagtagga 7020
   ggcttggtag gtttaagaat agtttttgct gtactttcta tagtgaatag agttaggcag 7080
   ggatattcac cattatcgtt tcagacccac ctcccaaccc cgaggggacc cgacaggccc 7140
   gaaggaatag aagaagaagg tggagagaga gacagagaca gatccattcg attagtgaac 7200
   ggatctcgac ggtatcgccg aattcacaaa tggcagtatt catccacaat tttaaaagaa 7260
   aaggggggat tggggggtac agtgcagggg aaagaatagt agacataata gcaacagaca 7320
   tacaaactaa agaattacaa aaacaaatta caaaaattca aaattttcgg gtttattaca 7380
   gggacagcag agatccactt tatcgatggt ccggtccgga attcctgcag ccccgataaa 7440
   ataaaagatt ttatttagtc tccagaaaaa ggggggaatg aaagacccca cctgtaggtt 7500
   tggcaagcta gctgcagtaa cgccattttg caaggcatgg aaaaatacca aaccaagaat 7560
   agagaagttc agatcaaggg cgggtacatg aaaatagcta acgttgggcc aaacaggata 7620
   tctgcggtga gcagtttcgg ccccggcccg gggccaagaa cagatggtca ccgcagtttc 7680
   ggccccggcc cgaggccaag aacagatggt ccccagatat ggcccaaccc tcagcagttt 7740
   cttaagaccc atcagatgtt tccaggctcc cccaaggacc tgaaatgacc ctgcgcctta 7800
   tttgaattaa ccaatcagcc tgcttctcgc ttctgttcgc gcgcttctgc ttcccgagct 7860
   ctataaaaga gctcacaacc cctcactcgg cgcgccagtc ctccgacaga ctgagtcgcc 7920
   cgggggggat ccaccgggct gcaggaattc ataacttcgt ataatgtatg ctatacgaag 7980
   ttatagttaa gccttattgt ttatcatcct cattcaacga cgcgaaggtg taccttggcg 8040
   agaataactt cgtataggat actttatacg aagttattta tctgtgcccc agtttgctag 8100
   ggaggtcgca gtacttggcc acagccatct cgtgctgctc gacgtaggtc tctttgtcgg 8160
   cctccttgat tctttccagt ctgtggtcca cgaagtggaa gccgggcatc ttgaggttct 8220
   tagcgggttt cttggatctg tatgtggtct tgaaggagca gtgcaggtag cccccgccca 8280
   cgagcttcag ggccatctgg ctgtggcctc tcaggccgcc gtcagcgggg tacagcatct 8340
   cggtgttggc ctcccagccg cgtgttttct tctgcatcac agggccgttg gatgggaagt 8400
   tcaccccgtt gatcttgacg ttgtagatga tgcagccgtt ctggaagctg gtgtcctggg 8460
   tagcggtcag cacgcccccg tcttcgtatg tggtgattct ctcccatgtg aagccctcag 8520
   ggaaggactg cttaaagaag tcggggatgc cctgggtgtg gttgatgaag gctttgctgc 8580
   cgtacatgaa gctggtagcc aggatgtcga aggcgaaggg gagagggccg ccctcgacca 8640
   ccttgatctt catggtctgg gtgccctcgt agggcttgcc ttcgccctcg gatgtgcact 8700
   tgaagtggtg gttgttcacg gtgccctcca tgtacagctt catgtgcatg ttctccttga 8760
   tcagctcgct catggtggca tatgataact tcgtatagca tacattatac gaagttatat 8820
   taagggttat tgaatatgat cggaagtcaa cgggtcgatg gtgatgcttg gctcgaataa 8880
   cttcgtataa agtatcctat acgaagttat actttggccg cggctcgaat ctacttaccg 8940
   ataagcttgg gagttccgcg ttacataact tacggtaaat ggcccgcctg gctgaccgcc 9000
   caacgacccc cgcccattga cgtcaataat gacgtatgtt cccatagtaa cgccaatagg 9060
   gactttccat tgacgtcaat gggtggagta tttacggtaa actgcccact tggcagtaca 9120
   tcaagtgtat catatgccaa gtacgccccc tattgacgtc aatgacggta aatggcccgc 9180
   ctggcattat gcccagtaca tgaccttacg ggactttcct acttggcagt acatctacgt 9240
   attagtcatc gctattacca tggtgatgcg gttttggcag tacaccaatg ggcgtggata 9300
   gcggtttgac tcacggggat ttccaagtct ccaccccatt gacgtcaatg ggagtttgtt 9360
   ttggcaccaa aatcaacggg actttccaaa atgtcgtaac aactccgccc cattgacgca 9420
   aatgggcggt aggcgtgtac ggtgggaggt ctatataagc agagctcgtt tagtgaaccg 9480
   tcagatcgcc tggagacgcc atccacgctg ttttgacctc catagaagac accgactcta 9540
   gctagaggat ccggactagt aactcgagga tggggactga cccggacgcg tgccgccacc 9600
   atggtgagca agggcgagga gctgttcacc ggggtggtgc ccatcctggt cgagctggac 9660
   ggcgacgtaa acggccacaa gttcagcgtg tccggcgagg gcgagggcga tgccacctac 9720
   ggcaagctga ccctgaagtt catctgcacc accggcaagc tgcccgtgcc ctggcccacc 9780
   ctcgtgacca ccctgaccta cggcgtgcag tgcttcagcc gctaccccga ccacatgaag 9840
   cagcacgact tcttcaagtc cgccatgccc gaaggctacg tccaggagcg caccatcttc 9900
   ttcaaggacg acggcaacta caagacccgc gccgaggtga agttcgaggg cgacaccctg 9960
   gtgaaccgca tcgagctgaa gggcatcgac ttcaaggagg acggcaacat cctggggcac 10020
   aagctggagt acaactacaa cagccacaac gtctatatca tggccgacaa gcagaagaac 10080
   ggcatcaagg tgaacttcaa gatccgccac aacatcgagg acggcagcgt gcagctcgcc 10140
   gaccactacc agcagaacac ccccatcggc gacggccccg tgctgctgcc cgacaaccac 10200
   tacctgagca cccagtccgc cctgagcaaa gaccccaacg agaagcgcga tcacatggtc 10260
   ctgctggagt tcgtgaccgc cgccgggatc actctcggca tggacgagct gtacaagtaa 10320
   gatatcaagc ttatcgataa tcaacctctg gattacaaaa tttgtgaaag attgactggt 10380
   attcttaact atgttgctcc ttttacgcta tgtggatacg ctgctttaat gcctttgtat 10440
   catgctattg cttcccgtat ggctttcatt ttctcctcct tgtataaatc ctggttgctg 10500
   tctctttatg aggagttgtg gcccgttgtc aggcaacgtg gcgtggtgtg cactgtgttt 10560
   gctgacgcaa cccccactgg ttggggcatt gccaccacct gtcagctcct ttccgggact 10620
   ttcgctttcc ccctccctat tgccacggcg gaactcatcg ccgcctgcct tgcccgctgc 10680
   tggacagggg ctcggctgtt gggcactgac aattccgtgg tgttgtcggg gaaatcatcg 10740
   tcctttcctt ggctgctcgc ctgtgttgcc acctggattc tgcgcgggac gtccttctgc 10800
   tacgtccctt cggccctcaa tccagcggac cttccttccc gcggcctgct gccggctctg 10860
   cggcctcttc cgcgtcttcg ccttcgccct cagacgagtc ggatctccct ttgggccgcc 10920
   tccccgcatc gataccgtcg acctcgagaa cctagaaaaa catggagcaa tcacaagtag 10980
   caatacagca gctaccaatg ctgattgtgc ctggctagaa gcacaagagg aggaggaggt 11040
   gggttttcca gtcacacctc aggtaccttt aagaccaatg acttacaagg cagctgtaga 11100
   tcttagccac tttttaaaag aaaagggggg actggaaggg ctaattcact cccaacgaag 11160
   acaagatctt tttgcttgta ctgggtctct ctggttagac cagatctgag cctgggagct 11220
   ctctggctaa ctagggaacc cactgcttaa gcctcaataa agcttgcctt gagtgcttca 11280
   agtagtgtgt gcccgtctgt tgtgtgactc tggtaactag agatccctca gaccctttta 11340
   gtcagtgtgg aaaatct 11357
<210> 35
   <211> 12293
   <212> DNA
   <213> Artificial Sequence
<220>
   <223> sequence for screening human cells
<400> 35
   ttaattccgt gtattctata gtgtcaccta aatcgtatgt gtatgataca taaggttatg      60
   tattaattgt agccgcgttc taacgacaat atgtacaagc ctaattgtgt agcatctggc     120
   ttactgaagc agaccctatc atctctctcg taaactgccg tcagagtcgg tttggttgga     180
   cgaaccttct gagtttctgg taacgccgtc ccgcacccgg aaatggtcag cgaaccaatc     240
   agcagggtca tcgctagcca gatcctctac gccggacgca tcgtggccgg catcaccggc     300
   gccacaggtg cggttgctgg cgcctatatc gccgacatca ccgatgggga agatcgggct     360
   cgccacttcg ggctcatgag cgcttgtttc ggcgtgggta tggtggcagg ccccgtggcc     420
   gggggactgt tgggcgccat ctccttgcat gcaccattcc ttgcggcggc ggtgctcaac     480
   ggcctcaacc tactactggg ctgcttccta atgcaggagt cgcataaggg agagcgtcga     540
   atggtgcact ctcagtacaa tctgctctga tgccgcatag ttaagccagc cccgacaccc     600
   gccaacaccc gctgacgcgc cctgacgggc ttgtctgctc ccggcatccg cttacagaca     660
   agctgtgacc gtctccggga gctgcatgtg tcagaggttt tcaccgtcat caccgaaacg     720
   cgcgagacga aagggcctcg tgatacgcct atttttatag gttaatgtca tgataataat     780
   ggtttcttag acgtcaggtg gcacttttcg gggaaatgtg cgcggaaccc ctatttgttt     840
   atttttctaa atacattcaa atatgtatcc gctcatgaga caataaccct gataaatgct     900
   tcaataatat tgaaaaagga agagtatgag tattcaacat ttccgtgtcg cccttattcc     960
   cttttttgcg gcattttgcc ttcctgtttt tgctcaccca gaaacgctgg tgaaagtaaa 1020
   agatgctgaa gatcagttgg gtgcacgagt gggttacatc gaactggatc tcaacagcgg 1080
   taagatcctt gagagttttc gccccgaaga acgttttcca atgatgagca cttttaaagt 1140
   tctgctatgt ggcgcggtat tatcccgtat tgacgccggg caagagcaac tcggtcgccg 1200
   catacactat tctcagaatg acttggttga gtactcacca gtcacagaaa agcatcttac 1260
   ggatggcatg acagtaagag aattatgcag tgctgccata accatgagtg ataacactgc 1320
   ggccaactta cttctgacaa cgatcggagg accgaaggag ctaaccgctt ttttgcacaa 1380
   catgggggat catgtaactc gccttgatcg ttgggaaccg gagctgaatg aagccatacc 1440
   aaacgacgag cgtgacacca cgatgcctgt agcaatggca acaacgttgc gcaaactatt 1500
   aactggcgaa ctacttactc tagcttcccg gcaacaatta atagactgga tggaggcgga 1560
   taaagttgca ggaccacttc tgcgctcggc ccttccggct ggctggttta ttgctgataa 1620
   atctggagcc ggtgagcgtg ggtctcgcgg tatcattgca gcactggggc cagatggtaa 1680
   gccctcccgt atcgtagtta tctacacgac ggggagtcag gcaactatgg atgaacgaaa 1740
   tagacagatc gctgagatag gtgcctcact gattaagcat tggtaactgt cagaccaagt 1800
   ttactcatat atactttaga ttgatttaaa acttcatttt taatttaaaa ggatctaggt 1860
   gaagatcctt tttgataatc tcatgaccaa aatcccttaa cgtgagtttt cgttccactg 1920
   agcgtcagac cccgtagaaa agatcaaagg atcttcttga gatccttttt ttctgcgcgt 1980
   aatctgctgc ttgcaaacaa aaaaaccacc gctaccagcg gtggtttgtt tgccggatca 2040
   agagctacca actctttttc cgaaggtaac tggcttcagc agagcgcaga taccaaatac 2100
   tgtccttcta gtgtagccgt agttaggcca ccacttcaag aactctgtag caccgcctac 2160
   atacctcgct ctgctaatcc tgttaccagt ggctgctgcc agtggcgata agtcgtgtct 2220
   taccgggttg gactcaagac gatagttacc ggataaggcg cagcggtcgg gctgaacggg 2280
   gggttcgtgc acacagccca gcttggagcg aacgacctac accgaactga gatacctaca 2340
   gcgtgagcat tgagaaagcg ccacgcttcc cgaagggaga aaggcggaca ggtatccggt 2400
   aagcggcagg gtcggaacag gagagcgcac gagggagctt ccagggggaa acgcctggta 2460
   tctttatagt cctgtcgggt ttcgccacct ctgacttgag cgtcgatttt tgtgatgctc 2520
   gtcagggggg cggagcctat ggaaaaacgc cagcaacgcg gcctttttac ggttcctggc 2580
   cttttgctgg ccttttgctc acatgttctt tcctgcgtta tcccctgatt ctgtggataa 2640
   ccgtattacc gcctttgagt gagctgatac cgctcgccgc agccgaacga ccgagcgcag 2700
   cgagtcagtg agcgaggaag cggaagagcg cccaatacgc aaaccgcctc tccccgcgcg 2760
   ttggccgatt cattaatgca gctgtggaat gtgtgtcagt tagggtgtgg aaagtcccca 2820
   ggctccccag caggcagaag tatgcaaagc atgcatctca attagtcagc aaccaggtgt 2880
   ggaaagtccc caggctcccc agcaggcaga agtatgcaaa gcatgcatct caattagtca 2940
   gcaaccatag tcccgcccct aactccgccc atcccgcccc taactccgcc cagttccgcc 3000
   cattctccgc cccatggctg actaattttt tttatttatg cagaggccga ggccgcctcg 3060
   gcctctgagc tattccagaa gtagtgagga ggcttttttg gaggcctagg cttttgcaaa 3120
   aagcttggac acaagacagg cttgcgagat atgtttgaga ataccacttt atcccgcgtc 3180
   agggagaggc agtgcgtaaa aagacgcgga ctcatgtgaa atactggttt ttagtgcgcc 3240
   agatctctat aatctcgcgc aacctatttt cccctcgaac actttttaag ccgtagataa 3300
   acaggctggg acacttcaca tgagcgaaaa atacatcgtc acctgggaca tgttgcagat 3360
   ccatgcacgt aaactcgcaa gccgactgat gccttctgaa caatggaaag gcattattgc 3420
   cgtaagccgt ggcggtctgt accgggtgcg ttactggcgc gtgaactggg tattcgtcat 3480
   gtcgataccg tttgtatttc cagctacgat cacgacaacc agcgcgagct taaagtgctg 3540
   aaacgcgcag aaggcgatgg cgaaggcttc atcgttattg atgacctggt ggataccggt 3600
   ggtactgcgg ttgcgattcg tgaaatgtat ccaaaagcgc actttgtcac catcttcgca 3660
   aaaccggctg gtcgtccgct ggttgatgac tatgttgttg atatcccgca agatacctgg 3720
   attgaacagc cgtgggatat gggcgtcgta ttcgtcccgc caatctccgg tcgctaatct 3780
   tttcaacgcc tggcactgcc gggcgttgtt ctttttaact tcaggcgggt tacaatagtt 3840
   tccagtaagt attctggagg ctgcatccat gacacaggca aacctgagcg aaaccctgtt 3900
   caaaccccgc tttaaacatc ctgaaacctc gacgctagtc cgccgcttta atcacggcgc 3960
   acaaccgcct gtgcagtcgg cccttgatgg taaaaccatc cctcactggt atcgcatgat 4020
   taaccgtctg atgtggatct ggcgcggcat tgacccacgc gaaatcctcg acgtccaggc 4080
   acgtattgtg atgagcgatg ccgaacgtac cgacgatgat ttatacgata cggtgattgg 4140
   ctaccgtggc ggcaactgga tttatgagtg ggccccggat ctttgtgaag gaaccttact 4200
   tctgtggtgt gacataattg gacaaactac ctacagagat ttaaagctct aaggtaaata 4260
   taaaattttt aagtgtataa tgtgttaaac tactgattct aattgtttgt gtattttaga 4320
   ttccaaccta tggaactgat gaatgggagc agtggtggaa tgcctttaat gaggaaaacc 4380
   tgttttgctc agaagaaatg ccatctagtg atgatgaggc tactgctgac tctcaacatt 4440
   ctactcctcc aaaaaagaag agaaaggtag aagaccccaa ggactttcct tcagaattgc 4500
   taagtttttt gagtcatgct gtgtttagta atagaactct tgcttgcttt gctatttaca 4560
   ccacaaagga aaaagctgca ctgctataca agaaaattat ggaaaaatat tctgtaacct 4620
   ttataagtag gcataacagt tataatcata acatactgtt ttttcttact ccacacaggc 4680
   atagagtgtc tgctattaat aactatgctc aaaaattgtg tacctttagc tttttaattt 4740
   gtaaaggggt taataaggaa tatttgatgt atagtgcctt gactagagat cataatcagc 4800
   cataccacat ttgtagaggt tttacttgct ttaaaaaacc tcccacacct ccccctgaac 4860
   ctgaaacata aaatgaatgc aattgttgtt gttaacttgt ttattgcagc ttataatggt 4920
   tacaaataaa gcaatagcat cacaaatttc acaaataaag catttttttc actgcattct 4980
   agttgtggtt tgtccaaact catcaatgta tcttatcatg tctggatcaa ctggataact 5040
   caagctaacc aaaatcatcc caaacttccc accccatacc ctattaccac tgccaattac 5100
   ctagtggttt catttactct aaacctgtga ttcctctgaa ttattttcat tttaaagaaa 5160
   ttgtatttgt taaatatgta ctacaaactt agtagttgga agggctaatt cactcccaaa 5220
   gaagacaaga tatccttgat ctgtggatct accacacaca aggctacttc cctgattagc 5280
   agaactacac accagggcca ggggtcagat atccactgac ctttggatgg tgctacaagc 5340
   tagtaccagt tgagccagat aaggtagaag aggccaataa aggagagaac accagcttgt 5400
   tacaccctgt gagcctgcat gggatggatg acccggagag agaagtgtta gagtggaggt 5460
   ttgacagccg cctagcattt catcacgtgg cccgagagct gcatccggag tacttcaaga 5520
   actgctgata tcgagcttgc tacaagggac tttccgctgg ggactttcca gggaggcgtg 5580
   gcctgggcgg gactggggag tggcgagccc tcagatcctg catataagca gctgcttttt 5640
   gcctgtactg ggtctctctg gttagaccag atctgagcct gggagctctc tggctaacta 5700
   gggaacccac tgcttaagcc tcaataaagc ttgccttgag tgcttcaagt agtgtgtgcc 5760
   cgtctgttgt gtgactctgg taactagaga tccctcagac ccttttagtc agtgtggaaa 5820
   atctctagca gtggcgcccg aacagggact tgaaagcgaa agggaaacca gaggagctct 5880
   ctcgacgcag gactcggctt gctgaagcgc gcacggcaag aggcgagggg cggcgactgg 5940
   tgagtacgcc aaaaattttg actagcggag gctagaagga gagagatggg tgcgagagcg 6000
   tcagtattaa gcgggggaga attagatcgc gatgggaaaa aattcggtta aggccagggg 6060
   gaaagaaaaa atataaatta aaacatatag tatgggcaag cagggagcta gaacgattcg 6120
   cagttaatcc tggcctgtta gaaacatcag aaggctgtag acaaatactg ggacagctac 6180
   aaccatccct tcagacagga tcagaagaac ttagatcatt atataataca gtagcaaccc 6240
   tctattgtgt gcatcaaagg atagagataa aagacaccaa ggaagcttta gacaagatag 6300
   aggaagagca aaacaaaagt aagaccaccg cacagcaagc ggccgctgat cttcagacct 6360
   ggaggaggag atatgaggga caattggaga agtgaattat ataaatataa agtagtaaaa 6420
   attgaaccat taggagtagc acccaccaag gcaaagagaa gagtggtgca gagagaaaaa 6480
   agagcagtgg gaataggagc tttgttcctt gggttcttgg gagcagcagg aagcactatg 6540
   ggcgcagcgt caatgacgct gacggtacag gccagacaat tattgtctgg tatagtgcag 6600
   cagcagaaca atttgctgag ggctattgag gcgcaacagc atctgttgca actcacagtc 6660
   tggggcatca agcagctcca ggcaagaatc ctggctgtgg aaagatacct aaaggatcaa 6720
   cagctcctgg ggatttgggg ttgctctgga aaactcattt gcaccactgc tgtgccttgg 6780
   aatgctagtt ggagtaataa atctctggaa cagatttgga atcacacgac ctggatggag 6840
   tgggacagag aaattaacaa ttacacaagc ttaatacact ccttaattga agaatcgcaa 6900
   aaccagcaag aaaagaatga acaagaatta ttggaattag ataaatgggc aagtttgtgg 6960
   aattggttta acataacaaa ttggctgtgg tatataaaat tattcataat gatagtagga 7020
   ggcttggtag gtttaagaat agtttttgct gtactttcta tagtgaatag agttaggcag 7080
   ggatattcac cattatcgtt tcagacccac ctcccaaccc cgaggggacc cgacaggccc 7140
   gaaggaatag aagaagaagg tggagagaga gacagagaca gatccattcg attagtgaac 7200
   ggatctcgac ggtatcgccg aattcacaaa tggcagtatt catccacaat tttaaaagaa 7260
   aaggggggat tggggggtac agtgcagggg aaagaatagt agacataata gcaacagaca 7320
   tacaaactaa agaattacaa aaacaaatta caaaaattca aaattttcgg gtttattaca 7380
   gggacagcag agatccactt tatcgataag cttgggagtt ccgcgttaca taacttacgg 7440
   taaatggccc gcctggctga ccgcccaacg acccccgccc attgacgtca ataatgacgt 7500
   atgttcccat agtaacgcca atagggactt tccattgacg tcaatgggtg gagtatttac 7560
   ggtaaactgc ccacttggca gtacatcaag tgtatcatat gccaagtacg ccccctattg 7620
   acgtcaatga cggtaaatgg cccgcctggc attatgccca gtacatgacc ttacgggact 7680
   ttcctacttg gcagtacatc tacgtattag tcatcgctat taccatggtg atgcggtttt 7740
   ggcagtacac caatgggcgt ggatagcggt ttgactcacg gggatttcca agtctccacc 7800
   ccattgacgt caatgggagt ttgttttggc accaaaatca acgggacttt ccaaaatgtc 7860
   gtaacaactc cgccccattg acgcaaatgg gcggtaggcg tgtacggtgg gaggtctata 7920
   taagcagagc tcgtttagtg aaccgtcaga tcgcctggag acgccatcca cgctgttttg 7980
   acctccatag aagacaccga ctctagctag aggatccgga ctagtaactc gaggatgggg 8040
   actgacccgg gctgcaggaa ttcataactt cgtataatgt atgctatacg aagttatagt 8100
   taagccttat tgtttatcat cctcattcaa cgacgcgaag gtgtaccttg gcgagaataa 8160
   cttcgtatag gatactttat acgaagttat ttacacggcg atctttccgc ccttcttggc 8220
   ctttatgagg atctctctga tttttcttgc gtcgagtttt ccggtaagac ctttcggtac 8280
   ttcgtccaca aacacaactc ctccgcgcaa ctttttcgcg gttgttactt gactggcgac 8340
   gtaatccacg atctcttttt ccgtcatcgt ctttccgtgc tccaaaacaa caacggcggc 8400
   gggaagttca ccggcgtcat cgtcgggaag acctgcgaca cctgcgtcga agatgttggg 8460
   gtgttggagc aagatggatt ccaattcagc gggagccacc tgatagcctt tgtacttaat 8520
   cagagacttc aggcggtcaa cgatgaagaa gtgttcgtct tcgtcccagt aagctatgtc 8580
   tccagaatgt agccatccat ccttgtcaat caaggcgttg gtcgcttccg gattgtttac 8640
   ataaccggac ataatcatag gacctctcac acacagttcg cctctttgat taacgcccag 8700
   cgttttcccg gtatccagat ccacaacctt cgcttcaaaa aatggaacaa ctttaccgac 8760
   cgcgcccggt ttatcatccc cctcgggtgt aatcagaata gctgatgtag tctcagtgag 8820
   cccatatcct tgcctgatac ctggcagatg gaacctcttg gcaaccgctt ccccgacttc 8880
   cttagagagg ggagcgccac cagaagcaat ttcgtgtaaa ttagataaat cgtatttgtc 8940
   aatcagagtg cttttggcga agaaggagaa tagggttggc accagcagcg cactttgaat 9000
   cttgtaatcc tgaaggctcc tcagaaacag ctcttcttca aatctataca ttaagacgac 9060
   tcgaaatcca catatcaaat atccgagtgt agtaaacatt ccaaaaccgt gatggaatgg 9120
   aacaacactt aaaatcgcag tatccggaat gatttgattg ccaaaaatag gatctctggc 9180
   atgcgagaat ctcacgcagg cagttctatg aggcagagcg acacctttag gcagaccagt 9240
   agatccagag gagttcatga tcagtgcaat tgtcttgtcc ctatcgaagg actctggcac 9300
   aaaatcgtat tcattaaaac cgggaggtag atgagatgtg acgaacgtgt acatcgactg 9360
   aaatccctgg taatccgttt tagaatccat gataataatt ttttggatga ttgggagctt 9420
   tttttgcacg ttcaaaattt tttgcaaccc ctttttggaa acgaacacca cggtaggctg 9480
   cgaaatgccc atactgttga gcaattcacg ttcattataa atgtcgttcg cgggcgcaac 9540
   tgcaactccg ataaataacg cgcccaacac cggcataaag aattgaagag agttttcact 9600
   gcatacgacg attctgtgat ttgtattcag cccatatcgt ttcatagctt ctgccaaccg 9660
   aacggacatt tcgaagtact cagcgtaagt gatgtccacc tcgatatgtg catctgtaaa 9720
   agcaattgtt ccaggaacca gggcgtatct cttcatagcc ttatgcagtt gctctccagc 9780
   ggttccatct tccagcggat agaatggcgc cgggcctttc tttatgtttt tggcgtcttc 9840
   catggtggca tatgataact tcgtatagca tacattatac gaagttatat taagggttat 9900
   tgaatatgat cggaagtcaa cgggtcgatg gtgatgcttg gctcgaataa cttcgtataa 9960
   agtatcctat acgaagttat actttggccg cggctcgagg gggttggggt tgcgcctttt 10020
   ccaaggcagc cctgggtttg cgcagggacg cggctgctct gggcgtggtt ccgggaaacg 10080
   cagcggcgcc gaccctgggt ctcgcacatt cttcacgtcc gttcgcagcg tcacccggat 10140
   cttcgccgct acccttgtgg gccccccggc gacgcttcct gctccgcccc taagtcggga 10200
   aggttccttg cggttcgcgg cgtgccggac gtgacaaacg gaagccgcac gtctcactag 10260
   taccctcgca gacggacagc gccagggagc aatggcagcg cgccgaccgc gatgggctgt 10320
   ggccaatagc ggctgctcag cagggcgcgc cgagagcagc ggccgggaag gggcggtgcg 10380
   ggaggcgggg tgtggggcgg tagtgtgggc cctgttcctg cccgcgcggt gttccgcatt 10440
   ctgcaagcct ccggagcgca cgtcggcagt cggctccctc gttgaccgaa tcaccgacct 10500
   ctctccccag ggggatccac cggtcgccac catggtgagc aagggcgagg agctgttcac 10560
   cggggtggtg cccatcctgg tcgagctgga cggcgacgta aacggccaca agttcagcgt 10620
   gtccggcgag ggcgagggcg atgccaccta cggcaagctg accctgaagt tcatctgcac 10680
   caccggcaag ctgcccgtgc cctggcccac cctcgtgacc accctgacct acggcgtgca 10740
   gtgcttcagc cgctaccccg accacatgaa gcagcacgac ttcttcaagt ccgccatgcc 10800
   cgaaggctac gtccaggagc gcaccatctt cttcaaggac gacggcaact acaagacccg 10860
   cgccgaggtg aagttcgagg gcgacaccct ggtgaaccgc atcgagctga agggcatcga 10920
   cttcaaggag gacggcaaca tcctggggca caagctggag tacaactaca acagccacaa 10980
   cgtctatatc atggccgaca agcagaagaa cggcatcaag gtgaacttca agatccgcca 11040
   caacatcgag gacggcagcg tgcagctcgc cgaccactac cagcagaaca cccccatcgg 11100
   cgacggcccc gtgctgctgc ccgacaacca ctacctgagc acccagtccg ccctgagcaa 11160
   agaccccaac gagaagcgcg atcacatggt cctgctggag ttcgtgaccg ccgccgggat 11220
   cactctcggc atggacgagc tgtacaagta agatatcaag cttatcgata atcaacctct 11280
   ggattacaaa atttgtgaaa gattgactgg tattcttaac tatgttgctc cttttacgct 11340
   atgtggatac gctgctttaa tgcctttgta tcatgctatt gcttcccgta tggctttcat 11400
   tttctcctcc ttgtataaat cctggttgct gtctctttat gaggagttgt ggcccgttgt 11460
   caggcaacgt ggcgtggtgt gcactgtgtt tgctgacgca acccccactg gttggggcat 11520
   tgccaccacc tgtcagctcc tttccgggac tttcgctttc cccctcccta ttgccacggc 11580
   ggaactcatc gccgcctgcc ttgcccgctg ctggacaggg gctcggctgt tgggcactga 11640
   caattccgtg gtgttgtcgg ggaaatcatc gtcctttcct tggctgctcg cctgtgttgc 11700
   cacctggatt ctgcgcggga cgtccttctg ctacgtccct tcggccctca atccagcgga 11760
   ccttccttcc cgcggcctgc tgccggctct gcggcctctt ccgcgtcttc gccttcgccc 11820
   tcagacgagt cggatctccc tttgggccgc ctccccgcat cgataccgtc gacctcgaga 11880
   acctagaaaa acatggagca atcacaagta gcaatacagc agctaccaat gctgattgtg 11940
   cctggctaga agcacaagag gaggaggagg tgggttttcc agtcacacct caggtacctt 12000
   taagaccaat gacttacaag gcagctgtag atcttagcca ctttttaaaa gaaaaggggg 12060
   gactggaagg gctaattcac tcccaacgaa gacaagatct ttttgcttgt actgggtctc 12120
   tctggttaga ccagatctga gcctgggagc tctctggcta actagggaac ccactgctta 12180
   agcctcaata aagcttgcct tgagtgcttc aagtagtgtg tgcccgtctg ttgtgtgact 12240
   ctggtaacta gagatccctc agaccctttt agtcagtgtg gaaaatctct aga 12293
<210> 36
   <211> 6135
   <212> DNA
   <213> Artificial Sequence
<220>
   <223> sequence for screening human cells
<400> 36
   gggctaattc actcccaaag aagacaagat atccttgatc tgtggatcta ccacacacaa 60
   ggctacttcc ctgattagca gaactacaca ccagggccag gggtcagata tccactgacc 120
   tttggatggt gctacaagct agtaccagtt gagccagata aggtagaaga ggccaataaa 180
   ggagagaaca ccagcttgtt acaccctgtg agcctgcatg ggatggatga cccggagaga 240
   gaagtgttag agtggaggtt tgacagccgc ctagcatttc atcacgtggc ccgagagctg 300
   catccggagt acttcaagaa ctgctgatat cgagcttgct acaagggact ttccgctggg 360
   gactttccag ggaggcgtgg cctgggcggg actggggagt ggcgagccct cagatcctgc 420
   atataagcag ctgctttttg cctgtactgg gtctctctgg ttagaccaga tctgagcctg 480
   ggagctctct ggctaactag ggaacccact gcttaagcct caataaagct tgccttgagt 540
   gcttcaagta gtgtgtgccc gtctgttgtg tgactctggt aactagagat ccctcagacc 600
   cttttagtca gtgtggaaaa tctctagcag tggcgcccga acagggactt gaaagcgaaa 660
   gggaaaccag aggagctctc tcgacgcagg actcggcttg ctgaagcgcg cacggcaaga 720
   ggcgaggggc ggcgactggt gagtacgcca aaaattttga ctagcggagg ctagaaggag 780
   agagatgggt gcgagagcgt cagtattaag cgggggagaa ttagatcgcg atgggaaaaa 840
   attcggttaa ggccaggggg aaagaaaaaa tataaattaa aacatatagt atgggcaagc 900
   agggagctag aacgattcgc agttaatcct ggcctgttag aaacatcaga aggctgtaga 960
   caaatactgg gacagctaca accatccctt cagacaggat cagaagaact tagatcatta 1020
   tataatacag tagcaaccct ctattgtgtg catcaaagga tagagataaa agacaccaag 1080
   gaagctttag acaagataga ggaagagcaa aacaaaagta agaccaccgc acagcaagcg 1140
   gccgctgatc ttcagacctg gaggaggaga tatgagggac aattggagaa gtgaattata 1200
   taaatataaa gtagtaaaaa ttgaaccatt aggagtagca cccaccaagg caaagagaag 1260
   agtggtgcag agagaaaaaa gagcagtggg aataggagct ttgttccttg ggttcttggg 1320
   agcagcagga agcactatgg gcgcagcgtc aatgacgctg acggtacagg ccagacaatt 1380
   attgtctggt atagtgcagc agcagaacaa tttgctgagg gctattgagg cgcaacagca 1440
   tctgttgcaa ctcacagtct ggggcatcaa gcagctccag gcaagaatcc tggctgtgga 1500
   aagataccta aaggatcaac agctcctggg gatttggggt tgctctggaa aactcatttg 1560
   caccactgct gtgccttgga atgctagttg gagtaataaa tctctggaac agatttggaa 1620
   tcacacgacc tggatggagt gggacagaga aattaacaat tacacaagct taatacactc 1680
   cttaattgaa gaatcgcaaa accagcaaga aaagaatgaa caagaattat tggaattaga 1740
   taaatgggca agtttgtgga attggtttaa cataacaaat tggctgtggt atataaaatt 1800
   attcataatg atagtaggag gcttggtagg tttaagaata gtttttgctg tactttctat 1860
   agtgaataga gttaggcagg gatattcacc attatcgttt cagacccacc tcccaacccc 1920
   gaggggaccc gacaggcccg aaggaataga agaagaaggt ggagagagag acagagacag 1980
   atccattcga ttagtgaacg gatctcgacg gtatcgccga attcacaaat ggcagtattc 2040
   atccacaatt ttaaaagaaa aggggggatt ggggggtaca gtgcagggga aagaatagta 2100
   gacataatag caacagacat acaaactaaa gaattacaaa aacaaattac aaaaattcaa 2160
   aattttcggg tttattacag ggacagcaga gatccacttt atcgataagc ttgggagttc 2220
   cgcgttacat aacttacggt aaatggcccg cctggctgac cgcccaacga cccccgccca 2280
   ttgacgtcaa taatgacgta tgttcccata gtaacgccaa tagggacttt ccattgacgt 2340
   caatgggtgg agtatttacg gtaaactgcc cacttggcag tacatcaagt gtatcatatg 2400
   ccaagtacgc cccctattga cgtcaatgac ggtaaatggc ccgcctggca ttatgcccag 2460
   tacatgacct tacgggactt tcctacttgg cagtacatct acgtattagt catcgctatt 2520
   accatggtga tgcggttttg gcagtacacc aatgggcgtg gatagcggtt tgactcacgg 2580
   ggatttccaa gtctccaccc cattgacgtc aatgggagtt tgttttggca ccaaaatcaa 2640
   cgggactttc caaaatgtcg taacaactcc gccccattga cgcaaatggg cggtaggcgt 2700
   gtacggtggg aggtctatat aagcagagct cgtttagtga accgtcagat cgcctggaga 2760
   cgccatccac gctgttttga cctccataga agacaccgac tctagctaga ggatccggac 2820
   tagtaactcg aggatgggga ctgacccggg ctgcaggaat tcataacttc gtataatgta 2880
   tgctatacga agttatagtt aagccttatt gtttatcatc ctcattcaac gacgcgaagg 2940
   tgtaccttgg cgagaataac ttcgtatagg atactttata cgaagttatt tatctgtgcc 3000
   ccagtttgct agggaggtcg cagtacttgg ccacagccat ctcgtgctgc tcgacgtagg 3060
   tctctttgtc ggcctccttg attctttcca gtctgtggtc cacgaagtgg aagccgggca 3120
   tcttgaggtt cttagcgggt ttcttggatc tgtatgtggt cttgaaggag cagtgcaggt 3180
   agcccccgcc cacgagcttc agggccatct ggctgtggcc tctcaggccg ccgtcagcgg 3240
   ggtacagcat ctcggtgttg gcctcccagc cgcgtgtttt cttctgcatc acagggccgt 3300
   tggatgggaa gttcaccccg ttgatcttga cgttgtagat gatgcagccg ttctggaagc 3360
   tggtgtcctg ggtagcggtc agcacgcccc cgtcttcgta tgtggtgatt ctctcccatg 3420
   tgaagccctc agggaaggac tgcttaaaga agtcggggat gccctgggtg tggttgatga 3480
   aggctttgct gccgtacatg aagctggtag ccaggatgtc gaaggcgaag gggagagggc 3540
   cgccctcgac caccttgatc ttcatggtct gggtgccctc gtagggcttg ccttcgccct 3600
   cggatgtgca cttgaagtgg tggttgttca cggtgccctc catgtacagc ttcatgtgca 3660
   tgttctcctt gatcagctcg ctcatggtgg catatgataa cttcgtatag catacattat 3720
   acgaagttat attaagggtt attgaatatg atcggaagtc aacgggtcga tggtgatgct 3780
   tggctcgaat aacttcgtat aaagtatcct atacgaagtt atactttggc cgcggctcga 3840
   gggggttggg gttgcgcctt ttccaaggca gccctgggtt tgcgcaggga cgcggctgct 3900
   ctgggcgtgg ttccgggaaa cgcagcggcg ccgaccctgg gtctcgcaca ttcttcacgt 3960
   ccgttcgcag cgtcacccgg atcttcgccg ctacccttgt gggccccccg gcgacgcttc 4020
   ctgctccgcc cctaagtcgg gaaggttcct tgcggttcgc ggcgtgccgg acgtgacaaa 4080
   cggaagccgc acgtctcact agtaccctcg cagacggaca gcgccaggga gcaatggcag 4140
   cgcgccgacc gcgatgggct gtggccaata gcggctgctc agcagggcgc gccgagagca 4200
   gcggccggga aggggcggtg cgggaggcgg ggtgtggggc ggtagtgtgg gccctgttcc 4260
   tgcccgcgcg gtgttccgca ttctgcaagc ctccggagcg cacgtcggca gtcggctccc 4320
   tcgttgaccg aatcaccgac ctctctcccc agggggatcc accggtcgcc accatggtga 4380
   gcaagggcga ggagctgttc accggggtgg tgcccatcct ggtcgagctg gacggcgacg 4440
   taaacggcca caagttcagc gtgtccggcg agggcgaggg cgatgccacc tacggcaagc 4500
   tgaccctgaa gttcatctgc accaccggca agctgcccgt gccctggccc accctcgtga 4560
   ccaccctgac ctacggcgtg cagtgcttca gccgctaccc cgaccacatg aagcagcacg 4620
   acttcttcaa gtccgccatg cccgaaggct acgtccagga gcgcaccatc ttcttcaagg 4680
   acgacggcaa ctacaagacc cgcgccgagg tgaagttcga gggcgacacc ctggtgaacc 4740
   gcatcgagct gaagggcatc gacttcaagg aggacggcaa catcctgggg cacaagctgg 4800
   agtacaacta caacagccac aacgtctata tcatggccga caagcagaag aacggcatca 4860
   aggtgaactt caagatccgc cacaacatcg aggacggcag cgtgcagctc gccgaccact 4920
   accagcagaa cacccccatc ggcgacggcc ccgtgctgct gcccgacaac cactacctga 4980
   gcacccagtc cgccctgagc aaagacccca acgagaagcg cgatcacatg gtcctgctgg 5040
   agttcgtgac cgccgccggg atcactctcg gcatggacga gctgtacaag taagatatca 5100
   agcttatcga taatcaacct ctggattaca aaatttgtga aagattgact ggtattctta 5160
   actatgttgc tccttttacg ctatgtggat acgctgcttt aatgcctttg tatcatgcta 5220
   ttgcttcccg tatggctttc attttctcct ccttgtataa atcctggttg ctgtctcttt 5280
   atgaggagtt gtggcccgtt gtcaggcaac gtggcgtggt gtgcactgtg tttgctgacg 5340
   caacccccac tggttggggc attgccacca cctgtcagct cctttccggg actttcgctt 5400
   tccccctccc tattgccacg gcggaactca tcgccgcctg ccttgcccgc tgctggacag 5460
   gggctcggct gttgggcact gacaattccg tggtgttgtc ggggaaatca tcgtcctttc 5520
   cttggctgct cgcctgtgtt gccacctgga ttctgcgcgg gacgtccttc tgctacgtcc 5580
   cttcggccct caatccagcg gaccttcctt cccgcggcct gctgccggct ctgcggcctc 5640
   ttccgcgtct tcgccttcgc cctcagacga gtcggatctc cctttgggcc gcctccccgc 5700
   atcgataccg tcgacctcga gaacctagaa aaacatggag caatcacaag tagcaataca 5760
   gcagctacca atgctgattg tgcctggcta gaagcacaag aggaggagga ggtgggtttt 5820
   ccagtcacac ctcaggtacc tttaagacca atgacttaca aggcagctgt agatcttagc 5880
   cactttttaa aagaaaaggg gggactggaa gggctaattc actcccaacg aagacaagat 5940
   ctttttgctt gtactgggtc tctctggtta gaccagatct gagcctggga gctctctggc 6000
   taactaggga acccactgct taagcctcaa taaagcttgc cttgagtgct tcaagtagtg 6060
   tgtgcccgtc tgttgtgtga ctctggtaac tagagatccc tcagaccctt ttagtcagtg 6120
   tggaaaatct ctaga 6135
<210> 37
   <211> 6022
   <212> DNA
   <213> Artificial Sequence
<220>
   <223> sequence for screening human cells
<400> 37
   gggctaattc actcccaaag aagacaagat atccttgatc tgtggatcta ccacacacaa      60
   ggctacttcc ctgattagca gaactacaca ccagggccag gggtcagata tccactgacc     120
   tttggatggt gctacaagct agtaccagtt gagccagata aggtagaaga ggccaataaa     180
   ggagagaaca ccagcttgtt acaccctgtg agcctgcatg ggatggatga cccggagaga     240
   gaagtgttag agtggaggtt tgacagccgc ctagcatttc atcacgtggc ccgagagctg     300
   catccggagt acttcaagaa ctgctgatat cgagcttgct acaagggact ttccgctggg     360
   gactttccag ggaggcgtgg cctgggcggg actggggagt ggcgagccct cagatcctgc     420
   atataagcag ctgctttttg cctgtactgg gtctctctgg ttagaccaga tctgagcctg     480
   ggagctctct ggctaactag ggaacccact gcttaagcct caataaagct tgccttgagt     540
   gcttcaagta gtgtgtgccc gtctgttgtg tgactctggt aactagagat ccctcagacc     600
   cttttagtca gtgtggaaaa tctctagcag tggcgcccga acagggactt gaaagcgaaa     660
   gggaaaccag aggagctctc tcgacgcagg actcggcttg ctgaagcgcg cacggcaaga     720
   ggcgaggggc ggcgactggt gagtacgcca aaaattttga ctagcggagg ctagaaggag     780
   agagatgggt gcgagagcgt cagtattaag cgggggagaa ttagatcgcg atgggaaaaa 840
   attcggttaa ggccaggggg aaagaaaaaa tataaattaa aacatatagt atgggcaagc 900
   agggagctag aacgattcgc agttaatcct ggcctgttag aaacatcaga aggctgtaga 960
   caaatactgg gacagctaca accatccctt cagacaggat cagaagaact tagatcatta 1020
   tataatacag tagcaaccct ctattgtgtg catcaaagga tagagataaa agacaccaag 1080
   gaagctttag acaagataga ggaagagcaa aacaaaagta agaccaccgc acagcaagcg 1140
   gccgctgatc ttcagacctg gaggaggaga tatgagggac aattggagaa gtgaattata 1200
   taaatataaa gtagtaaaaa ttgaaccatt aggagtagca cccaccaagg caaagagaag 1260
   agtggtgcag agagaaaaaa gagcagtggg aataggagct ttgttccttg ggttcttggg 1320
   agcagcagga agcactatgg gcgcagcgtc aatgacgctg acggtacagg ccagacaatt 1380
   attgtctggt atagtgcagc agcagaacaa tttgctgagg gctattgagg cgcaacagca 1440
   tctgttgcaa ctcacagtct ggggcatcaa gcagctccag gcaagaatcc tggctgtgga 1500
   aagataccta aaggatcaac agctcctggg gatttggggt tgctctggaa aactcatttg 1560
   caccactgct gtgccttgga atgctagttg gagtaataaa tctctggaac agatttggaa 1620
   tcacacgacc tggatggagt gggacagaga aattaacaat tacacaagct taatacactc 1680
   cttaattgaa gaatcgcaaa accagcaaga aaagaatgaa caagaattat tggaattaga 1740
   taaatgggca agtttgtgga attggtttaa cataacaaat tggctgtggt atataaaatt 1800
   attcataatg atagtaggag gcttggtagg tttaagaata gtttttgctg tactttctat 1860
   agtgaataga gttaggcagg gatattcacc attatcgttt cagacccacc tcccaacccc 1920
   gaggggaccc gacaggcccg aaggaataga agaagaaggt ggagagagag acagagacag 1980
   atccattcga ttagtgaacg gatctcgacg gtatcgccga attcacaaat ggcagtattc 2040
   atccacaatt ttaaaagaaa aggggggatt ggggggtaca gtgcagggga aagaatagta 2100
   gacataatag caacagacat acaaactaaa gaattacaaa aacaaattac aaaaattcaa 2160
   aattttcggg tttattacag ggacagcaga gatccacttt atcgatggtc cggtccggaa 2220
   ttcctgcagc cccgataaaa taaaagattt tatttagtct ccagaaaaag gggggaatga 2280
   aagaccccac ctgtaggttt ggcaagctag ctgcagtaac gccattttgc aaggcatgga 2340
   aaaataccaa accaagaata gagaagttca gatcaagggc gggtacatga aaatagctaa 2400
   cgttgggcca aacaggatat ctgcggtgag cagtttcggc cccggcccgg ggccaagaac 2460
   agatggtcac cgcagtttcg gccccggccc gaggccaaga acagatggtc cccagatatg 2520
   gcccaaccct cagcagtttc ttaagaccca tcagatgttt ccaggctccc ccaaggacct 2580
   gaaatgaccc tgcgccttat ttgaattaac caatcagcct gcttctcgct tctgttcgcg 2640
   cgcttctgct tcccgagctc tataaaagag ctcacaaccc ctcactcggc gcgccagtcc 2700
   tccgacagac tgagtcgccc gggggggatc caccgggctg caggaattca taacttcgta 2760
   taatgtatgc tatacgaagt tatagttaag ccttattgtt tatcatcctc attcaacgac 2820
   gcgaaggtgt accttggcga gaataacttc gtataggata ctttatacga agttatttat 2880
   ctgtgcccca gtttgctagg gaggtcgcag tacttggcca cagccatctc gtgctgctcg 2940
   acgtaggtct ctttgtcggc ctccttgatt ctttccagtc tgtggtccac gaagtggaag 3000
   ccgggcatct tgaggttctt agcgggtttc ttggatctgt atgtggtctt gaaggagcag 3060
   tgcaggtagc ccccgcccac gagcttcagg gccatctggc tgtggcctct caggccgccg 3120
   tcagcggggt acagcatctc ggtgttggcc tcccagccgc gtgttttctt ctgcatcaca 3180
   gggccgttgg atgggaagtt caccccgttg atcttgacgt tgtagatgat gcagccgttc 3240
   tggaagctgg tgtcctgggt agcggtcagc acgcccccgt cttcgtatgt ggtgattctc 3300
   tcccatgtga agccctcagg gaaggactgc ttaaagaagt cggggatgcc ctgggtgtgg 3360
   ttgatgaagg ctttgctgcc gtacatgaag ctggtagcca ggatgtcgaa ggcgaagggg 3420
   agagggccgc cctcgaccac cttgatcttc atggtctggg tgccctcgta gggcttgcct 3480
   tcgccctcgg atgtgcactt gaagtggtgg ttgttcacgg tgccctccat gtacagcttc 3540
   atgtgcatgt tctccttgat cagctcgctc atggtggcat atgataactt cgtatagcat 3600
   acattatacg aagttatatt aagggttatt gaatatgatc ggaagtcaac gggtcgatgg 3660
   tgatgcttgg ctcgaataac ttcgtataaa gtatcctata cgaagttata ctttggccgc 3720
   ggctcgaggg ggttggggtt gcgccttttc caaggcagcc ctgggtttgc gcagggacgc 3780
   ggctgctctg ggcgtggttc cgggaaacgc agcggcgccg accctgggtc tcgcacattc 3840
   ttcacgtccg ttcgcagcgt cacccggatc ttcgccgcta cccttgtggg ccccccggcg 3900
   acgcttcctg ctccgcccct aagtcgggaa ggttccttgc ggttcgcggc gtgccggacg 3960
   tgacaaacgg aagccgcacg tctcactagt accctcgcag acggacagcg ccagggagca 4020
   atggcagcgc gccgaccgcg atgggctgtg gccaatagcg gctgctcagc agggcgcgcc 4080
   gagagcagcg gccgggaagg ggcggtgcgg gaggcggggt gtggggcggt agtgtgggcc 4140
   ctgttcctgc ccgcgcggtg ttccgcattc tgcaagcctc cggagcgcac gtcggcagtc 4200
   ggctccctcg ttgaccgaat caccgacctc tctccccagg gggatccacc ggtcgccacc 4260
   atggtgagca agggcgagga gctgttcacc ggggtggtgc ccatcctggt cgagctggac 4320
   ggcgacgtaa acggccacaa gttcagcgtg tccggcgagg gcgagggcga tgccacctac 4380
   ggcaagctga ccctgaagtt catctgcacc accggcaagc tgcccgtgcc ctggcccacc 4440
   ctcgtgacca ccctgaccta cggcgtgcag tgcttcagcc gctaccccga ccacatgaag 4500
   cagcacgact tcttcaagtc cgccatgccc gaaggctacg tccaggagcg caccatcttc 4560
   ttcaaggacg acggcaacta caagacccgc gccgaggtga agttcgaggg cgacaccctg 4620
   gtgaaccgca tcgagctgaa gggcatcgac ttcaaggagg acggcaacat cctggggcac 4680
   aagctggagt acaactacaa cagccacaac gtctatatca tggccgacaa gcagaagaac 4740
   ggcatcaagg tgaacttcaa gatccgccac aacatcgagg acggcagcgt gcagctcgcc 4800
   gaccactacc agcagaacac ccccatcggc gacggccccg tgctgctgcc cgacaaccac 4860
   tacctgagca cccagtccgc cctgagcaaa gaccccaacg agaagcgcga tcacatggtc 4920
   ctgctggagt tcgtgaccgc cgccgggatc actctcggca tggacgagct gtacaagtaa 4980
   gatatcaagc ttatcgataa tcaacctctg gattacaaaa tttgtgaaag attgactggt 5040
   attcttaact atgttgctcc ttttacgcta tgtggatacg ctgctttaat gcctttgtat 5100
   catgctattg cttcccgtat ggctttcatt ttctcctcct tgtataaatc ctggttgctg 5160
   tctctttatg aggagttgtg gcccgttgtc aggcaacgtg gcgtggtgtg cactgtgttt 5220
   gctgacgcaa cccccactgg ttggggcatt gccaccacct gtcagctcct ttccgggact 5280
   ttcgctttcc ccctccctat tgccacggcg gaactcatcg ccgcctgcct tgcccgctgc 5340
   tggacagggg ctcggctgtt gggcactgac aattccgtgg tgttgtcggg gaaatcatcg 5400
   tcctttcctt ggctgctcgc ctgtgttgcc acctggattc tgcgcgggac gtccttctgc 5460
   tacgtccctt cggccctcaa tccagcggac cttccttccc gcggcctgct gccggctctg 5520
   cggcctcttc cgcgtcttcg ccttcgccct cagacgagtc ggatctccct ttgggccgcc 5580
   tccccgcatc gataccgtcg acctcgagaa cctagaaaaa catggagcaa tcacaagtag 5640
   caatacagca gctaccaatg ctgattgtgc ctggctagaa gcacaagagg aggaggaggt 5700
   gggttttcca gtcacacctc aggtaccttt aagaccaatg acttacaagg cagctgtaga 5760
   tcttagccac tttttaaaag aaaagggggg actggaaggg ctaattcact cccaacgaag 5820
   acaagatctt tttgcttgta ctgggtctct ctggttagac cagatctgag cctgggagct 5880
   ctctggctaa ctagggaacc cactgcttaa gcctcaataa agcttgcctt gagtgcttca 5940
   agtagtgtgt gcccgtctgt tgtgtgactc tggtaactag agatccctca gaccctttta 6000
   gtcagtgtgg aaaatctcta ga 6022
<210> 38
   <211> 6156
   <212> DNA
   <213> Artificial Sequence
<220>
   <223> sequence for screening human cells
<400> 38
   gggctaattc actcccaaag aagacaagat atccttgatc tgtggatcta ccacacacaa 60
   ggctacttcc ctgattagca gaactacaca ccagggccag gggtcagata tccactgacc 120
   tttggatggt gctacaagct agtaccagtt gagccagata aggtagaaga ggccaataaa 180
   ggagagaaca ccagcttgtt acaccctgtg agcctgcatg ggatggatga cccggagaga 240
   gaagtgttag agtggaggtt tgacagccgc ctagcatttc atcacgtggc ccgagagctg 300
   catccggagt acttcaagaa ctgctgatat cgagcttgct acaagggact ttccgctggg 360
   gactttccag ggaggcgtgg cctgggcggg actggggagt ggcgagccct cagatcctgc 420
   atataagcag ctgctttttg cctgtactgg gtctctctgg ttagaccaga tctgagcctg 480
   ggagctctct ggctaactag ggaacccact gcttaagcct caataaagct tgccttgagt 540
   gcttcaagta gtgtgtgccc gtctgttgtg tgactctggt aactagagat ccctcagacc 600
   cttttagtca gtgtggaaaa tctctagcag tggcgcccga acagggactt gaaagcgaaa 660
   gggaaaccag aggagctctc tcgacgcagg actcggcttg ctgaagcgcg cacggcaaga 720
   ggcgaggggc ggcgactggt gagtacgcca aaaattttga ctagcggagg ctagaaggag 780
   agagatgggt gcgagagcgt cagtattaag cgggggagaa ttagatcgcg atgggaaaaa 840
   attcggttaa ggccaggggg aaagaaaaaa tataaattaa aacatatagt atgggcaagc 900
   agggagctag aacgattcgc agttaatcct ggcctgttag aaacatcaga aggctgtaga 960
   caaatactgg gacagctaca accatccctt cagacaggat cagaagaact tagatcatta 1020
   tataatacag tagcaaccct ctattgtgtg catcaaagga tagagataaa agacaccaag 1080
   gaagctttag acaagataga ggaagagcaa aacaaaagta agaccaccgc acagcaagcg 1140
   gccgctgatc ttcagacctg gaggaggaga tatgagggac aattggagaa gtgaattata 1200
   taaatataaa gtagtaaaaa ttgaaccatt aggagtagca cccaccaagg caaagagaag 1260
   agtggtgcag agagaaaaaa gagcagtggg aataggagct ttgttccttg ggttcttggg 1320
   agcagcagga agcactatgg gcgcagcgtc aatgacgctg acggtacagg ccagacaatt 1380
   attgtctggt atagtgcagc agcagaacaa tttgctgagg gctattgagg cgcaacagca 1440
   tctgttgcaa ctcacagtct ggggcatcaa gcagctccag gcaagaatcc tggctgtgga 1500
   aagataccta aaggatcaac agctcctggg gatttggggt tgctctggaa aactcatttg 1560
   caccactgct gtgccttgga atgctagttg gagtaataaa tctctggaac agatttggaa 1620
   tcacacgacc tggatggagt gggacagaga aattaacaat tacacaagct taatacactc 1680
   cttaattgaa gaatcgcaaa accagcaaga aaagaatgaa caagaattat tggaattaga 1740
   taaatgggca agtttgtgga attggtttaa cataacaaat tggctgtggt atataaaatt 1800
   attcataatg atagtaggag gcttggtagg tttaagaata gtttttgctg tactttctat 1860
   agtgaataga gttaggcagg gatattcacc attatcgttt cagacccacc tcccaacccc 1920
   gaggggaccc gacaggcccg aaggaataga agaagaaggt ggagagagag acagagacag 1980
   atccattcga ttagtgaacg gatctcgacg gtatcgccga attcacaaat ggcagtattc 2040
   atccacaatt ttaaaagaaa aggggggatt ggggggtaca gtgcagggga aagaatagta 2100
   gacataatag caacagacat acaaactaaa gaattacaaa aacaaattac aaaaattcaa 2160
   aattttcggg tttattacag ggacagcaga gatccacttt atcgatggtc cggtccggaa 2220
   ttcctgcagc cccgataaaa taaaagattt tatttagtct ccagaaaaag gggggaatga 2280
   aagaccccac ctgtaggttt ggcaagctag ctgcagtaac gccattttgc aaggcatgga 2340
   aaaataccaa accaagaata gagaagttca gatcaagggc gggtacatga aaatagctaa 2400
   cgttgggcca aacaggatat ctgcggtgag cagtttcggc cccggcccgg ggccaagaac 2460
   agatggtcac cgcagtttcg gccccggccc gaggccaaga acagatggtc cccagatatg 2520
   gcccaaccct cagcagtttc ttaagaccca tcagatgttt ccaggctccc ccaaggacct 2580
   gaaatgaccc tgcgccttat ttgaattaac caatcagcct gcttctcgct tctgttcgcg 2640
   cgcttctgct tcccgagctc tataaaagag ctcacaaccc ctcactcggc gcgccagtcc 2700
   tccgacagac tgagtcgccc gggggggatc caccgggctg caggaattca taacttcgta 2760
   taatgtatgc tatacgaagt tatagttaag ccttattgtt tatcatcctc attcaacgac 2820
   gcgaaggtgt accttggcga gaataacttc gtataggata ctttatacga agttatttat 2880
   ctgtgcccca gtttgctagg gaggtcgcag tacttggcca cagccatctc gtgctgctcg 2940
   acgtaggtct ctttgtcggc ctccttgatt ctttccagtc tgtggtccac gaagtggaag 3000
   ccgggcatct tgaggttctt agcgggtttc ttggatctgt atgtggtctt gaaggagcag 3060
   tgcaggtagc ccccgcccac gagcttcagg gccatctggc tgtggcctct caggccgccg 3120
   tcagcggggt acagcatctc ggtgttggcc tcccagccgc gtgttttctt ctgcatcaca 3180
   gggccgttgg atgggaagtt caccccgttg atcttgacgt tgtagatgat gcagccgttc 3240
   tggaagctgg tgtcctgggt agcggtcagc acgcccccgt cttcgtatgt ggtgattctc 3300
   tcccatgtga agccctcagg gaaggactgc ttaaagaagt cggggatgcc ctgggtgtgg 3360
   ttgatgaagg ctttgctgcc gtacatgaag ctggtagcca ggatgtcgaa ggcgaagggg 3420
   agagggccgc cctcgaccac cttgatcttc atggtctggg tgccctcgta gggcttgcct 3480
   tcgccctcgg atgtgcactt gaagtggtgg ttgttcacgg tgccctccat gtacagcttc 3540
   atgtgcatgt tctccttgat cagctcgctc atggtggcat atgataactt cgtatagcat 3600
   acattatacg aagttatatt aagggttatt gaatatgatc ggaagtcaac gggtcgatgg 3660
   tgatgcttgg ctcgaataac ttcgtataaa gtatcctata cgaagttata ctttggccgc 3720
   ggctcgaatc tacttaccga taagcttggg agttccgcgt tacataactt acggtaaatg 3780
   gcccgcctgg ctgaccgccc aacgaccccc gcccattgac gtcaataatg acgtatgttc 3840
   ccatagtaac gccaataggg actttccatt gacgtcaatg ggtggagtat ttacggtaaa 3900
   ctgcccactt ggcagtacat caagtgtatc atatgccaag tacgccccct attgacgtca 3960
   atgacggtaa atggcccgcc tggcattatg cccagtacat gaccttacgg gactttccta 4020
   cttggcagta catctacgta ttagtcatcg ctattaccat ggtgatgcgg ttttggcagt 4080
   acaccaatgg gcgtggatag cggtttgact cacggggatt tccaagtctc caccccattg 4140
   acgtcaatgg gagtttgttt tggcaccaaa atcaacggga ctttccaaaa tgtcgtaaca 4200
   actccgcccc attgacgcaa atgggcggta ggcgtgtacg gtgggaggtc tatataagca 4260
   gagctcgttt agtgaaccgt cagatcgcct ggagacgcca tccacgctgt tttgacctcc 4320
   atagaagaca ccgactctag ctagaggatc cggactagta actcgaggat ggggactgac 4380
   ccggacgcgt gccgccacca tggtgagcaa gggcgaggag ctgttcaccg gggtggtgcc 4440
   catcctggtc gagctggacg gcgacgtaaa cggccacaag ttcagcgtgt ccggcgaggg 4500
   cgagggcgat gccacctacg gcaagctgac cctgaagttc atctgcacca ccggcaagct 4560
   gcccgtgccc tggcccaccc tcgtgaccac cctgacctac ggcgtgcagt gcttcagccg 4620
   ctaccccgac cacatgaagc agcacgactt cttcaagtcc gccatgcccg aaggctacgt 4680
   ccaggagcgc accatcttct tcaaggacga cggcaactac aagacccgcg ccgaggtgaa 4740
   gttcgagggc gacaccctgg tgaaccgcat cgagctgaag ggcatcgact tcaaggagga 4800
   cggcaacatc ctggggcaca agctggagta caactacaac agccacaacg tctatatcat 4860
   ggccgacaag cagaagaacg gcatcaaggt gaacttcaag atccgccaca acatcgagga 4920
   cggcagcgtg cagctcgccg accactacca gcagaacacc cccatcggcg acggccccgt 4980
   gctgctgccc gacaaccact acctgagcac ccagtccgcc ctgagcaaag accccaacga 5040
   gaagcgcgat cacatggtcc tgctggagtt cgtgaccgcc gccgggatca ctctcggcat 5100
   ggacgagctg tacaagtaag atatcaagct tatcgataat caacctctgg attacaaaat 5160
   ttgtgaaaga ttgactggta ttcttaacta tgttgctcct tttacgctat gtggatacgc 5220
   tgctttaatg cctttgtatc atgctattgc ttcccgtatg gctttcattt tctcctcctt 5280
   gtataaatcc tggttgctgt ctctttatga ggagttgtgg cccgttgtca ggcaacgtgg 5340
   cgtggtgtgc actgtgtttg ctgacgcaac ccccactggt tggggcattg ccaccacctg 5400
   tcagctcctt tccgggactt tcgctttccc cctccctatt gccacggcgg aactcatcgc 5460
   cgcctgcctt gcccgctgct ggacaggggc tcggctgttg ggcactgaca attccgtggt 5520
   gttgtcgggg aaatcatcgt cctttccttg gctgctcgcc tgtgttgcca cctggattct 5580
   gcgcgggacg tccttctgct acgtcccttc ggccctcaat ccagcggacc ttccttcccg 5640
   cggcctgctg ccggctctgc ggcctcttcc gcgtcttcgc cttcgccctc agacgagtcg 5700
   gatctccctt tgggccgcct ccccgcatcg ataccgtcga cctcgagaac ctagaaaaac 5760
   atggagcaat cacaagtagc aatacagcag ctaccaatgc tgattgtgcc tggctagaag    5820
   cacaagagga ggaggaggtg ggttttccag tcacacctca ggtaccttta agaccaatga    5880
   cttacaaggc agctgtagat cttagccact ttttaaaaga aaagggggga ctggaagggc    5940
   taattcactc ccaacgaaga caagatcttt ttgcttgtac tgggtctctc tggttagacc    6000
   agatctgagc ctgggagctc tctggctaac tagggaaccc actgcttaag cctcaataaa    6060
   gcttgccttg agtgcttcaa gtagtgtgtg cccgtctgtt gtgtgactct ggtaactaga    6120
   gatccctcag acccttttag tcagtgtgga aaatct                              6156
<210> 39
   <211> 7092
   <212> DNA
   <213> Artificial Sequence
<220>
   <223> sequence for screening human cells
<400> 39
   gggctaattc actcccaaag aagacaagat atccttgatc tgtggatcta ccacacacaa      60
   ggctacttcc ctgattagca gaactacaca ccagggccag gggtcagata tccactgacc     120
   tttggatggt gctacaagct agtaccagtt gagccagata aggtagaaga ggccaataaa     180
   ggagagaaca ccagcttgtt acaccctgtg agcctgcatg ggatggatga cccggagaga     240
   gaagtgttag agtggaggtt tgacagccgc ctagcatttc atcacgtggc ccgagagctg     300
   catccggagt acttcaagaa ctgctgatat cgagcttgct acaagggact ttccgctggg     360
   gactttccag ggaggcgtgg cctgggcggg actggggagt ggcgagccct cagatcctgc     420
   atataagcag ctgctttttg cctgtactgg gtctctctgg ttagaccaga tctgagcctg     480
   ggagctctct ggctaactag ggaacccact gcttaagcct caataaagct tgccttgagt     540
   gcttcaagta gtgtgtgccc gtctgttgtg tgactctggt aactagagat ccctcagacc     600
   cttttagtca gtgtggaaaa tctctagcag tggcgcccga acagggactt gaaagcgaaa     660
   gggaaaccag aggagctctc tcgacgcagg actcggcttg ctgaagcgcg cacggcaaga     720
   ggcgaggggc ggcgactggt gagtacgcca aaaattttga ctagcggagg ctagaaggag     780
   agagatgggt gcgagagcgt cagtattaag cgggggagaa ttagatcgcg atgggaaaaa     840
   attcggttaa ggccaggggg aaagaaaaaa tataaattaa aacatatagt atgggcaagc     900
   agggagctag aacgattcgc agttaatcct ggcctgttag aaacatcaga aggctgtaga     960
   caaatactgg gacagctaca accatccctt cagacaggat cagaagaact tagatcatta    1020
   tataatacag tagcaaccct ctattgtgtg catcaaagga tagagataaa agacaccaag    1080
   gaagctttag acaagataga ggaagagcaa aacaaaagta agaccaccgc acagcaagcg    1140
   gccgctgatc ttcagacctg gaggaggaga tatgagggac aattggagaa gtgaattata 1200
   taaatataaa gtagtaaaaa ttgaaccatt aggagtagca cccaccaagg caaagagaag 1260
   agtggtgcag agagaaaaaa gagcagtggg aataggagct ttgttccttg ggttcttggg 1320
   agcagcagga agcactatgg gcgcagcgtc aatgacgctg acggtacagg ccagacaatt 1380
   attgtctggt atagtgcagc agcagaacaa tttgctgagg gctattgagg cgcaacagca 1440
   tctgttgcaa ctcacagtct ggggcatcaa gcagctccag gcaagaatcc tggctgtgga 1500
   aagataccta aaggatcaac agctcctggg gatttggggt tgctctggaa aactcatttg 1560
   caccactgct gtgccttgga atgctagttg gagtaataaa tctctggaac agatttggaa 1620
   tcacacgacc tggatggagt gggacagaga aattaacaat tacacaagct taatacactc 1680
   cttaattgaa gaatcgcaaa accagcaaga aaagaatgaa caagaattat tggaattaga 1740
   taaatgggca agtttgtgga attggtttaa cataacaaat tggctgtggt atataaaatt 1800
   attcataatg atagtaggag gcttggtagg tttaagaata gtttttgctg tactttctat 1860
   agtgaataga gttaggcagg gatattcacc attatcgttt cagacccacc tcccaacccc 1920
   gaggggaccc gacaggcccg aaggaataga agaagaaggt ggagagagag acagagacag 1980
   atccattcga ttagtgaacg gatctcgacg gtatcgccga attcacaaat ggcagtattc 2040
   atccacaatt ttaaaagaaa aggggggatt ggggggtaca gtgcagggga aagaatagta 2100
   gacataatag caacagacat acaaactaaa gaattacaaa aacaaattac aaaaattcaa 2160
   aattttcggg tttattacag ggacagcaga gatccacttt atcgataagc ttgggagttc 2220
   cgcgttacat aacttacggt aaatggcccg cctggctgac cgcccaacga cccccgccca 2280
   ttgacgtcaa taatgacgta tgttcccata gtaacgccaa tagggacttt ccattgacgt 2340
   caatgggtgg agtatttacg gtaaactgcc cacttggcag tacatcaagt gtatcatatg 2400
   ccaagtacgc cccctattga cgtcaatgac ggtaaatggc ccgcctggca ttatgcccag 2460
   tacatgacct tacgggactt tcctacttgg cagtacatct acgtattagt catcgctatt 2520
   accatggtga tgcggttttg gcagtacacc aatgggcgtg gatagcggtt tgactcacgg 2580
   ggatttccaa gtctccaccc cattgacgtc aatgggagtt tgttttggca ccaaaatcaa 2640
   cgggactttc caaaatgtcg taacaactcc gccccattga cgcaaatggg cggtaggcgt 2700
   gtacggtggg aggtctatat aagcagagct cgtttagtga accgtcagat cgcctggaga 2760
   cgccatccac gctgttttga cctccataga agacaccgac tctagctaga ggatccggac 2820
   tagtaactcg aggatgggga ctgacccggg ctgcaggaat tcataacttc gtataatgta 2880
   tgctatacga agttatagtt aagccttatt gtttatcatc ctcattcaac gacgcgaagg 2940
   tgtaccttgg cgagaataac ttcgtatagg atactttata cgaagttatt tacacggcga 3000
   tctttccgcc cttcttggcc tttatgagga tctctctgat ttttcttgcg tcgagttttc 3060
   cggtaagacc tttcggtact tcgtccacaa acacaactcc tccgcgcaac tttttcgcgg 3120
   ttgttacttg actggcgacg taatccacga tctctttttc cgtcatcgtc tttccgtgct 3180
   ccaaaacaac aacggcggcg ggaagttcac cggcgtcatc gtcgggaaga cctgcgacac 3240
   ctgcgtcgaa gatgttgggg tgttggagca agatggattc caattcagcg ggagccacct 3300
   gatagccttt gtacttaatc agagacttca ggcggtcaac gatgaagaag tgttcgtctt 3360
   cgtcccagta agctatgtct ccagaatgta gccatccatc cttgtcaatc aaggcgttgg 3420
   tcgcttccgg attgtttaca taaccggaca taatcatagg acctctcaca cacagttcgc 3480
   ctctttgatt aacgcccagc gttttcccgg tatccagatc cacaaccttc gcttcaaaaa 3540
   atggaacaac tttaccgacc gcgcccggtt tatcatcccc ctcgggtgta atcagaatag 3600
   ctgatgtagt ctcagtgagc ccatatcctt gcctgatacc tggcagatgg aacctcttgg 3660
   caaccgcttc cccgacttcc ttagagaggg gagcgccacc agaagcaatt tcgtgtaaat 3720
   tagataaatc gtatttgtca atcagagtgc ttttggcgaa gaaggagaat agggttggca 3780
   ccagcagcgc actttgaatc ttgtaatcct gaaggctcct cagaaacagc tcttcttcaa 3840
   atctatacat taagacgact cgaaatccac atatcaaata tccgagtgta gtaaacattc 3900
   caaaaccgtg atggaatgga acaacactta aaatcgcagt atccggaatg atttgattgc 3960
   caaaaatagg atctctggca tgcgagaatc tcacgcaggc agttctatga ggcagagcga 4020
   cacctttagg cagaccagta gatccagagg agttcatgat cagtgcaatt gtcttgtccc 4080
   tatcgaagga ctctggcaca aaatcgtatt cattaaaacc gggaggtaga tgagatgtga 4140
   cgaacgtgta catcgactga aatccctggt aatccgtttt agaatccatg ataataattt 4200
   tttggatgat tgggagcttt ttttgcacgt tcaaaatttt ttgcaacccc tttttggaaa 4260
   cgaacaccac ggtaggctgc gaaatgccca tactgttgag caattcacgt tcattataaa 4320
   tgtcgttcgc gggcgcaact gcaactccga taaataacgc gcccaacacc ggcataaaga 4380
   attgaagaga gttttcactg catacgacga ttctgtgatt tgtattcagc ccatatcgtt 4440
   tcatagcttc tgccaaccga acggacattt cgaagtactc agcgtaagtg atgtccacct 4500
   cgatatgtgc atctgtaaaa gcaattgttc caggaaccag ggcgtatctc ttcatagcct 4560
   tatgcagttg ctctccagcg gttccatctt ccagcggata gaatggcgcc gggcctttct 4620
   ttatgttttt ggcgtcttcc atggtggcat atgataactt cgtatagcat acattatacg 4680
   aagttatatt aagggttatt gaatatgatc ggaagtcaac gggtcgatgg tgatgcttgg 4740
   ctcgaataac ttcgtataaa gtatcctata cgaagttata ctttggccgc ggctcgaggg 4800
   ggttggggtt gcgccttttc caaggcagcc ctgggtttgc gcagggacgc ggctgctctg 4860
   ggcgtggttc cgggaaacgc agcggcgccg accctgggtc tcgcacattc ttcacgtccg 4920
   ttcgcagcgt cacccggatc ttcgccgcta cccttgtggg ccccccggcg acgcttcctg     4980
   ctccgcccct aagtcgggaa ggttccttgc ggttcgcggc gtgccggacg tgacaaacgg     5040
   aagccgcacg tctcactagt accctcgcag acggacagcg ccagggagca atggcagcgc     5100
   gccgaccgcg atgggctgtg gccaatagcg gctgctcagc agggcgcgcc gagagcagcg     5160
   gccgggaagg ggcggtgcgg gaggcggggt gtggggcggt agtgtgggcc ctgttcctgc     5220
   ccgcgcggtg ttccgcattc tgcaagcctc cggagcgcac gtcggcagtc ggctccctcg     5280
   ttgaccgaat caccgacctc tctccccagg gggatccacc ggtcgccacc atggtgagca     5340
   agggcgagga gctgttcacc ggggtggtgc ccatcctggt cgagctggac ggcgacgtaa     5400
   acggccacaa gttcagcgtg tccggcgagg gcgagggcga tgccacctac ggcaagctga     5460
   ccctgaagtt catctgcacc accggcaagc tgcccgtgcc ctggcccacc ctcgtgacca     5520
   ccctgaccta cggcgtgcag tgcttcagcc gctaccccga ccacatgaag cagcacgact     5580
   tcttcaagtc cgccatgccc gaaggctacg tccaggagcg caccatcttc ttcaaggacg     5640
   acggcaacta caagacccgc gccgaggtga agttcgaggg cgacaccctg gtgaaccgca     5700
   tcgagctgaa gggcatcgac ttcaaggagg acggcaacat cctggggcac aagctggagt     5760
   acaactacaa cagccacaac gtctatatca tggccgacaa gcagaagaac ggcatcaagg     5820
   tgaacttcaa gatccgccac aacatcgagg acggcagcgt gcagctcgcc gaccactacc     5880
   agcagaacac ccccatcggc gacggccccg tgctgctgcc cgacaaccac tacctgagca     5940
   cccagtccgc cctgagcaaa gaccccaacg agaagcgcga tcacatggtc ctgctggagt     6000
   tcgtgaccgc cgccgggatc actctcggca tggacgagct gtacaagtaa gatatcaagc     6060
   ttatcgataa tcaacctctg gattacaaaa tttgtgaaag attgactggt attcttaact     6120
   atgttgctcc ttttacgcta tgtggatacg ctgctttaat gcctttgtat catgctattg     6180
   cttcccgtat ggctttcatt ttctcctcct tgtataaatc ctggttgctg tctctttatg     6240
   aggagttgtg gcccgttgtc aggcaacgtg gcgtggtgtg cactgtgttt gctgacgcaa     6300
   cccccactgg ttggggcatt gccaccacct gtcagctcct ttccgggact ttcgctttcc     6360
   ccctccctat tgccacggcg gaactcatcg ccgcctgcct tgcccgctgc tggacagggg     6420
   ctcggctgtt gggcactgac aattccgtgg tgttgtcggg gaaatcatcg tcctttcctt     6480
   ggctgctcgc ctgtgttgcc acctggattc tgcgcgggac gtccttctgc tacgtccctt     6540
   cggccctcaa tccagcggac cttccttccc gcggcctgct gccggctctg cggcctcttc     6600
   cgcgtcttcg ccttcgccct cagacgagtc ggatctccct ttgggccgcc tccccgcatc     6660
   gataccgtcg acctcgagaa cctagaaaaa catggagcaa tcacaagtag caatacagca     6720
   gctaccaatg ctgattgtgc ctggctagaa gcacaagagg aggaggaggt gggttttcca     6780
   gtcacacctc aggtaccttt aagaccaatg acttacaagg cagctgtaga tcttagccac     6840
   tttttaaaag aaaagggggg actggaaggg ctaattcact cccaacgaag acaagatctt 6900
   tttgcttgta ctgggtctct ctggttagac cagatctgag cctgggagct ctctggctaa 6960
   ctagggaacc cactgcttaa gcctcaataa agcttgcctt gagtgcttca agtagtgtgt 7020
   gcccgtctgt tgtgtgactc tggtaactag agatccctca gaccctttta gtcagtgtgg 7080
   aaaatctcta ga 7092
<210> 40
   <211> 5672
   <212> DNA
   <213> Artificial Sequence
<220>
   <223> sequence for screening simian cells
<400> 40
   cctagtcgcc gcctggtcaa ctcggtactc ggtaataaga agaccctggt ctgttaggac 60
   cctttctgct ttgagaaacc gaagcaggaa aatccctagc agattggcgc ccgaacaggg 120
   acttgaagga gagtgagaga ctcctgagta cggctgagtg aaggcagtaa gggcggcagg 180
   aaccaaccac gacggagtgc tcctataaag gcgcgggtcg gtaccagacg gcgtgaggag 240
   cgggagagga ggaggcctcc ggttgcagta agtgcaacac aaaaaagaaa tagctgtctt 300
   gttatccagg aagggataat aagatagagt gggagatggg cgcgagaaac tccgtcttgt 360
   cagggaagaa agcagatgaa ttgaaaaaat tattaatcgc atgaatttta aaagaagggg 420
   aggaataggg gatatgactc cagcagaaag attaattaac atgatcacta cagaacaaga 480
   aatacaattt caacaatcaa aaaactcaaa atttaaaaat tttcgggtct attacagagc 540
   tcacgcgtga ttggagttgg gagattataa attagtagag atcactccga ttggcttggc 600
   ccccacagat gtgaagaggt acactactgg tggcacctca agaaataaaa gaggggtctt 660
   tgtgctaggg ttcttgggtt ttctcgcaac ggcaggttct gcaatgggcg cggcgtcgtt 720
   gacgctgacc gctcagtccc ggactttatt ggctgggata gtgcagcaac agcaacagct 780
   gttggacgtg gtcaagagac aacaagaatt gttgcgactg accgtctggg gaacaaagaa 840
   cctccagact agggtcactg ccatcgagaa gtacttaaag gaccaggcgc agctaaatgc 900
   ttggggatgt gcgtttagac aagtctgcca cactactgta ccatggccaa atgcaagtct 960
   aacaccagac tggaacaatg atacttggca agagtgggag cgaaaggttg acttcttgga 1020
   ggaaaatata acagccctcc tagaagaggc acaaattcaa caagagaaga acatgtatga 1080
   attacaaaag ttgaatagct gggatgtgtt tggcaattgg tttgaccttg cttcttggat 1140
   aaagtatata caatatggaa tttatgtagt tgtaggagta atactgttaa gaatagtgat 1200
   ctatatagta caaatgctag ctaagttaag gcaggggtat aggccagtgt tctcttcccc 1260
   accctcttat ttccagtaga ctcataccca acaggacccg gcactgccaa ccagagaagg 1320
   caaagaagga gacggtggag aaggcggtgg atcctattat cgaattcctg cagccccgat 1380
   aaaataaaag attttattta gtctccagaa aaagggggga atgaaagacc ccacctgtag 1440
   gtttggcaag ctagctgcag taacgccatt ttgcaaggca tggaaaaata ccaaaccaag 1500
   aatagagaag ttcagatcaa gggcgggtac atgaaaatag ctaacgttgg gccaaacagg 1560
   atatctgcgg tgagcagttt cggccccggc ccggggccaa gaacagatgg tcaccgcagt 1620
   ttcggccccg gcccgaggcc aagaacagat ggtccccaga tatggcccaa ccctcagcag 1680
   tttcttaaga cccatcagat gtttccaggc tcccccaagg acctgaaatg accctgcgcc 1740
   ttatttgaat taaccaatca gcctgcttct cgcttctgtt cgcgcgcttc tgcttcccga 1800
   gctctataaa agagctcaca acccctcact cggcgcgcca gtcctccgac agactgagtc 1860
   gcccgggggg gatccaccgg gctgcaggaa ttcgatatca agcttatcga tgattctaga 1920
   catgcatatc tagcgtcccg ggctgcagga attcataact tcgtataatg tatgctatac 1980
   gaagttatag ttaagcctta ttgtttatca tcctcattca acgacgcgaa ggtgtacctt 2040
   ggcgagaata acttcgtata ggatacttta tacgaagtta tttatctgtg ccccagtttg 2100
   ctagggaggt cgcagtactt ggccacagcc atctcgtgct gctcgacgta ggtctctttg 2160
   tcggcctcct tgattctttc cagtctgtgg tccacgaagt ggaagccggg catcttgagg 2220
   ttcttagcgg gtttcttgga tctgtatgtg gtcttgaagg agcagtgcag gtagcccccg 2280
   cccacgagct tcagggccat ctggctgtgg cctctcaggc cgccgtcagc ggggtacagc 2340
   atctcggtgt tggcctccca gccgcgtgtt ttcttctgca tcacagggcc gttggatggg 2400
   aagttcaccc cgttgatctt gacgttgtag atgatgcagc cgttctggaa gctggtgtcc 2460
   tgggtagcgg tcagcacgcc cccgtcttcg tatgtggtga ttctctccca tgtgaagccc 2520
   tcagggaagg actgcttaaa gaagtcgggg atgccctggg tgtggttgat gaaggctttg 2580
   ctgccgtaca tgaagctggt agccaggatg tcgaaggcga aggggagagg gccgccctcg 2640
   accaccttga tcttcatggt ctgggtgccc tcgtagggct tgccttcgcc ctcggatgtg 2700
   cacttgaagt ggtggttgtt cacggtgccc tccatgtaca gcttcatgtg catgttctcc 2760
   ttgatcagct cgctcatggt ggcatatgat aacttcgtat agcatacatt atacgaagtt 2820
   atattaaggg ttattgaata tgatcggaag tcaacgggtc gatggtgatg cttggctcga 2880
   ataacttcgt ataaagtatc ctatacgaag ttatactttg gccgcggctc gagggggttg 2940
   gggttgcgcc ttttccaagg cagccctggg tttgcgcagg gacgcggctg ctctgggcgt 3000
   ggttccggga aacgcagcgg cgccgaccct gggtctcgca cattcttcac gtccgttcgc 3060
   agcgtcaccc ggatcttcgc cgctaccctt gtgggccccc cggcgacgct tcctgctccg 3120
   cccctaagtc gggaaggttc cttgcggttc gcggcgtgcc ggacgtgaca aacggaagcc 3180
   gcacgtctca ctagtaccct cgcagacgga cagcgccagg gagcaatggc agcgcgccga 3240
   ccgcgatggg ctgtggccaa tagcggctgc tcagcagggc gcgccgagag cagcggccgg 3300
   gaaggggcgg tgcgggaggc ggggtgtggg gcggtagtgt gggccctgtt cctgcccgcg 3360
   cggtgttccg cattctgcaa gcctccggag cgcacgtcgg cagtcggctc cctcgttgac 3420
   cgaatcaccg acctctctcc ccagggggat ccaccggtcg ccaccatggt gagcaagggc 3480
   gaggagctgt tcaccggggt ggtgcccatc ctggtcgagc tggacggcga cgtaaacggc 3540
   cacaagttca gcgtgtccgg cgagggcgag ggcgatgcca cctacggcaa gctgaccctg 3600
   aagttcatct gcaccaccgg caagctgccc gtgccctggc ccaccctcgt gaccaccctg 3660
   acctacggcg tgcagtgctt cagccgctac cccgaccaca tgaagcagca cgacttcttc 3720
   aagtccgcca tgcccgaagg ctacgtccag gagcgcacca tcttcttcaa ggacgacggc 3780
   aactacaaga cccgcgccga ggtgaagttc gagggcgaca ccctggtgaa ccgcatcgag 3840
   ctgaagggca tcgacttcaa ggaggacggc aacatcctgg ggcacaagct ggagtacaac 3900
   tacaacagcc acaacgtcta tatcatggcc gacaagcaga agaacggcat caaggtgaac 3960
   ttcaagatcc gccacaacat cgaggacggc agcgtgcagc tcgccgacca ctaccagcag 4020
   aacaccccca tcggcgacgg ccccgtgctg ctgcccgaca accactacct gagcacccag 4080
   tccgccctga gcaaagaccc caacgagaag cgcgatcaca tggtcctgct ggagttcgtg 4140
   accgccgccg ggatcactct cggcatggac gagctgtaca agtaaagcgg ccgcgactct 4200
   agagtcgacc tgcaggcatg caagcttgat atcaagctta tcgataatca acctctggat 4260
   tacaaaattt gtgaaagatt gactggtatt cttaactatg ttgctccttt tacgctatgt 4320
   ggatacgctg ctttaatgcc tttgtatcat gctattgctt cccgtatggc tttcattttc 4380
   tcctccttgt ataaatcctg gttgctgtct ctttatgagg agttgtggcc cgttgtcagg 4440
   caacgtggcg tggtgtgcac tgtgtttgct gacgcaaccc ccactggttg gggcattgcc 4500
   accacctgtc agctcctttc cgggactttc gctttccccc tccctattgc cacggcggaa 4560
   ctcatcgccg cctgccttgc ccgctgctgg acaggggctc ggctgttggg cactgacaat 4620
   tccgtggtgt tgtcggggaa atcatcgtcc tttccttggc tgctcgcctg tgttgccacc 4680
   tggattctgc gcgggacgtc cttctgctac gtcccttcgg ccctcaatcc agcggacctt 4740
   ccttcccgcg gcctgctgcc ggctctgcgg cctcttccgc gtcttcgcct tcgccctcag 4800
   acgagtcgga tctccctttg ggccgcctcc ccgcatcgat accgtcgacc tcgagtttta 4860
   taaaagaaaa ggggggactg gaagggattt attacagtgc aagaagacat agaatcttag 4920
   acatgtactt agaaaaggaa gaaggcatca taccagattg gcaggattac acctcaggac 4980
   caggaattag atacccaaag acatttggct ggctatggaa attagtccct gtaaatgtat 5040
   cagatgaggc acaggaggat gagaggcatt atttaatgca gccagctcaa acttccaagt 5100
   gggatgaccc ttggggagag gttctagcgt ggaagtttga tccaactcta gcctacactt 5160
   atgaggcata tgctagatac ccagaagagt tggaagcaag tcaggcctgt cagaactgca 5220
   tttcgctctg tattcagtcg ctctgcggag aggctggcag attgagccct gggaggttct 5280
   ctccagcact agcaggtaga gcctgggtgt tccctgctag actctcacca gcacttggcc 5340
   agtgctgggc agagtggctc cacgcttgct tgcttaaaga cctcttcaat aaagctgcca 5400
   ttttagaagt aagccagtgt gtgttcccat ctctcctagt cgccgcctgg tcaactcggt 5460
   actcggtaat aagaagaccc tggtctgtta ggaccctttc tgctttgaga aaccgaagca 5520
   ggaaaatccc tagcatggta cccagctttt gttcccttta gtgagggtta attccgagct 5580
   tggcgtaatc atggtcatag ctgtttcctg tgtgaaattg ttatccgctc acaattccac 5640
   acaacatacg agccggaagc ataaagtgta aa 5672
<210> 41
   <211> 5950
   <212> DNA
   <213> Artificial Sequence
<220>
   <223> sequence for screening human cells floxed
<400> 41
   gggctaattc actcccaaag aagacaagat atccttgatc tgtggatcta ccacacacaa      60
   ggctacttcc ctgattagca gaactacaca ccagggccag gggtcagata tccactgacc     120
   tttggatggt gctacaagct agtaccagtt gagccagata aggtagaaga ggccaataaa     180
   ggagagaaca ccagcttgtt acaccctgtg agcctgcatg ggatggatga cccggagaga     240
   gaagtgttag agtggaggtt tgacagccgc ctagcatttc atcacgtggc ccgagagctg     300
   catccggagt acttcaagaa ctgctgatat cgagcttgct acaagggact ttccgctggg     360
   gactttccag ggaggcgtgg cctgggcggg actggggagt ggcgagccct cagatcctgc     420
   atataagcag ctgctttttg cctgtactgg gtctctctgg ttagaccaga tctgagcctg     480
   ggagctctct ggctaactag ggaacccact gcttaagcct caataaagct tgccttgagt     540
   gcttcaagta gtgtgtgccc gtctgttgtg tgactctggt aactagagat ccctcagacc     600
   cttttagtca gtgtggaaaa tctctagcag tggcgcccga acagggactt gaaagcgaaa     660
   gggaaaccag aggagctctc tcgacgcagg actcggcttg ctgaagcgcg cacggcaaga     720
   ggcgaggggc ggcgactggt gagtacgcca aaaattttga ctagcggagg ctagaaggag     780
   agagatgggt gcgagagcgt cagtattaag cgggggagaa ttagatcgcg atgggaaaaa     840
   attcggttaa ggccaggggg aaagaaaaaa tataaattaa aacatatagt atgggcaagc     900
   agggagctag aacgattcgc agttaatcct ggcctgttag aaacatcaga aggctgtaga     960
   caaatactgg gacagctaca accatccctt cagacaggat cagaagaact tagatcatta 1020
   tataatacag tagcaaccct ctattgtgtg catcaaagga tagagataaa agacaccaag 1080
   gaagctttag acaagataga ggaagagcaa aacaaaagta agaccaccgc acagcaagcg 1140
   gccgctgatc ttcagacctg gaggaggaga tatgagggac aattggagaa gtgaattata 1200
   taaatataaa gtagtaaaaa ttgaaccatt aggagtagca cccaccaagg caaagagaag 1260
   agtggtgcag agagaaaaaa gagcagtggg aataggagct ttgttccttg ggttcttggg 1320
   agcagcagga agcactatgg gcgcagcgtc aatgacgctg acggtacagg ccagacaatt 1380
   attgtctggt atagtgcagc agcagaacaa tttgctgagg gctattgagg cgcaacagca 1440
   tctgttgcaa ctcacagtct ggggcatcaa gcagctccag gcaagaatcc tggctgtgga 1500
   aagataccta aaggatcaac agctcctggg gatttggggt tgctctggaa aactcatttg 1560
   caccactgct gtgccttgga atgctagttg gagtaataaa tctctggaac agatttggaa 1620
   tcacacgacc tggatggagt gggacagaga aattaacaat tacacaagct taatacactc 1680
   cttaattgaa gaatcgcaaa accagcaaga aaagaatgaa caagaattat tggaattaga 1740
   taaatgggca agtttgtgga attggtttaa cataacaaat tggctgtggt atataaaatt 1800
   attcataatg atagtaggag gcttggtagg tttaagaata gtttttgctg tactttctat 1860
   agtgaataga gttaggcagg gatattcacc attatcgttt cagacccacc tcccaacccc 1920
   gaggggaccc gacaggcccg aaggaataga agaagaaggt ggagagagag acagagacag 1980
   atccattcga ttagtgaacg gatctcgacg gtatcgccga attcacaaat ggcagtattc 2040
   atccacaatt ttaaaagaaa aggggggatt ggggggtaca gtgcagggga aagaatagta 2100
   gacataatag caacagacat acaaactaaa gaattacaaa aacaaattac aaaaattcaa 2160
   aattttcggg tttattacag ggacagcaga gatccacttt atcgataagc ttgggagttc 2220
   cgcgttacat aacttacggt aaatggcccg cctggctgac cgcccaacga cccccgccca 2280
   ttgacgtcaa taatgacgta tgttcccata gtaacgccaa tagggacttt ccattgacgt 2340
   caatgggtgg agtatttacg gtaaactgcc cacttggcag tacatcaagt gtatcatatg 2400
   ccaagtacgc cccctattga cgtcaatgac ggtaaatggc ccgcctggca ttatgcccag 2460
   tacatgacct tacgggactt tcctacttgg cagtacatct acgtattagt catcgctatt 2520
   accatggtga tgcggttttg gcagtacacc aatgggcgtg gatagcggtt tgactcacgg 2580
   ggatttccaa gtctccaccc cattgacgtc aatgggagtt tgttttggca ccaaaatcaa 2640
   cgggactttc caaaatgtcg taacaactcc gccccattga cgcaaatggg cggtaggcgt 2700
   gtacggtggg aggtctatat aagcagagct cgtttagtga accgtcagat cgcctggaga 2760
   cgccatccac gctgttttga cctccataga agacaccgac tctagctaga ggatccggac 2820
   tagtaactcg aggatgggga ctgacccggg ctgcaggaat tcataacttc gtataatgta 2880
   tgctatacga agttatcata tgccaccatg agcgagctga tcaaggagaa catgcacatg 2940
   aagctgtaca tggagggcac cgtgaacaac caccacttca agtgcacatc cgagggcgaa 3000
   ggcaagccct acgagggcac ccagaccatg aagatcaagg tggtcgaggg cggccctctc 3060
   cccttcgcct tcgacatcct ggctaccagc ttcatgtacg gcagcaaagc cttcatcaac 3120
   cacacccagg gcatccccga cttctttaag cagtccttcc ctgagggctt cacatgggag 3180
   agaatcacca catacgaaga cgggggcgtg ctgaccgcta cccaggacac cagcttccag 3240
   aacggctgca tcatctacaa cgtcaagatc aacggggtga acttcccatc caacggccct 3300
   gtgatgcaga agaaaacacg cggctgggag gccaacaccg agatgctgta ccccgctgac 3360
   ggcggcctga gaggccacag ccagatggcc ctgaagctcg tgggcggggg ctacctgcac 3420
   tgctccttca agaccacata cagatccaag aaacccgcta agaacctcaa gatgcccggc 3480
   ttccacttcg tggaccacag actggaaaga atcaaggagg ccgacaaaga gacctacgtc 3540
   gagcagcacg agatggctgt ggccaagtac tgcgacctcc ctagcaaact ggggcacaga 3600
   taaataactt cgtataaagt atcctatacg aagttatact ttggccgcgg ctcgaggggg 3660
   ttggggttgc gccttttcca aggcagccct gggtttgcgc agggacgcgg ctgctctggg 3720
   cgtggttccg ggaaacgcag cggcgccgac cctgggtctc gcacattctt cacgtccgtt 3780
   cgcagcgtca cccggatctt cgccgctacc cttgtgggcc ccccggcgac gcttcctgct 3840
   ccgcccctaa gtcgggaagg ttccttgcgg ttcgcggcgt gccggacgtg acaaacggaa 3900
   gccgcacgtc tcactagtac cctcgcagac ggacagcgcc agggagcaat ggcagcgcgc 3960
   cgaccgcgat gggctgtggc caatagcggc tgctcagcag ggcgcgccga gagcagcggc 4020
   cgggaagggg cggtgcggga ggcggggtgt ggggcggtag tgtgggccct gttcctgccc 4080
   gcgcggtgtt ccgcattctg caagcctccg gagcgcacgt cggcagtcgg ctccctcgtt 4140
   gaccgaatca ccgacctctc tccccagggg gatccaccgg tcgccaccat ggtgagcaag 4200
   ggcgaggagc tgttcaccgg ggtggtgccc atcctggtcg agctggacgg cgacgtaaac 4260
   ggccacaagt tcagcgtgtc cggcgagggc gagggcgatg ccacctacgg caagctgacc 4320
   ctgaagttca tctgcaccac cggcaagctg cccgtgccct ggcccaccct cgtgaccacc 4380
   ctgacctacg gcgtgcagtg cttcagccgc taccccgacc acatgaagca gcacgacttc 4440
   ttcaagtccg ccatgcccga aggctacgtc caggagcgca ccatcttctt caaggacgac 4500
   ggcaactaca agacccgcgc cgaggtgaag ttcgagggcg acaccctggt gaaccgcatc 4560
   gagctgaagg gcatcgactt caaggaggac ggcaacatcc tggggcacaa gctggagtac 4620
   aactacaaca gccacaacgt ctatatcatg gccgacaagc agaagaacgg catcaaggtg 4680
   aacttcaaga tccgccacaa catcgaggac ggcagcgtgc agctcgccga ccactaccag 4740
   cagaacaccc ccatcggcga cggccccgtg ctgctgcccg acaaccacta cctgagcacc 4800
   cagtccgccc tgagcaaaga ccccaacgag aagcgcgatc acatggtcct gctggagttc 4860
   gtgaccgccg ccgggatcac tctcggcatg gacgagctgt acaagtaaga tatcaagctt 4920
   atcgataatc aacctctgga ttacaaaatt tgtgaaagat tgactggtat tcttaactat 4980
   gttgctcctt ttacgctatg tggatacgct gctttaatgc ctttgtatca tgctattgct 5040
   tcccgtatgg ctttcatttt ctcctccttg tataaatcct ggttgctgtc tctttatgag 5100
   gagttgtggc ccgttgtcag gcaacgtggc gtggtgtgca ctgtgtttgc tgacgcaacc 5160
   cccactggtt ggggcattgc caccacctgt cagctccttt ccgggacttt cgctttcccc 5220
   ctccctattg ccacggcgga actcatcgcc gcctgccttg cccgctgctg gacaggggct 5280
   cggctgttgg gcactgacaa ttccgtggtg ttgtcgggga aatcatcgtc ctttccttgg 5340
   ctgctcgcct gtgttgccac ctggattctg cgcgggacgt ccttctgcta cgtcccttcg 5400
   gccctcaatc cagcggacct tccttcccgc ggcctgctgc cggctctgcg gcctcttccg 5460
   cgtcttcgcc ttcgccctca gacgagtcgg atctcccttt gggccgcctc cccgcatcga 5520
   taccgtcgac ctcgagaacc tagaaaaaca tggagcaatc acaagtagca atacagcagc 5580
   taccaatgct gattgtgcct ggctagaagc acaagaggag gaggaggtgg gttttccagt 5640
   cacacctcag gtacctttaa gaccaatgac ttacaaggca gctgtagatc ttagccactt 5700
   tttaaaagaa aaggggggac tggaagggct aattcactcc caacgaagac aagatctttt 5760
   tgcttgtact gggtctctct ggttagacca gatctgagcc tgggagctct ctggctaact 5820
   agggaaccca ctgcttaagc ctcaataaag cttgccttga gtgcttcaag tagtgtgtgc 5880
   ccgtctgttg tgtgactctg gtaactagag atccctcaga cccttttagt cagtgtggaa 5940
   aatctctaga 5950
<210> 42
   <211> 5837
   <212> DNA
   <213> Artificial Sequence
<220>
   <223> sequence fr screening human cells floxed
<400> 42
   gggctaattc actcccaaag aagacaagat atccttgatc tgtggatcta ccacacacaa      60
   ggctacttcc ctgattagca gaactacaca ccagggccag gggtcagata tccactgacc     120
   tttggatggt gctacaagct agtaccagtt gagccagata aggtagaaga ggccaataaa     180
   ggagagaaca ccagcttgtt acaccctgtg agcctgcatg ggatggatga cccggagaga     240
   gaagtgttag agtggaggtt tgacagccgc ctagcatttc atcacgtggc ccgagagctg     300
   catccggagt acttcaagaa ctgctgatat cgagcttgct acaagggact ttccgctggg 360
   gactttccag ggaggcgtgg cctgggcggg actggggagt ggcgagccct cagatcctgc 420
   atataagcag ctgctttttg cctgtactgg gtctctctgg ttagaccaga tctgagcctg 480
   ggagctctct ggctaactag ggaacccact gcttaagcct caataaagct tgccttgagt 540
   gcttcaagta gtgtgtgccc gtctgttgtg tgactctggt aactagagat ccctcagacc 600
   cttttagtca gtgtggaaaa tctctagcag tggcgcccga acagggactt gaaagcgaaa 660
   gggaaaccag aggagctctc tcgacgcagg actcggcttg ctgaagcgcg cacggcaaga 720
   ggcgaggggc ggcgactggt gagtacgcca aaaattttga ctagcggagg ctagaaggag 780
   agagatgggt gcgagagcgt cagtattaag cgggggagaa ttagatcgcg atgggaaaaa 840
   attcggttaa ggccaggggg aaagaaaaaa tataaattaa aacatatagt atgggcaagc 900
   agggagctag aacgattcgc agttaatcct ggcctgttag aaacatcaga aggctgtaga 960
   caaatactgg gacagctaca accatccctt cagacaggat cagaagaact tagatcatta 1020
   tataatacag tagcaaccct ctattgtgtg catcaaagga tagagataaa agacaccaag 1080
   gaagctttag acaagataga ggaagagcaa aacaaaagta agaccaccgc acagcaagcg 1140
   gccgctgatc ttcagacctg gaggaggaga tatgagggac aattggagaa gtgaattata 1200
   taaatataaa gtagtaaaaa ttgaaccatt aggagtagca cccaccaagg caaagagaag 1260
   agtggtgcag agagaaaaaa gagcagtggg aataggagct ttgttccttg ggttcttggg 1320
   agcagcagga agcactatgg gcgcagcgtc aatgacgctg acggtacagg ccagacaatt 1380
   attgtctggt atagtgcagc agcagaacaa tttgctgagg gctattgagg cgcaacagca 1440
   tctgttgcaa ctcacagtct ggggcatcaa gcagctccag gcaagaatcc tggctgtgga 1500
   aagataccta aaggatcaac agctcctggg gatttggggt tgctctggaa aactcatttg 1560
   caccactgct gtgccttgga atgctagttg gagtaataaa tctctggaac agatttggaa 1620
   tcacacgacc tggatggagt gggacagaga aattaacaat tacacaagct taatacactc 1680
   cttaattgaa gaatcgcaaa accagcaaga aaagaatgaa caagaattat tggaattaga 1740
   taaatgggca agtttgtgga attggtttaa cataacaaat tggctgtggt atataaaatt 1800
   attcataatg atagtaggag gcttggtagg tttaagaata gtttttgctg tactttctat 1860
   agtgaataga gttaggcagg gatattcacc attatcgttt cagacccacc tcccaacccc 1920
   gaggggaccc gacaggcccg aaggaataga agaagaaggt ggagagagag acagagacag 1980
   atccattcga ttagtgaacg gatctcgacg gtatcgccga attcacaaat ggcagtattc 2040
   atccacaatt ttaaaagaaa aggggggatt ggggggtaca gtgcagggga aagaatagta 2100
   gacataatag caacagacat acaaactaaa gaattacaaa aacaaattac aaaaattcaa 2160
   aattttcggg tttattacag ggacagcaga gatccacttt atcgatggtc cggtccggaa 2220
   ttcctgcagc cccgataaaa taaaagattt tatttagtct ccagaaaaag gggggaatga 2280
   aagaccccac ctgtaggttt ggcaagctag ctgcagtaac gccattttgc aaggcatgga 2340
   aaaataccaa accaagaata gagaagttca gatcaagggc gggtacatga aaatagctaa 2400
   cgttgggcca aacaggatat ctgcggtgag cagtttcggc cccggcccgg ggccaagaac 2460
   agatggtcac cgcagtttcg gccccggccc gaggccaaga acagatggtc cccagatatg 2520
   gcccaaccct cagcagtttc ttaagaccca tcagatgttt ccaggctccc ccaaggacct 2580
   gaaatgaccc tgcgccttat ttgaattaac caatcagcct gcttctcgct tctgttcgcg 2640
   cgcttctgct tcccgagctc tataaaagag ctcacaaccc ctcactcggc gcgccagtcc 2700
   tccgacagac tgagtcgccc gggggggatc caccgggctg caggaattca taacttcgta 2760
   taatgtatgc tatacgaagt tatcatatgc caccatgagc gagctgatca aggagaacat 2820
   gcacatgaag ctgtacatgg agggcaccgt gaacaaccac cacttcaagt gcacatccga 2880
   gggcgaaggc aagccctacg agggcaccca gaccatgaag atcaaggtgg tcgagggcgg 2940
   ccctctcccc ttcgccttcg acatcctggc taccagcttc atgtacggca gcaaagcctt 3000
   catcaaccac acccagggca tccccgactt ctttaagcag tccttccctg agggcttcac 3060
   atgggagaga atcaccacat acgaagacgg gggcgtgctg accgctaccc aggacaccag 3120
   cttccagaac ggctgcatca tctacaacgt caagatcaac ggggtgaact tcccatccaa 3180
   cggccctgtg atgcagaaga aaacacgcgg ctgggaggcc aacaccgaga tgctgtaccc 3240
   cgctgacggc ggcctgagag gccacagcca gatggccctg aagctcgtgg gcgggggcta 3300
   cctgcactgc tccttcaaga ccacatacag atccaagaaa cccgctaaga acctcaagat 3360
   gcccggcttc cacttcgtgg accacagact ggaaagaatc aaggaggccg acaaagagac 3420
   ctacgtcgag cagcacgaga tggctgtggc caagtactgc gacctcccta gcaaactggg 3480
   gcacagataa ataacttcgt ataaagtatc ctatacgaag ttatactttg gccgcggctc 3540
   gagggggttg gggttgcgcc ttttccaagg cagccctggg tttgcgcagg gacgcggctg 3600
   ctctgggcgt ggttccggga aacgcagcgg cgccgaccct gggtctcgca cattcttcac 3660
   gtccgttcgc agcgtcaccc ggatcttcgc cgctaccctt gtgggccccc cggcgacgct 3720
   tcctgctccg cccctaagtc gggaaggttc cttgcggttc gcggcgtgcc ggacgtgaca 3780
   aacggaagcc gcacgtctca ctagtaccct cgcagacgga cagcgccagg gagcaatggc 3840
   agcgcgccga ccgcgatggg ctgtggccaa tagcggctgc tcagcagggc gcgccgagag 3900
   cagcggccgg gaaggggcgg tgcgggaggc ggggtgtggg gcggtagtgt gggccctgtt 3960
   cctgcccgcg cggtgttccg cattctgcaa gcctccggag cgcacgtcgg cagtcggctc 4020
   cctcgttgac cgaatcaccg acctctctcc ccagggggat ccaccggtcg ccaccatggt 4080
   gagcaagggc gaggagctgt tcaccggggt ggtgcccatc ctggtcgagc tggacggcga 4140
   cgtaaacggc cacaagttca gcgtgtccgg cgagggcgag ggcgatgcca cctacggcaa 4200
   gctgaccctg aagttcatct gcaccaccgg caagctgccc gtgccctggc ccaccctcgt 4260
   gaccaccctg acctacggcg tgcagtgctt cagccgctac cccgaccaca tgaagcagca 4320
   cgacttcttc aagtccgcca tgcccgaagg ctacgtccag gagcgcacca tcttcttcaa 4380
   ggacgacggc aactacaaga cccgcgccga ggtgaagttc gagggcgaca ccctggtgaa 4440
   ccgcatcgag ctgaagggca tcgacttcaa ggaggacggc aacatcctgg ggcacaagct 4500
   ggagtacaac tacaacagcc acaacgtcta tatcatggcc gacaagcaga agaacggcat 4560
   caaggtgaac ttcaagatcc gccacaacat cgaggacggc agcgtgcagc tcgccgacca 4620
   ctaccagcag aacaccccca tcggcgacgg ccccgtgctg ctgcccgaca accactacct 4680
   gagcacccag tccgccctga gcaaagaccc caacgagaag cgcgatcaca tggtcctgct 4740
   ggagttcgtg accgccgccg ggatcactct cggcatggac gagctgtaca agtaagatat 4800
   caagcttatc gataatcaac ctctggatta caaaatttgt gaaagattga ctggtattct 4860
   taactatgtt gctcctttta cgctatgtgg atacgctgct ttaatgcctt tgtatcatgc 4920
   tattgcttcc cgtatggctt tcattttctc ctccttgtat aaatcctggt tgctgtctct 4980
   ttatgaggag ttgtggcccg ttgtcaggca acgtggcgtg gtgtgcactg tgtttgctga 5040
   cgcaaccccc actggttggg gcattgccac cacctgtcag ctcctttccg ggactttcgc 5100
   tttccccctc cctattgcca cggcggaact catcgccgcc tgccttgccc gctgctggac 5160
   aggggctcgg ctgttgggca ctgacaattc cgtggtgttg tcggggaaat catcgtcctt 5220
   tccttggctg ctcgcctgtg ttgccacctg gattctgcgc gggacgtcct tctgctacgt 5280
   cccttcggcc ctcaatccag cggaccttcc ttcccgcggc ctgctgccgg ctctgcggcc 5340
   tcttccgcgt cttcgccttc gccctcagac gagtcggatc tccctttggg ccgcctcccc 5400
   gcatcgatac cgtcgacctc gagaacctag aaaaacatgg agcaatcaca agtagcaata 5460
   cagcagctac caatgctgat tgtgcctggc tagaagcaca agaggaggag gaggtgggtt 5520
   ttccagtcac acctcaggta cctttaagac caatgactta caaggcagct gtagatctta 5580
   gccacttttt aaaagaaaag gggggactgg aagggctaat tcactcccaa cgaagacaag 5640
   atctttttgc ttgtactggg tctctctggt tagaccagat ctgagcctgg gagctctctg 5700
   gctaactagg gaacccactg cttaagcctc aataaagctt gccttgagtg cttcaagtag 5760
   tgtgtgcccg tctgttgtgt gactctggta actagagatc cctcagaccc ttttagtcag 5820
   tgtggaaaat ctctaga 5837
<210> 43
   <211> 5971
   <212> DNA
   <213> Artificial Sequence
<220>
   <223> sequence for screening human cells floxed
<400> 43
   gggctaattc actcccaaag aagacaagat atccttgatc tgtggatcta ccacacacaa 60
   ggctacttcc ctgattagca gaactacaca ccagggccag gggtcagata tccactgacc 120
   tttggatggt gctacaagct agtaccagtt gagccagata aggtagaaga ggccaataaa 180
   ggagagaaca ccagcttgtt acaccctgtg agcctgcatg ggatggatga cccggagaga 240
   gaagtgttag agtggaggtt tgacagccgc ctagcatttc atcacgtggc ccgagagctg 300
   catccggagt acttcaagaa ctgctgatat cgagcttgct acaagggact ttccgctggg 360
   gactttccag ggaggcgtgg cctgggcggg actggggagt ggcgagccct cagatcctgc 420
   atataagcag ctgctttttg cctgtactgg gtctctctgg ttagaccaga tctgagcctg 480
   ggagctctct ggctaactag ggaacccact gcttaagcct caataaagct tgccttgagt 540
   gcttcaagta gtgtgtgccc gtctgttgtg tgactctggt aactagagat ccctcagacc 600
   cttttagtca gtgtggaaaa tctctagcag tggcgcccga acagggactt gaaagcgaaa 660
   gggaaaccag aggagctctc tcgacgcagg actcggcttg ctgaagcgcg cacggcaaga 720
   ggcgaggggc ggcgactggt gagtacgcca aaaattttga ctagcggagg ctagaaggag 780
   agagatgggt gcgagagcgt cagtattaag cgggggagaa ttagatcgcg atgggaaaaa 840
   attcggttaa ggccaggggg aaagaaaaaa tataaattaa aacatatagt atgggcaagc 900
   agggagctag aacgattcgc agttaatcct ggcctgttag aaacatcaga aggctgtaga 960
   caaatactgg gacagctaca accatccctt cagacaggat cagaagaact tagatcatta 1020
   tataatacag tagcaaccct ctattgtgtg catcaaagga tagagataaa agacaccaag 1080
   gaagctttag acaagataga ggaagagcaa aacaaaagta agaccaccgc acagcaagcg 1140
   gccgctgatc ttcagacctg gaggaggaga tatgagggac aattggagaa gtgaattata 1200
   taaatataaa gtagtaaaaa ttgaaccatt aggagtagca cccaccaagg caaagagaag 1260
   agtggtgcag agagaaaaaa gagcagtggg aataggagct ttgttccttg ggttcttggg 1320
   agcagcagga agcactatgg gcgcagcgtc aatgacgctg acggtacagg ccagacaatt 1380
   attgtctggt atagtgcagc agcagaacaa tttgctgagg gctattgagg cgcaacagca 1440
   tctgttgcaa ctcacagtct ggggcatcaa gcagctccag gcaagaatcc tggctgtgga 1500
   aagataccta aaggatcaac agctcctggg gatttggggt tgctctggaa aactcatttg 1560
   caccactgct gtgccttgga atgctagttg gagtaataaa tctctggaac agatttggaa 1620
   tcacacgacc tggatggagt gggacagaga aattaacaat tacacaagct taatacactc 1680
   cttaattgaa gaatcgcaaa accagcaaga aaagaatgaa caagaattat tggaattaga 1740
   taaatgggca agtttgtgga attggtttaa cataacaaat tggctgtggt atataaaatt 1800
   attcataatg atagtaggag gcttggtagg tttaagaata gtttttgctg tactttctat 1860
   agtgaataga gttaggcagg gatattcacc attatcgttt cagacccacc tcccaacccc 1920
   gaggggaccc gacaggcccg aaggaataga agaagaaggt ggagagagag acagagacag 1980
   atccattcga ttagtgaacg gatctcgacg gtatcgccga attcacaaat ggcagtattc 2040
   atccacaatt ttaaaagaaa aggggggatt ggggggtaca gtgcagggga aagaatagta 2100
   gacataatag caacagacat acaaactaaa gaattacaaa aacaaattac aaaaattcaa 2160
   aattttcggg tttattacag ggacagcaga gatccacttt atcgatggtc cggtccggaa 2220
   ttcctgcagc cccgataaaa taaaagattt tatttagtct ccagaaaaag gggggaatga 2280
   aagaccccac ctgtaggttt ggcaagctag ctgcagtaac gccattttgc aaggcatgga 2340
   aaaataccaa accaagaata gagaagttca gatcaagggc gggtacatga aaatagctaa 2400
   cgttgggcca aacaggatat ctgcggtgag cagtttcggc cccggcccgg ggccaagaac 2460
   agatggtcac cgcagtttcg gccccggccc gaggccaaga acagatggtc cccagatatg 2520
   gcccaaccct cagcagtttc ttaagaccca tcagatgttt ccaggctccc ccaaggacct 2580
   gaaatgaccc tgcgccttat ttgaattaac caatcagcct gcttctcgct tctgttcgcg 2640
   cgcttctgct tcccgagctc tataaaagag ctcacaaccc ctcactcggc gcgccagtcc 2700
   tccgacagac tgagtcgccc gggggggatc caccgggctg caggaattca taacttcgta 2760
   taatgtatgc tatacgaagt tatcatatgc caccatgagc gagctgatca aggagaacat 2820
   gcacatgaag ctgtacatgg agggcaccgt gaacaaccac cacttcaagt gcacatccga 2880
   gggcgaaggc aagccctacg agggcaccca gaccatgaag atcaaggtgg tcgagggcgg 2940
   ccctctcccc ttcgccttcg acatcctggc taccagcttc atgtacggca gcaaagcctt 3000
   catcaaccac acccagggca tccccgactt ctttaagcag tccttccctg agggcttcac 3060
   atgggagaga atcaccacat acgaagacgg gggcgtgctg accgctaccc aggacaccag 3120
   cttccagaac ggctgcatca tctacaacgt caagatcaac ggggtgaact tcccatccaa 3180
   cggccctgtg atgcagaaga aaacacgcgg ctgggaggcc aacaccgaga tgctgtaccc 3240
   cgctgacggc ggcctgagag gccacagcca gatggccctg aagctcgtgg gcgggggcta 3300
   cctgcactgc tccttcaaga ccacatacag atccaagaaa cccgctaaga acctcaagat 3360
   gcccggcttc cacttcgtgg accacagact ggaaagaatc aaggaggccg acaaagagac 3420
   ctacgtcgag cagcacgaga tggctgtggc caagtactgc gacctcccta gcaaactggg 3480
   gcacagataa ataacttcgt ataaagtatc ctatacgaag ttatactttg gccgcggctc 3540
   gaatctactt accgataagc ttgggagttc cgcgttacat aacttacggt aaatggcccg 3600
   cctggctgac cgcccaacga cccccgccca ttgacgtcaa taatgacgta tgttcccata 3660
   gtaacgccaa tagggacttt ccattgacgt caatgggtgg agtatttacg gtaaactgcc 3720
   cacttggcag tacatcaagt gtatcatatg ccaagtacgc cccctattga cgtcaatgac 3780
   ggtaaatggc ccgcctggca ttatgcccag tacatgacct tacgggactt tcctacttgg 3840
   cagtacatct acgtattagt catcgctatt accatggtga tgcggttttg gcagtacacc 3900
   aatgggcgtg gatagcggtt tgactcacgg ggatttccaa gtctccaccc cattgacgtc 3960
   aatgggagtt tgttttggca ccaaaatcaa cgggactttc caaaatgtcg taacaactcc 4020
   gccccattga cgcaaatggg cggtaggcgt gtacggtggg aggtctatat aagcagagct 4080
   cgtttagtga accgtcagat cgcctggaga cgccatccac gctgttttga cctccataga 4140
   agacaccgac tctagctaga ggatccggac tagtaactcg aggatgggga ctgacccgga 4200
   cgcgtgccgc caccatggtg agcaagggcg aggagctgtt caccggggtg gtgcccatcc 4260
   tggtcgagct ggacggcgac gtaaacggcc acaagttcag cgtgtccggc gagggcgagg 4320
   gcgatgccac ctacggcaag ctgaccctga agttcatctg caccaccggc aagctgcccg 4380
   tgccctggcc caccctcgtg accaccctga cctacggcgt gcagtgcttc agccgctacc 4440
   ccgaccacat gaagcagcac gacttcttca agtccgccat gcccgaaggc tacgtccagg 4500
   agcgcaccat cttcttcaag gacgacggca actacaagac ccgcgccgag gtgaagttcg 4560
   agggcgacac cctggtgaac cgcatcgagc tgaagggcat cgacttcaag gaggacggca 4620
   acatcctggg gcacaagctg gagtacaact acaacagcca caacgtctat atcatggccg 4680
   acaagcagaa gaacggcatc aaggtgaact tcaagatccg ccacaacatc gaggacggca 4740
   gcgtgcagct cgccgaccac taccagcaga acacccccat cggcgacggc cccgtgctgc 4800
   tgcccgacaa ccactacctg agcacccagt ccgccctgag caaagacccc aacgagaagc 4860
   gcgatcacat ggtcctgctg gagttcgtga ccgccgccgg gatcactctc ggcatggacg 4920
   agctgtacaa gtaagatatc aagcttatcg ataatcaacc tctggattac aaaatttgtg 4980
   aaagattgac tggtattctt aactatgttg ctccttttac gctatgtgga tacgctgctt 5040
   taatgccttt gtatcatgct attgcttccc gtatggcttt cattttctcc tccttgtata 5100
   aatcctggtt gctgtctctt tatgaggagt tgtggcccgt tgtcaggcaa cgtggcgtgg 5160
   tgtgcactgt gtttgctgac gcaaccccca ctggttgggg cattgccacc acctgtcagc 5220
   tcctttccgg gactttcgct ttccccctcc ctattgccac ggcggaactc atcgccgcct 5280
   gccttgcccg ctgctggaca ggggctcggc tgttgggcac tgacaattcc gtggtgttgt 5340
   cggggaaatc atcgtccttt ccttggctgc tcgcctgtgt tgccacctgg attctgcgcg 5400
   ggacgtcctt ctgctacgtc ccttcggccc tcaatccagc ggaccttcct tcccgcggcc 5460
   tgctgccggc tctgcggcct cttccgcgtc ttcgccttcg ccctcagacg agtcggatct 5520
   ccctttgggc cgcctccccg catcgatacc gtcgacctcg agaacctaga aaaacatgga 5580
   gcaatcacaa gtagcaatac agcagctacc aatgctgatt gtgcctggct agaagcacaa 5640
   gaggaggagg aggtgggttt tccagtcaca cctcaggtac ctttaagacc aatgacttac 5700
   aaggcagctg tagatcttag ccacttttta aaagaaaagg ggggactgga agggctaatt 5760
   cactcccaac gaagacaaga tctttttgct tgtactgggt ctctctggtt agaccagatc 5820
   tgagcctggg agctctctgg ctaactaggg aacccactgc ttaagcctca ataaagcttg 5880
   ccttgagtgc ttcaagtagt gtgtgcccgt ctgttgtgtg actctggtaa ctagagatcc 5940
   ctcagaccct tttagtcagt gtggaaaatc t 5971
<210> 44
   <211> 6907
   <212> DNA
   <213> Artificial Sequence
<220>
   <223> sequence for screening human cells floxed
<400> 44
   gggctaattc actcccaaag aagacaagat atccttgatc tgtggatcta ccacacacaa      60
   ggctacttcc ctgattagca gaactacaca ccagggccag gggtcagata tccactgacc     120
   tttggatggt gctacaagct agtaccagtt gagccagata aggtagaaga ggccaataaa     180
   ggagagaaca ccagcttgtt acaccctgtg agcctgcatg ggatggatga cccggagaga     240
   gaagtgttag agtggaggtt tgacagccgc ctagcatttc atcacgtggc ccgagagctg     300
   catccggagt acttcaagaa ctgctgatat cgagcttgct acaagggact ttccgctggg     360
   gactttccag ggaggcgtgg cctgggcggg actggggagt ggcgagccct cagatcctgc     420
   atataagcag ctgctttttg cctgtactgg gtctctctgg ttagaccaga tctgagcctg     480
   ggagctctct ggctaactag ggaacccact gcttaagcct caataaagct tgccttgagt     540
   gcttcaagta gtgtgtgccc gtctgttgtg tgactctggt aactagagat ccctcagacc     600
   cttttagtca gtgtggaaaa tctctagcag tggcgcccga acagggactt gaaagcgaaa     660
   gggaaaccag aggagctctc tcgacgcagg actcggcttg ctgaagcgcg cacggcaaga     720
   ggcgaggggc ggcgactggt gagtacgcca aaaattttga ctagcggagg ctagaaggag     780
   agagatgggt gcgagagcgt cagtattaag cgggggagaa ttagatcgcg atgggaaaaa     840
   attcggttaa ggccaggggg aaagaaaaaa tataaattaa aacatatagt atgggcaagc     900
   agggagctag aacgattcgc agttaatcct ggcctgttag aaacatcaga aggctgtaga     960
   caaatactgg gacagctaca accatccctt cagacaggat cagaagaact tagatcatta    1020
   tataatacag tagcaaccct ctattgtgtg catcaaagga tagagataaa agacaccaag    1080
   gaagctttag acaagataga ggaagagcaa aacaaaagta agaccaccgc acagcaagcg 1140
   gccgctgatc ttcagacctg gaggaggaga tatgagggac aattggagaa gtgaattata 1200
   taaatataaa gtagtaaaaa ttgaaccatt aggagtagca cccaccaagg caaagagaag 1260
   agtggtgcag agagaaaaaa gagcagtggg aataggagct ttgttccttg ggttcttggg 1320
   agcagcagga agcactatgg gcgcagcgtc aatgacgctg acggtacagg ccagacaatt 1380
   attgtctggt atagtgcagc agcagaacaa tttgctgagg gctattgagg cgcaacagca 1440
   tctgttgcaa ctcacagtct ggggcatcaa gcagctccag gcaagaatcc tggctgtgga 1500
   aagataccta aaggatcaac agctcctggg gatttggggt tgctctggaa aactcatttg 1560
   caccactgct gtgccttgga atgctagttg gagtaataaa tctctggaac agatttggaa 1620
   tcacacgacc tggatggagt gggacagaga aattaacaat tacacaagct taatacactc 1680
   cttaattgaa gaatcgcaaa accagcaaga aaagaatgaa caagaattat tggaattaga 1740
   taaatgggca agtttgtgga attggtttaa cataacaaat tggctgtggt atataaaatt 1800
   attcataatg atagtaggag gcttggtagg tttaagaata gtttttgctg tactttctat 1860
   agtgaataga gttaggcagg gatattcacc attatcgttt cagacccacc tcccaacccc 1920
   gaggggaccc gacaggcccg aaggaataga agaagaaggt ggagagagag acagagacag 1980
   atccattcga ttagtgaacg gatctcgacg gtatcgccga attcacaaat ggcagtattc 2040
   atccacaatt ttaaaagaaa aggggggatt ggggggtaca gtgcagggga aagaatagta 2100
   gacataatag caacagacat acaaactaaa gaattacaaa aacaaattac aaaaattcaa 2160
   aattttcggg tttattacag ggacagcaga gatccacttt atcgataagc ttgggagttc 2220
   cgcgttacat aacttacggt aaatggcccg cctggctgac cgcccaacga cccccgccca 2280
   ttgacgtcaa taatgacgta tgttcccata gtaacgccaa tagggacttt ccattgacgt 2340
   caatgggtgg agtatttacg gtaaactgcc cacttggcag tacatcaagt gtatcatatg 2400
   ccaagtacgc cccctattga cgtcaatgac ggtaaatggc ccgcctggca ttatgcccag 2460
   tacatgacct tacgggactt tcctacttgg cagtacatct acgtattagt catcgctatt 2520
   accatggtga tgcggttttg gcagtacacc aatgggcgtg gatagcggtt tgactcacgg 2580
   ggatttccaa gtctccaccc cattgacgtc aatgggagtt tgttttggca ccaaaatcaa 2640
   cgggactttc caaaatgtcg taacaactcc gccccattga cgcaaatggg cggtaggcgt 2700
   gtacggtggg aggtctatat aagcagagct cgtttagtga accgtcagat cgcctggaga 2760
   cgccatccac gctgttttga cctccataga agacaccgac tctagctaga ggatccggac 2820
   tagtaactcg aggatgggga ctgacccggg ctgcaggaat tcataacttc gtataatgta 2880
   tgctatacga agttatcata tgccaccatg gaagacgcca aaaacataaa gaaaggcccg 2940
   gcgccattct atccgctgga agatggaacc gctggagagc aactgcataa ggctatgaag 3000
   agatacgccc tggttcctgg aacaattgct tttacagatg cacatatcga ggtggacatc 3060
   acttacgctg agtacttcga aatgtccgtt cggttggcag aagctatgaa acgatatggg 3120
   ctgaatacaa atcacagaat cgtcgtatgc agtgaaaact ctcttcaatt ctttatgccg 3180
   gtgttgggcg cgttatttat cggagttgca gttgcgcccg cgaacgacat ttataatgaa 3240
   cgtgaattgc tcaacagtat gggcatttcg cagcctaccg tggtgttcgt ttccaaaaag 3300
   gggttgcaaa aaattttgaa cgtgcaaaaa aagctcccaa tcatccaaaa aattattatc 3360
   atggattcta aaacggatta ccagggattt cagtcgatgt acacgttcgt cacatctcat 3420
   ctacctcccg gttttaatga atacgatttt gtgccagagt ccttcgatag ggacaagaca 3480
   attgcactga tcatgaactc ctctggatct actggtctgc ctaaaggtgt cgctctgcct 3540
   catagaactg cctgcgtgag attctcgcat gccagagatc ctatttttgg caatcaaatc 3600
   attccggata ctgcgatttt aagtgttgtt ccattccatc acggttttgg aatgtttact 3660
   acactcggat atttgatatg tggatttcga gtcgtcttaa tgtatagatt tgaagaagag 3720
   ctgtttctga ggagccttca ggattacaag attcaaagtg cgctgctggt gccaacccta 3780
   ttctccttct tcgccaaaag cactctgatt gacaaatacg atttatctaa tttacacgaa 3840
   attgcttctg gtggcgctcc cctctctaag gaagtcgggg aagcggttgc caagaggttc 3900
   catctgccag gtatcaggca aggatatggg ctcactgaga ctacatcagc tattctgatt 3960
   acacccgagg gggatgataa accgggcgcg gtcggtaaag ttgttccatt ttttgaagcg 4020
   aaggttgtgg atctggatac cgggaaaacg ctgggcgtta atcaaagagg cgaactgtgt 4080
   gtgagaggtc ctatgattat gtccggttat gtaaacaatc cggaagcgac caacgccttg 4140
   attgacaagg atggatggct acattctgga gacatagctt actgggacga agacgaacac 4200
   ttcttcatcg ttgaccgcct gaagtctctg attaagtaca aaggctatca ggtggctccc 4260
   gctgaattgg aatccatctt gctccaacac cccaacatct tcgacgcagg tgtcgcaggt 4320
   cttcccgacg atgacgccgg tgaacttccc gccgccgttg ttgttttgga gcacggaaag 4380
   acgatgacgg aaaaagagat cgtggattac gtcgccagtc aagtaacaac cgcgaaaaag 4440
   ttgcgcggag gagttgtgtt tgtggacgaa gtaccgaaag gtcttaccgg aaaactcgac 4500
   gcaagaaaaa tcagagagat cctcataaag gccaagaagg gcggaaagat cgccgtgtaa 4560
   ataacttcgt ataaagtatc ctatacgaag ttatactttg gccgcggctc gagggggttg 4620
   gggttgcgcc ttttccaagg cagccctggg tttgcgcagg gacgcggctg ctctgggcgt 4680
   ggttccggga aacgcagcgg cgccgaccct gggtctcgca cattcttcac gtccgttcgc 4740
   agcgtcaccc ggatcttcgc cgctaccctt gtgggccccc cggcgacgct tcctgctccg 4800
   cccctaagtc gggaaggttc cttgcggttc gcggcgtgcc ggacgtgaca aacggaagcc 4860
   gcacgtctca ctagtaccct cgcagacgga cagcgccagg gagcaatggc agcgcgccga 4920
   ccgcgatggg ctgtggccaa tagcggctgc tcagcagggc gcgccgagag cagcggccgg 4980
   gaaggggcgg tgcgggaggc ggggtgtggg gcggtagtgt gggccctgtt cctgcccgcg 5040
   cggtgttccg cattctgcaa gcctccggag cgcacgtcgg cagtcggctc cctcgttgac 5100
   cgaatcaccg acctctctcc ccagggggat ccaccggtcg ccaccatggt gagcaagggc 5160
   gaggagctgt tcaccggggt ggtgcccatc ctggtcgagc tggacggcga cgtaaacggc 5220
   cacaagttca gcgtgtccgg cgagggcgag ggcgatgcca cctacggcaa gctgaccctg 5280
   aagttcatct gcaccaccgg caagctgccc gtgccctggc ccaccctcgt gaccaccctg 5340
   acctacggcg tgcagtgctt cagccgctac cccgaccaca tgaagcagca cgacttcttc 5400
   aagtccgcca tgcccgaagg ctacgtccag gagcgcacca tcttcttcaa ggacgacggc 5460
   aactacaaga cccgcgccga ggtgaagttc gagggcgaca ccctggtgaa ccgcatcgag 5520
   ctgaagggca tcgacttcaa ggaggacggc aacatcctgg ggcacaagct ggagtacaac 5580
   tacaacagcc acaacgtcta tatcatggcc gacaagcaga agaacggcat caaggtgaac 5640
   ttcaagatcc gccacaacat cgaggacggc agcgtgcagc tcgccgacca ctaccagcag 5700
   aacaccccca tcggcgacgg ccccgtgctg ctgcccgaca accactacct gagcacccag 5760
   tccgccctga gcaaagaccc caacgagaag cgcgatcaca tggtcctgct ggagttcgtg 5820
   accgccgccg ggatcactct cggcatggac gagctgtaca agtaagatat caagcttatc 5880
   gataatcaac ctctggatta caaaatttgt gaaagattga ctggtattct taactatgtt 5940
   gctcctttta cgctatgtgg atacgctgct ttaatgcctt tgtatcatgc tattgcttcc 6000
   cgtatggctt tcattttctc ctccttgtat aaatcctggt tgctgtctct ttatgaggag 6060
   ttgtggcccg ttgtcaggca acgtggcgtg gtgtgcactg tgtttgctga cgcaaccccc 6120
   actggttggg gcattgccac cacctgtcag ctcctttccg ggactttcgc tttccccctc 6180
   cctattgcca cggcggaact catcgccgcc tgccttgccc gctgctggac aggggctcgg 6240
   ctgttgggca ctgacaattc cgtggtgttg tcggggaaat catcgtcctt tccttggctg 6300
   ctcgcctgtg ttgccacctg gattctgcgc gggacgtcct tctgctacgt cccttcggcc 6360
   ctcaatccag cggaccttcc ttcccgcggc ctgctgccgg ctctgcggcc tcttccgcgt 6420
   cttcgccttc gccctcagac gagtcggatc tccctttggg ccgcctcccc gcatcgatac 6480
   cgtcgacctc gagaacctag aaaaacatgg agcaatcaca agtagcaata cagcagctac 6540
   caatgctgat tgtgcctggc tagaagcaca agaggaggag gaggtgggtt ttccagtcac 6600
   acctcaggta cctttaagac caatgactta caaggcagct gtagatctta gccacttttt 6660
   aaaagaaaag gggggactgg aagggctaat tcactcccaa cgaagacaag atctttttgc 6720
   ttgtactggg tctctctggt tagaccagat ctgagcctgg gagctctctg gctaactagg 6780
   gaacccactg cttaagcctc aataaagctt gccttgagtg cttcaagtag tgtgtgcccg 6840
   tctgttgtgt gactctggta actagagatc cctcagaccc ttttagtcag tgtggaaaat 6900
   ctctaga 6907
<210> 45
   <211> 5872
   <212> DNA
   <213> Artificial Sequence
<220>
   <223> sequence for screening simian cells floxed
<400> 45
   cagtcgctct gcggagaggc tggcagattg agccctggga ggttctctcc agcactagca 60
   ggtagagcct gggtgttccc tgctagactc tcaccagcac ttggccagtg ctgggcagag 120
   tggctccacg cttgcttgct taaagacctc ttcaataaag ctgccatttt agaagtaagc 180
   cagtgtgtgt tcccatctct cctagtcgcc gcctggtcaa ctcggtactc ggtaataaga 240
   agaccctggt ctgttaggac cctttctgct ttgagaaacc gaagcaggaa aatccctagc 300
   agattggcgc ccgaacaggg acttgaagga gagtgagaga ctcctgagta cggctgagtg 360
   aaggcagtaa gggcggcagg aaccaaccac gacggagtgc tcctataaag gcgcgggtcg 420
   gtaccagacg gcgtgaggag cgggagagga ggaggcctcc ggttgcagta agtgcaacac 480
   aaaaaagaaa tagctgtctt gttatccagg aagggataat aagatagagt gggagatggg 540
   cgcgagaaac tccgtcttgt cagggaagaa agcagatgaa ttgaaaaaat tattaatcgc 600
   atgaatttta aaagaagggg aggaataggg gatatgactc cagcagaaag attaattaac 660
   atgatcacta cagaacaaga aatacaattt caacaatcaa aaaactcaaa atttaaaaat 720
   tttcgggtct attacagagc tcacgcgtga ttggagttgg gagattataa attagtagag 780
   atcactccga ttggcttggc ccccacagat gtgaagaggt acactactgg tggcacctca 840
   agaaataaaa gaggggtctt tgtgctaggg ttcttgggtt ttctcgcaac ggcaggttct 900
   gcaatgggcg cggcgtcgtt gacgctgacc gctcagtccc ggactttatt ggctgggata 960
   gtgcagcaac agcaacagct gttggacgtg gtcaagagac aacaagaatt gttgcgactg 1020
   accgtctggg gaacaaagaa cctccagact agggtcactg ccatcgagaa gtacttaaag 1080
   gaccaggcgc agctaaatgc ttggggatgt gcgtttagac aagtctgcca cactactgta 1140
   ccatggccaa atgcaagtct aacaccagac tggaacaatg atacttggca agagtgggag 1200
   cgaaaggttg acttcttgga ggaaaatata acagccctcc tagaagaggc acaaattcaa 1260
   caagagaaga acatgtatga attacaaaag ttgaatagct gggatgtgtt tggcaattgg 1320
   tttgaccttg cttcttggat aaagtatata caatatggaa tttatgtagt tgtaggagta 1380
   atactgttaa gaatagtgat ctatatagta caaatgctag ctaagttaag gcaggggtat 1440
   aggccagtgt tctcttcccc accctcttat ttccagtaga ctcataccca acaggacccg 1500
   gcactgccaa ccagagaagg caaagaagga gacggtggag aaggcggtgg atcctattat 1560
   cgaattcctg cagccccgat aaaataaaag attttattta gtctccagaa aaagggggga 1620
   atgaaagacc ccacctgtag gtttggcaag ctagctgcag taacgccatt ttgcaaggca 1680
   tggaaaaata ccaaaccaag aatagagaag ttcagatcaa gggcgggtac atgaaaatag 1740
   ctaacgttgg gccaaacagg atatctgcgg tgagcagttt cggccccggc ccggggccaa 1800
   gaacagatgg tcaccgcagt ttcggccccg gcccgaggcc aagaacagat ggtccccaga 1860
   tatggcccaa ccctcagcag tttcttaaga cccatcagat gtttccaggc tcccccaagg 1920
   acctgaaatg accctgcgcc ttatttgaat taaccaatca gcctgcttct cgcttctgtt 1980
   cgcgcgcttc tgcttcccga gctctataaa agagctcaca acccctcact cggcgcgcca 2040
   gtcctccgac agactgagtc gcccgggggg gatccaccgg gctgcaggaa ttcgatatca 2100
   agcttatcga tgattctaga catgcatatc tagcgtcccg ggctgcagga attcataact 2160
   tcgtataatg tatgctatac gaagttatca tatgccacca tgagcgagct gatcaaggag 2220
   aacatgcaca tgaagctgta catggagggc accgtgaaca accaccactt caagtgcaca 2280
   tccgagggcg aaggcaagcc ctacgagggc acccagacca tgaagatcaa ggtggtcgag 2340
   ggcggccctc tccccttcgc cttcgacatc ctggctacca gcttcatgta cggcagcaaa 2400
   gccttcatca accacaccca gggcatcccc gacttcttta agcagtcctt ccctgagggc 2460
   ttcacatggg agagaatcac cacatacgaa gacgggggcg tgctgaccgc tacccaggac 2520
   accagcttcc agaacggctg catcatctac aacgtcaaga tcaacggggt gaacttccca 2580
   tccaacggcc ctgtgatgca gaagaaaaca cgcggctggg aggccaacac cgagatgctg 2640
   taccccgctg acggcggcct gagaggccac agccagatgg ccctgaagct cgtgggcggg 2700
   ggctacctgc actgctcctt caagaccaca tacagatcca agaaacccgc taagaacctc 2760
   aagatgcccg gcttccactt cgtggaccac agactggaaa gaatcaagga ggccgacaaa 2820
   gagacctacg tcgagcagca cgagatggct gtggccaagt actgcgacct ccctagcaaa 2880
   ctggggcaca gataaataac ttcgtataaa gtatcctata cgaagttata ctttggccgc 2940
   ggctcgaggg ggttggggtt gcgccttttc caaggcagcc ctgggtttgc gcagggacgc 3000
   ggctgctctg ggcgtggttc cgggaaacgc agcggcgccg accctgggtc tcgcacattc 3060
   ttcacgtccg ttcgcagcgt cacccggatc ttcgccgcta cccttgtggg ccccccggcg 3120
   acgcttcctg ctccgcccct aagtcgggaa ggttccttgc ggttcgcggc gtgccggacg 3180
   tgacaaacgg aagccgcacg tctcactagt accctcgcag acggacagcg ccagggagca 3240
   atggcagcgc gccgaccgcg atgggctgtg gccaatagcg gctgctcagc agggcgcgcc 3300
   gagagcagcg gccgggaagg ggcggtgcgg gaggcggggt gtggggcggt agtgtgggcc 3360
   ctgttcctgc ccgcgcggtg ttccgcattc tgcaagcctc cggagcgcac gtcggcagtc 3420
   ggctccctcg ttgaccgaat caccgacctc tctccccagg gggatccacc ggtcgccacc 3480
   atggtgagca agggcgagga gctgttcacc ggggtggtgc ccatcctggt cgagctggac 3540
   ggcgacgtaa acggccacaa gttcagcgtg tccggcgagg gcgagggcga tgccacctac 3600
   ggcaagctga ccctgaagtt catctgcacc accggcaagc tgcccgtgcc ctggcccacc 3660
   ctcgtgacca ccctgaccta cggcgtgcag tgcttcagcc gctaccccga ccacatgaag 3720
   cagcacgact tcttcaagtc cgccatgccc gaaggctacg tccaggagcg caccatcttc 3780
   ttcaaggacg acggcaacta caagacccgc gccgaggtga agttcgaggg cgacaccctg 3840
   gtgaaccgca tcgagctgaa gggcatcgac ttcaaggagg acggcaacat cctggggcac 3900
   aagctggagt acaactacaa cagccacaac gtctatatca tggccgacaa gcagaagaac 3960
   ggcatcaagg tgaacttcaa gatccgccac aacatcgagg acggcagcgt gcagctcgcc 4020
   gaccactacc agcagaacac ccccatcggc gacggccccg tgctgctgcc cgacaaccac 4080
   tacctgagca cccagtccgc cctgagcaaa gaccccaacg agaagcgcga tcacatggtc 4140
   ctgctggagt tcgtgaccgc cgccgggatc actctcggca tggacgagct gtacaagtaa 4200
   agcggccgcg actctagagt cgacctgcag gcatgcaagc ttgatatcaa gcttatcgat 4260
   aatcaacctc tggattacaa aatttgtgaa agattgactg gtattcttaa ctatgttgct 4320
   ccttttacgc tatgtggata cgctgcttta atgcctttgt atcatgctat tgcttcccgt 4380
   atggctttca ttttctcctc cttgtataaa tcctggttgc tgtctcttta tgaggagttg 4440
   tggcccgttg tcaggcaacg tggcgtggtg tgcactgtgt ttgctgacgc aacccccact 4500
   ggttggggca ttgccaccac ctgtcagctc ctttccggga ctttcgcttt ccccctccct 4560
   attgccacgg cggaactcat cgccgcctgc cttgcccgct gctggacagg ggctcggctg 4620
   ttgggcactg acaattccgt ggtgttgtcg gggaaatcat cgtcctttcc ttggctgctc 4680
   gcctgtgttg ccacctggat tctgcgcggg acgtccttct gctacgtccc ttcggccctc 4740
   aatccagcgg accttccttc ccgcggcctg ctgccggctc tgcggcctct tccgcgtctt 4800
   cgccttcgcc ctcagacgag tcggatctcc ctttgggccg cctccccgca tcgataccgt 4860
   cgacctcgag ttttataaaa gaaaaggggg gactggaagg gatttattac agtgcaagaa 4920
   gacatagaat cttagacatg tacttagaaa aggaagaagg catcatacca gattggcagg 4980
   attacacctc aggaccagga attagatacc caaagacatt tggctggcta tggaaattag 5040
   tccctgtaaa tgtatcagat gaggcacagg aggatgagag gcattattta atgcagccag 5100
   ctcaaacttc caagtgggat gacccttggg gagaggttct agcgtggaag tttgatccaa 5160
   ctctagccta cacttatgag gcatatgcta gatacccaga agagttggaa gcaagtcagg      5220
   cctgtcagaa ctgcatttcg ctctgtattc agtcgctctg cggagaggct ggcagattga      5280
   gccctgggag gttctctcca gcactagcag gtagagcctg ggtgttccct gctagactct      5340
   caccagcact tggccagtgc tgggcagagt ggctccacgc ttgcttgctt aaagacctct      5400
   tcaataaagc tgccatttta gaagtaagcc agtgtgtgtt cccatctctc ctagtcgccg      5460
   cctggtcaac tcggtactcg gtaataagaa gaccctggtc tgttaggacc ctttctgctt      5520
   tgagaaaccg aagcaggaaa atccctagca tggtacccag cttttgttcc ctttagtgag      5580
   ggttaattcc gagcttggcg taatcatggt catagctgtt tcctgtgtga aattgttatc      5640
   cgctcacaat tccacacaac atacgagccg gaagcataaa gtgtaaagcc tggggtgcct      5700
   aatgagtgag ctaactcaca ttaattgcgt tgcgctcact gcccgctttc cagtcgggaa      5760
   acctgtcgtg ccagctgcat taatgaatcg gccaacgcgc ggggagaggc ggtttgcgta      5820
   ttgggcgctc ttccgcttcc tcgctcactg actcgctgcg ctcggtcgtt cg              5872
<210> 46
   <211> 1653
   <212> DNA
   <213> Lampyris noctiluca
<220>
   <221> CDS
   <222> (1)..(1653)
<400> 46
   atg gaa gac gcc aaa aac ata aag aaa ggc ccg gcg cca ttc tat ccg        48
   Met Glu Asp Ala Lys Asn Ile Lys Lys Gly Pro Ala Pro Phe Tyr Pro
   1               5                   10                  15
   ctg gaa gat gga acc gct gga gag caa ctg cat aag gct atg aag aga        96
   Leu Glu Asp Gly Thr Ala Gly Glu Gln Leu His Lys Ala Met Lys Arg
               20                  25                  30
   tac gcc ctg gtt cct gga aca att gct ttt aca gat gca cat atc gag       144
   Tyr Ala Leu Val Pro Gly Thr Ile Ala Phe Thr Asp Ala His Ile Glu
           35                  40                  45
   gtg gac atc act tac gct gag tac ttc gaa atg tcc gtt cgg ttg gca       192
   Val Asp Ile Thr Tyr Ala Glu Tyr Phe Glu Met Ser Val Arg Leu Ala
       50                  55                  60
   gaa gct atg aaa cga tat ggg ctg aat aca aat cac aga atc gtc gta       240
   Glu Ala Met Lys Arg Tyr Gly Leu Asn Thr Asn His Arg Ile Val Val
   65                  70                  75                  80
   tgc agt gaa aac tct ctt caa ttc ttt atg ccg gtg ttg ggc gcg tta       288
   Cys Ser Glu Asn Ser Leu Gln Phe Phe Met Pro Val Leu Gly Ala Leu
                   85                  90                  95
   ttt atc gga gtt gca gtt gcg ccc gcg aac gac att tat aat gaa cgt       336
   Phe Ile Gly Val Ala Val Ala Pro Ala Asn Asp Ile Tyr Asn Glu Arg
               100                 105                 110
   gaa ttg ctc aac agt atg ggc att tcg cag cct acc gtg gtg ttc gtt        384
   Glu Leu Leu Asn Ser Met Gly Ile Ser Gln Pro Thr Val Val Phe Val
           115                 120                 125
   tcc aaa aag ggg ttg caa aaa att ttg aac gtg caa aaa aag ctc cca        432
   Ser Lys Lys Gly Leu Gln Lys Ile Leu Asn Val Gln Lys Lys Leu Pro
       130                 135                 140
   atc atc caa aaa att att atc atg gat tct aaa acg gat tac cag gga        480
   Ile Ile Gln Lys Ile Ile Ile Met Asp Ser Lys Thr Asp Tyr Gln Gly
   145                 150                 155                 160
   ttt cag tcg atg tac acg ttc gtc aca tct cat cta cct ccc ggt ttt        528
   Phe Gln Ser Met Tyr Thr Phe Val Thr Ser His Leu Pro Pro Gly Phe
                   165                 170                 175
   aat gaa tac gat ttt gtg cca gag tcc ttc gat agg gac aag aca att        576
   Asn Glu Tyr Asp Phe Val Pro Glu Ser Phe Asp Arg Asp Lys Thr Ile
               180                 185                 190
   gca ctg atc atg aac tcc tct gga tct act ggt ctg cct aaa ggt gtc        624
   Ala Leu Ile Met Asn Ser Ser Gly Ser Thr Gly Leu Pro Lys Gly Val
           195                 200                 205
   gct ctg cct cat aga act gcc tgc gtg aga ttc tcg cat gcc aga gat        672
   Ala Leu Pro His Arg Thr Ala Cys Val Arg Phe Ser His Ala Arg Asp
       210                 215                 220
   cct att ttt ggc aat caa atc att ccg gat act gcg att tta agt gtt        720
   Pro Ile Phe Gly Asn Gln Ile Ile Pro Asp Thr Ala Ile Leu Ser Val
   225                 230                 235                 240
   gtt cca ttc cat cac ggt ttt gga atg ttt act aca ctc gga tat ttg        768
   Val Pro Phe His His Gly Phe Gly Met Phe Thr Thr Leu Gly Tyr Leu
                   245                 250                 255
   ata tgt gga ttt cga gtc gtc tta atg tat aga ttt gaa gaa gag ctg        816
   Ile Cys Gly Phe Arg Val Val Leu Met Tyr Arg Phe Glu Glu Glu Leu
               260                 265                 270
   ttt ctg agg agc ctt cag gat tac aag att caa agt gcg ctg ctg gtg        864
   Phe Leu Arg Ser Leu Gln Asp Tyr Lys Ile Gln Ser Ala Leu Leu Val
           275                 280                 285
   cca acc cta ttc tcc ttc ttc gcc aaa agc act ctg att gac aaa tac        912
   Pro Thr Leu Phe Ser Phe Phe Ala Lys Ser Thr Leu Ile Asp Lys Tyr
       290                 295                 300
   gat tta tct aat tta cac gaa att gct tct ggt ggc gct ccc ctc tct        960
   Asp Leu Ser Asn Leu His Glu Ile Ala Ser Gly Gly Ala Pro Leu Ser
   305                 310                 315                 320
   aag gaa gtc ggg gaa gcg gtt gcc aag agg ttc cat ctg cca ggt atc       1008
   Lys Glu Val Gly Glu Ala Val Ala Lys Arg Phe His Leu Pro Gly Ile
                   325                 330                 335
   agg caa gga tat ggg ctc act gag act aca tca gct att ctg att aca       1056
   Arg Gln Gly Tyr Gly Leu Thr Glu Thr Thr Ser Ala Ile Leu Ile Thr
               340                 345                 350
   ccc gag ggg gat gat aaa ccg ggc gcg gtc ggt aaa gtt gtt cca ttt       1104
   Pro Glu Gly Asp Asp Lys Pro Gly Ala Val Gly Lys Val Val Pro Phe
           355                 360                 365
   ttt gaa gcg aag gtt gtg gat ctg gat acc ggg aaa acg ctg ggc gtt      1152
   Phe Glu Ala Lys Val Val Asp Leu Asp Thr Gly Lys Thr Leu Gly Val
       370                 375                 380
   aat caa aga ggc gaa ctg tgt gtg aga ggt cct atg att atg tcc ggt      1200
   Asn Gln Arg Gly Glu Leu Cys Val Arg Gly Pro Met Ile Met Ser Gly
   385                 390                 395                 400
   tat gta aac aat ccg gaa gcg acc aac gcc ttg att gac aag gat gga      1248
   Tyr Val Asn Asn Pro Glu Ala Thr Asn Ala Leu Ile Asp Lys Asp Gly
                   405                 410                 415
   tgg cta cat tct gga gac ata gct tac tgg gac gaa gac gaa cac ttc      1296
   Trp Leu His Ser Gly Asp Ile Ala Tyr Trp Asp Glu Asp Glu His Phe
               420                 425                 430
   ttc atc gtt gac cgc ctg aag tct ctg att aag tac aaa ggc tat cag      1344
   Phe Ile Val Asp Arg Leu Lys Ser Leu Ile Lys Tyr Lys Gly Tyr Gln
           435                 440                 445
   gtg gct ccc gct gaa ttg gaa tcc atc ttg ctc caa cac ccc aac atc      1392
   Val Ala Pro Ala Glu Leu Glu Ser Ile Leu Leu Gln His Pro Asn Ile
       450                 455                 460
   ttc gac gca ggt gtc gca ggt ctt ccc gac gat gac gcc ggt gaa ctt      1440
   Phe Asp Ala Gly Val Ala Gly Leu Pro Asp Asp Asp Ala Gly Glu Leu
   465                 470                 475                 480
   ccc gcc gcc gtt gtt gtt ttg gag cac gga aag acg atg acg gaa aaa      1488
   Pro Ala Ala Val Val Val Leu Glu His Gly Lys Thr Met Thr Glu Lys
                   485                 490                 495
   gag atc gtg gat tac gtc gcc agt caa gta aca acc gcg aaa aag ttg      1536
   Glu Ile Val Asp Tyr Val Ala Ser Gln Val Thr Thr Ala Lys Lys Leu
               500                 505                 510
   cgc gga gga gtt gtg ttt gtg gac gaa gta ccg aaa ggt ctt acc gga      1584
   Arg Gly Gly Val Val Phe Val Asp Glu Val Pro Lys Gly Leu Thr Gly
           515                 520                 525
   aaa ctc gac gca aga aaa atc aga gag atc ctc ata aag gcc aag aag      1632
   Lys Leu Asp Ala Arg Lys Ile Arg Glu Ile Leu Ile Lys Ala Lys Lys
       530                 535                 540
   ggc gga aag atc gcc gtg taa                                          1653
   Gly Gly Lys Ile Ala Val
   545                 550
<210> 47
   <211> 550
   <212> PRT
   <213> Lampyris noctiluca
<400> 47
   Met Glu Asp Ala Lys Asn Ile Lys Lys Gly Pro Ala Pro Phe Tyr Pro
   1               5                   10                  15
   Leu Glu Asp Gly Thr Ala Gly Glu Gln Leu His Lys Ala Met Lys Arg
               20                  25                  30
   Tyr Ala Leu Val Pro Gly Thr Ile Ala Phe Thr Asp Ala His Ile Glu
           35                  40                  45
   Val Asp Ile Thr Tyr Ala Glu Tyr Phe Glu Met Ser Val Arg Leu Ala
       50                  55                  60
   Glu Ala Met Lys Arg Tyr Gly Leu Asn Thr Asn His Arg Ile Val Val
   65                  70                  75                  80
   Cys Ser Glu Asn Ser Leu Gln Phe Phe Met Pro Val Leu Gly Ala Leu
                   85                  90                  95
   Phe Ile Gly Val Ala Val Ala Pro Ala Asn Asp Ile Tyr Asn Glu Arg
               100                 105                 110
   Glu Leu Leu Asn Ser Met Gly Ile Ser Gln Pro Thr Val Val Phe Val
           115                 120                 125
   Ser Lys Lys Gly Leu Gln Lys Ile Leu Asn Val Gln Lys Lys Leu Pro
       130                 135                 140
   Ile Ile Gln Lys Ile Ile Ile Met Asp Ser Lys Thr Asp Tyr Gln Gly
   145                 150                 155                 160
   Phe Gln Ser Met Tyr Thr Phe Val Thr Ser His Leu Pro Pro Gly Phe
                   165                 170                 175
   Asn Glu Tyr Asp Phe Val Pro Glu Ser Phe Asp Arg Asp Lys Thr Ile
               180                 185                 190
   Ala Leu Ile Met Asn Ser Ser Gly Ser Thr Gly Leu Pro Lys Gly Val
           195                 200                 205
   Ala Leu Pro His Arg Thr Ala Cys Val Arg Phe Ser His Ala Arg Asp
       210                 215                 220
   Pro Ile Phe Gly Asn Gln Ile Ile Pro Asp Thr Ala Ile Leu Ser Val
   225                 230                 235                 240
   Val Pro Phe His His Gly Phe Gly Met Phe Thr Thr Leu Gly Tyr Leu
                   245                 250                 255
   Ile Cys Gly Phe Arg Val Val Leu Met Tyr Arg Phe Glu Glu Glu Leu
               260                 265                 270
   Phe Leu Arg Ser Leu Gln Asp Tyr Lys Ile Gln Ser Ala Leu Leu Val
           275                 280                 285
   Pro Thr Leu Phe Ser Phe Phe Ala Lys Ser Thr Leu Ile Asp Lys Tyr
       290                 295                 300
   Asp Leu Ser Asn Leu His Glu Ile Ala Ser Gly Gly Ala Pro Leu Ser
   305                 310                 315                 320
   Lys Glu Val Gly Glu Ala Val Ala Lys Arg Phe His Leu Pro Gly Ile
                   325                 330                 335
   Arg Gln Gly Tyr Gly Leu Thr Glu Thr Thr Ser Ala Ile Leu Ile Thr
               340                 345                 350
   Pro Glu Gly Asp Asp Lys Pro Gly Ala Val Gly Lys Val Val Pro Phe
           355                 360                 365
   Phe Glu Ala Lys Val Val Asp Leu Asp Thr Gly Lys Thr Leu Gly Val
       370                 375                 380
   Asn Gln Arg Gly Glu Leu Cys Val Arg Gly Pro Met Ile Met Ser Gly
   385                 390                 395                 400
   Tyr Val Asn Asn Pro Glu Ala Thr Asn Ala Leu Ile Asp Lys Asp Gly
                   405                 410                 415
   Trp Leu His Ser Gly Asp Ile Ala Tyr Trp Asp Glu Asp Glu His Phe
               420                 425                 430
   Phe Ile Val Asp Arg Leu Lys Ser Leu Ile Lys Tyr Lys Gly Tyr Gln
           435                 440                 445
   Val Ala Pro Ala Glu Leu Glu Ser Ile Leu Leu Gln His Pro Asn Ile
       450                 455                 460
   Phe Asp Ala Gly Val Ala Gly Leu Pro Asp Asp Asp Ala Gly Glu Leu
   465                 470                 475                 480
   Pro Ala Ala Val Val Val Leu Glu His Gly Lys Thr Met Thr Glu Lys
                   485                 490                 495
   Glu Ile Val Asp Tyr Val Ala Ser Gln Val Thr Thr Ala Lys Lys Leu
               500                 505                 510
   Arg Gly Gly Val Val Phe Val Asp Glu Val Pro Lys Gly Leu Thr Gly
           515                 520                 525
   Lys Leu Asp Ala Arg Lys Ile Arg Glu Ile Leu Ile Lys Ala Lys Lys
       530                 535                 540
   Gly Gly Lys Ile Ala Val
   545                 550